# EUROPEAN PATENT SPECIFICATION

(11) **EP 0 873 123 B1**
(45) Date of publication and mention of the grant of the patent: **09.04.2003**
(21) Application number: 96938647.3
(22) Date of filing: 05.11.1996
(51) Int. Cl.: A61K 31/415, A61K 31/44, C07D 213/34, C07D 213/30, C07D 213/62, C07D 213/72, C07D 233/70, C07D 233/84, C07D 233/88, C07D 213/82, C07D 213/56, C07D 233/54, C07D 233/90, C07D 405/12, C07D 405/04, C07D 417/12, C07D 401/12

(54) **INHIBITORS OF PROTEIN ISOPRENYL TRANSFERASES**
INHIBITOREN DER PROTEIN-ISOPRENYL-TRANSFERASE
INHIBITEURS DE PROTEINE-ISOPRENYLE-TRANSFERASES

(30) Priority: 06.11.1995 US 7247 P
(43) Date of publication of application: 28.10.1998
(73) Proprietor: UNIVERSITY OF PITTSBURGH, Pittsburgh, Pennsylvania 15260 (US)
(72) Inventor: SEBTI, Said, M., Tampa, FL 33647 (US); HAMILTON, Andrew, D., Pittsburgh, PA 15215 (US); ROSENBERG, Saul, H., Grayslake, IL 60030 (US); AUGERI, David, J., Kenosha, WI 53143 (US); BARR, Kenneth, J., Chicago, IL 60657-2906 (US); DONNER, Bernard, G., Mundelein, IL 60060 (US); FAKHHOURY, Stephen, A., Mundelein, IL 60060 (US); JANOWICK, David, A., Beach Park, IL 60087 (US); KALVIN, Douglas, M., Buffalo Grove, IL 60089 (US); LARSEN, John, J., Melrose Park, IL 60164 (US); LIU, Gang, Waukegan, IL 60085 (US); O'CONNOR, Stephen, J., Wilmette, IL 60091 (US); SHEN, Wang, Gurnee, IL 60031 (US); SWENSON, Rolf, E., Grayslake, IL 60030 (US); SORENSON, Bryan, K., Waukegan, IL 60087 (US); SULLIVAN, Gerard, M., Round Lake Beach, IL 60073 (US); SZCZEPANKIEWICZ, Bruce, Gages Lake, IL 60030 (US); TASKER, Andrew, S., Gurnee, IL 60031 (US); WASICAK, James, T., Waterford, WI 53185 (US); WINN, Martin, Deerfield, IL 60015 (US)
(74) Representative: Smart, Peter John
(86) International application number: US9617092
(87) International publication number: WO97017070

(56) References cited:
- EP-A- 0 675 112
- THE JOURNAL OF BIOLOGICAL CHEMISTRY, Vol. 266, No. 22, 05 August 1991, MOORES, pages 14603-14610.
- JOURNAL OF MEDICINAL CHEMISTRY, 1989, Vol. 32, No.8, TILLEY, pages 1814-1820.

## Description

### Technical Field

The present invention relates to novel compounds which are useful in inhibiting protein isoprenyl transferases (for example, protein farnesyltransferase and protein geranylgeranyltransferase) and the farnesylation or geranylgeranylation of the oncogene protein Ras, compositions containing such compounds and to methods of using such compounds.

### Background of the Invention

Ras oncogenes are the most frequently identified activated oncogenes in human tumors. Transformed protein Ras is involved in the proliferation of cancer cells. Ras must be farnesylated before this proliferation can occur. Farnesylation of Ras by farnesyl pyrophosphate (FPP) is effected by protein farnesyltransferase. Inhibition of protein farnesyltransferase and, thereby, of farnesylation of the Ras protein, blocks the ability of transformed cells to proliferate. Inhibition of protein geranylgeranyltransferase and, thereby, of geranylgeranylation of Ras proteins, also results in down regulation of Ras protein function.

Activation of Ras also partially mediates smooth muscle cell proliferation (Circulation, 1-3: 88 (1993)). Inhibition of protein isoprenyl transferases and, thereby, of farnesylation or geranylgeranylation of the Ras protein, also aids in the prevention of restenosis following percutaneous transluminal coronary angioplasty.

Therefore, there is a need for compounds which are inhibitors of protein farnesyltransferase and protein geranylgeranyltransferase.

### Summary of the Invention

In its principal embodiment, the present invention provides compounds of formula 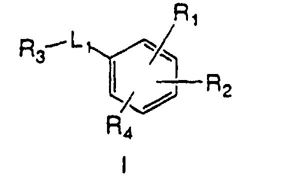 or a pharmaceutically acceptable salt thereof wherein R₁ is selected from the group consisting of (a) hydrogen, (b) lower alkyl, (c) alkenyl, (d) alkoxy, (e) thioalkoxy, (f) halogen, (g) haloalkyl, (h) aryl - L₂ - wherein L₂ is absent or is selected from the group consisting of -CH₂-, -CH₂CH₂-, -CH(CH₃)-, -O-, -S(O)_{q} wherein q is 0, 1 or 2, -N(R)- wherein R is hydrogen or loweralkyl, and -C(O)-, and aryl is selected from the group consisting of phenyl, naphthyl, tetrahydronaphthyl, indanyl and indenyl and the aryl group is unsubstituted or substituted, and (i) heterocyclic - L₃ - wherein L₃ is absent or is selected from the group consisting of -CH₂-, -CH₂CH₂-, -CH(CH₃)-, - O-, -S(O)_{q} wherein q is 0, 1 or 2, -N(R)- wherein R is hydrogen or lower alkyl, and -C(O)-,and heterocyclic is a monocyclic heterocyclic wherein the heterocyclic is unsubstituted or substituted with one, two or three substituents independently selected from the group consisting of lower alkyl, hydroxy, hydroxyalkyl, halo, nitro, oxo (=0), amino, N-protected amino, alkoxy, thioalkoxy and haloalkyl.

R₂ is -C(O)NH-CH(R₁₄)-C(O)OR₁₅ wherein R₁₄ is selected from the group consisting of lower alkyl, cycloalkyl, cycloalkylalkyl, alkoxyalkyl, thioalkoxyalkyl, hydroxyalkyl, aminoalkyl, carboxyalkyl, alkoxycarbonylalkyl, arylalkyl or alkylsulfonylalkyl and R₁₅ is hydrogen or a carboxy-protecting group.

R₃ is pyridyl, substituted pyridyl, imidazolyl, or substituted imidazolyl.

R₄ is selected from the group consisting of hydrogen, lower alkyl, haloalkyl, halogen, aryl, arylalkyl, heterocyclic, and (heterocyclic)alkyl.

L₁ is absent or is selected from the group consisting of (a) -L₄-N(R₄)-L₅- wherein L₄ is absent or selected from C₁-to-C₁₀-alkylene and C₂-to-C₁₀-alkenylene wherein the alkylene group or the alkenylene group is unsubstituted or substituted with one, two, three or four substituents independently selected from (1) lower alkyl, (2) alkenyl, (3)hydroxy, (4) amino, (5) N-protected amino, (6) carboxy, (7) alkoxycarbonyl, (8) oxo, (9) thioxo, (10) imino, (11) =N-OH, (12) =N-O-lower alkyl, (13) =N-O-aryl, and (14) =N-O-hetercyclic wherein the heterocyclic is unsubstituted or substituted with one, two or three substituents independently selected from the group consisting of lower alkyl, halo, nitro, haloalkyl, oxo, hydroxyl, hydroxyalkyl, amino, N-protected amino, alkoxy, and thioalkoxy, R₄ is hydrogen or lower alkyl; and L₅ is absent or is selected from, -CH₂-, -CH₂CH₂- and -CH (CH₃)-, (b) -L₄-O-L₅- wherein L₄ and L₅ are defined above, (c) -L₄-S(O)ₙ-L₅- wherein n is 0, 1 or 2 and L₄ and L₅ are defined above, (d) -L₄-L₆C(W)-N(R₅)-L₅ wherein L₄, L₅ and R₅ are defined above, W is O or S, and L₆ is absent or is selected from -O-, -S- and -N(R₆) - wherein R₆ is hydrogen or lower alkyl, (e) -L₄-L₆-S(O)ₘ-N(R₇)-L₅ -wherein L₄, L₅ and L₆ are defined above, m is 1 or 2, and R₇ is hydrogen or lower alkyl, (f) -L₄-N(R₇)-C(W)-L₇-L₅ -wherein W, R₇, L₄ and L₆ are defined above, and L₇ is absent or is selected from -O- and --S-, (g) -L₄-N(R₇)-S(O)ₚ-L₇-L₅- wherein p is 1 or 2 and L₄, R₇, L₅ and L₇ are defined above, (h) C₂-C₄-alkylene optionally substituted with 1 or 2 hydroxy groups, (i) C₂-to-C₄-alkenylene, and (j) C₂-to-C₄-alkynylene, wherein "lower alkyl" indicates C₁ to C₁₀ alkyl.

The compounds disclosed may be used in a method for inhibiting protein isoprenyl transferases (i.e. protein farnesyltransferase and/or geranylgeranyltransferase) in a human or lower mammal, comprising administering to the patient a therapeutically effective amount of a compound of formula 1.

This may result in inhibiting post-translational modification of the oncogenic Ras protein by protein farnesyltransferase, protein geranylgeranyltransferase or both.

The compounds may be used for treatment of conditions mediated by farnesylated or geranylgeranylated proteins, for example, treatment of Ras associated tumours in humans and other animals.

Thus they may be used in a method for inhibiting or treating cancer in a human or lower mammal, comprising administering to the patient a therapeutically effective amount of a compound of the invention alone or in combination with another chemotherapeutic agent.

They may be used also in a method for treating or preventing restenosis in a human or lower mammal, comprising administering to the patient a therapeutically effective amount of a compound of the invention.

The compounds of the invention can comprise asymmetrically substituted carbon atoms. As a result, all stereoisomers of the compounds of the invention are meant to be included in the invention, including racemic mixtures, mixtures of diastereomers, as well as single diastereomers of the compounds of the invention. The terms "S" and "R" configuration, as used herein, are as defined by the IUPAC 1974 Recommendations for Section E, Fundamental Stereochemistry, Pure Appl. Chem. (1976) 45, 13-30.

### Detailed Description

### Definitions of Terms

As used herein the terms "Cys", "Glu", "Leu", "Lys", "Met", "nor-Leu", "nor-Val", "Phe", "Ser" and "Val" refer to cysteine, glutamine, leucine, lysine, methionine, nor-leucine, nor-valine, phenylalanine, serine and valine in their L-, D- or DL forms. In general, unless otherwise specified, as used herein these amino acids are in their naturally occuring L- form.

The term "carboxy protecting group" as used herein refers to a carboxylic acid protecting ester group employed to block or protect the carboxylic acid functionality while the reactions involving other functional sites of the compound are carried out. Carboxy protecting groups are disclosed in Greene, "Protective Groups in Organic Synthesis" pp. 152-186 (1981), which is hereby incorporated herein by reference. In addition, a carboxy protecting group can be used as a prodrug whereby the carboxy protecting group can be readily cleaved *in vivo,* for example by enzymatic hydrolysis, to release the biologically active parent. T. Higuchi and V. Stella provide a thorough discussion of the prodrug concept in "Pro-drugs as Novel Delivery Systems", Vol 14 of the A.C.S. Symposium Series, American Chemical Society (1975), which is hereby incorporated herein by reference. Such carboxy protecting groups are well known to those skilled in the art, having been extensively used in the protection of carboxyl groups in the penicillin and cephalosporin fields, as described in U.S. Pat. No. 3,840,556 and 3,719,667, the disclosures of which are hereby incorporated herein by reference. Examples of esters useful as prodrugs for compounds containing carboxyl groups can be found on pages 14-21 of "Bioreversible Carriers in Drug Design: Theory and Application", edited by E.B. Roche, Pergamon Press, New York (1987), which is hereby incorporated herein by reference. Representative carboxy protecting groups are C₁ to C₈ alkyl (e.g. methyl, ethyl or tertiary butyl and the like); arylalkyl, for example, phenethyl or benzyl and substituted derivatives thereof such as alkoxybenzyl or nitrobenzyl groups and the like; arylalkenyl, for example, phenylethenyl and the like; aryl and substituted derivatives thereof, for example, 5-indanyl and the like; dialkylaminoalkyl (e.g., dimethylaminoethyl and the like); alkanoyloxyalkyl groups such as acetoxymethyl, butyryloxymethyl, valeryloxymethyl, isobutyryloxymethyl, isovaleryloxymethyl, 1-(propionyloxy)-1-ethyl, 1-(pivaloyloxyl)-1-ethyl, 1-methyl-1-(propionyloxy)-1-ethyl, pivaloyloxymethyl, propionyloxymethyl and the like; cycloalkanoyloxyalkyl groups such as cyclopropylcarbonyloxymethyl, cyclobutylcarbonyloxymethyl, cyclopentylcarbonyloxymethyl, cyclohexylcarbonyloxymethyl and the like; aroyloxyalkyl, such as benzoyloxymethyl, benzoyloxyethyl and the like; arylalkylcarbonyloxyalkyl, such as benzylcarbonyloxymethyl, 2-benzylcarbonyloxyethyl and the like; alkoxycarbonylalkyl or cycloalkyloxycarbonylalkyl, such as methoxycarbonylmethyl, cyclohexyloxycarbonylmethyl, 1-methoxycarbonyl-1-ethyl, and the like; alkoxycarbonyloxyalkyl or cycloalkyloxycarbonyloxyalkyl, such as methoxycarbonyloxymethyl, t-butyloxycarbonyloxymethyl, 1-ethoxycarbonyloxy-1-ethyl, 1-cyclohexyloxycarbonyloxy-1-ethyl and the like; aryloxycarbonyloxyalkyl, such as 2-(phenoxycarbonyloxy)ethyl, 2-(5-indanyloxycarbonyloxy)ethyl and the like; alkoxyalkylcarbonyloxyalkyl, such as 2-(1-methoxy-2-methylpropan-2-oyloxy)ethyl and like; arylalkyloxycarbonyloxyalkyl, such as 2-(benzyloxycarbonyloxy)ethyl and the like; arylalkenyloxycarbonyloxyalkyl, such as 2-(3-phenylpropen-2-yloxycarbonyloxy)ethyl and the like; alkoxycarbonylaminoalkyl, such as t-butyloxycarbonylaminomethyl and the like; alkylaminocarbonylaminoalkyl, such as methylaminocarbonylaminomethyl and the like; alkanoylaminoalkyl, such as acetylaminomethyl and the like; heterocycliccarbonyloxyalkyl, such as 4-methylpiperazinylcarbonyloxymethyl and the like; dialkylaminocarbonylalkyl, such as dimethylaminocarbonylmethyl, diethylaminocarbonylmethyl and the like; (5-(loweralkyl)-2-oxo-1,3-dioxolen-4-yl)alkyl, such as (5-t-butyl-2-oxo-1,3-dioxolen-4-yl)methyl and the like; and (5-phenyl-2-oxo-1,3-dioxolen-4-yl)alkyl, such as (5-phenyl-2-oxo-1,3-dioxolen-4-yl)methyl and the like.

Preferred carboxy-protected compounds of the invention are compounds wherein the protected carboxy group is a loweralkyl, cycloalkyl or arylalkyl ester, for example, methyl ester, ethyl ester, propyl ester, isopropyl ester, butyl ester, sec-butyl ester, isobutyl ester, amyl ester, isoamyl ester, octyl ester, cyclohexyl ester, phenylethyl ester and the like or an alkanoyloxyalkyl, cycloalkanoyloxyalkyl, aroyloxyalkyl or an arylalkylcarbonyloxyalkyl ester.

The term "N-protecting group" or "N-protected" as used herein refers to those groups intended to protect the N-terminus of an amino acid or peptide or to protect an amino group against undesirable reactions during synthetic procedures. Commonly used N-protecting groups are disclosed in Greene, "Protective Groups In Organic Synthesis," (John Wiley & Sons, New York (1981)), which is hereby incorporated herein by reference. N-protecting groups comprise acyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl and the like; alkyl groups such as benzyl, triphenylmethyl, benzyloxymethyl and the like; and silyl groups such as trimethylsilyl and the like. Preferred N-protecting groups are formyl, acetyl, benzoyl, pivaloyl, t-butylacetyl, phenylsulfonyl, benzyl, t-butyfoxycarbonyl (Boc) and benzyloxycarbonyl (Cbz).

The term "alkanoyl" as used herein refers to R₂₉C(O)- wherein R₂₉ is a loweralkyl group.

The term "alkanoylaminoalkyl" as used herein refers to a loweralkyl radical to which is appended R₇₁-NH- wherein R₇₁ is an alkanoyl group.

The term "alkanoyloxy" as used herein refers to R₂₉C(O)-O- wherein R₂₉ is a loweralkyl group.

The term "alkanoyloxyalkyl" as used herein refers to a loweralkyl radical to which is appended an alkanoyloxy group.

The term "alkenyl" as used herein refers to a straight or branched chain hydrocarbon containing from 2 to 10 carbon atoms and also containing at least one carbon-carbon double bond. Examples of alkenyl include -CH=CH₂, -CH₂CH=CH₂, -C(CH₃)=CH₂, -CH₂CH=CHCH₃, and the like.

The term "alkenylene" as used herein refers to a divalent group derived from a straight or branched chain hydrocarbon containing from 2 to 10 carbon atoms and also containing at least one carbon-carbon double bond. Examples of alkenylene include -CH=CH-, -CH₂CH=CH-, -C(CH₃)=CH-, -CH₂CH=CHCH₂-, and the like.

The term "alkoxy" as used herein refers to R₃₀O- wherein R₃₀ is loweralkyl as defined above. Representative examples of alkoxy groups include methoxy, ethoxy, t-butoxy and the like.

The term "alkoxyalkoxy" as used herein refers to R₃₁O-R₃₂O- wherein R₃₁ is loweralkyl as defined above and R₃₂ is an alkylene radical. Representative examples of alkoxyalkoxy groups include methoxymethoxy, ethoxymethoxy, t-butoxymethoxy and the like.

The term "alkoxyalkyl" as used herein refers to an alkoxy group as previously defined appended to an alkyl group as previously defined. Examples of alkoxyalkyl include, but are not limited to, methoxymethyl, methoxyethyl, isopropoxymethyl and the like.

The term "alkoxyalkylcarbonyloxyalkyl" as used herein refers to a loweralkyl radical to which is appended R₆₆-C(O)-O- wherein R₆₆ is an alkoxyalkyl group.

The term "alkoxycarbonyl" as used herein refers to an alkoxy group as previously defined appended to the parent molecular moiety through a carbonyl group. Examples of alkoxycarbonyl include methoxycarbonyl, ethoxycarbonyl, isopropoxycarbonyl and the like.

The term "alkoxycarbonylalkyl" as used herein refers to an alkoxylcarbonyl group as previously defined appended to a loweralkyl radical. Examples of alkoxycarbonylalkyl include methoxycarbonylmethyl, 2-ethoxycarbonylethyl and the like.

The term "alkoxycarbonylaminoalkyl" as used herein refers to a loweralkyl radical to which is appended R₆₉-NH- wherein R₆₉ is an alkoxycarbonyl group.

The term "alkoxycarbonyloxyalkyl" as used herein refers to a loweralkyl radical to which is appended R₆₃-O- wherein R₆₃ is an alkoxycarbonyl group.

The term "alkylamino" as used herein refers to R₃₅NH- wherein R₃₅ is a loweralkyl group, for example, methylamino, ethylamino, butylamino, and the like.

The term "alkylaminoalkyl" as used herein refers a loweralkyl radical to which is appended an alkylamino group.

The term "alkylaminocarbonylaminoalkyl" as used herein refers to a loweralkyl radical to which is appended R₇₀-C(O)-NH- wherein R₇₀ is an alkylamino group.

The term "alkylene" as used herein refers to a divalent group derived from a straight or branched chain saturated hydrocarbon having from 1 to 10 carbon atoms by the removal of two hydrogen atoms, for example methylene, 1,2-ethylene, 1,1-ethylene, 1,3-propylene, 2,2-dimethylpropylene, and the like.

The term "alkylsulfinyl" as used herein refers to R₃₃S(O)- wherein R₃₃ is a loweralkyl group.

The term "alkylsulfonyl" as used herein refers to R₃₄S(O)₂- wherein R₃₄ is a loweralkyl group.

The term "alkylsulfonylalkyl" as used herein refers to a loweralkyl radical to which is appended an alkylsulfonyl group.

The term "alkynyl" as used herein refers to a straight or branched chain hydrocarbon containing from 2 to 10 carbon atoms and also containing at least one carbon-carbon triple bond. Examples of alkynyl include -C≡CH, -CH₂C≡CH, -CH₂C≡CCH₃, and the like.

The term "alkynylene" as used herein refers to a divalent group derived from a straight or branched chain hydrocarbon containing from 2 to 10 carbon atoms and also containing at least one carbon-carbon triple bond. Examples of alkynylene include -C≡C-, -CH₂C≡C-, -CH₂C≡CCH₂-, and the like.

The term "amino" as used herein refers to -NH₂.

The term "aminoalkyl" as used herein refers to a loweralkyl radical to which is appended an amino group.

The term "aroyloxyalkyl" as used herein refers to a loweralkyl radical to which is appended an aroyloxy group (i.e., R₆₁-C(O)O- wherein R₆₁ is an aryl group).

The term "aryl" as used herein refers to a mono- or bicyclic carbocyclic ring system having one or two aromatic rings including, but not limited to, phenyl, naphthyl, tetrahydronaphthyl, indanyl, indenyl and the like. Aryl groups (including bicyclic aryl groups) can be unsubstituted or substituted with one, two or three substituents independently selected from loweralkyl, haloalkyl, alkoxy, thioalkoxy, amino, alkylamino, dialkylamino, hydroxy, halo, mercapto, nitro, cyano, carboxaldehyde, carboxy, alkoxycarbonyl, haloalkyl-C(O)-NH-, haloalkenyl-C(O)-NH- and carboxamide. In addition, substituted aryl groups include tetrafluorophenyl and pentafluorophenyl.

The term "arylalkenyl" as used herein refers to an alkenyl radical to which is appended an aryl group.

The term "arylalkenyloxycarbonyloxyalkyl" as used herein refers to a loweralkyl radical to which is appended R₆₈-O-C(O)-O- wherein R₆₈ is an arylalkenyl group.

The term "arylalkyl" as used herein refers to a loweralkyl radical to which is appended an aryl group. Representative arylalkyl groups include benzyl, phenylethyl, hydroxybenzyl, fluorobenzyl, fluorophenylethyl and the like.

The term "arylalkylcarbonyloxyalkyl" as used herein refers to a loweralkyl radical to which is appended an arylalkylcarbonyloxy group (i.e., R₆₂C(O)O- wherein R₆₂ is an arylalkyl group).

The term "arylalkyloxycarbonyloxyalkyl" as used herein refers to a loweralkyl radical to which is appended R₆₇-O-C(O)-O- wherein R₆₇ is an arylalkyl group.

The term "aryloxyalkyl" as used herein refers to a loweralkyl radical to which is appended R₆₅-O- wherein R₆₅ is an aryl group.

The term "aryloxythioalkoxyalkyl" as used herein refers to a loweralkyl radical to which is appended R₇₅-S- wherein R₇₅ is an aryloxyalkyl group.

The term "aryloxycarbonyloxyalkyl" as used herein refers to a loweralkyl radical to which is appended R₆₅-O-C(O)-O- wherein R₆₅ is an aryl group.

The term "arylsulfonyl" as used herein refers to R₃₆S(O)₂- wherein R₃₆ is an aryl group.

The term "arylsulfonyloxy" as used herein refers to R₃₇S(O)₂O- wherein R₃₇ is an aryl group.

The term "carboxyalkyl" as used herein refers to a loweralkyl radical to which is appended a carboxy (-COOH) group.

The term "carboxaldehyde" as used herein refers to the group -C(O)H.

The term "carboxamide" as used herein refers to the group -C(O)NH₂.

The term "cyanoalkyl" as used herein refers to a loweralkyl radical to which is appended a cyano (-CN) group.

The term "cycloalkanoylalkyl" as used herein refers to a loweralkyl radical to which is appended a cycloalkanoyl group (i.e., R₆₀-C(O)- wherein R₆₀ is a cycloalkyl group).

The term "cycloalkenyl" as used herein refers to an alicyclic group comprising from 3 to 10 carbon atoms and containing a carbon-carbon double bond including, but not limited to, cyclopentenyl, cyclohexenyl and the like.

The term "cycloalkyl" as used herein refers to an alicyclic group comprising from 3 to 10 carbon atoms including, but not limited to, cyclopropyl, cyclobutyl, cyclopentyl, cyclohexyl, norbomyl, adamantyl and the like.

The term "cycloalkylalkyl" as used herein refers to a loweralkyl radical to which is appended a cycloalkyl group. Representative examples of cycloalkylalkyl include cyclopropylmethyl, cyclohexylmethyl,
2-(cyclopropyl)ethyl, adamantylmethyl and the like.

The term "cycloalkyloxycarbonyloxyalkyl" as used herein refers to a loweralkyl radical to which is appended R₆₄-O-C(O)-O- wherein R₆₄ is a cycloalkyl group.

The term "dialkoxyalkyl" as used herein refers to a loweralkyl radical to which is appended two alkoxy groups.

The term "dialkylamino" as used herein refers to R₃₈R₃₉N- wherein R₃₈ and R₃₉ are independently selected from loweralkyl, for example dimethylamino, diethylamino, methyl propylamino, and the like.

The term "dialkylaminoalkyl" as used herein refers to a loweralkyl radical to which is appended a dialkylamino group.

The term "dialkyaminocarbonylalkyl" as used herein refers to a loweralkyl radical to which is appended R₇₃-C(O)- wherein R₇₃ is a dialkylamino group.

The term "dioxoalkyl" as used herein refers to a loweralkyl radical which is substituted with two oxo (=O) groups.

The term "dithioalkoxyalkyl" as used herein refers to a loweralkyl radical to which is appended two thioalkoxy groups.

The term "halogen" or "halo" as used herein refers to I, Br, Cl or F.

The term "haloalkenyl" as used herein refers to an alkenyl radical, as defined above, bearing at least one halogen substituent.

The term "haloalkyl" as used herein refers to a lower alkyl radical, as defined above, bearing at least one halogen substituent, for example, chloromethyl, fluoroethyl or trifluoromethyl and the like.

The term "heterocyclic ring" or "heterocyclic" or "heterocycle" as used herein refers to a 5-, 6- or 7-membered ring containing one, two or three heteroatoms independently selected from the group consisting of nitrogen, oxygen and sulfur or a 5-membered ring containing 4 nitrogen atoms; and includes a 5-, 6- or 7-membered ring containing one, two or three nitrogen atoms; one oxygen atom; one sulfur atom; one nitrogen and one sulfur atom; one nitrogen and one oxygen atom; two oxygen atoms in non-adjacent positions; one oxygen and one sulfur atom in non-adjacent positions; two sulfur atoms in non-adjacent positions; two sulfur atoms in adjacent positions and one nitrogen atom; two adjacent nitrogen atoms and one sulfur atom; two non-adjacent nitrogen atoms and one sulfur atom; two non-adjacent nitrogen atoms and one oxygen atom. The 5-membered ring has 0-2 double bonds and the 6- and 7-membered rings have 0-3 double bonds. The term "heterocyclic" also includes bicyclic, tricyclic and tetracyclic groups in which any of the above heterocyclic rings is fused to one or two rings independently selected from the group consisting of an aryl ring, a cyclohexane ring, a cyclohexene ring, a cyclopentane ring, a cyclopentene ring and another monocyclic heterocyclic ring (for example, indolyl, quinolyl, isoquinolyl, tetrahydroquinolyl, benzofuryl or benzothienyl and the like). Heterocyclics include: pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidinyl, morpholinyl, thiomorpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, thiazolidinyl, isothiazolyl, triazolyl, tetrazolyl, oxadiazolyl, thiadiazolyl, pyrimidyl, tetrahydrofuranyl, dihydrofuranyl, tetrahydrothienyl, dihydrothienyl, dihydroindolyl, tetrahydroquinolyl, tetrahydroisoquinolyl, pyranyl, dihydropyranyl, dithiazolyl, benzofuranyl and benzothienyl. Heterocyclics also include bridged bicyclic groups wherein a monocyclic heterocyclic group is bridged by an alkylene group, for example, 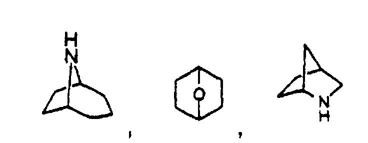 and the like.

Heterocyclics also include compounds of the formula 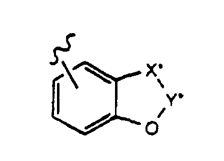 wherein X* is -CH₂-, -CH₂O- or -O- and Y* is -C(O)- or -(C(R")₂)ᵥ- wherein R" is hydrogen or C₁-C₄-alkyl and v is 1, 2 or 3 such as 1,3-benzodioxolyl, 1,4-benzodioxanyl and the like.

Heterocyclics can be unsubstituted or substituted. In the context of a heterocycle, the term "substituted" means a heterocyle substituted with one, two, three, four or five substituents independently selected from the group consisting of a) hydroxy, b) -SH, c) halo, d) oxo (=O), e) thioxo (=S), f) amino,g) -NHOH, h) alkylamino, i) dialkylamino, j) alkoxy, k) alkoxyalkoxy, I) haloalkyl, m) hydroxyalkyl, n) alkoxyalkyl, o) cycloalkyl which is unsubstituted or substituted with one, two, three or four loweralkyl groups, p) cycloalkenyl which is unsubstituted or substituted with one, two, three or four loweralkyl groups, q) alkenyl, r) alkynyl, s) aryl, t) arylalkyl, u) -COOH, v) -SO₃H, w) loweralkyl, x) alkoxycarbonyl, y) -C(O)NH₂, z) -C(S)NH₂, aa) -C(=N-OH)NH₂, bb) aryl-L₁₆-C(O)- wherein L₁₆ is an alkenylene radical, cc) -S-L₁₇-C(O)OR₄₀ wherein L₁₇ is an alkylene radical which is unsubstituted or substituted with one or two substitutents independently selected from the group consisting of alkanoyl, oxo (=O) or methinylamino (=CHNR₄₁R₄₂ wherein R₄₁ is hydrogen or loweralkyl and R₄₂ is loweralkyl) and R₄₀ is hydrogen or a carboxy-protecting group, dd) -S-L₁₈-C(O)NR₄₃R₄₄ wherein L₁₈ is an alkylene radical which is unsubstituted or substituted with one or two substitutents independently selected from the group consisting of alkanoyl, oxo (=O) or methinylamino (=CHNR₄₁R₄₂ wherein R₄₁ is hydrogen or loweralkyl and R₄₃ and

R₄₄ are independently selected from the group consisting of hydrogen, loweralkyl and aryl, ee) -S-L₁₉-CN wherein L₁₉ is an alkylene radical, ff) -S-L₂₀-R₄₅ wherein L₂₀ is absent or is an alkylene radical or an alkenylene radical or an alkynylene radical wherein the alkylene, alkenylene or alkynylene radical is unsubstituted or substituted with oxo (=O) and R₄₅ is hydrogen, aryl, arylalkyl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, gg) -O-L₂₁-R₄₆ wherein L₂₁ is absent or is an alkylene radical or an alkenylene radical or an alkynylene radical wherein the alkylene, alkenylene or alkynylene radical is unsubstituted or substituted with one or two substitutents independently selected from the group consisting of alkanoyl, oxo (=O) or methinylamino (=CHNR₄₁R₄₂ wherein R₄₁ is hydrogen or loweralkyl and R₄₆ is hydrogen, aryl, arylalkyl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, hh) -O-S(O)₂-R₄₇ wherern R₄₇ is aryl, arylalkyl, heterocyclic or heterocyclicalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, ii) -S(O)₂-NH-R₄₈ wherein R₄₈ is aryl, arylalkyl, heterocyclic or heterocyclicalkyl wherein the heterocyctic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, jj) alkylsufinyl, kk) alkylsufonyl, II) arylsulfonyl, mm) arylsulfonyloxy, nn) -C(=NOR₄₉)C(O)OR₅₀ wherein R₄₉ is hydrogen or loweralkyl and R₅₀ is hydrogen or a carboxy-protecting group, oo) alkoxycarbonylalkyl, pp) carboxyalkyl, qq) cyanoalkyl, rr) alkylaminoalkyl, ss) N-protected alkylaminoalkyl, tt) dialkylaminoalkyl, uu) dioxoalkyl, vv) loweralkyl-C(O)-, ww) loweralkyl-C(S)-, xx) aryl-C(O)-, yy) aryl-C(S)-, zz) loweralkyl-C(O)-O-, aaa) loweralkyl-S-C(S)- bbb) N-protected amino, ccc) aminoalkyl-C(O)-, ddd) N-protected aminoalkyl-C(O)- eee) aminoalkyl-C(S)-, fff) N-protected aminoalkyl-C(S)-, ggg) aminoalkyl, hhh) N-protected aminoalkyl, iii) formyl, jjj) cyano, kkk) nitro, III) spiroalkyl, mmm) oxoalkyloxy, nnn) R₅₃-L₂₂- wherein L₂₂ is alkenylene or alkynylene and R₅₃ is aryl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, ooo) aryl-NH-C(O)-, ppp) R₅₄-N=N- wherein R₅₄ is aryl or heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, qqq) =N-R₅₅ wherein R₅₅ is hydrogen, aryl, heterocyclic, -S(O)₂-aryl or -S(O)₂-heterocyclic wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, rrr) diarylalkyl-N=N-, sss) aryl-N(R₅₆)- or arylalkyl-N(R₅₆)- wherein R₅₆ is hydrogen or an N-protecting group, ttt) arylsulfonylalkyl, uuu) heterocyclicsulfonylalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, vvv) =C(CN)(C(O)NH₂), www) =C(CN)(C(O)O-loweralkyl), xxx) heterocyclic or heterocyclicalkyl wherein the heterocyclic is unsubstituted or substituted with one, two or three substituents independently selected from the group consisting of loweralkyl, hydroxy, hydroxyalkyl, halo, nitro, oxo (=O), amino, N-protected amino, alkoxy, thioalkoxy and haloalkyl, yyy) hydroxythioalkoxy, zzz) aryloxyalkyl, aaaa) aryloxyalkylthioalkoxy, bbbb) dialkoxyalkyl, cccc) dithioalkoxyalkyl, dddd) arylalkyl-NH-L₂₃- wherein L₂₃ is an alkylene group, eeee) heterocyclicalkyl-NH-L₂₄- wherein L₂₄ is an alkylene group, ffff) aryl-S(O)₂-NH-L₂₅- wherein L₂₅ is an alkylene group, gggg) heterocyclic-S(O)₂-NH-L₂₆- wherein L₂₆ is an alkylene group, hhhh) aryl-C(O)-NH-L₂₇- wherein L₂₇ is an alkylene group and iiii) heterocyclic-C(O)-NH-L₂₈- wherein L₂₈ is an alkylene group.

The term "(heterocyclic)alkyl" as used herein refers to a heterocyclic group as defined above appended to a loweralkyl radical as defined above. Examples of heterocyclic alkyl include 2-pyridylmethyl, 4-pyridylmethyl, 4-quinolinylmethyl and the like.

The term "heterocycliccarbonyloxyalkyl" as used herein refers to a loweralkyl radical to which is appended R₇₂-C(O)-O- wherein R₇₂ is a heterocyclic group.

The term "hydroxyalkyl" as used herein refers to a loweralkyl radicat to which is appended an hydroxy group.

The term "hydroxythioalkoxy" as used herein refers to R₅₁S- wherein R₅₁ is a hydroxyalkyl group.

The term "loweralkyl" as used herein refers to branched or straight chain alkyl groups comprising one to ten carbon atoms, including methyl, ethyl, propyl, isopropyl, n-butyl, t-butyl, neopentyl and the like.

The term "N-protected alkylaminoalkyl" as used herein refers to an alkylaminoalkyl group wherein the nitrogen is N-protected.

The term "oxoalkyloxy" as used herein refers to an alkoxy radical wherein the loweralkyl moiety is substituted with an oxo (=O) group.

The term "spiroalkyl" as used herein refers to an alkylene diradical, both ends of which are bonded to the same carbon atom of the parent group to form a spirocyclic group.

The term "thioalkoxy" as used herein refers to R₅₂S- wherein R₅₂ is loweralkyl. Examples of thioalkoxy include, but are not limited to, methylthio, ethylthio and the like.

The term "thioalkoxyalkyl" as used herein refers to a thioalkoxy group as previously defined appended to a loweralkyl group as previously defined. Examples of thioalkoxyalkyl include thiomethoxymethyl, 2-thiomethoxyethyl and the like.

### Preferred embodiments

Preferred compounds of the invention are compounds of formula 1 wherein R₁ is aryl-L₂ -wherein L₂ is absent or is selected from -CH₂- and -O- and aryl is selected from phenyl and naphthyl wherein the aryl group is unsubstituted or substituted.

More preferred compounds of the invention are compounds of formula 1 wherein R₁ is unsubstituted or substituted phenyl.

The most preferred compounds of the invention are compounds of formula 1 wherein R₁ is unsubstituted or substituted phenyl and R₂ is 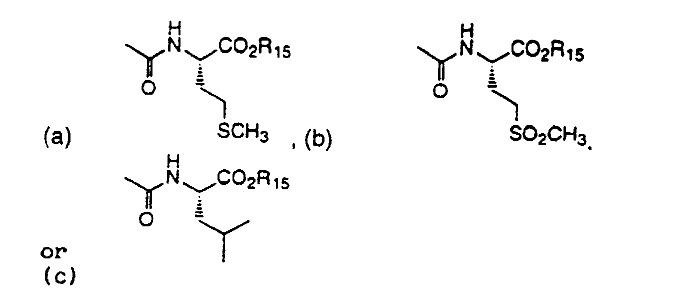 R₃ may be pyridyl or pyridyl substituted with 1 or 2 substituents independently selected from
lower alkyl,
halogen,
haloalkyl,
hydroxy,
alkoxy,
alkoxycarbonyl,
aryl, and
arylalkyl.

Alternatively, R₃ may be imidazolyl or imidazolyl substituted with lower alkyl.

In a second aspect, the present invention relates to a compound as described herein for use in a method of inhibiting protein isoprenyl transferases in a mammal in need of such treatment.

In a third aspect, the present invention relates to a composition for inhibiting protein isoprenyl transferases comprising a pharmaceutical carrier and a therapeutically effective amount of a compound of formula 1.

In a fourth aspect, the present invention relates to a compound as described herein alone or in combination with another chemotherapeutic agent for use in a method for inhibiting or treating cancer in a mammal.

In a fifth aspect, the present invention relates to a compound of formula 1 in combination with another chemotherapeutic agent and a pharmaceutically acceptable carrier.

In a sixth aspect, the present invention relates to a compound as described herein, for use in a method for inhibiting post-translational modification of the oncogenic Ras protein by protein farnesyltransferase, protein geranylgeranyltransferase or both in a mammal.

In a seventh aspect, the present invention relates to a composition for inhibiting post-translational modification of the oncognenic Ras protein by protein farnesyltransferase, protein geranylgeranyltransferase or both comprising a compound of formula 1 in combination with a pharmaceutically acceptable carrier.

In an eighth aspect, the present invention relates to a compound as described herein, for use in a method for treating or preventing restenosis in a mammal.

In a ninth aspect, the present invention relates to a composition for treating or preventing restenosis in a mammal comprising a compound of formula 1 in combination with a pharmaceutically acceptable carrier.

### Protein Farnesyltransferase Inhibition

The ability of the compounds of the invention to inhibit protein farnesyltransferase or protein geranylgeranyltransferase can be measured according to the method of Moores, et al., J. Biol. Chem. 266: 14603 (1991) or the method of Vogt, et al., J. Biol. Chem. 270:660-664 (1995). In addition, procedures for determination of the inhibition of farnesylation of the oncogene protein Ras are described by Goldstein, et al., J. Biol. Chem., 266:15575-15578 (1991) and by Singh in United States Patent No. 5,245,061.

In addition, in vitro inhibition of protein farnesyltransferase may be measured by the following procedure. Rat brain protein farnesyltransferase activity is measured using an Amersham Life Science commercial scintillation proximity assay kit and substituting a biotin-K Ras B fragment (biotin-Lys-Lys-Ser-Lys-Thr-Lys-Cys-Val-Ile-Met-CO₂H), 0.1 µM final concentration, for the biotin-lamin substrate provided by Amersham. The enzyme is purified according to Reiss, Y., et al., Cell, 62: 81-88 (1990), utilizing steps one through three. The specific activity of the enzyme is approximately 10 nmol substrate farnesylated/mg enzyme/hour. The percent inhibition of the farnesylation caused by the compounds of the invention (at 10 x 10⁻⁶ M) compared to an uninhibited control sample is evaluated in the same Amersham test system.

The % inhibition of protein farnesyltransferase was determined for representative compounds of the invention. The results are summarised in Table 1.

**Table 1**

| *In Vivo* Potencies of Representative Compounds | | | | | |
|---|---|---|---|---|---|
| Example | % inhibition @ 10 µM | % inhibition @ 1 µM | Example | % inhibition @ 10 µM | % inhibition @ 1 µM |
| 36 | - | 92 | 140 | - | 99 |
| 39 | - | 30 | 141 | - | 98 |
| 40 | - | 54 | 142 | - | 98 |
| 41 | - | 98 | 143 | - | 76 |
| 42 | - | 92 | 144 | - | 83 |
| 51 | - | 98 | 145 | - | 36 |
| 52 | - | 36 | 147 | - | 98 |
| 53 | - | 86 | 148 | - | 94 |
| 54 | - | 68 | 149 | - | 89 |
| 55 | - | 40 | 150 | - | 65 |
| 56 | - | 88 | 151 | - | 43 |
| 58 | - | 28 | 152 | - | 22 |
| 59 | - | 95 | 153 | - | 98 |
| 67 | 93 | - | 154 | - | 99 |
| 68 | - | 63 | 155 | - | 99 |
| 69 | - | 76 | 156 | - | 99 |
| 70 | - | 98 | 157 | - | 82 |
| 71 | - | 98 | 158 | - | 62 |
| 72 | - | 67 | 159 | - | 98 |
| 73 | - | 98 | 160 | - | 98 |
| 74 | - | 98 | 161 | - | 97 |
| 75 | - | 74 | 163 | - | 94 |
| 77 | - | 98 | 167 | - | 54 |
| 79 | - | 98 | 169 | - | 98 |
| 80 | - | 97 | 170 | - | 93 |
| 81 | - | 82 | 171 | - | 98 |
| 82 | - | 67 | 172 | - | 98 |
| 83 | - | 99 | 175 | - | 98 |
| 84 | - | 89 | 176 | - | 98 |
| 85 | - | 92 | 177 | - | 98 |
| 86 | - | 55 | 178 | - | 26 |
| 87 | - | 41 | 179 | - | 99 |
| 88 | - | 63 | 180 | - | 93 |
| 89 | - | 93 | 190 | - | 72 |
| 90 | - | 23 | 191 | - | 95 |
| 91 | - | 94 | 192 | - | 91 |
| 96 | - | 65 | 193 | - | 98 |
| 98 | - | 95 | 194 | - | 95 |
| 100 | - | 98 | 195 | - | 66 |
| 101 | - | 22 | 196 | 53 | - |
| 102 | - | 97 | 197 | 82 | - |
| 103 | - | 98 | 198 | 52 | - |
| 104 | - | 41 | 199 | 62 | - |
| 105 | - | 99 | 200 | 56 | - |
| 106 | - | 23 | 201 | 36 | - |
| 107 | - | 21 | 203 | 96 | - |
| 108 | - | 50 | 204 | 81 | - |
| 137 | - | 98 | 205 | 71 | - |
| 139 | - | 99 | 206 | 80 | - |

Additional methods for the measurement of in vitro inhibition of protein prenylation (i.e., inhibition of farnesyltransferase or geranygeranyltransferase) are described below.

Assays are performed using the glass fiber filter binding assay procedure with either rabbit reticulocyte lysate, or FTase or GGTase I fractions isolated from bovine brains using a combination of hydrophobic and DEAE column chromatography procedures. Protein substrates are purchased from Panvera Corporation (H-ras for FTase, H-ras-CVLL for GGTase I) and the tritium labeled prenyl lipid substrates(FPP or GGPP) are obtained from Amersham Life Science.

### FTase

³H-Farnesyldiphosphate (final concentration 0.6 µM), H-Ras (final concentration 5.0 µM), and the test compound (various final concentrations from a stock solution in 50% DMSO/water; final concentration DMSO < 2%) are mixed in buffer (50 mM HEPES (pH 7.5), 30 mM MgCl₂, 20 mM KCI, 10 µM ZnCl₂, 5 mM DTT, 0.01% Triton X-100) to give a final volume of 50 µL. The mixture is brought to 37 °C, enzyme is added, and the reaction is incubated for 30 minutes. 1 mL of 1 M HCl/ethanol is added to stop the reaction, the mixture is allowed to stand for 15 minutes at room temperature and then is diluted with 2 mL ethanol. The reaction mixture is filtered through a 2.5 cm glass microfiber filter from Whatman and washed with four 2 mL portions of ethanol. The glass filter is transferred to a scintillation vial and 5 mL scintillation fluid is added. The radioisotope retained on the glass fiber filter is counted and this reflects the activity of the enzymes. An IC₅₀ value can be calculated by measuring the activity of the enzyme over a suitable range of inhibitor concentrations.

### GGTase I

³H-geranylgeranyldiphosphate (final concentration 0.5 µM), H-Ras-CVLL (final concentration 5.0 µM), and the test compound (various final concentrations from a stock solution in 50% DMSO/water; final concentration DMSO < 2%) are mixed in buffer (50 mM Tris-HCl (pH 7.2), 30 mM MgCl₂, 20 mM KCl, 10 µM ZnCl₂, 5 mM DTT, 0.01% Triton X-100) to give a final volume of 50 µL. The mixture is brought to 37 °C, enzyme is added, and the reaction is incubated for 30 minutes. 1 mL of 1 M HCl/ethanol is added to stop the reaction, the mixture is allowed to stand for 15 minutes at room temperature and then is diluted with 2 mL ethanol. The reaction mixture is filtered through a 2.5 cm glass microfiber filter from Whatman and washed with four 2 mL portions of ethanol. The glass filter is transferred to a scintillation vial and 5 mL scintillation fluid is added. The radioisotope retained on the glass fiber lifter is counted and this reflects the activity of the enzymes. An IC₅₀ value can be calculated by measuring the activity of the enzyme over a suitable range of inhibitor concentrations.

In addition, the ability of the compounds of the invention to inhibit prenylation in whole cells, inhibit anchorage-independent tumor cell growth and inhibit human tumor xenograft in mice can be demonstrated according to the methods described in PCT Patent Application No. WO95/25086, published September 21, 1995, which is hereby incorporated herein by reference.

### Pharmaceutical Compositions

The compounds of the present invention can be used in the form of pharmaceutically acceptable salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate,
3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides (such as methyl, ethyl, propyl, and butyl chloride, bromides, and iodides), dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid.

Basic addition salts can be prepared *in situ* during the final isolation and purification of the compounds of formula (I)-(XII), or separately by reacting the carboxylic acid function with a suitable base such as the hydroxide, carbonate or bicarbonate of a pharmaceutically acceptable metal cation or with ammonia, or an organic primary, secondary or tertiary amine. Such pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

The compounds of the invention are useful (in humans and other mammals) for inhibiting protein isoprenyltransferases (i.e, protein farnesyltransferase and/or protein geranylgeranyltransferase) and the isoprenylation (i.e., farnesylation and/or geranylgeranylation) of Ras. These inhibitors of protein isoprenyltransferases are also useful for inhibiting or treating cancer in humans and other mammals. Examples of the kinds of cancers which may be treated with the compounds of the invention include, but are not limited to, carcinomas, such as lung, colorectal, bladder, breast, kidney, ovarian, liver, exocrine pancreatic, cervical, esophageal, stomach, and small intestinal; sarcomas, such as oesteroma, osteosarcoma, lepoma, liposarcoma, hemanioma, and hemangiosarcoma; melanomas, such as amelanotic and melanotic; mixed types of cancers such as carcinosarcoma, lymphoid tissue type, follicular reticulum, cell sarcoma and Hodgkins disease; and leukemias, such as myeloid, acute lymphoblastic, chronic lymphocytic, acute myloblastic and chronic mylocytic.

The ability of the compounds of the invention to inhibit or treat cancer can be demonstrated according to the methods of Mazerska Z., Woynarowska B., Stefanska B., Borowski S., Drugs Exptl. Clin. Res. 13(6), 345-351 (1987); Bissery, MC, Guenard F, Guerritte-Voegelein F, Lavelle F., Cancer Res. 51, 4845-4852 (1991); and Rygaard J, and Povlsen C., Acta Pathol. Microbiol. Scand. 77, 758 (1969).

These inhibitors of protein isoprenyltransferases are also useful for treating or preventing restenosis in humans and other mammals. The ability of the compounds of the invention to treat or prevent restenosis can be demonstrated according to the methods described by Kranzhofer, R. et al. Circ. Res. 73: 264-268 (1993), Mitsuka, M. et al. Circ. Res. 73: 269-275 (1993) and Santoian, E.C. et al. Circulation 88: 11-14 (1993).

For use as a chemotherapeutic agent, the total daily dose administered to a host in single or divided doses may be in amounts, for example, from 0.01 to 500 mg/kg body weight daily, preferably in amounts from 0.1 to 20 mg/kg body weight daily and more preferably in amounts from 0.5 to 10 mg/kg body weight daily. Dosage unit compositions may contain such amounts of submultiples thereof to make up the daily dose.

For treatment or prevention of restenosis, the total daily dose administered to a host in single or divided doses may be in amounts, for example, from 0.001 to 1000 mg/kg body weight daily and more preferred from 1.0 to 50 mg/kg body weight daily. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, sublingually, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically aceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

While the compounds of the invention can be administered as the sole active pharmaceutical agent for the treatment of cancer, they can also be used in combination with one or more other chemotherapeutic agents.

Representative examples of chemotherapeutic agents are described in Holleb, et al., Clinical Oncology, American Cancer Society, United States (1991) p 56 et seq. These agents include alkylating agents such as the nitrogen mustards (mechloethamine, melphalan, chlorambucil, cyclophosphamide and ifosfamide), nitrosoureas (carmustine, lomustine, semustine, streptozocin), alkyl sulfonates (busulfan), triazines (dacarbazine) and ethyenimines (thiotepa, hexamethylmelamine); folic acid analogues (methotrexate); pyrimidine analogues (5-fluorouracil, cytosine arabinoside); purine analogues (6-mercaptopurine, 6-thioguanine); antitumor antibiotics (actinomycin D, the anthracyclines (doxorubicin), bleomycin, mitomycin C, methramycin); plant alkaloids such as vinca alkaloids (vincristine, vinblastine) and etoposide (VP-16); hormones and hormone antagonists (tamoxifen and corticosteroids); and miscellaneous agents (cisplatin, taxol, brequinar).

The above compounds to be employed in combination with the isoprenyl protein transferase inhibitor of the invention will be used in therapeutic amounts as indicated in the Physicians' Desk Reference (PDR) 47th Edition (1993), which is incorporated herein by reference, or such therapeutically useful amounts as would be known to one of ordinary skill in the art.

The compounds of the invention and the other chemotherapeutic agent can be administered at the recommended maximum clinical dosage or at lower doses. Dosage levels of the active compounds in the compositions of the invention may be varied so as to obtain a desired therapeutic response depending on the route of administration, severity of the disease and the response of the patient.

When administered as a combination, the therapeutic agents can be formulated as separate compositions which are given at the same time or different times, or the therapeutic agents can be given as a single composition.

### Preparation of the Compounds of the Invention

In general, the compounds of the invention can be prepared by the processes illustrated in the following Schemes 1-16. In these general schemes compounds of the formula I are used to exemplify the methods, but the methods are intended to be applicable to all of the compounds of the invention.

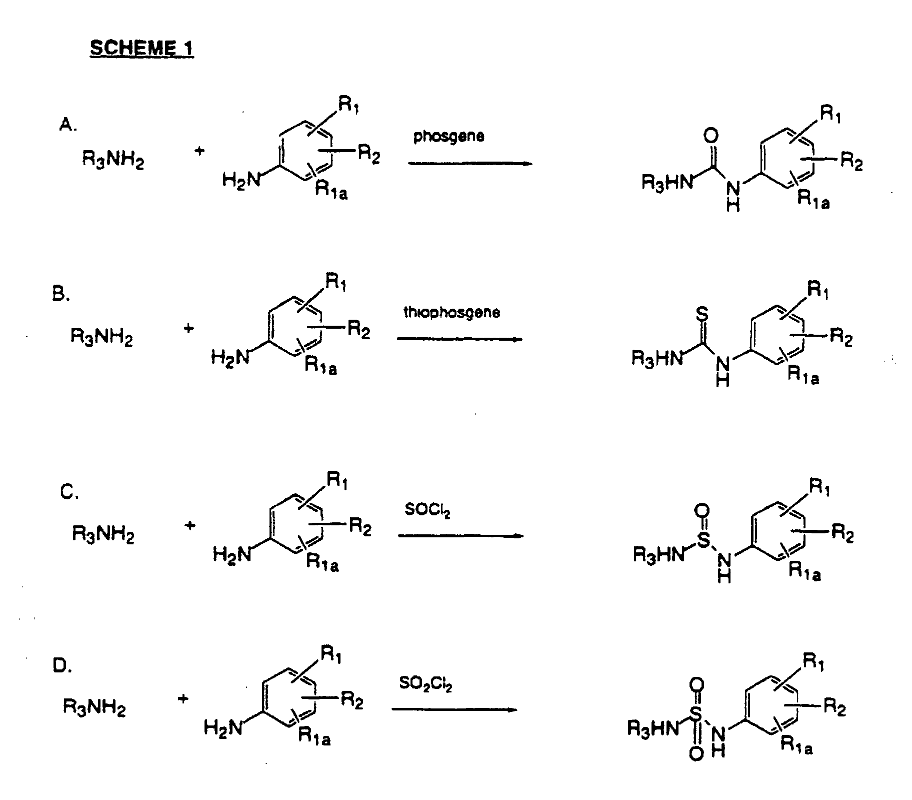

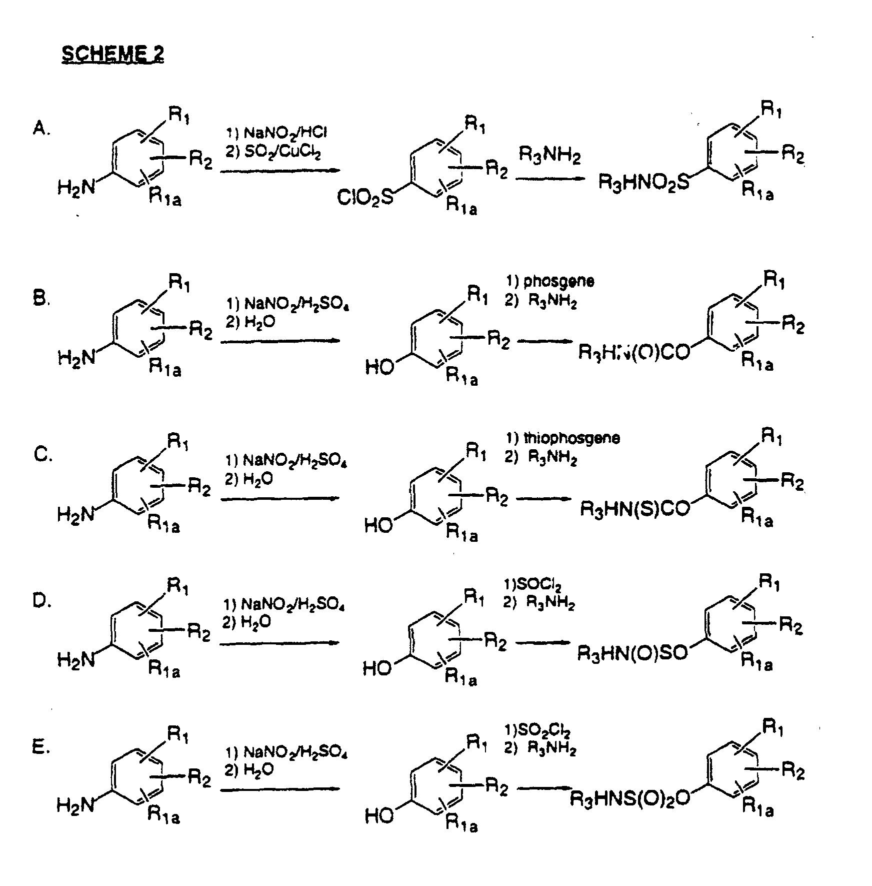

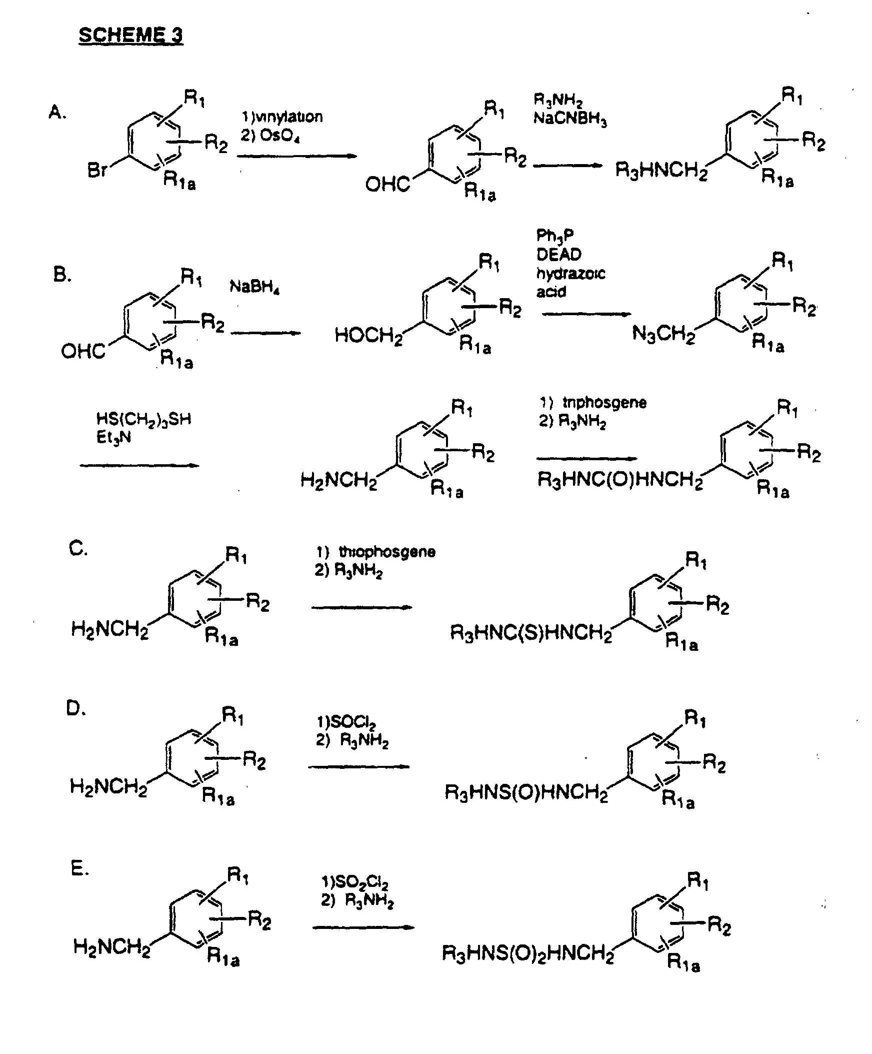

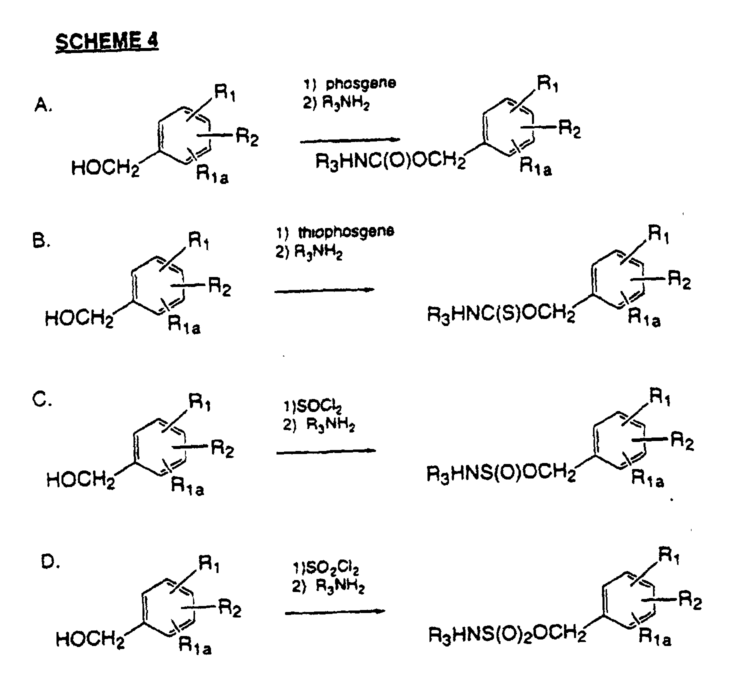

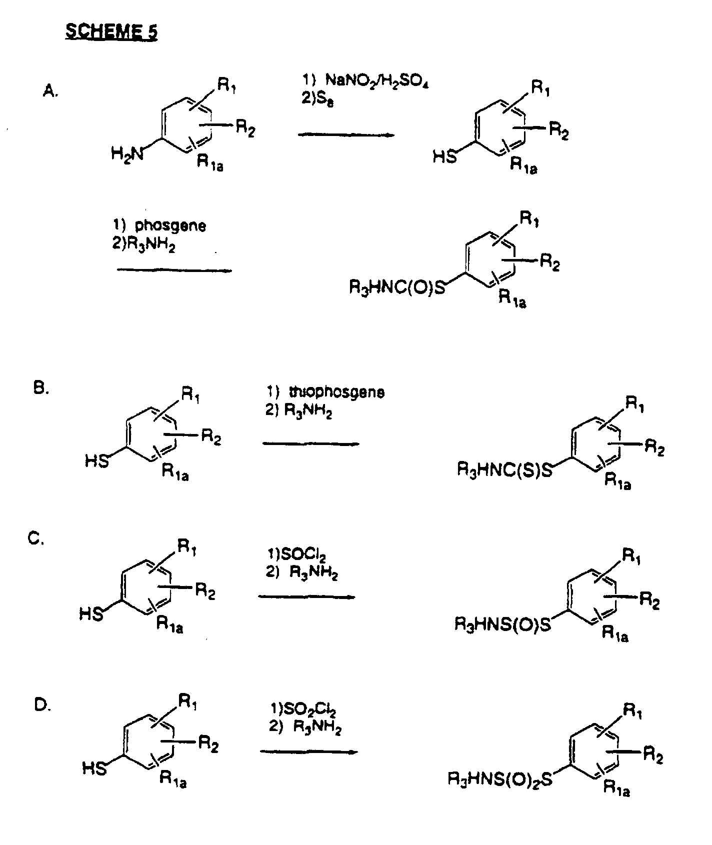

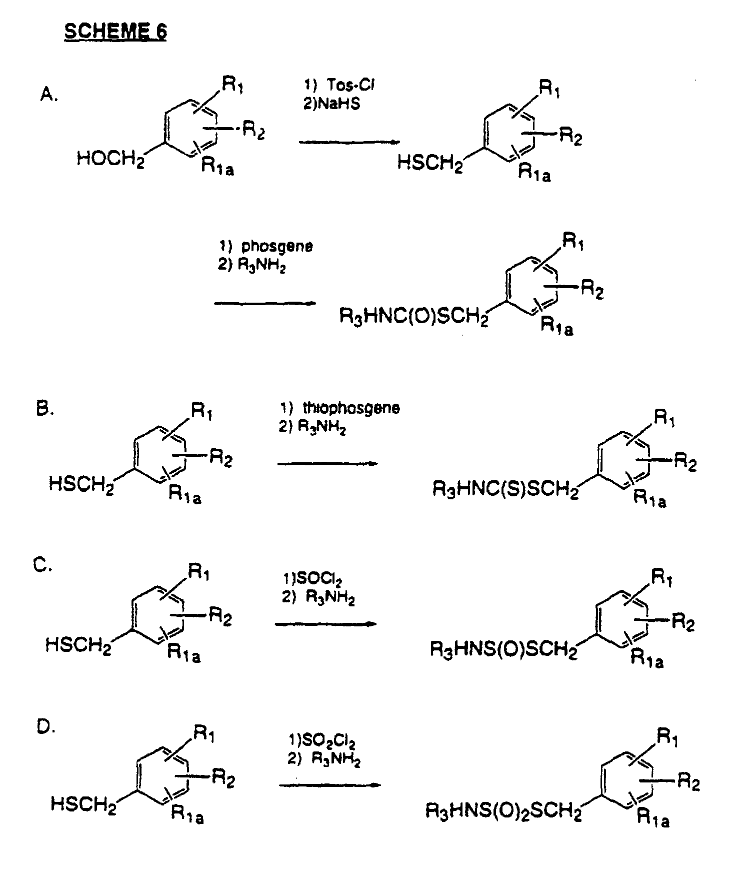

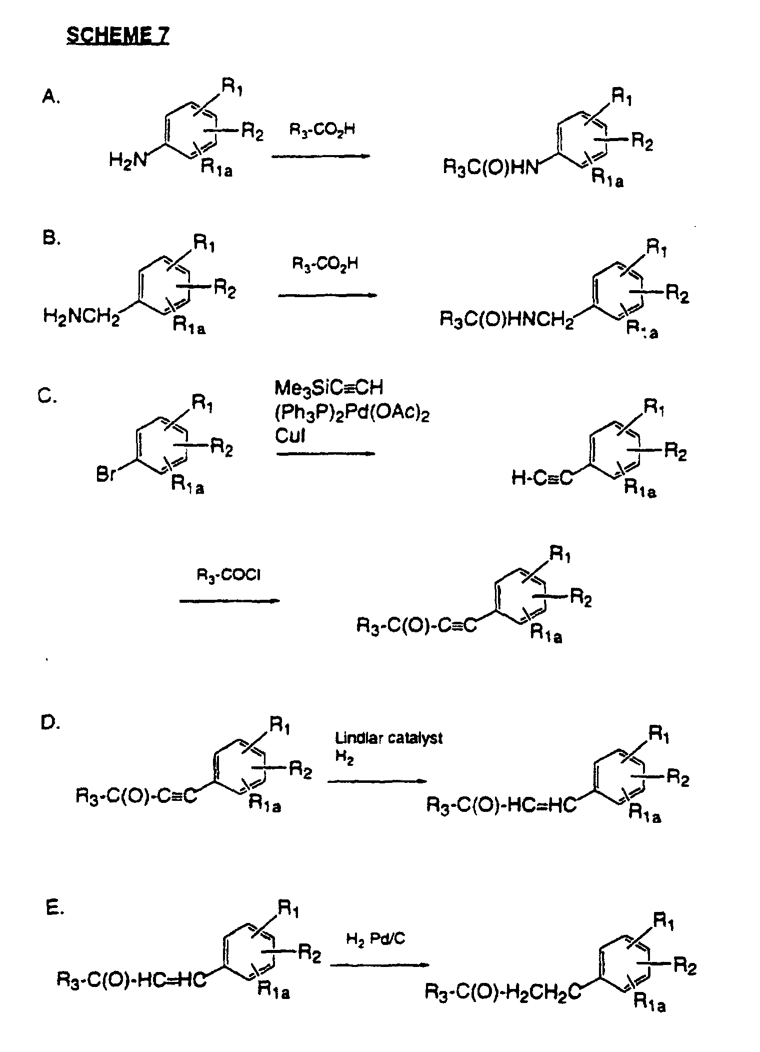

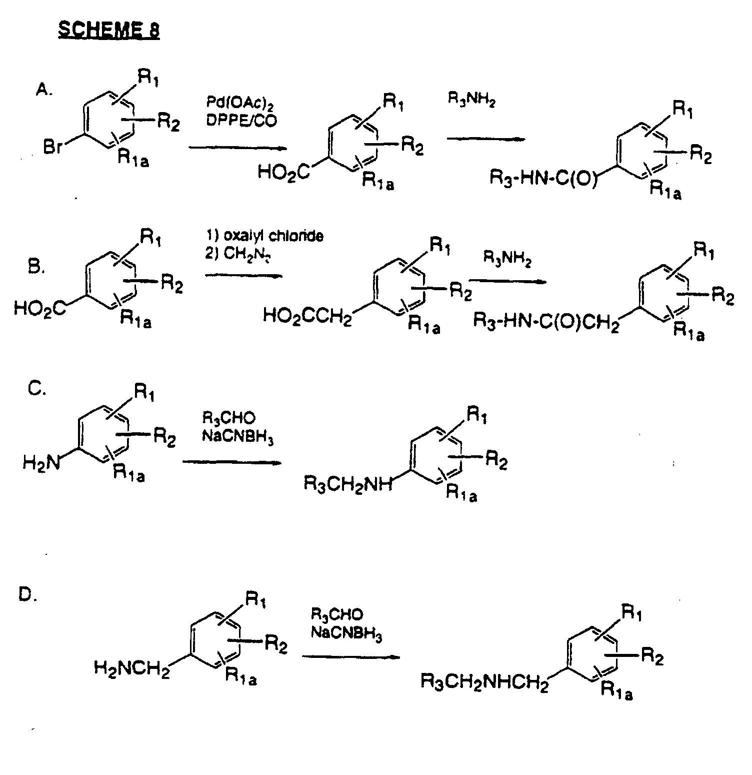

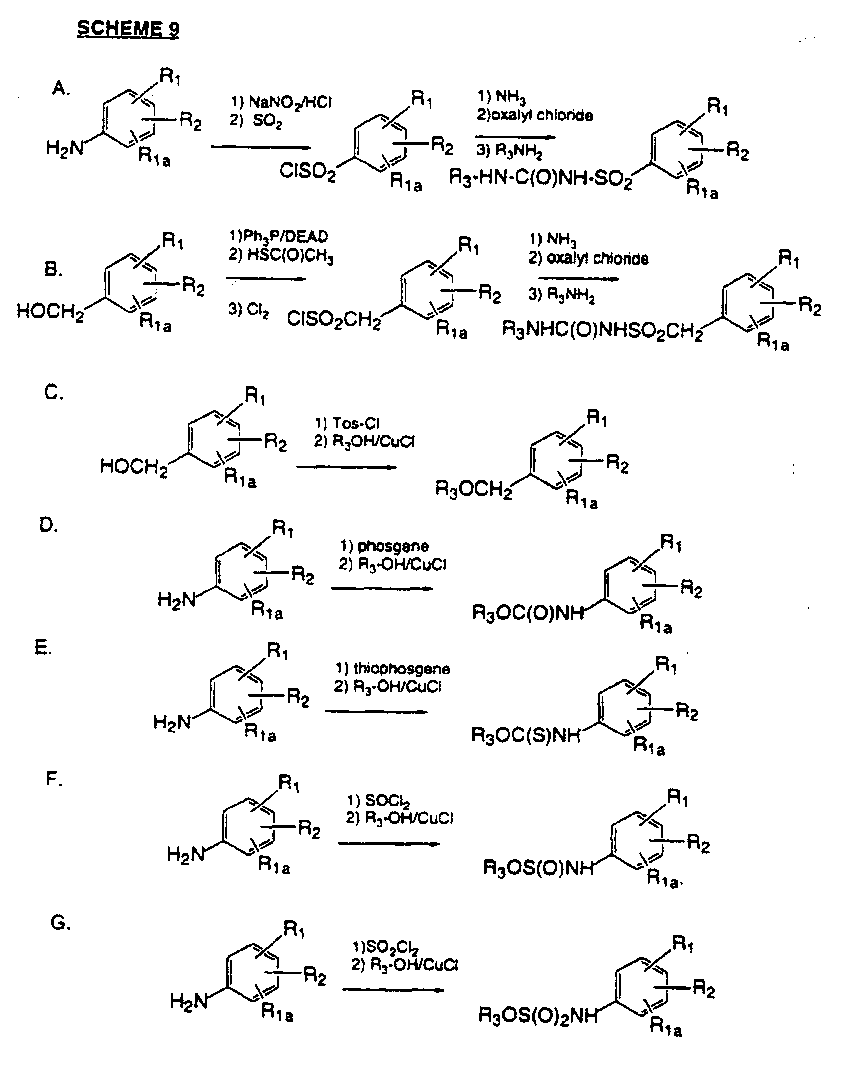

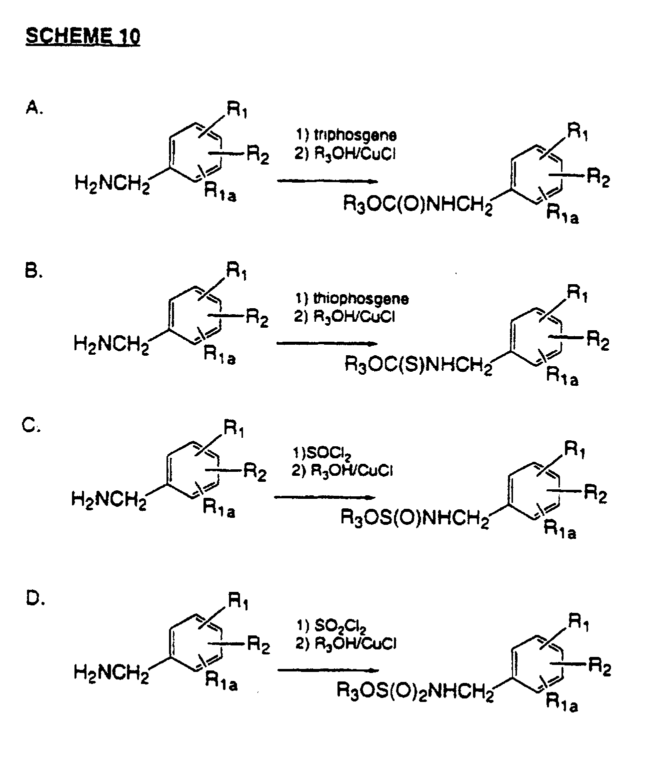

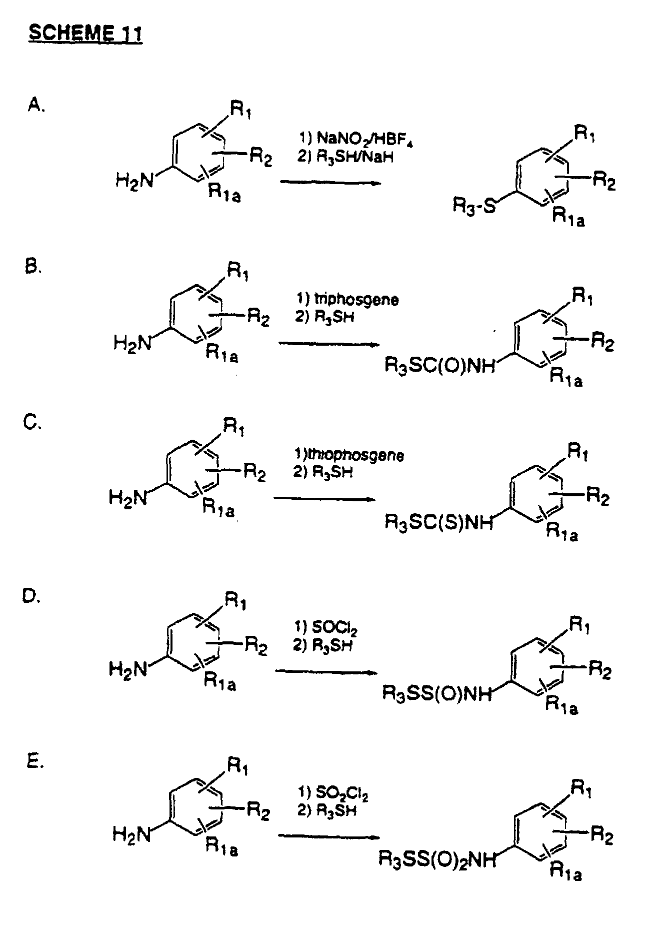

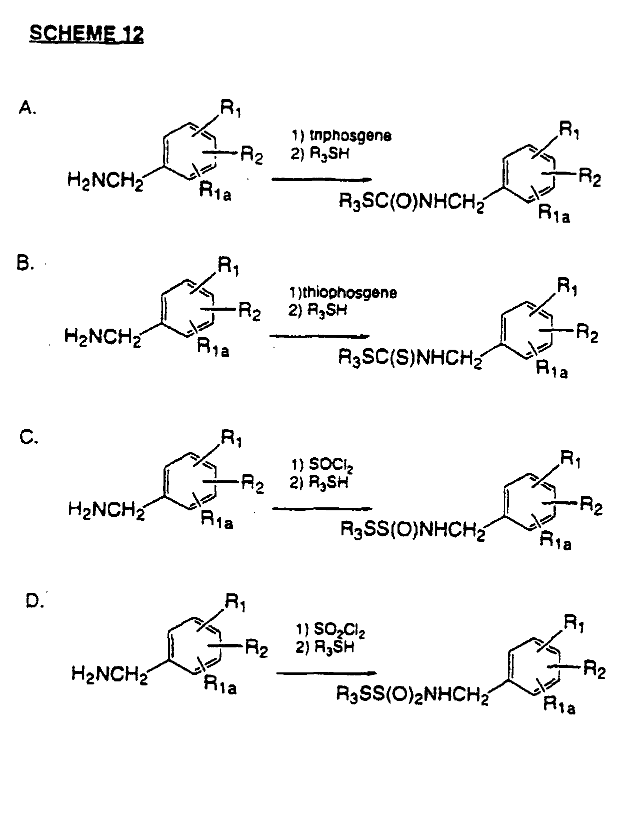

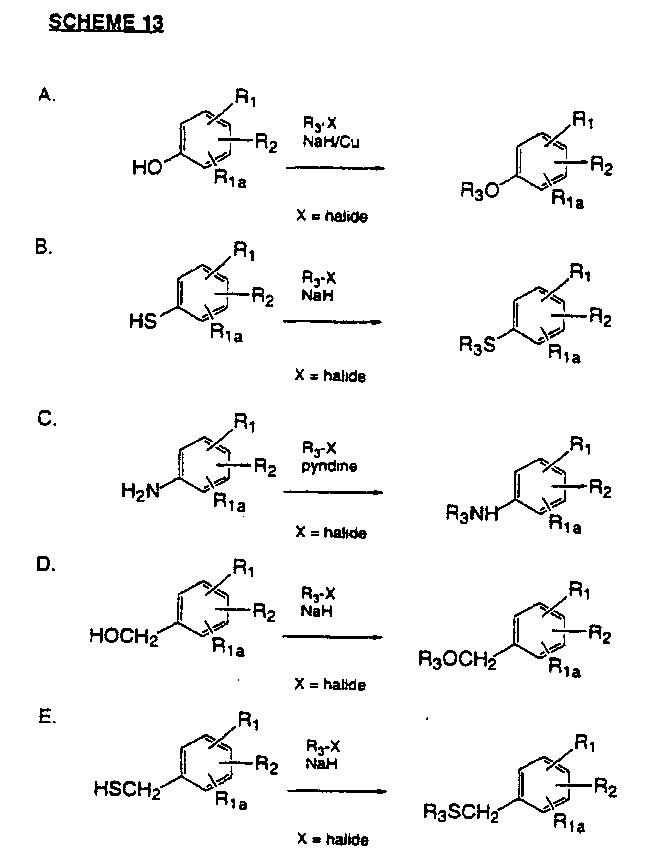

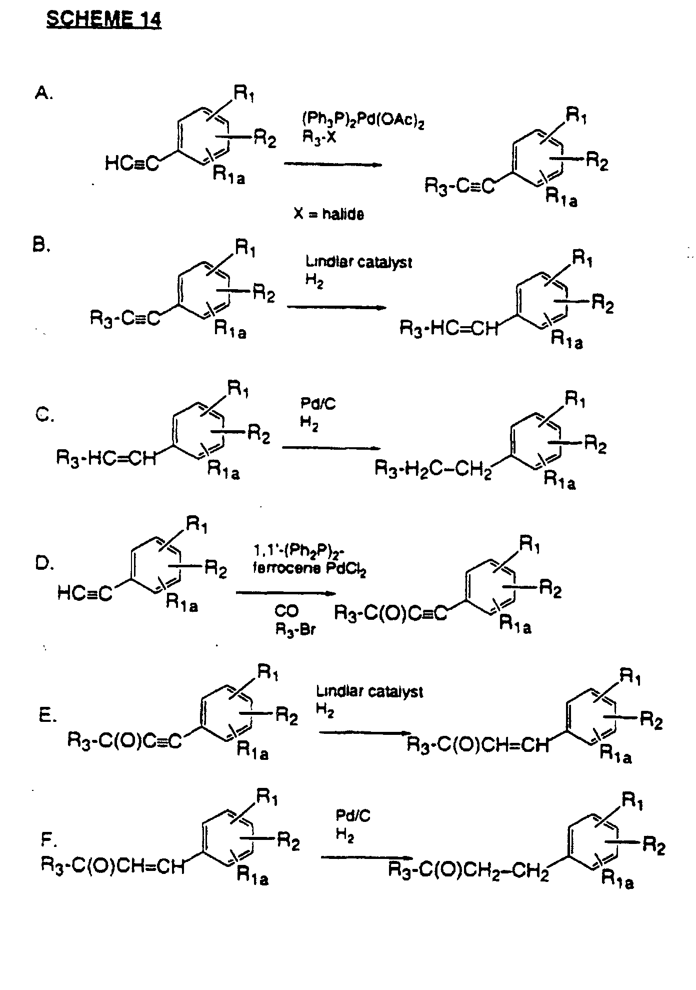

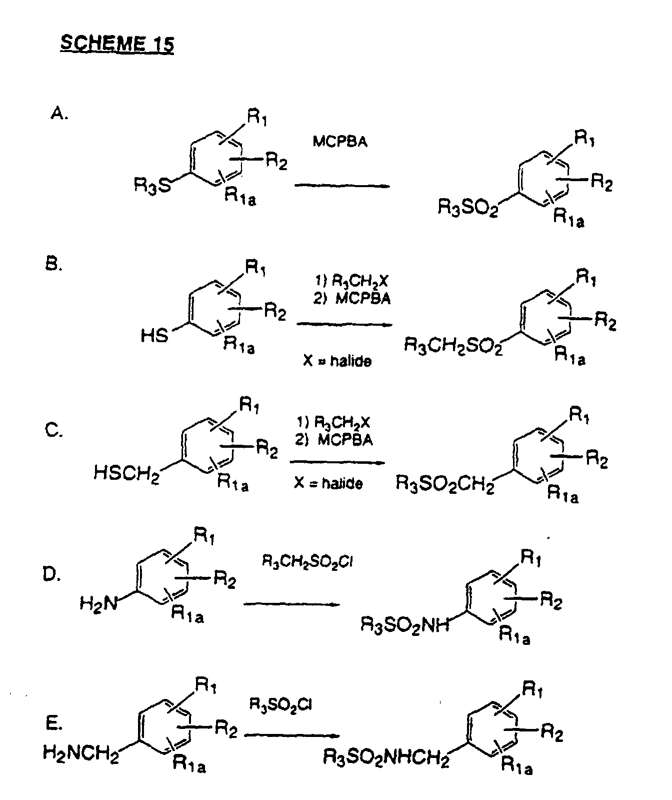

Scheme 16 illustrates an alternative method for preparing the compounds.

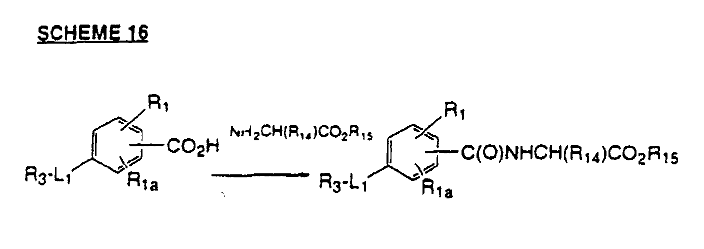

The foregoing may be better understood by reference to the following examples which are provided for illustration and not intended to limit the scope of the inventive concept.

### Compound 1

### (3-(Aminomethyl)benzoyl)-Met-OCH₃

### Step A

### (3-(Chloromethyl)benzoyl)-Met-OCH₃

To a solution of methionine methyl ester hydrochloride (2.0 g, 10 mmol) and 3-(chloromethyl)benzoyl chloride (2.08 g, 11.0 mmol) in methylene chloride (50 mL) was slowly added triethylamine (3.07 mL, 22.0 mmol) at ice bath temperature for 2 hours. The mixture was washed with 0.5 N HCl (50 mL x 2), brine solution (50 mL x 2) and water (50 mL x 2). The organic phase was dried over anhydrous MgSO₄ and concentrated under reduced pressure. The residue was purified by flash column chromatography (30% ethyl acetate in hexanes) to give the desired product (3.03 g) as a white solid: m.p. 82-83°C;
¹H NMR (CDCl₃) δ 7.82 (1H, s), 7.74 (1H, d, *J*=7.7 Hz), 7.53 (1H, d, *J*=7.7 Hz), 7.42 (1H, t, *J*=7.7 Hz), 7.06 (1H, br d, *J*=7.6Hz), 4.92 (1H, ddd, *J*=7.6, 7.1, 5.1 Hz), 4.59 (2H, s), 3.78 (3H, s), 2.58 (2H, t, *J*=7.1Hz) 2.26 (1H, sm), 2.15 (1H, m), 2.10 (3H, s); ¹³C NMR (CDCl₃) δ 172.59, 166.54, 138.13, 134.25, 131.95, 129.12, 127.42, 126.97, 52.72. 52.14, 45.55, 31.47, 30.12, 15.55.

### Step B

### (3-(Azidomethyl)benzoyl)-Met-OCH₃

A suspension of (3-(chloromethyl)benzoyl)-Met-OCH₃ (1.58 g, 5.0 mmol) and sodium azide (1.3 g, 20.0 mmol) in DMSO (40 mL) was stirred at 80°C for 7 hours. The mixture was diluted with methylene chloride (100 mL), washed with brine (70 mL x 2) and water (70 mL x 2), and then dried over anhydrous MgSO₄. The solvent was evaporated under reduced pressure to give a yellow residue. Chromatography on silica gel (30% ethyl acetate in hexanes) to provide the desired product (1.45 g) as a colorless solid: m.p. 48-49°C; ¹H NMR (CDCl₃) δ 7.78 (2H, m), 7.49 (2H, m), 6.99 (1H, br d, *J*=7.4 Hz), 4.49 (1H, ddd, *J*=7.4, 7.1, 5.2 Hz), 4.42 (2H, s), 3.80 (3H,s), 2.60 (2H, t, *J*=7.4 Hz), 2.29 (1H, m), 2.17 (1H, m), 2.12 (3H, s); ¹³C NMR (CDCl₃) δ 177.50. 166.54. 135.97, 134.06, 131.18, 128.89, 126.84, 126.71, 54.09, 52.47, 51.95, 31.38, 30.00,15.30.

### Step C

### (3-(Aminomethyl)benzoyl)-Met-OCH₃

A suspension of (3-(azidomethyl)benzoyl)-Met-OCH₃ (1.29 g, 4.0 mmol) and 5% palladium on carbon (0.2 g) in methanol (40 mL) was stirred under a hydrogen atmosphere (1 atm) for two days at room temperature. The catalyst was removed by filtration through celite (1.5 g) and the solvent was evaporated in vacuo. The residue was washed with water (5 mL x 2) and dried to give the desired product (1.12 g) as a colorless foam. ¹H NMR (CDCl₃) δ 7.81 (1H, s), 7.68 (1H, d, *J*=7.4 Hz), 7.45 (1H, d, *J*=6.5 Hz), 7.36 (1H, t, *J*=7.4 Hz), 4.91 (1H, ddd, J=7.3, 7.1, 5.1 Hz), 3.90 (2H, s), 3.77 (3H, s), 3.21 (2H, br s), 2.59 (2H, t, *J*=7.4 Hz), 2.20 (1H, m), 2.12 (1H, m), 2.09 (3H, s).

### Compound 2

### (4-(Aminomethyl)benzoyl)-Met-OCH₃

The title compound is prepared according to the procedure used to prepare Compound 1 but replacing 3-(chloromethyl)benzoyl chloride with 4-(chloromethyl)benzoyl chloride.

### Compound 3

### (3-Aminobenzoyl)-Met-OCH₃

The title compound was prepared according to the procedure described in J. Biol. Chem. 269 12410-12413 (1994).

### Compound 4

### (4-Aminobenzoyl)-Met-OCH₃

### Step A

### N-BOC-4-Aminobenzoic acid

4-Aminobenzoic acid (10 g, 72.9 mmol) was placed into a mixture of dioxane (145.8 mL) and 0.5 M NaOH (145.8 mL). The solution was cooled to 0°C and di-t-butyl dicarbonate (23.87 g, 109.5 mmol) was added. The reaction mixture was allowed to warm to room temperature and stirred overnight. The next day, the dioxane was removed, the residue was made acidic and extracted into ethyl acetate. The ethyl acetate fractions were combined and washed with 1N HCl to remove any unreacted starting material. The solution was dried over Na₂SO₄ and the solvent was removed in vacuo. The crude material was recrystallized from ethyl acetate/hexanes to provide the desired product (12.2 g): m.p. 189-190°C; ¹H NMR (CD₃OD) δ 1.52 (9H, s), 7.49 (2H, d, *J*=8.6 Hz), 7.91 (2H, d, *J*=8.6 Hz), 9.28 (1H, s); ¹³C NMR (CD₃OD) δ 28.59, 81.29, 118.54, 125.30, 131.81, 145.70, 155.00, 169.80;
Anal. Calc. for C₁₂H₁₅NO₄, C: 60.76, H: 6.37, N: 5.90; Found, C: 60.52, H: 6.43, N: 5.83; HRMS Calc. for C₁₂H₁₅NO₄, 237.0961, Found, 237.1001.

### Step B

### (N-BOC-4-Aminobenzoyl)-Met-OCH₃

Into a dried, nitrogen filled flask was placed N-BOC-4-aminobenzoic acid (8.77 g, 36.97 mmol) in dry methylene chloride (148 mL) along with methionine methyl ester hydrochloride (8.12 g, 40.66 mmol). This solution was cooled in an ice bath and triethylamine (6.7 mL), EDCI (7.80 g, 40.66 mmol) and hydroxybenzotriazole (HOBT, 5.50 g, 40.66 mmol) were added. The mixture was stirred overnight, diluted with more methylene chloride and was extracted three times each with 1 M HCl, 1M NaHCO₃ and water. The methylene chloride was dried over MgSO₄ and the solvent was removed in vacuo. The resulting solid was recrystallized from ethyl acetate/hexanes to yield the desired product (9.72 g): m.p. 184-185°C; ¹H NMR (CDCl₃) δ 1.53 (9H, s), 2.06-2.18 (4H, m), 2.23-2.33 (1H, m), 2.59 (2H, t, *J*=7.6 Hz), 3.80 (3H, s), 4.92 (1H, m), 7.45 (2H, d, *J*=8.7 Hz), 7.77 (2H, d, *J*=8.7 Hz), ¹³C NMR (CDCl₃) δ 15.59, 28.34, 30.15, 31.64, 52.10, 52.73, 81.20, 117.73, 127.8, 128.33, 141.88, 152.33, 166.50, 172.75;
Anal. Calc. for C₁₈H₂₆N₂O₅S, C: 56.53, H: 6.85, N: 7.29; Found, C: 56.47, H: 6.86, N: 7.29; m/z (El) 382 (M).

### Step C

### (4-Aminobenzoyl)-Met-OCH₃ hydrochloride

N-BOC-4-aminobenzoyl-Met-OCH₃ (3.53 g, 9.59 mmol) was placed into methylene chloride (30-35 mL) and to it was added 3M HCl/EtO₂ (38.4 mL). After standing, a white precipitate formed. After two hours the solution was decanted and the crystals were collected by centrifugation. The crystals were then washed several times with fresh ether and dried overnight on the vacuum pump. Meanwhile, the filtrate was left to stand overnight to allow additional product to precipitate. The second fraction was washed with ether and dried overnight on the vacuum pump. The total yield of the desired product was 2.87 g: m.p. 158-164°C; ¹H NMR (CDCl₃) δ 2.10 (3H, s), 2.12-2.29 (1H, m), 2.52-2.71 (1H, m), 2.59 (2H, t, *J*=7.6 Hz), 3.75 (3H, s), 4.79 (1H, m), 7.02 (2H, d, *J*=8.6 Hz), 7.55 (2H, d, *J*=8.6 Hz); ¹³C NMR (CDCl₃) δ 15.23, 31.43, 31.53, 52.91, 52.43, 124.35, 130.56, 135.31, 135.76, 168.95, 173.87; HRMS Calc. for C₁₃H₁₈N₂O₃S, 282.1038, Found 282.1009.

### Compound 5

### (4-Amino-3-methylbenzoyl)-Met-OCH₃

### Step A

### N-BOC-4-Amino-3-methylbenzoic acid

4-Amino-3-methylbenzoic acid (5 g, 33.1 mmol) was reacted according to the same procedure as that used in the process for preparing N-BOC-4-aminobenzoic acid. The resulting orange-brown solid was recrystallized from ethyl acetate and hexanes to provide the desired product (4.99 g) as tan prismatic crystals: m.p. 180-182°C; 1H NMR (CD₃OD) δ 1.51 (9h, s), 2.27 (3H, s), 7.66 (1H, d, *J*=8.1 Hz), 7.79-7.82 (2H, m), 8.32 (1H, s); 13C NMR (CD3OD) δ 17.98, 28.62, 81.47, 123.12, 127.05, 129.14, 130.65, 132.99, 142.45, 155.33, 168.70; Anal. Calc. for C₁₃H₁₇NO₄, C: 62.15, H: 6.82, N: 5.58; Found C: 62.07, H: 6.86, N: 5.46; m/z (El) 251; HRMS Calc. for C₁₃H₁₇NO₄, 251.1158; Found, 251.1153.

### Step B

### (N-BOC-4-Amino-3-methylbenzoyl)-Met-OCH₃

N-BOC-4-amino-3-methylbenzoic acid (2.00 g, 7.96 mmol) was reacted with with methionine methyl ester hydrochloride (1.75 g, 8.76 mmol), triethylamine (1.4 mL), EDCl (1.68 g, 8.76 mmol) and hydroxybenzotriazole (HOBT, 1.18 g, 8.76 mmol) in dry methylene chloride (31.8 mL) according to the procedure described for the preparation of N-BOC-4-aminobenzoyl)-Met-OCH₃. The resulting solid was recrystallized from ethyl acetate/hexanes to yield the desired product (2.61 g): m.p. 163-165°C; ¹H NMR (CDCl₃) δ 1.54 (9H, s), 2.06-2.18 (4H, m), 2.23-2.34 (4H, m), 2.59 (2H, t, *J*=6.8 Hz), 3.80 (3H, s), 4.92 (1H, m), 6.45 (1H, s), 6.88 (1H, d, *J*=7.5 Hz), 7.63 (1H, d, *J*=8.6 Hz), 7.66 (1H, s), 8.05 (1H, d, *J*=8.6 Hz); ¹³C NMR (CDCl₃) δ 15.47, 17.61, 28.22, 30.03, 31.55, 51.93, 52.57, 81.04, 118.73, 125.62, 127.66, 129.54, 139.89, 152.34, 166.58, 172.66.

### Step C

### (4-Amino-3-methylbenzoyl)-Met-OCH₃ hydrochloride

N-BOC-4-Amino-3-methylbenzoyl-Met-OCH₃ (0.99 g, 2.59 mmol) was dissolved in methylene chloride (15-20 mL) and precipitated with 3M HCl/Et₂O (20.7 mL). A pale orange precipitate was obtained, washed with ether and dried overnight on the vacuum pump. The total yield of the desired product was 0.83 g: m.p. 157-159°C; ¹H NMR (CD₃OD) δ 2.04 (3H, s), 2.11-2.25 (1H, m), 2.47 (3H, s), 2.52-2.68 (3H, m), 3.74 (3H, s), 4.75-4.80 (1H, m), 7.48 (1H, d, *J*=8.2 Hz), 7.81 (2H, d, *J*=8.2 Hz), 7.87 (1H, s); ¹³C NMR (CD₃OD) δ 15.23, 17.28, 31.43, 31.51, 52.91, 53.37, 124.41, 127.85, 131.99, 133.63, 134.14, 135.65, 169.05, 173.84; Anal. Calc, for C₁₄H₂₁N₂O₃S, C: 50.52, H: 6.36, N: 8.42; Found C: 50.71, H: 6.40, N: 8.34.

### Compound 6

### (4-Amino-3-methoxybenzoyl)-Met-OCH₃

### Step A

### N-BOC-4-Amino-3-methoxybenzoic acid

4-Amino-3-methoxybenzoic acid (1 g, 5.98 mmol) was reacted according to the same procedure as that used in the process for preparing N-BOC-4-aminobenzoic acid. The resulting solid was recrystallized from ethyl acetate and hexanes to provide the desired product (1.5 g) as tan crystals: m.p. 176-178°C; ¹H NMR (CD₃OD) δ 1.52 (9H, s), 3.92 (3H, s), 7.56 (1H, s), 7.62 (1H, d, *J*=8.4Hz), 7.96 (1H, s), 8.03 (1H, d, *J*=8.4 Hz); ¹³C NMR (CD₃OD) δ 28.53, 56.35, 81.78, 172.01, 118.58, 124.20, 125.76, 133.84, 149.04, 154.20, 169.60; HRMS Calc. for C₁₃H₁₇NO₅, 267.1107; Found, 267.1103.

### Step B

### (N-BOC-4-Amino-3-methoxybenzoyl)-Met-OCH₃

N-BOC-4-amino-3-methoxybenzoic acid (0.35 g, 1.31 mmol) was reacted with with methionine methyl aster hydrochloride (0.9 g, 1.43 mmol) using EDCI according to the procedure described for the preparation of (N-BOC-4-aminobenzoyl)-Met-OCH₃. The resulting solid was recrystallized from ethyl acetate/hexanes to yield the desired product (0.36 g): m.p. 163-165°C; ¹H NMR (CDCl₃) δ 1.53 (9H, s), 2.09-2.18 (4H, m), 2.23-2.35 (1H, m), 2.60 (2H, t, *J*=6.9 Hz), 3.80 (3H, s), 3.93 (3H, s), 4.92 (1H, br s), 6.93 (1H, d, *J*=7.6 Hz), 7.25(1H, m), 7.31 (1H, d, *J*=10.2 Hz), 7.44 (1H, s), 8.15 (1H, d, *J*=8.5 Hz); ¹³C NMR (CDCl₃) δ 15.47, 28.23, 30.09, 31.48, 52.06, 52.54, 55.81, 80.82, 98.06, 109.38, 116.66, 119.31, 131.52, 147.23, 152.31, 166.57, 172.58; m/z (FAB) 413 (M + 1).

### Step C

### (4-Amino-3-methoxybenzoyl)-Met-OCH₃ hydrochloride

N-HOC-4-Amino-3-methoxybenzoyl-Met-OCH₃ (0.71 g, 1.79 mmol) was dissolved in methylene chloride (4 mL) and precipitated with 3M HCl/Et₂O (12 mL). A reddish precipitate was obtained, washed with ether and dried overnight on the vacuum pump. The total yield of the desired product was 0.55 g: m.p. 176-177°C; ¹H NMR (CD₃OD) δ 2.08 (3H, s), 2.21 (2H, m), 2.61 (2H, m), 3.74 (3H, s), 4.02 (3H, s), 4.79 (1H, m), 7.50 (1H, d, *J*=8.2 Hz), 7.57 (1H, d, *J*=4.1 Hz), 7.67 (1H, s); ¹³C NMR (CD₃OD) δ 15.26, 31.34, 31.42, 52.95, 53.38, 57.12, 112.29, 121.43, 124.57, 124.77, 136.15, 153.67, 168.79, 173.81.

### Compound 7

### (4-Amino-1-naphthoyl)-Met -OCH₃

### Step A

### 4-Amino-1-naphthoic acid

4-Amino-1-naphthalenecarbonitrile (1.5 g, 8.91 mmol) was suspended in a 50% KOH solution (18 mL). The heterogeneous solution was heated at reflux for 2-3 days. Once the solution became homogeneous and TLC showed no more starting material, the deep red solution was cooled and poured over 200 mL of water. The resulting solution was then filtered and the desired product was precipitated with concentrated HCl. The resulting red crystals were filtered and the filtrate was refiltered to give pink crystals. The first fraction of crystals was treated with activated carbon to remove some of the red color. A total of 1.51 g of the desired product was obtained: m.p. 169-171°C; ¹H NMR (CD₃OD) δ 6.69 (1H, d, *J*=8.2 Hz), 7.38-7.43 (1H, m), 7.48-7.54 (1H, m), 8.03 (1H, d, *J*=8.5 Hz), 8.13 (1H, d, *J*=8.2 Hz), 9.09 (1H, d, *J*=8.5 Hz); ¹³C NMR (CD₃OD) δ 107.39, 114.61, 122.99, 123.92, 125.21, 127.40, 128.48, 135.04, 151.35, 171.44; HRMS Calc. for C₁₁H₇NO₂, 187.0633; Found, 187.0642.

### Step B

### N-BOC-4-Amino-1-naphthoic acid

4-Amino-1-naphthoic acid (0.86 g, 4.61 mmol) was dissolved in dioxane (9.2 mL). Di-t-butyl dicarbonate (1.11 g, 5.07 mmol) was added and the mixture was stirred overnight. The reaction mixture was worked up as described above for N-BOC-4-aminobenzoic acid to give 0.76 g of the desired product as a reddish pink solid: m.p. 194-195°C; ¹H NMR (CD₃OD) δ 1.56 (9H, s), 7.53-7.62 (2H, m), 7.79 (1H, d, *J*=8.1 Hz), 8.12 (1H, d, *J*=8.0 Hz), 8.22 (1H, d, *J*=8.18 Hz), 9.02 (1H, d, *J*=8.9 Hz); ¹³C NMR (CD₃OD) δ 26.68, 81.62, 119.06, 123.40, 124.57, 127.03, 127.37, 128.49, 128.77, 131.89, 133.76, 139.86, 155.95, 170.73; Anal. Calc. for C₁₇H₁₇NO₄, C: 66.90, H: 5.96, N: 4.88; Found C: 66.49, H: 6.08, N: 4.79; m/z (El), 289; HRMS Calc, for C₁₆H₁₇NO₄, 287.1158; Found, 287.1151.

### Step C

### (N-BOC-4-Amino-1-naphthoyl)-Met-OCH₃

N-BOC-4-Amino-naphthoic acid (0.46 g, 1.60 mmol), methionine methyl ester hydrochloride (0.35 g, 1.76 mmol), EDCl (0.43 g, 1.76 mmol), HOBT (0.24 g, 1.76 mmol) and triethylamine (0.27 mL) in methylene chloride (6.4 mL) were reacted as described above for N-BOC-4-aminobenzoyl-Met-OCH3. After workup and recrystallization from ethyl acetate hexanes, the desired product (0.44 g) was obtained as pale pink crystals: m.p. 131-132°C; ¹H NMR (CDCl₃) δ 1.57 (9H, s), 2.11-2.21 (4H, m), 2.29-2.41 (1H, m), 2.65 (2H, t, *J*=7.1 Hz), 3.83 (3H, s), 4.99-5.06 (1H, m), 6.68 (1H, d, *J*=8.0 Hz), 7.02 (1H, s), 7.56-7.59 (2H, m) 7.69 (1H, d, *J*=7.9 Hz), 7.87-7.90 (1H, m), 8.02 (1H, d, *J*=7.9 Hz), 8.44-8.48 (1H, m); ¹³C NMR (CDCl₃) δ 15.56, 28.31, 30.19, 31.65, 52.06, 52.64, 81.17, 115.82, 120.18, 125.79, 126.37, 126.53, 127.18, 131.02, 135.65, 152.93, 169.04, 172.40; HRMS Calc. for C₂₂H₂₈N₂O₅S, 432.1719; Found, 432.1702; m/z (FAB) 433 (M+1).

### Step D

### (4-Amino-1-naphthoyl)-Met-OCH₃ hydrochloride

(N-BOC-4-Amino-1-naphtholyl)-Met-OCH₃ (0.57 g, 1.31 mmol) was deprotected with HCl/ether to yield the desired product (0.31 g) as a white solid: m.p. 178-181°C; ¹H NMR (CD₃OD) δ 2.08-2.16 (4H, m), 2.20-2.30 (1H, m) 2.57-2.75 (2H, m) 3.82 (3H, s), 4.87-4.91 (1H, m), 7.59 (1H, d, *J*=7.5 Hz), 7.67 (1H, d, *J*=7.5 Hz) 7.71-7.80 (2H, m), 8.03 (1H, dd, *J*=7.1, 2.0 Hz), 8.35 (1H, dd, *J*=6.8, 1.8 Hz); ¹³C NMR (CD₃OD) δ 15.23, 31.40, 53.01, 53.33, 119.90, 122.20, 126.15, 127.41, 127.77, 129.09, 129.31, 131.50, 132.33, 135.64, 171.77, 173.83; m/z (FAB), 369 (M+1).

### Compound 8

### (4-Amino-2-phenylbenzoyl)-Met-OCH₃

### Step A

### 4-Nitro-2-phenyltoluene

2-Bromo-4-nitrotoluene (2.16 g, 10.00 mmol) and phenylboric acid (1.46 g, 12.00 mmol) were dissolved in anhydrous DMF (25 mL) under nitrogen. To this mixture was added Pd(Ph₃P)₄ (0.58 g, 5%). The mixture was heated at 100°C overnight. The solution was poured onto 1 N HCI and extracted with Et₂O. The crude product was chromatographed on silica gel using hexanes as eluent. After recrystallization from ethanol, the desired product (1.23 g) was obtained as pale orange needles: m.p. 69-71°C; ¹H NMR (CDCl₃) δ 2.36 (3H, s), 7.29-7.40 (2H, m), 7.41-7.49 (5H, m), 8.07-8.10 (2H, m); ¹³C NMR (CDCl₃) δ 20.68, 121.96, 124.51, 127.78, 128.41, 128.83, 131.06, 139.06, 139.44, 142.97, 143.48, 146.05; Anal. Calc. for C₁₃H₁₁NO₂, C: 73.26, H: 5.20, N: 6.57; Found, C: 73.10, H: 5.12, N: 6.50; m/z (El) 213; HRMS Calc. for C₁₃H₁₁NO₂, 213.0790; Found, 213.0793.

### Step B

### 4-Nitro-2-phenylbenzoic acid

4-Nitro-2-phenyltoluene (0.5 g, 2.34 mmol) was dissolved in water (4.6 mL) and pyridine (2.3 mL). The mixture was heated to reflex and KMnO₄ (1.85 g, 11.7 mmol) was added. The reaction mixture was heated overnight and the solution was filtered and washed several times with boiling water. The aqueous solution was made acidic and the product was extracted into ethyl acetate. The ethyl acetate solution was dried over Na₂SO₄ and the solvent removed in vacuo to provide the desired product (0.37 g): m.p. 174-176°C, ¹H NMR (CD₃OD) δ 7.38-7.48 (5H, m), 7.96 (1H, d, *J*=8.5 Hz), 8.21 (1H, d, *J*=2.3 Hz), 8.28 (1H, dd, *J*=8.48, 2.37 Hz); ¹³C NMR (CD₃OD) δ 122.95, 126.09, 129.27, 129.42, 129.49, 131.56, 139.26, 140.42, 144.41, 150.17, 170.52; m/z (EI) 243 (M).

### Step C

### (4-Nitro-2-phenylbenzoyl)-Met-OCH₃

4-Nitro-2-phenylbenzoic acid (0.3 g, 1.23 mmol), methionine methyl ester hydrochloride salt (0.27 g, 1.35 mmol), EDCI (0.26 g, 1.35 mmol), HOST (0.18 g, 1.35 mmol) and triethylamine (0.19 mL) in dry methylene chloride (4.9 mL) were reacted according the procedure described above for (N-BOC-4-aminobenzoyl)-Met-OCH₃. After recrystallization of the product from ethyl acetate hexanes, the desired product (0.41 g) was obtained: m.p. 98-101°C; ¹H NMR (CDCl₃) δ 1.62-1.73 (1H, m), 1.79-1.88 (1H, m), 1.91 (3H, s), 1.99 (2H, t, *J*=7.2 Hz), 3.59 (3H, s), 4.53 (1H, m), 6.45 (1H, d, *J*=7.8 Hz), 7.33-7.40 (5H, m), 7.67 (1H, d, *J*=8.3 Hz), 8.07-8.12 (2H, m); ¹³C NMR (CDCl₃) δ 14.92, 29.11, 30.67, 51.51, 52.29, 121.86, 124.74, 128.27, 128.60; 128.69, 129.52, 137.50, 140.56, 147.02, 148.09, 167.23, 171.23; m/z (FAB), 389 (M+1).

### Step D

### (4-Amino-2-phenylbenzoyl)-Met-OCH₃

(4-Nitro-2-phenylbenzoyl)-Met-OCH₃ (0.35 g, 0.90 mmol) was dissolved in ethyl acetate (9.0 mL). To this mixture was added SnCl₂ · 2H₂O (1.02 g, 4.5 mmol) and the reaction mixture was heated under nitrogen at reflux for one hour. The mixture was poured onto ice, the solution was made basic using NaHCO₃ and the product was extracted into ethyl acetate several times (7-8). The ethyl acetate solutions were combined, washed with brine and dried over Na₂SO₄. The solvent was removed in vacuo to the desired product (0.24 g) as a yellow solid: ¹H NMR (CDCl₃) δ 1.58-1.70 (1H, m), 1.80-1.92 (1H, m), 1.98 (3H, s), 2.06 (2H, t, *J*=7.7 Hz), 3.62 (3H, s), 4.00 (2H, br s), 4.56-4.63 (1H, m), 5.84 (1H, d, *J*=7.7 Hz), 6.50 (1H, s), 6.61 (1H, d, *J*=8.4 Hz) 7.29-7.42 (5H, m), 7.58 (1H, d, *J*=8.3 Hz); ¹³C NMR (CDCl₃) δ 15.02, 29.25, 31.25, 51.57, 52.15, 113.27, 115.88, 123.52, 127.56, 128.37, 128.44, 130.92, 140.66, 141.44, 148.53, 168.58, 171.91.

### Compound 9

### (4-Amino-2-(2-thienyl)benzoyl)-Met-OCH₃

The title compound can be prepared according to the method used to prepare Compound 8, only substituting thiophene-2-boronic acid for phenyl boronic acid.

### Compound 10

### (4-Amino-1-(1-naphthyl)benzoyl)-Met-OCH₃

The title compound can be prepared according to the method used to prepare Compound 8, only substituting 1-naphthylboronic acid for phenylboronic acid.

The next compound is Compound 17.

### Compound 17

### (4-Amino-2-(3,5-dimethylphenyl)benzoyl)-Met-OCH₃

### Step A

### 2-Bromo-4-nitrobenzoic acid

2-Bromo-4-nitrotoluene (5.0 g, 23.14 mmol) was dissolved in pyridine (23 mL) and water (46 mL). The heterogeneous mixture was heated to 60°C and KMnO₄ (18.29 g, 115.7 mmol) was added carefully. The mixture was then heated under reflux overnight. The reaction mixture was filtered and washed with boiling water. The solution was then made acidic and extracted into ethyl acetate, dried over Na₂SO₄ and the solvent was removed in vacuo. The crude product was dissolved in aqueous NaOH and washed with hexanes. The aqueous phase was made acidic and the product was extracted into ethyl acetate. The ethyl acetate solutions were combined and dried over Na₂SO₄ and the solvent was removed in vacuo to provide the desired product (3.72 g): m.p. 158-160°C; ¹H NMR (CD₃OD) δ 7.81 (1H, d, *J*=8.5 Hz), 8.08 (1H, d, *J*=8.5 Hz), 8.30 (1H, s); ¹³C NMR (CD₃OD) δ 121.96, 122.75, 129.36, 132.24, 139.52, 149.54, 167.75; Anal. Calc. for C₇H₄BrNO₄ •0.1 ethyl acetate, C: 34.88, H: 1.90, N: 5.50; Found, C: 34.68, H: 1.86, N: 5.82.

### Step B

### 3,5-Dimethylphenylboronic acid

Magnesium turnings (1.44 g, 59.43 mmol) were coverd with dry THF (18.8 mL) in a dried, nitrogen filled flask fitted with an addition funnel and reflux condenser. To this was added 5-bromo-m-xylene (10 g, 54.03 mmol) in THF (15 mL) after initiation of the Grignard reaction. The addition was carried out over several minutes and the reacton mixture was heated at reflux for 1-2 hours until most of the magnesium had reacted. The reaction mixture was then cooled and transferred to an addition funnel fitted to an nitrogen filled flask containing triisopropyl borate (24.9 mL) at -70°C. The dropwise addition was carried out over several minutes and the mixture warmed to room temperature and stirred overnight. The grey solution was poured onto 2 M HCl and immediately turned yellow. The solution was extracted with Et₂O and the Et₂O fractions were combined, dried over MgSO₄ and the solvent was removed in vacuo to provide the desired product (2.41 g): m.p.249-251°C; ¹H NMR (CDCl₃) δ 2.44 (6H, s), 7.23 (1H, s), 7.84 (2H, s); ¹³C NMR (CD₃OD) δ 21.36, 133.28, 134.39, 137.48.

### Step C

### 4-Nitro-2-(3,5-dimethylphenyl)benzoic acid

2-Bromo-4-nitrobenzoic acid (0.43 g, 2.03 mmol) and 3,5-dimethylphenyl boronic acid (0.334 g, 2.23 mmol) were dissolved in anhydrous DMF (25 mL) under nitrogen. To this mixture was added Cs₂CO₃ (1.66 g, 5.08 mmol) followed by Pd(Ph₃P)₄ (0.12 g, 5%). The mixture was heated at 100°C overnight. The solution was poured onto 1N HCl and extracted with Et₂O. it was dried over MgSO₄ and the solvent was removed in vacuo. The crude product was chromatographed on silica gel using a 9:1 mixture of hexanes and ethyl acetate to provide the desired product (0.34 g): ¹H NMR (CDCl₃) δ 2.36 (6H, s), 6.99 (2H, s), 7.07 (1H, s), 8.03 (1H, d, *J*=9.0 Hz), 8.23-8.25 (2H, m); ¹³C NMR (CDCl₃) δ 21.28, 121.68, 123.68, 125.74, 126.07, 130.22, 131.19, 131.31, 135.04, 138.21. 144.74, 170.75.

### Step D

### (4-Nitro-2-(3.5-dimethylphenyl)benzoyl)-Met-OCH₃

4-Nitro-2-(3,5-dimethylphenyl)benzoic acid (0.15 g, 0.55 mmol), methionine methyl ester hydrochloride (0.11 g, 0.55 mmol), EDCl (0.11 g, 0.55 mmol), HOBT (0.07 g, 0.55 mmol) and triethylamine (0.08 mL) in dry methylene chloride (2.2 mL) were reacted and worked up according to the procedure for (N-BOC-4-aminobenzoyl )- Met-OCH₃ as described above. After recrystattization from ethyl acetate and hexanes, the desired product was obtained (0.13 g): m.p. 122-124°C; ¹H NMR (CDCl₃) δ 1.2-1.84 (1H, m), 1.85-1.97 (1H, m), 2.01 (3H, s), 2.05 (3H, t, *J*=7.7Hz), 2.38 (6H, s), 3.70 (3H, s), 4.67-4.74 (1H, m), 6.03 (1H, d, *J*=7.9 Hz), 7.05 (2H, s), 7.09 (1H, s), 7.84-7.87 (1H, m), 7.84-7.87 (1H, m) 8.23-8.26 (2H, m); ¹³C NMR (CDCl₃) δ 15.20, 21.26, 29.22, 31.15, 51.79, 52.57, 122.07, 125.11, 126.27, 130.03, 130.53, 137.77, 138.82, 140.29, 141.56, 148.41, 167.14, 171.53.

### Step E

### (4-Amino-2-(3,5-dimethylphenyl)benzoyl)-Met-OCH₃

(4-Nitro-2-(3,5-dimethylphenyl)benzoyl)-Met-OCH₃ (0.11 g, 0.26 mmol) was dissolved in ethyl acetate (3.0 mL). To this mixture was added SnCl₂ · 2H₂O (0.3 g, 1.30 mmol) and the reacton was heated under nitrogen at reflux for 6 hours. The mixture was worked up as described above for (4-amino-2-phenylbenzoyl)-Met-OCH₃ to give the desired product (0.15 g): ¹H NMR (CDCl₃) δ 1.60-1.70 (1H, m), 1.80-1.90 (1H, m), 1.99 (3H, s), 2.05 (2H, t, *J*=7.6 Hz), 2.33 (6H, s), 3.64 (3H. s), 3.93 (2H, br s), 4.61-4.64 (1H, m), 5.82 (1H, d, *J*=7.7 Hz), 6.49 (1H, d, *J*=2.3 Hz) 6.62 (1H, dd, *J*=8.4, 2.4 Hz), 6.98 (2H, s), 7.00 (1H, s), 7.65 (1H, d, *J*=8.3 Hz); ¹³C NMR (CDCl₃) δ 15.08, 21.17, 29.28, 31.49, 51.70, 52.18, 113.30, 115.94, 123.55, 126.36, 129.32, 131.23, 138.15, 140.72, 141.92, 148.40, 168.45, 172.01.

### Preparation 1

### Anilines of the formula B-NH₂

The anilines from Table 2 entries 10-54 and 64-126 (B-NH₂) are prepared using the procedures for Compounds 1-10 and 17 with the exception that methionine methyl ester is replaced by methioninesulfone methyl ester, (S-Me)cysteine methyl ester, serine methyl ester, (O-Me)serine methyl ester, (O-Me)homoserine methyl ester, homoserine lactone, isoleucine methyl ester, leucine methyl ester, norleucine methyl ester, norvaline methyl ester, cyclohexylalanine methyl ester, phenylalanine methyl ester, or glutamic acid dimethyl ester.

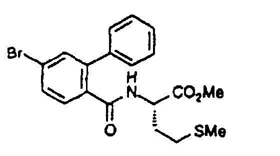

### Preparation 2

### 4-Bromo-2-phenylbenzoyl methionine methyl ester

### Preparation 2A

### 4-Bromo-2-phenylbenzoic acid methyl ester

A solution of methyl 4-amino-2-phenylbenzoic acid (1.0 equivalent) in dilute aqueous HBr is treated with NaNO₂ (1.1 equivalents) to form the diazonium salt. The reaction is treated with CuBr (1.1 equivalents) and heated. When judged complete by TLC analysis, the mixture is extracted into ethyl acetate which is dried and evaporated. The title arylbromide is purified by chromatography on silica gel.

### Preparation 2B

### 4-Bromo-2-phenylbenzoic acid

To a solution of the resultant compound from Preparation 2A (1.0 equivalent) in a 3:1 mixture of tetrahydrofuran (THF) and water is added an excess (1.5 equivalents) of LiOH. When hydrolysis is judged complete by TLC analysis, the solvent is evaporated and the remaining aqueous layer is acidified to pH = 3 and extracted into ethyl acetate which is dried and evaporated prior to purification by chromatography on silica gel.

### Preparation 2C

### 4-Bromo-2-phenylbenzoyl methionine methyl ester

To a solution of the resultant compound from Preparation 2B (1.0 equivalent) in dimethylformamide (DMF) is added 3-hydroxy-1,2,3-benzotriazin-4(3H)-one (1.5 equivalents) followed by methionine methyl ester (1.0 equivalent) and 1-(3-dimehtylaminopropyl)-3-ethylcarbodiimide hydrochloride (1.5 equivalents). When judged complete by TLC analysis, the reaction is taken up in ethyl acetate which is washed by 1N HCl and saturated brine, and then is dried and evaporated. The crude reaction mixture is purified by column chromatography to afford the title product.

### Preparation 2D

### 4-Bromo-2-phenylbenzoyl methionine methyl ester alternate procedure

A solution of 4-amino-2-phenylbenzoyl methionine methyl ester (1.0 equivalent) in dilute aqueous HBr is treated with NaNO₂ (1.1 equivalents) to form the diazonium salt. The reaction is treated with CuBr (1.1 equivalents) and heated. When judged complete by TLC analysis, the mixture is extracted into ethyl acetate which is dried and evaporated. The title arylbromide is purified by chromatography on silica gel.

### Preparation 3

### Arylbromides of the formula B-Br

The anilines from Table 2 (B-NH₂) are reacted according to the procedures of Preparation 2 to provide the arylbromides listed in Table 3.

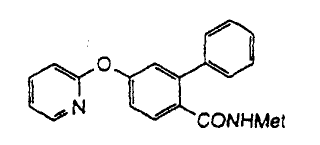

### Example 1

### [4-(2-pyridyloxy)-2-phenylbenzoyl]methionine

### Example 1A

### 4-Hydroxy-2-phenylbenzoic acid methyl ester

A solution of methyl 4-amino-2-phenylbenzoate (1.0 equivalent) in dilute aqueous H₂SO₄ is treated with NaNO₂ (1.1 equivalents) until an excess of nitrous acid persists to form the diazonium salt. This salt is then diluted further with water and heated. The mixture is extracted into ethyl acetate which is dried and evaporated. The title ester is purified by chromatography on silica gel.

### Example 1B

### 4-(2-Pyridyloxy)-2-phenylbenzoic acid methyl ester

A solution of the resultant phenol from Example 1A (1.0 equivalent) is treated with 2-bromopyridine (1.0 equivalent) in the presence of a NaH (1.0 equivalent), or K₂CO₃ (2.0 equivalents) and copper (1.0 equivalent) in DMF or pyridine. The product is isolated by removal of the solvent and chromatography on silica gel.

### Example 1C

### 4-(2-Pyridyloxy)-2-phenylbenzoic acid

A solution of the resultant ester from Example 1B (1.0 equivalent) in aqueous methanol is treated with NaOH (2.0 equivalents) and stirred until the reaction is deemed complete by TLC analysis. The mixture is acidified, diluted with water, and extracted into ethyl acetate which is dried and evaporated. Chromatography on silica gel provides the title product.

### Example 1D

### [4-(2-Pyridyloxy)-2-phenylbenzoyl]methionine methyl ester

To a solution of the resultant compound from Example 1C (1.0 equivalent) in dimethylformamide (DMF) is added 3-hydroxy-1,2,3-benzotriazin-4(3H)-one (1.5 equivalents) followed by methionine methyl ester (1.0 equivalent) and 1-(3-dimethylaminopropyl)-3-ethylcarbodiimide hydrochloride (1.5 equivalents). When judged complete by TLC analysis, the reaction is taken up in ethyl acetate which is washed with 1N HCl and saturated brine, and then is dried and evaporated. The crude reaction mixture is purified by column chromatography to afford the title product.

### Example 1E

### [4-(2-Pyridyloxy)-2-phenylbenzoyl]methionine methyl ester, alternate procedure

A solution of 4-amino-2-phenylbenzoyl methionine methyl ester (1.0 equivalent) in dilute aqueous H₂SO₄ is treated with NaNO₂ (1.1 equivalents) until an excess of nitrous acid persists to form the diazonium salt. This salt is then diluted further with water and heated to form the phenol which is purified by chromatography on silica gel. A solution of this phenol (1.0 equivalent) is treated with 3-bromopyridine (1.0 equivalent) in the presence of a NaH (1.0 equivalent), or K₂CO₃ (2.0 equivalents) and copper (1.0 equivalent) in DMF or pyridine. The product is isolated by removal of the solvent and chromatography on silica gel.

### Example 1F

### [4-(2-pyridyloxy)-2-phenyl]benzoylmethionine

The resultant compound from Example 1E is hydrolyzed according to the procedure of Example 1C to give the title product.

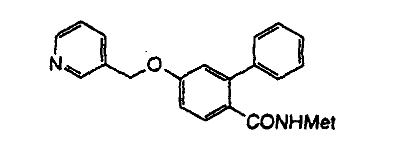

### Example 2

### [4-(3-pyridylmethyloxy)-2-phenylbenzoyl]methionine

The title compound is prepared as described in Example 1 with the exception that 2-bromopyridine is replaced by 3-chloromethylpyridine hydrochloride.

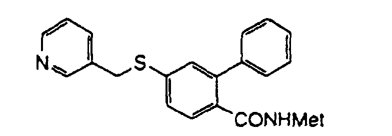

### Example 3

### [4-(3-Pyridylmethylthio)-2-phenylbenzoyl]methionine

### Example 3A

### 4-Mercapto-2-phenylbenzoic acid methyl ester

A solution of methyl 4-amino-2-phenylbenzoic acid (1.0 equivalent) in dilute aqueous H₂SO₄ is treated with NaNO₂ (1.1 equivalents) to form the diazonium salt. The reaction is treated with S₈ (10 equivalents) and heated. The mixture is extracted into ethyl acetate which is dried and evaporated. The title thiophenol is purified by chromatography on silica gel.

### Example 3B

### 4-(2-Pyridylmethylthio)-2-phenylbenzoic acid methyl ester

A solution of the resultant thiophenol (1.0 equivalent) from Example 3A is treated with 2-chloromethylpyridine hydrochloride (1.0 equivalent) in the presence of a NaH (2.0 equivalents), or K₂CO₃ (3.0 equivalent)s in DMF or pyridine. The product is isolated by removal of the solvent and chromatography on silica gel.

### Example 3C

### 4-(2-Pyridylmethylthio)-2-phenylbenzoic acid

The resultant compound from Example 3B is hydrolyzed according to the procedure of Example 1C to give the title acid.

### Example 3D

### [4-(2-Pyridylmethylthio)-2-phenylbenzoyl]methionine methyl ester

To a solution of the resultant compound from Example 3C (1.0 equivalent) in dimethylformamide (DMF) is added 3-hydroxy-1,2,3-benzotriazin-4(3H)-one (1.5 equivalents) followed by methionine methyl ester (1.0 equivalent) and 1-(3-dimehtylaminopropyl)-3-ethylcarbodiimide hydrochloride (1.5 equivalents). When judged complete by TLC analysis, the reaction is taken up in ethyl acetate which is washed with 1N HCl and saturated brine, and then is dried and evaporated. The crude reaction mixture is purified by column chromatography to afford the title product.

### Example 3E

### [4-(2-Pyridylmethylthio)-2-phenylbenzoyl]methionine methyl ester, alternate procedure 1

A solution of 4-amino-2-phenylbenzoyl methionine methyl ester (1.0 equivalent) in dilute aqueous H₂SO₄ is treated with NaNO₂ (1.1 equivalents) to form the diazonium salt. The reaction is treated with S₈ (10 equivalents) and heated. The mixture is extracted into ethyl acetate which is dried and evaporated to afford 2-phenyl-4-mercaptobenzoyl-methionine methyl ester. The thiophenol is purified by chromatography on silica gel. A solution of this thiophenol (1.0 equivalent) is treated with 2-chloromethylpyridine hydrochloride (1.0 equivalent) in the presence of a NaH (2.0 equivalents), or K₂CO₃ (3.0 equivalents) in DMF or pyridine. The product is isolated by removal of the solvent and chromatography on silica gel.

### Example 3F

### [4-(2-Pyridylmethylthio)-2-phenylbenzoyl]methionine methyl ester, alternate procedure 2

Methyl 4-amino-2-phenylbenzoate (100 mmol) is mixed in 50% sulfuric acid, and is cooled by a ice-water bath. To the above mixture with good stirring is added slowly a cold solution of sodium nitrite (110 mmol) in water, the reaction temperature is kept under 10 °C. Powdered anhydrous sodium carbonate (100 mmol) is carefully added to the cold reaction mixture in small portions, until the reaction mixture reaches pH 7 to 8. Then, the reaction mixture is added in small portions to a solution of sodium p-methoxybenzylsulfide (prepared from reaction 110 mmol of p-methoxybenzylthiol with 55 mmol of 2.0 M NaOH aqueous solution). After completion of the addition, the reaction mixture is refluxed until judged complete by TLC analysis. The reaction mixture is then extracted with ether, and the organic extracts are washed sequentially with aqueous sodium carbonate solution, water and brine, dried with anhydrous magnesium sulfate, filtered, and concentrated in vacuo. The residue is then purified by column chromatography on silica gel.

The product thus obtained is dissolved in methanol and water, followed by addition of lithium hydroxide (200 mmol), and the mixture is refluxed until hydrolysis is judged complete by TLC analysis. The reaction mixture is then acidified with 6 N HCl, and extracted into ethyl acetate. The organic extracts are washed with brine, dried with anhydrous sodium sulfate, and concentrated in vacuo. The crude product obtained is redissolved in methylene chloride, followed by addition of 1-(3-dimethylaminopropyl)-3-ethylcarbodiimide (1.1 equivalent) and 1-hydroxybenzotriazol (1.2 equivalent). The reaction is stirred until it is judged complete by TLC analysis, and then is diluted with ether. The mixture is washed with water, brine, dried over anhydrous magnesium sulfate, filtered, and concentrated in vacuo. The residue is then purred by column chromatography on silica gel.

The resulting product is dissolved in trifluoroacetic acid and anisole (1.5 equivalent), and mercury diacetate (1.2 equivalent) is added. After TLC shows no starting material left, the reaction mixture is diluted with ether, washed with water, brine, dried over anhydrous magnesium sulfate, filtered, and concentrated in vacuo. The resulting crude material is purified by column chromatography to afford 2-phenyl-4-mercaptobenzoyl-methionine methyl ester. A solution of this thiophenol (1.0 equivalent) is treated with 2-chloromethylpyridine hydrochloride (1.0 equivalent) in the presence of a NaH (2.0 equivalents), or K₂CO₃ (3.0 equivalents) in DMF or pyridine. The product is isolated by removal of the solvent and chromatography on silica gel.

### Example 3G

### [4-(3-Pyridylthiomethyl)-2-phenylbenzoyl]methionine

To a solution of the resultant compound from Example 3D (1.0 equivalent) in a 3:1 mixture of tetrahydrofuran (THF) and water is added an excess (1.5 equivalents) of LiOH. When hydrolysis is judged complete by TLC analysis, the solvent is evaporated and the remaining aqueous layer is acidified to pH = 3 and extracted into ethyl acetate which is dried and evaporated prior to purification by chromatography on silica gel.

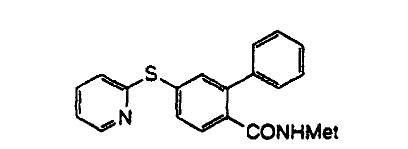

### Example 4

### 4-(2-Pyridylthio)-2-phenylbenzoylmethionine

### Example 4A

### 4-Fluoro-2-phenyl benzoic acid methyl ester

A solution of methyl 4-amino-2-phenylbenzoate (1.0 equivalent) in dilute aqueous HBF₄ is treated with NaNO₂ (1.1 equivalents) until an excess of nitrous acid persists. The mixture is extracted into ethyl acetate which is dried and evaporated. The title ester is purified by chromatography on silica gel.

### Example 4B

### 4-Fluoro-2-phenyl benzoic acid

The resultant compound from Example 4A is hydrolyzed according to the procedure of Example 1C to give the title acid.

### Example 4C

### 4-Fluoro-2-phenyl benzoyl methionine methyl ester

The resultant product from Example 4B is coupled to methionine methyl ester according to the procedure of Example 3D to give the title compound.

### Example 4D

### 4-(2-Pyridylthio)-2-phenyl benzoyl methionine methyl ester

A mixture of the resultant fluorobenzoate from Example 4C (1.0 equivalent) and 2-mercaptopyridine (1.0 equivalent) is treated with K₂CO₃ (2.0 equivalents) or NaH (1.0 equivalent) in DMF or DMSO and is stirred until the reaction is judged complete by TLC analysis. The mixture is diluted with water and extracted into ethyl acetate which is dried and evaporated. Chromatography of the residue on silica gel affords the title compound.

### Example 4E

### 4-(2-Pyridylthio)-2-phenyl benzoyl methionine methyl ester, alternate procedure 1

A solution of 4-amino-2-phenylbenzoyl methionine methyl ester (1.0 equivalent) in dilute aqueous H₂SO₄ is treated with NaNO₂ (1.1 equivalents) to form the diazonium salt. The reaction is treated with S₈ (10 equivalents) and heated. The mixture is extracted into ethyl acetate which is dried and evaporated. The title thiophenol is purified by chromatography on silica gel. A solution of this thiophenol (1.0 equivalent) is treated with 2-bromopyridine hydrobromide (1.0 equivalent) in the presence of a NaH (2.0 equivalent), or K₂CO₃ (3.0 equivalent)s in DMF or pyridine. The product is isolated by removal of the solvent and chromatography on silica gel.

### Example 4F

### 4-(2-Pyridylthio)-2-phenyl benzoyl methionine methyl ester, alternate procedure 2

A solution of the resultant thiophenol from Example 3A (1.0 equivalent) is treated with 2-bromopyridine hydrobromide (1.0 equivalent) in the presence of a NaH (2.0 equivalents), or K₂CO₃ (3.0 equivalents) in DMF or pyridine. The product is isolated by removal of the solvent and chromatography on silica gel. The resultant ester is hydrolyzed according to the procedure of Example 1 C and then is coupled to methionine methyl ester according to the procedure of Example 4 C to give the title compound.

### Example 4G

### 4-(2-Pyridylthio)-2-phenylbenzoylmethionine

To a solution of the resultant compound from Example 4D (1.0 equivalent) in a 3:1 mixture of tetrahydrofuran (THF) and water is added an excess (1.5 equivalents) of LiOH. When hydrolysis is judged compete by TLC analysis, the solvent is evaporated and the remaining aqueous layer is acidified to pH = 3 and extracted into ethyl acetate which is dried and evaporated prior to purification by chromatography on silica gel.

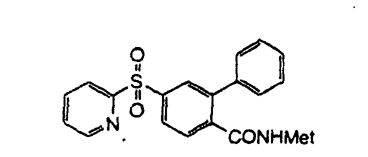

### Example 5

### 4-(2-Pyridylsulfonyl)-2-phenylbenzoylmethionine

### Example 5A

### 4-(2-Pyridylsulfonyl)-2-phenylbenzoic acid methyl ester

A solution of 4-(2-pyridylthio)-2-phenylbenzoic acid methyl ester is carefully treated with two equivalents of *meta*-chloroperbenzoic acid in methylene chloride at low temperature and the reaction is then quenched with aqueous Na₂SO₃ when judged complete by TLC analysis. The layers are separated and the organic phase is extracted with aqueous NaHCO₃ to remove the *m*-chlorobenzoic acid. The product is isolated by removal of the solvent and is purified by chromatography on silica gel.

### Example 5B

### 4-(2-Pyridylsulfonyl)-2-phenylbenzoylmethionine

The desired compound is prepared by hydrolysis of the product of Example 5A, followed by coupling with methionine methyl ester and saponification of the Methyl ester according to the method of Examples 4C, D and G.

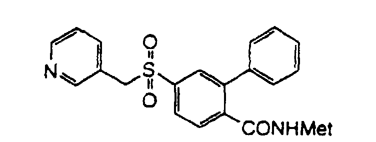

### Example 6

### 4-(3-Pyridylthiomethylen)-2-phenylbenzoylmethionine

The title compound is prepared from the resultant product of Example 3B using the procedures from Example 5.

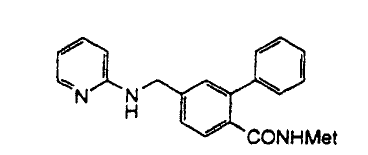

### Example 7

### 4-[(2-Aminopyridyl)methylene]-2-phenylbenzoylmethionine

### Example 7A

### 2-Phenylterephthalic acid mono methyl ester

A solution of 4-bromo-2-phenylbenzoic acid methyl ester (1.0 equivalent), Pd(OAc)₂ (0.05 equivalent) and DPPE (1.0 equivalent) is heated in DMF to 65° C under 4 atm. of carbon monoxide until TLC analysis indicates that the reaction is complete. The reaction mixture is poured into water and extracted with ethyl acetate which is dried and evaporated. The product is purified by chromatography on silica gel.

### Example 7B

### 4-(Hydroxymethyl)-2-phenylbenzoic acid methyl ester

The resultant acid from Example 7A (1.0 equivalent) is treated with a slight excess of N-methylmorpholine (1.1 equivalent) and isobutylchloroformate (1.0 equivalent) in THF at 0° C. The mixture is then treated with NaBH₄ (1.0 equivalent) and aqueous NaHCO₃ and stirred at 0° C until the reaction is judged complete by TLC analysis. The mixture is poured into dilute aqueous acid and extracted into ethyl acetate which is dried and evaporated. The product is purified by chromatography on silica gel.

### Example 7C

### 4-(Hydroxymethyl)-2-phenylbenzoic acid

The resultant compound from Example 7B is hydrolyzed according to the procedure of Example 1 C to give the title acid.

### Example 7D

### 4-(Hydroxymethyl)-2-phenylbenzoyl methionine methyl ester

The resultant product from Example 7C is coupled to methionine methyl ester according to the procedure of Example 1D to give the title compound.

### Example 7E

### 4-formyl-2-phenylbenzoyl methionine methyl ester

A mixture of the resultant alcohol from Example 7D (1.0 equivalent), N-methylmorpholine-N-oxide (1.5 equivalents), molecular sieves, and a catalytic amount of TPAP is stirred in a CH₂Cl₂/acetonitrile mixture until the reaction is judged complete by TLC analysis. The mixture is diluted with ethyl ether and filtered through SiO₂. The product is purified by chromatography on silica gel.

### Example 7F

### 4-(formyl)-2-phenylbenzoyl methionine methyl ester, alternate procedure

A mixture of (2-phenyl-4-bromobenzoyl) methionine methyl ester (100 mmol), 4,4,6-trimethyl-2-vinyl-1,3,2-dioxaborinane (100 mmol), tetrakis(triphenylphosphine)palladium (0) (3 mmol) in toluene and 2 M sodium carbonate in water (100 mL) is heated at 80 °C until the starting methyl ester disappears. The resulting mixture is extracted with ether, and washed with water, brine, dried over anhydrous magnesium sulfate, filtered, and concentrated in vacuo. The residue is then purified by column chromatography on silica gel.

To a solution of the resulting vinyl compound in dioxane/water (4/1) is added osmium tetraoxide (0.03 equivalent), N-methylmorpholine N-oxide (3 equivalents), and the reaction is stirred at 25 °C until TLC analysis shows the reaction to be complete. The reaction mixture is extracted with ether, which is washed with water and brine, dried over anhydrous magnesium sulfate, filtered, and concentrated in vacuo. The residue is then purified by column chromatography on silica gel to afford the title product.

### Example 7G

### 4-(Hydroxymethyl)-2-phenylbenzoyl methionine methyl ester, alternate procedure

To a solution of the resultant compound from Example 7E in ethanol at 0 °C is added sodium borohydride (0.5 equivalent), and the reaction is stirred at 0 °C until TLC analysis shows the reaction to be complete. The reaction mixture is extracted with ether, which is washed with water and brine, dried over anhydrous magnesium sulfate, filtered, and concentrated in vacuo. The residue is then purified by column chromatography on silica gel to afford the title product.

### Example 7H

### 4-[(2-Aminopyridyl)methylene]-2-phenylbenzoylmethionine methyl ester

A mixture of the resultant aldehyde from Example 7E (1.0 equivalent), 2-aminopyridine (1.0 equivalent) and NaCNBH₃ (1.5 equivalents) in methanol/acetic acid is stirred until the reaction is judged complete by TLC analysis. The mixture is poured into aqueous NaHCO₃ and extracted into ethyl acetate which is dried and evaporated. Chromatography of the residue on silica gel affords the title compound.

### Example 7I

### 4-[(2-Aminopyridyl)methylene]-2-phenylbenzoylmethionine

The resultant compound from Example 7H is hydrolyzed according to the procedure of Example 4G to give the title product.

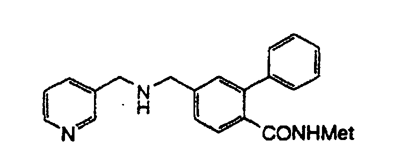

### Example 8

### 4-[(3-aminomethylpyridyl)methylene]-2-phenylbenzoylmethionine

Using the procedures of Examples 7F-G and replacing 2-aminopyridine with 3-aminomethylpyridine affords the title product.

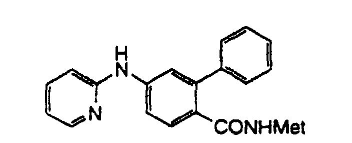

### Example 9

### 4-(2-Aminopyridyl)-2-phenylbenzoylmethionine

### Example 9A

### 4-(2-Aminopyridyl)-2-phenylbenzoylmethionine methyl ester

4-Amino-2-phenylbenzoyl methionine (1.0 equivalent) methyl ester and 2-bromopyridine hydrobromide (1.0 equivalent) in pyridine are heated until the reaction is judged complete by TLC analysis. The solvent is evaporated and the residue is taken up in ethyl acetate which is washed with water and brine, dried, and evaporated. Chromatography on silica gel affords the title product.

### Example 9B

### 4-(2-Aminopyridyl)-2-phenylbenzoylmethionine

The resultant compound from Example 9A is hydrolyzed according to the procedure of Example 4G to give the title product.

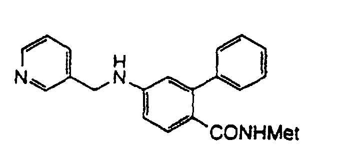

### Example 10

### 4-(3-Aminomethylpyridyl)-2-phenylbenzoylmethionine

### Example 10A

### 4-(3-Aminomethylpyridyl)-2-phenylbenzoylmethionine methyl ester

A mixture of 3-pyridinecarboxaldehyde (1.0 equivalent), 4-amino-2-phenylbenzoyl methionine methyl ester (1.0 equivalent) and NaCNBH₃ (1.0 equivalent) in methanol/acetic acid is stirred until the reaction is judged complete by TLC analysis. The mixture is poured into aqueous NaHCO₃ and extracted into ethyl acetate which is dried and evaporated. Chromatography of the residue on silica gel affords the title compound.

### Example 10B

### 4-(3-Aminomethylpyridyl)-2-phenylbenzoylmethionine

The resultant compound from Example 10A is hydrolyzed according to the procedure of Example 4G to give the title product.

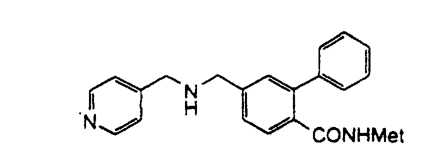

### Example 11

### Example 11A

### 4-(Azidomethyl)-2-phenylbenzoyl methionine methyl ester

To triphenylphosphine (1.0 equivalent) in tetrahydrofuran (THF) at -78° C is added diethyl azodicarboxylate (1.0 equivalent) in THF. To this mixture is added a solution of hydrazoic acid in benzene (2.0 equivalents) and then the resultant compound from Example 7D (1.0 equivalent). After one hour the mixture was warmed to room temperature, stirred until the reaction is judged complete by TLC analysis, evaporated and chromatographed on silica gel to afford the title product.

### Example 11B

### 4-(Aminomethyl)-2-phenylbenzoyl methionine methyl ester

To the resultant compound from Example 11A in methanol is added triethylamine (3.0 equivalent) and propane 1,3-dithiol (3.0 equivalents). After the reaction is judged complete by TLC analysis, the mixture is filtered and evaporated. Chromatography of the residue on silica gel provides the title product.

### Example 11C

### 4-[(4-aminomethylpyridyl)methylene]-2-phenylbenzoylmethionine

Using the procedures of Example 10 with the resultant amine from Example 11B and 3-pyridinecarboxaldehyde affords the title product.

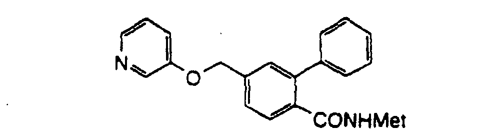

### Example 12

### 4-(3-Pyridyloxymethylene)-2-phenylbenzoylmethionine

### Example 12A

### 4-(p-Toluenesulfonyloxy)-2-phenylbenzoylmethionine methyl ester

The resultant compound from Example 7D (1.0 equivalent) and *p*-toluenesulfonyl chloride (1.0 equivalent) in pyridine are stirred until the reaction is judged complete by TLC analysis. The solvent is evaporated and the residue is taken up in ethyl acetate which is washed with water and brine, dried, and evaporated. Chromatography on silica gel affords the title product.

### Examgle 12B

### 4-(3-Pyridyloxymethylene)-2-phenylbenzoylmethionine methyl ester

3-Hydroxypyridine (1.0 equivalent) is treated with sodium hydride (1.0 equivalent) in DMSO, then the resultant compound from Example 12A (1.0 equivalent) is added. When judged complete by TLC analysis, the reaction is diluted with water and ethyl acetate, the organic layer is dried and concentrated, and the crude title compound is purified by chromatography on silica gel.

### Example 12C

### 4-(3-Pyridyloxymethylene)-2-phenylbenzoylmethionine

The resultant compound from Example 12B is hydrolyzed according to the procedure of Example 4G to give the title product.

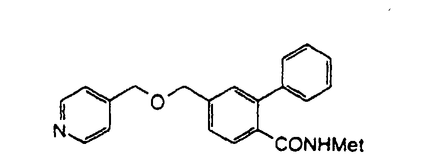

### Example 13

### 4-(3-Pyridylmethoxymethylene)-2-phenylbenzoylmethionine

### Example 13A

### 4-(3-Pyridylmethoxymethylene)-2-phenylbenzoylmethionine methyl ester

Using the procedure of Example 12B but replacing 3-hydroxypyridine with 3-hydroxymethylpyridine affords the title compound.

### Example 13B

### 4-(3-Pyridylmethoxymethylene)-2-phenylbenzoylmethionine methyl ester, alternate procedure

The resultant compound from Example 7D (1.0 equivalent) is treated with sodium hydride (2.0 equivalents) in DMSO, then 3-chloromethylpyridine hydrochloride (1.0 equivalent) is added. When judged complete by TLC analysis, the reaction is diluted with water and ethyl acetate, the organic layer is dried and concentrated, and the crude title compound is purified by chromatography on silica gel.

### Example 13C

### 4-(3-Pyridylmethoxymethylene)-2-phenylbenzoylmethionine

The resultant compound from Example 13A is hydrolyzed according to the procedure of Example 4G to give the title product.

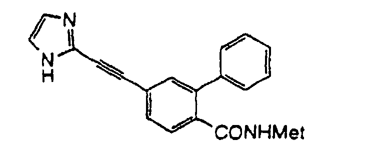

### Example 14

### {4-[2-(Imidazol-2-yl)ethynyl]-2-phenylbenzoyl}-methionine

### Example 14A

### (4-Ethynyl-2-phenylbenzoyl)-methionine methyl ester

A mixture of (2-phenyl-4-bromobenzoyl)-methionine methyl ester (100 mmol), diethylamine (300 mmol), trimethylsilylacetylene (110 mmol), bis(triphenylphosphine) palladium diacetate (5 mmol) and copper(I) iodide (3 mmol) in toluene is heated at 60 °C until TLC analysis indicates the starting methyl ester has disappeared. The reaction mixture is concentrated in vacuo, redissolved in ether, filtered through silica gel, and concentrated. The residue is then dissolved in THF, and is treated with tetrabutylammonium fluoride (120 mmol). After TLC analysis indicates that no starting material is left, the reaction mixture is diluted with ether, washed with water and brine, dried over anhydrous magnesium sulfate, filtered, and concentrated in vacuo. The residue is then purified with column chromatography on silica gel to give the title product.

### Example 14B

### {4-[2-(Imidazol-2-yl)ethynyl]-2-phenylbenzoyl}-methionine methyl ester

The resultant product from Example 14A (5 mmol) is mixed with 4-bromoimidazole (5 mmol), diethylamine (1 mL), bis(triphenylphosphine) palladium diacetate (0.1 mmol) and copper(I) iodide (0.1 mmol) in toluene. The mixture is stirred at 25 °C until TLC analysis indicates the reaction is complete. The reaction mixture is concentrated in vacuo, and the residue is purified with column chromatography on silica gel to give the title product.

### Example 14C

### {4-[2-(Imidazol-2-yl)ethynyl]-2-phenylbenzoyl}-methionine

The resultant compound from Example 14B is hydrolyzed according to the procedure of Example 4G to give the title product.

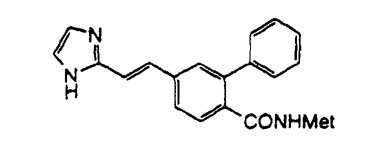

### Example 15

### {4-[2-(Imidazol-4-yl)ethenyl]-2-phenylbenzoyl}-methionine

The resultant acetylene (3 mmol) from Example 14 is mixed with Lindlar catalyst (50 mg), 5 drops of quinoline in ethyl acetate. The reaction mixture is attached to a hydrogenation apparatus, and then is detached from the apparatus after about 95% of the theoretical hydrogen has been absorbed. The reaction mixture is filtered and concentrated in vacuo. The crude product is purified with a column chromatography on silica gel to give the title compound.

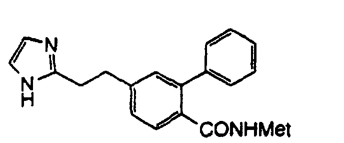

### Example 16

### {4-[2-(Imidazol-4-yl)ethyl]-2-phenylbenzoyl}-methionine

The resultant olefin (1 mmol) from Example 15 is mixed with 5% palladium on carbon (100 mg) in ethyl acetate. The reaction mixture is attached to a hydrogenation apparatus, and then is detached from the apparatus after about 95% of the theoretical hydrogen has been absorbed. The reaction mixture is filtered and concentrated in vacuo. The crude product is purified with a column chromatography on silica gel to give the title compound.

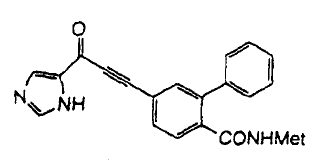

### Example 17

### {4-[2-(Imidazol-4-ylcarbonyl)ethynyl]-2-phenylbenzoyl}-methionine

### Example 17A

### {4-[2-(Imidazol-4-ylcarbonyl)ethynyl]-2-phenylbenzoyl}methionine methyl ester

A stainless autoclave containing the resultant product from Example 14A (5 mmol), 4-bromoimidazole (5 mmol), 1,1'-bis(diphenylphosphine)-ferrocenepalladium dichloride (0.1 mmol), and triethylamine (10 mL) is flushed with nitrogen, and pressurized to 20 atm with carbon monoxide. The reaction mixture is stirred at 120 °C until judged complete by TLC analysis. After cooling, the triethylamine is evaporated in vacuo, and the residue is purified by column chromatography on silica gel to give the title compound.

### Example 17B

### {4-[2-(Imidazol-4-ylcarbonyl)ethynyl]-2-phenylbenzoyl}-methionine

The resultant compound from Example 17A is hydrolyzed according to the procedure of Example 4G to give the title product.

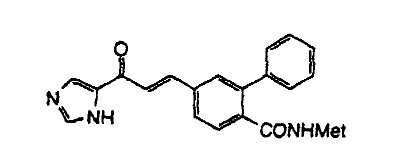

### Example 18

### {4-[2-(Imidazol-4-ylcarbonyl)ethenyl]-2-phenylbenzoyl}-methionine

Using the procedure of Example 15 with the resultant compound from Example 17 affords the title product.

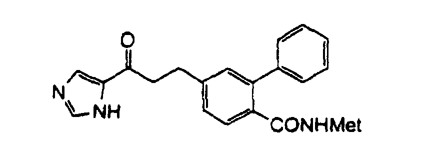

### Example 19

### {4-[2-(Imidazol-4-ylcarbonyl)ethyl]-2-phenylbenzoyl}methionine

Using the procedure of Example 16 with the resultant compound from Example 18 affords the title product.

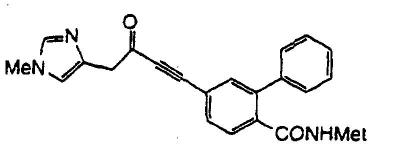

### Example 20

### {4-[4-(1-Methylimidazol-4-yl)-3-keto-1-butynyl]-2-phenylbenzoyl}methionine

### Example 20A

### {4-[4-(1-Methylimidazol-4-yl)-3-keto-1-butynyl]-2-phenylbenzoyl}methionine methyl ester

To a solution of 1-methyl-4-imidazoleacetic acid (5 mmol) in methylene chloride at 0 °C is added oxalyl chloride (6 mmol) and DMF (0.05 mmol). After 30 minute, the solvent is evaporated in vacuo. The residue is redissolved in dichloromethane, followed by the addition of the resultant acetylene from Example 14A (5 mmol), triethylamine (10 mmol), and copper(I) iodide (1 mmol). The reaction is stirred at 25 °C until TLC analysis indicates no starting material is left in the reaction mixture. The reaction is diluted with ether, washed with water and brine, dried over anhydrous magnesium sulfate, filtered, and concentrated in vacuo. The residue is then purified by column chromatography on silica gel to give the title compound.

### Example 20B

### {4-[4-(1-Methylimidazol-4-yl)-3-keto-1-butynyl]-2-phenylbenzoyl}methionine

The resultant compound from Example 20A is hydrolyzed according to the procedure of Example 4G to give the title product.

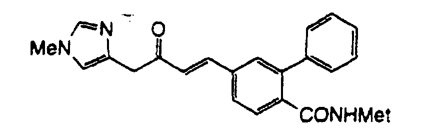

### Example 21

### {4-[4-(1-Methylimidazol-4-yl)-3-keto-1-butenyl]-2-phenylbenzoyl}-methionine

Using the procedure of Example 15 with the resultant compound from Example 20 affords the title product.

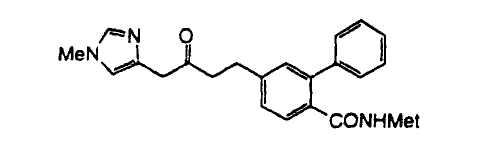

### Example 22

### {4-[4-(1-Methylimidazol-4-yl)-3-keto-1-butyl]-2-phenylbenzoyl}methionine

Using the procedure of Example 16 with the resultant compound from Example 20 affords the title product.

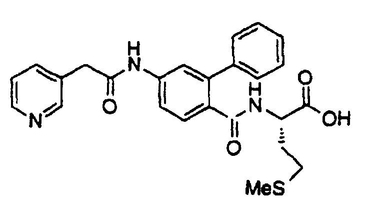

### Example 23

### [4-(3-pyridylmethylcarbonylamino-2-phenylbenzoyl]methionine

### Example 23A

### [4-(3-pyridylmethylcarbonylamino-2-phenylbenzoyl]methionine methyl ester

To a solution of 4-amino-2-phenylbenzoyl methionine methyl ester (1.0 equivalent) in dimethylformamide (DMF) is added 3-hydroxy-1,2,3-benzotriazin-4(3H)-one (1.5 equivalents) followed by 3-pyridylacetic acid (1.0 equivalent) and 1-(3-dimehtylaminopropyl)-3-ethylcarbodiimide hydrochloride (1.5 equivalents). When judged complete by TLC analysis, the reaction is taken up in ethyl acetate which is washed with 1N HCl and saturated brine, and then is dried and evaporated. The crude reaction mixture is purified by column chromatography to afford the title product.

### Example 23B

### [4-(3-pyridylmethylcarbonylamino)-2-phenylbenzoyl]methionine

The resultant compound from Example 23A is hydrolyzed according to the procedure of Example 4G to give the title product.

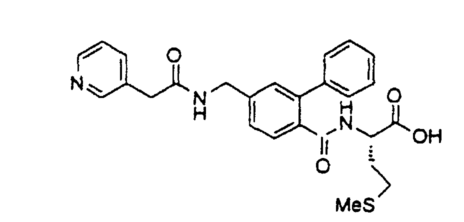

### Example 24

### [4-(3-pyridylmethylcarbonylaminomethyl)-2-phenylbenzoyl]methionine

### Example 24A

### 4-(Azidomethyl)-2-phenylbenzoyl methionine methyl ester

To triphenylphosphine (1.0 equivalent) in tetrahydrofuran (THF) at -78° C is added diethyl azodicarboxylate (1.0 equivalent) in THF. To this mixture is added a solution of hydrazoic acid in benzene (2.0 equivalents) and then the resultant compound from Example 7D (1.0 equivalent). After one hour the mixture was warmed to room temperature, stirred until the reaction is judged complete by TLC analysis, evaporated and chromatographed on silica gel to afford the title product.

### Example 24B

### 4-(Aminomethyl)-2-phenylbenzoyl methionine methyl ester

To the resultant compound from Example 24A in methanol is added triethylamine (3.0 equivalent) and propane 1,3-dithiol (3.0 equivalents). After the reaction is judged complete by TLC analysis, the mixture is filtered and evaporated. Chromatography of the residue on silica get provides the title product.

### Example 24C

### [4-(3-pyridylmethylcarbonylaminomethyl)-2-phenylbenzoyl]methionine

To a solution of the resultant amine from Example 24B (1.0 equivalent) in dimethylformamide (DMF) is added 3-hydroxy-1,2,3-benzotriazin-4(3H)-one (1.5 equivalents) followed by 3-pyridylacetic acid (1.0 equivalent) and 1-(3-dimethylaminopropyl)-3-ethylcarbodiimide hydrochloride (1.5 equivalents). When judged complete by TLC analysis, the reaction is taken up in ethyl acetate which is washed with 1N HCI and saturated brine, and then is dried and evaporated. The crude reaction mixture is purified by column chromatography to afford the title product.

### Example 24D

### (S)Pyroglutamyl-(4-aminomethyl-2-phenyl)benzoyl methionine

The resultant compound from Example 24C is hydrolyzed according to the procedure of Example 4G to give the title product.

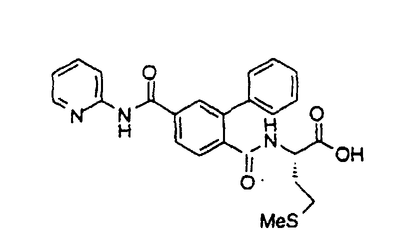

### Example 25

### [4-(2-pyridylaminocarbonyl)-2-phenylbenzoyl]methionine

### Example 25A

### 4-Carboxy-2-phenylbenzoyl methionine methyl ester

A solution of 4-bromo-2-phenylbenzoyl methionine methyl ester (1.0 equivalent), Pd(OAc)₂ (0.05 equivalent) and DPPE (1.0 equivalent) is heated in DMF to 65° C under 4 atm. of carbon monoxide until TLC analysis indicates that the reaction is complete. The reaction mixture is poured into water and extracted with ethyl acetate which is dried and evaporated. The product is purified by chromatography on silica gel.

### Example 25B

### [4-(2-pyridylaminocarbonyl)-2-phenylbenzoyl]methionine methyl ester

To a solution of the resultant acid from Example 25A (1.0 equivalent) in DMF is added 3-hydroxy-1,2,3-benzotriazin-4(3H)-one (1.5 equivalents) followed by 2-aminopyridine (1.0 equivalent) and 1-(3-dimethylaminopropyl)-3-ethylcarbodiimide hydrochloride (1.5 equivalents). When judged complete by TLC analysis, the reaction is taken up in ethyl acetate which is washed by 1 N HCl and saturated brine, and then is dried and evaporated. The crude reaction mixture is purified by column chromatography to afford the title product.

### Example 25C

### 4-[(Pyridin-2-ylamino)carbonyl]-2-phenylbenzoyl methionine

The resultant compound from Example 25B is hydrolyzed according to the procedure of Example 4G to give the title product.

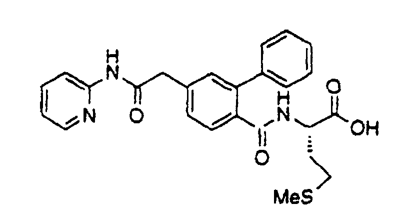

### Example 26

### [4-(2-pyridylaminocarbonylmethyl)-2-phenylbenzoyl methionine

### Example 26A

### 4-Diazocarbonyl-2-phenylbenzoyl methionine methyl ester

The resultant acid from Example 25A (1 equivalent) in dichloromethane is treated with oxalyl chloride (1 equivalent) and DMF (0.05 equivalent). When gas evolution has ceased, the acid chloride solution is added to an ether solution of diazomethane: The reaction is stirred until judged complete by TLC analysis, and then is concentrated to give the crude title compound which is purified by chromatography on silica gel.

### Example 26B

### 4-carboxymethyl-2-phenylbenzoyl methionine methyl ester

The resultant compound from Example 26A (1 equivalent) in dioxane is added to a slurry of sodium thiosulfate (1.1 equivalents) and silver (I) oxide (0.5 equivalent) in water. The reaction is stirred until judged complete by TLC analysis, filtered, acidified, and extracted into ethyl acetate which is dried and evaporated. Chromatography of the residue on silica gel affords the title product.

### Example 26C

### 4-[(Pyridin-2-ylamino)carbonylmethyl]-2-phenylbenzoyl methionine methyl ester

To a solution of the resultant acid from Example 26B (1.0 equivalent) in dimethylformamide (DMF) is added 3-hydroxy-1,2,3-benzotriazin-4(3H)-one (1.5 equivalents) followed by 2-aminopyridine (1.0 equivalent) and 1-(3-dimehtylaminopropyl)-3-ethylcarbodiimide hydrochloride (1.5 equivalents). When judged complete by TLC analysis, the reaction is taken up in ethyl acetate which is washed with 1 N HCl and saturated brine, and then is dried and evaporated. The crude reaction mixture is purified by column chromatography to afford the title product.

### Example 26D

### 4-[(Pyridin-2-ylamino)carbonylmethyl]-2-phenylbenzoyl methionine

The resultant compound from Example 26C is hydrolyzed according to the procedure of Example 1C to give the title product.

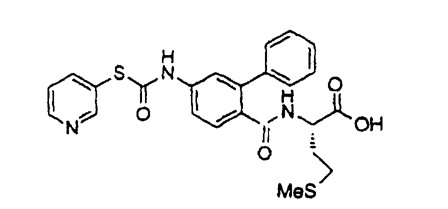

### Example 27

### [4-(3-pyridylthiocarbonylamino)-2-phenylbenzoyl methionine

### Example 27A

### 4-(3-pyridylthiocarbonylamino)-2-phenylbenzoic acid methyl ester

To a solution of 4-amino-2-phenylbenzoic acid methyl ester hydrochloride (1.0 equivalent) in toluene is added triphosgene (0.33 equivalent) and the mixture is heated at reflux until judged complete by TLC analysis. The intermediate is reacted without further purification with 3-mercaptopyridine (1.0 equivalent) and triethylamine (2.0 equivalents). When judged complete by TLC analysis, the reaction is taken up in ethyl acetate and washed with 1N HCl and brine, evaporated, and purified by chromatography on silica gel.

### Example 27B

### 4-(3-pyridylthiocarbonylamino)-2-phenylbenzoic acid

To a solution of the resultant compound from Example 27A (1.0 equivalent) in a 3:1 mixture of tetrahydrofuran (THF) and water is added an excess (1.5 equivalents) of LiOH. When hydrolysis is judged complete by TLC analysis, the solvent is evaporated and the remaining aqueous layer is acidified to pH = 3 and extracted into ethyl acetate which is dried and evaporated prior to purification by chromatography on silica gel.

### Example 27C

### [4-(3-pyridylthiocarbonylamino)-2-phenylbenzoyl methionine methyl ester

To a solution of the resultant compound from Example 27B (1.0 equivalent) in dimethylformamide (DMF) is added 3-hydroxy-1,2,3-benzotriazin-4(3H)-one (1.5 equivalents) followed by methionine methyl ester (1.0 equivalent) and 1-(3-dimethylaminopropyl)-3-ethylcarbodiimide hydrochloride (1.5 equivalents). When judged complete by TLC analysis, the reaction is taken up in ethyl acetate which is washed with 1N HCl and saturated brine, and then is dried and evaoporated. The crude reation mixture is purified by column chromatography to afford the title product.

### Example 27D

### 4-((S)-2-Pyrrolidone-5-aminomethylcarbonyl)amino-2-phenylbenzoyl methionine methyl ester, alternate preparation

To a solution of 4-amino-2-phenylbenzoyl methionine methyl ester (1.0 equivalent) in methylene chloride is added a solution of phosgene in toluene (1.0 equivalent) and triethylamine (2.0 equivalents). The intermediate is reacted without further purification with (*S*)-5-aminomethyl-2-pyrrolidone (1.0 equivalent) and triethylamine (1.0 equivalent). When judged complete by TLC analysis, the reaction is taken up in ethyl acetate and washed with 1N HCl and brine, evaporated, and purified by chromatography on silica gel.

### Example 27E

### [4-(3-pyridylthiocarbonylamino)-2-phenylbenzoyl methionine

To a solution of the resultant compound from Example 27C in a 3:1 mixture of THF and water is added an excess of LiOH (1.5 equivalents). When hydrolysis is judged complete by TLC analysis, the solvent is evaporated and the remaining aqueous layer is acidified to pH = 3 and extracted into ethyl acetate which is dried and evaporated prior to purification by chromatography on silica gel.

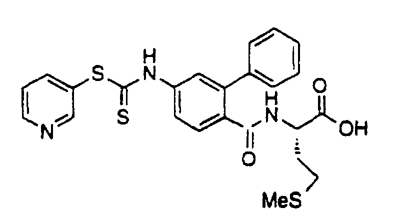

### Example 28

### [4-(3-pyridylthiosulfinylamino)-2-phenylbenzoyl methionine

The title compound is prepared as described in Example 27 with the exception that triphosgene (0.33 equivalent) is replaced by thiophosgene (1.0 equivalent).

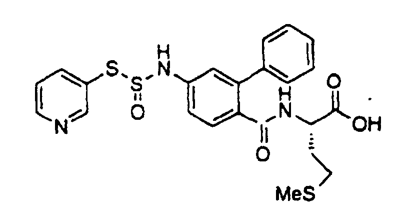

### Example 29

### [4-3-pyridylmercaptosulfinyl)amino-2-phenylbenzoyl]methionine

### Example 29A

### [4-3-pyridylmercaptosulfinyl)amino-2-phenylbenzoyl]methionine methyl ester

To a solution of 4-amino-2-phenylbenzoyl methionine methyl ester (1.0 equivalent) in methylene chloride is added thionyl chloride (1.0 equivalent) and triethylamine (2.0 equivalents). After the amine has fully reacted,3-mercaptopyridine (1.0 equivalent) is added. When the reaction is judged complete by TLC analysis, the product is isolated as described in Example 1A and purified by chromatography on silica gel.

### Example 29B

### 4-((S)-2-Pyrrolidone-5-aminomethylsulfinyl)amino-2-phenylbenzoyl methionine

To a solution of the resultant compound from Example 29A in a 3:1 mixture of THF and water is added an excess of LiOH (1.5 equivalents). When hydrolysis is judged complete by TLC analysis, the solvent is evaporated and the remaining aqueous layer is acidified to pH = 3 and extracted into ethyl acetate which is dried and evaporated prior to purification by chromatography on silica gel.

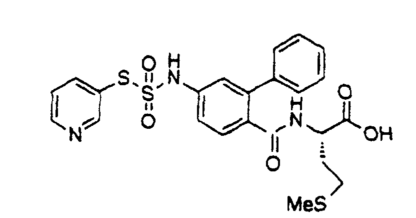

### Example 30

### [4-(3-pyridylmercaptosulfonylamino)-2-phenylbenzoyl]methionine

### Example 30A

### [4-(3-pyridylmercaptosulfonylamino)-2-phenylbenzoyl]methionine methyl ester

To a solution of 4-amino-2-phenylbenzoyl methionine methyl ester (1.0 equivalent) in methylene chloride is added sulfuryl chloride (1.0 equivalent) and triethylamine (2.0 equivalents). After the amine has fully reacted, 3-mercaptopyridine (1.0 equivalent) is added. When the reaction is judged complete by TLC analysis, the product is isolated as described in Example 27A and purified by chromatography on silica gel.

### Example 30B

### [4-(3-pyridylmercaptosulfonylamino)-2-phenylbenzoyl]methionine methyl ester, alternate procedure

A solution of 1 equivalent of 4-amino-2-phenylbenzoyl methionine methyl ester (1.0 equivalent) and sulfuryl chloride (1.0 equivalent) in acetonitrile with a catalytic amount of antimony(V) chloride is heated to reflux until judged complete by TLC analysis. The solution is then cooled, filtered, and all volatiles are removed under reduced pressure. The residue is taken up in dichloromethane and treated with triethylamine (1 equivalent and (*S*)-5-aminomethyl-2-pyrrolidone (1.0 equivalent). When the reaction is judged complete by TLC analysis, the product is isolated as described in Example 27A and purified by chromatography on silica gel.

### Example 30C

### 4-((S)-2-Pyrrolidone-5-aminomethylsulfonyl)amino-2-phenylbenzoyl methionine methyl ester

The resultant compound from Example 30A is hydrolyzed according to the procedure of Example 27B to give the title product.

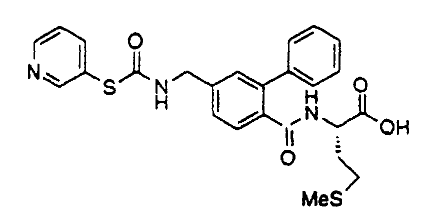

### Example 31

### 4-(Pyridin-3-ylmercaptocarbonyl)aminomethyl-2-phenylbenzoyl methionine

The title compound is prepared as described in Example 24 with the exception that 3-pyridylacetic acid (1.0 equivalent) is replaced by 3-mercaptopyridine (1.0 equivalent).

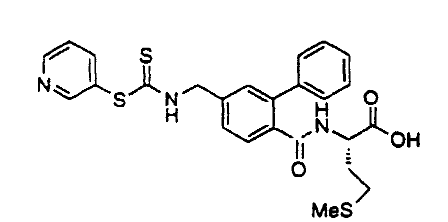

### Example 32

### 4-(Pyridin-3-ylmercaptocarbonyl)aminomethyl-2-phenylbenzoyl methionine

The title compound is prepared as described in Example 24 with the exception that 3-pyridylacetic acid (1.0 equivalent) is replaced by 3-mercaptopyridine (1.0 equivalent), and triphosgene (0.33 equivalent) is replaced by thiophosgene (1.0 equivalent).

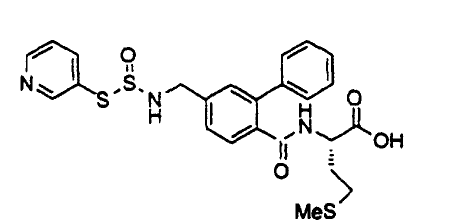

### Example 33

### 4-(3-pyridylmercaptosulfinyl)aminomethyl)-2-phenylbenzoyl]methionine

The title compound is prepared as described in Example 29 using the resultant amine from Example 24B.

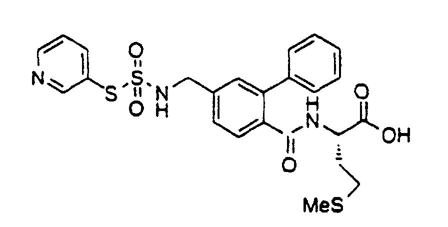

### Example 34

### 4-(Pyridin-3-ylmercaptosulfonyl)aminomethyl-2-phenylbenzoyl methionine

The title compound is prepared as described in Example 30 using the resultant amine from Example 24B.

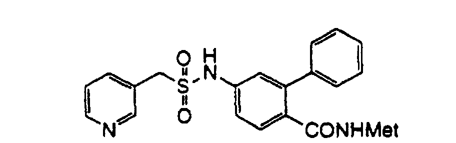

### Example 35

### [4-(3-pyridylmethylsulfonylamino)-2-phenylbenzoyl}methionine

### Example 35A

### [4-(3-pyridylmethylsulfonylamino)-2-phenylbenzoyl}methionine methyl ester

A mixture of 3-chlorosulfonylmethylpyridine hydrochloride (1.0 equivalent) and (4-amino-2-phenylbenzoyl)methionine methyl ester (1.0 equivalent) in dichloromethane is treated with triethylamine (2.2 equivalents). When judged complete by TLC analysis, the reaction is diluted with ethyl acetate, and then is washed with pH 4 water, saturated NaHCO₃, and brine. The mixture is dried and concentrated to give the crude title compound which is purified by chromatography on silica gel.

### Example 35B

### {4-[(3-sulfonylmethylpyridyl)amino]-2-phenylbenzoyl}methionine

The resultant compound from Example 35A is hydrolyzed according to the procedure of Example 27B to give the title product.

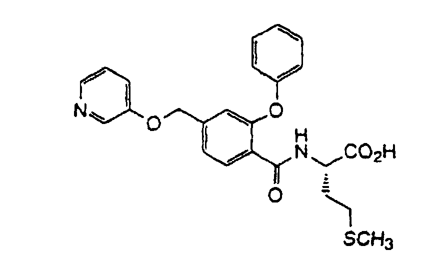

### Example 36

### [4-(3-pyridyloxymethylene)-2-phenoxybenzoyl]methionine

### Example 36A

### dimethyl phenoxyterephthalate

To a solution of phenol (10.8 g) in anisole (40 mL) and DMF (90 mL) was added potassium tert-butoxide (12.1 g). The mixture was heated for 40 minutes while continuously passing a nitrogen stream through the reaction flask (∼ 20 mL of solvent distilled over). The mixture was cooled to ambient temperature and dimethyl nitroterephthalate (23.9 g) was added. The resulting black mixture was stirred for 1 hour at ambient temperature and two hours at 100 - 105 °C. The reaction mixture was poured into ice containing 2 mL of concentrated HCI. The mixture was extracted with ether. The ether layer was washed with water (3x) and saturated aqueous NaHCO₃ (2x). The organic phase was dried over MgSO₄, filtered, and concentrated *in vacuo* to give 24.1 g of an oil. Chromatography on silica gel (10% ethyl acetate-hexanes) gave dimethy phenoxyterephthalate (15.9 g).

### Example 36B

### 4-carbomethoxy-3-phenoxybenzoic acid

To a mixture of dimethyl phenoxyterephthalate (10.4 g) in water (50 mL) was added 50% aqueous NaOH (2.32 g) and water (4 mL) and the reaction mixture was stirred overnight at ambient temperature. The reaction mixture was concentrated *in vacuo* and the residue was partitioned between water and ether. The aqueous phase was acidified (a solid formed) and extracted with dichloromethane. The organic phase was dried over Na₂SO₄, filtered, and concentrated *in vacuo* to give 4-carbomethoxy-3-phenoxybenzoic acid (6.6 g) as a 68:32 mixture of hydrolysis isomers.

### Example 36C

### 4-hydroxymethyl-2-phenoxybenzoic acid methyl ester

To a 0 °C solution in THF (9 mL) of 4-carbomethoxy-3-phenoxybenzoic acid (5.88 g), prepared as in Example 36B, was added borane-THF (1.0 M, 30 mL). The reaction mixture was stirred for 1 hour at 0 °C and 1.5 hours at ambient temperature. The reaction mixture was cooled in an ice bath and water (20 mL) was added slowly, followed by slow addition of 1:1 conc. HCl-water. The mixture was stirred for 10 minutes at ambient temperature and then toluene was added. The layers were separated and the aqueous phase was extracted with toluene. The combined organic layers were washed with saturated aqueous KHCO₃, dried over Na₂SO₄, filtered, and concentrated *in vacuo* to give 5.32 g of colorless oil. Chromatography on silica gel (30% ethyl acetate-hexanes) gave 4-hydroxymethyl-2-phenoxybenzoic acid methyl ester (4.77 g).

### Example 36D

### 4-bromomethyl-2-phenoxybenzoic acid methyl ester

To a sotution in DMF (10 mL) of 4-hydroxymethyl-2-phenoxybenzoic acid methyl ester (2.82 g), prepared as in Example 36C, was added LiBr (1.04 g) and PBr₃ (3.65 g) and the reaction mixture was stirred for 20 minutes at ambient temperature. The reaction mixture was poured into water and extracted with toluene. The organic phase was washed twice with water, dried over Na₂SO₄, filtered, and concentrated *in vacuo* to give 4-bromomethyl-2-phenoxybenzoic acid methyl ester (3.37 g).

### Example 36E

### 4-(3-pyridyloxymethyl)-2-phenoxybenzoic acid methyl ester

To a solution in toluene (25 mL) of 4-bromomethyl-2-phenoxybenzoic acid methyl ester (3.37 g), prepared as in Example 36D, was added 18-crown-6 (0.52 g) and the potassium 3-pyridyloxide (2.20 g). The reaction mixture was heated at 70 °C for 1 hour during which time a black, insoluble tar formed. The reaction mixture was cooled to ambient temperature and diluted with toluene. The toluene was decanted from the tar and the tar was dissolved in 1:1 THF-water (20 mL). The aqueous THF was added to the toluene and the mixture was washed twice with brine, dried over Na₂SO₄, filtered, and concentrated in vacuo to give a black oil. Chromatography on silica gel (1:1 ethyl acetate-hexanes) gave 4-(3-pyridyloxymethyl)-2-phenoxybenzoic acid methyl ester (2.47 g).

### Example 36F

### 4-(3-pyridyloxymethylene)-2-phenoxybenzoic acid

To a solution in methanol (15 mL) of 4-(3-pyridyloxymethylene)-2-phenoxybenzoic acid methyl ester (2.46 g), prepared as in Example 36E, was added a solution of 5% aqeuous KOH (2.00 g) in water (3 mL). The reaction mixture was heated at reflux for 1.5 hours. The reaction mixture was concentrated *in vacuo* and the residue was dissolved in water. The aqueous phase was acidified with acetic acid with cooling and stirring. The resulting solid was filtered, washed with water, and dissolved in THF. The THF solution was dried over Na₂SO₄, filtered, and concentrated *in vacuo* to give 4-(3-pyridyloxymethyl)-2-phenoxybenzoic acid (2.17 g).

### Example 36G

### [4-(3-pyridyloxymethyl)-2-phenoxybenzoyl]methionine methyl ester

To a solution in DMF (3 mL) of 4-(3-pyridyloxymethylene)-2-phenoxybenzoic acid (321 mg), prepared as in Exampte ^{36F}, was added 3-hydroxy-1,2,3-benzotriazin-4(3H)-one (345 mg, 1.5 mmol) followed by methionine methyl ester hydrochloride (300 mg, 1.5 mmol), ethyl dimethylaminopropyl carbodiimide hydrochloride (288 mg, 1.5 mmol), and triethylamine (280 mg). The reaction mixture was stirred for 15 hours at ambient temperature. The reaction mixture was diluted with water and extracted with toluene. The toluene solution was washed with, dried over Na₂SO₄, filtered, and concentrated in vacuo. Chromatography on silica gel (75% ethyl acetate-hexanes) gave [4-(3-pyridyloxymethyl)-2-phenoxybenzoyl]methionine methyl ester (425 mg, 98%).

### Example 36H

### [4-(3-pyridyloxymethyl)-2-phenoxybenzoyl]methionine

To a solution in 5:1 methanol-H₂O (3.5 mL) of [4-(3-pyridyloxymethyl)-2-phenoxybenzoyl]methionine methyl ester (440 mg), prepared as in Example 36G, was added a solution of 50% NaOH (354 mg) in water (0.8 mL) and the reaction mixture was heated at 60 °C for 15 minutes. The reaction mixture was concentrated in vacuo and the residue was taken up in H₂O (3 mL). The aqueous solution was acidified with concentrated HCl (415 mg) and 2 drops of ethyl acetate were added. The resulting solid was filtered and dried in a vacuum oven at 60 °C for 3 hours to give [4-(3-pyridyloxymethyl)-2-phenoxybenzoyl]methionine (373 mg). mp 195 °C; ¹H NMR (300 MHz, DMSO-d6) δ 1.90 (m.. 4H), 2.37 (m, 2H), 4.44 (m, 1H), 5.20 (s, 2H), 7.01 (s, 2H), 7.04 (s, 1H), 7.16 (t, 1H, *J*= 7.4 Hz), 3.66 (m, 5H), 7.70 (d, 1H, *J*= 9.0 Hz), 8.17 (dd, 1H, *J*= 4.4, 1.5 Hz), 8.30 (d, 1H, *J*= 3 Hz). Anal calcd for C₂₄H₂₄N₂O₅S: C, 63.70; H. 5.35; N, 6.59. Found: C, 63.46; H, 5.11; N, 6.08.
The next example is Example 39.

### Example 39

### [4-(2-Pyridylaminomethyl)-2-phenylbenzoyl]methionine

### Example 39A

### dimethyl 2-phenylterephthalate

A mixture of dimethyl 2-iodoterephthalate (22.8 g, 71.4 mmol) and tetrakis(triphenylphosphine) palladium(0) (4.14g, 3.38 mmol) in toluene (120 mL) was stirred for 10 minutes. Aqueous sodium carbonate (2 M, 160 mL) and a solution in methanol (40 mL) of phenylboronic acid (10.4 g, 85.3 mmol) were then added and the reaction mixture was stirred at reflux for 15 hours. The reaction mixture was cooled to ambient temperature and extracted with ether. The organic phase was washed with water (2x) and brine, and concentrated in vacuo to give dimethyl 2-phenylterephthalate as a dark brown oil (18.4 g) which was used without further purification.

### Example 39B

### 2-phenylmonomethylterephthalate

To a solution in 1:1 THF-methanol of the 2-phenylterephthalate prepared in Example 39A (18.4 g) was added a solution of KOH (4.56 g, 71.5 mmol) in water (30 mL). The reaction mixture was stirred overnight at ambient temperature and then was diluted in water and the methanol was evaporated in vacuo. The residue was filtered through a pad of Celite with a water rinse. The filtrate was extracted twice with ethyl acetate and the aqueous phase was cooled in an ice-water bath and concentrated HCl (10 mL) was added. The resulting suspension was stirred for 30 minutes and then was filtered. The solid was recrystallised from 25% agueous ethanol to give 2-phenylmonomethyl-terephthalate (10.4 g). The mother liquor was concentrated and the residue was purified by chromatography on silica gel (97:2:1, then 96:3:1 chloroform-methanol-acetic acid) to give an additional 1.75 g of the desired compound.

### Example 39C

### 4-hydroxymethyl-2-phenylbenzoic acid and methyl ester

To a 0°C solution in THF (40 mL) of 2-phenylmonomethylterephthalate (10.5 g, 41 mmol) was added borane-THE (1.0 M, 83 mL, 82 mmol) such that the reaction temperature remained below 6°C. The reaction mixture was stirred for 0.5 hours, then the cold bath was removed and stirring was continued for 2 hours. The reaction mixture was again cooled to 0°C and aqueous HCl (3 M, 100 mL) was added slowly. The cold bath was removed and the reaction mixture was stirred for 1 hour. The THE was evaporated and the residue was extracted with ethyl acetate (3x). The combined organic extracts were washed with 1M aqueous NaOH (2x), water (2x) and brine, dried, filtered, and concentrated in vacuo to give 4-hydroxymethyl-2-phenylbenzoic acid methyl ester (9.75).

### Example 39D

### 4-hydroxymethyl-2-phenylbenzoic acid

The desired compound was prepared by saponification of 4-hydroxymethyl-2-phenylbenzoic acid methyl ester, prepared as in Example 39C using the procedure of Example 39B.

### Example 39E

### (4-hydroxymethyl-2-phenylbenzoyl)methionine methyl ester

To a solution in 1:3 DMF-dichloromethane (100 mL) of 4-hydroxymethyl-2-phenylbenzoic acid (5.2 g, 23 mmol), prepared as in Example 39D, at 5-10°C was added 1-(3-dimethylaminopropyl)-3-ethylcarbodiimide hydrochloride (4.8 g, 25 mmol), 3-hydroxy-1,2,3-benzotriazin-4(3H)-one (4.1 g, 25 mmol), methionine methyl ester hydrochloride (5.0 g, 25 mmol) and 4-methylmorpholine (2.8 mL, 25 mmol). The reaction was warmed slowly to ambient temperature and stirred overnight. The reaction mixture was diluted with ethyl acetate and washed with aqueous 1M H₃PO₄. The organic phase was washed with brine, dried over Na₂SO₄, filtered, and concentrated in vacuo. Chromatography on silica gel (65% ethyl acetate-hexanes) gave [4-hydroxymethyl-2-phenylbenzoyl]methionine methyl ester (5.15 g, 60%).

### Example 39F

### (4-carboxylaldehyde-2-phenylbenzoyl)methionine methyl ester

A solution of DMSO (1.95 g, 27 mmol) in dichloromethane (100 mL) was cooled to -78°C and oxalyl chloride (1.8 mL, 20 mmol) was added dropwise. After 15 minutes, a solution in dichloromethane (35 mL) of [4-hdroxymethyl-2-phenylbenzoyl]methionine methyl ester (5.1 g, 13.7 mmol), prepared as in Example 39E, was added dropwise and the reaction mixture was stirred for one hour at -78°C. Triethylamine (7.6 mL, 55 mmol) was then added and the reaction mixture was warmed to ambient temperature. The reaction mixture was diluted with ethyl ether, washed twice with brine, dried over Na₂SO₄, filtered, and concentrated in vacuo to give an orange gum. The gum was dissolved in hot ethyl acetate (50 mL) and hexanes (10-20 mL) were added. The cloudy suspension was cooled in the refrigerator and the supernatant was concentrated in vacuo and the residue was left under high vacuum for three days to give (4-carboxyaldehyde-2-phenylbenzoyl)methionine methyl ester (4.9 g) as a light-orange solid.

### Example 39G

### [4-(2-Pyridylaminomethyl)-2-phenylbenzoyl]methionine methyl ester

A suspension in toluene (17 mL) of [4-carboxyaldehyde-2-phenylbenzoyl]methylmethionine methyl ester (800 mg, 2.15 mmol), prepared as in Example 39F, 2-aminopyridine (253 mg, 2.69 mmol) and *p*-toluenesulfonic acid hydrate (22 mg, 0.11 mmol) were heated under reflux overnight using a Dean-Stark trap containing 10 mL of toluene. The reaction mixture was concentrated in vacuo. The residue was taken up in isopropanol (20 mL) and *p*-toluenesulfonic acid hydrate was added to get to pH 4. Sodium cyanoborohydride (625 mg, 10 mmol) was added along with absolute ethanol (20 mL) to obtain a clear solution. The pH was then adjusted to pH 4 using *p*-toluenesulfonic acid hydrate and the reaction mixture was stirred for 2 hours while the pH was periodically readjusted to about 4. The reaction mixture was then partitioned between ethyl acetate and aqueous 2N sodium hydroxide. The organic phase was washed with aqueous 2N sodium hydroxide, twice with brine, dried over Na₂SO₄, filtered, and concentrated in vacuo. Chromatography on silica gel (55:45 chloroform-ethyl acetate) gave [4-(2-Pyridylaminomethyl)-2-phenyl-benzoyl]methionine methyl ester (125 mg).

### Example 39H

### [4-(2-Pyridylaminomethyl)-2-phenyl]benzoylmethionine

To a 0°C solution in methanol (3.0 mL) of [4-(2-Pyridylaminomethyl)-2-phenylbenzoyl]methionine methyl ester (0.29 mmol), prepared as in Example 39G, was added a solution of lithium hydroxide hydrate (20 mg, 0.49 mmol) in water (1 mL). Methanol (1 mL) and water (0.5 mL) were then added to obtain a clear solution. The reaction mixture was stirred for 1.5 hours and then the cold bath was removed and stirring was continued for 2.5 hours. The reaction mixture was partitioned between water which was taken to pH4 with HCl and ethyl acetate. The aqueous phase was extracted three times with chloroform. The combined organic layers were dried over Na₂SO₄, filtered, and concentrated in vacuo. The residue was dissolved in acetonitrile-methanol. The solution was diluted with water, frozen, and lyophilised to give [4-2-pyridylaminomethyl)-2-2phenylbenzoyl]-methionine as a white powder. ¹H NMR (300 MHz, DMSO-d₆) d 8.54 (d, 1H), 7.97 (d, 1H), 7.86 (m, 1H), 7.50-7.30 (envelope, 7H), 7.04 (d, 1H), 6.83 (m, 1H), 4.70 (d, 2H), 4.30 (m, 1H), 2.24 (m, 2H), 2.00 (s. 3H), 1.85 (m, 2H); MS (DC1-NH³) m/e 436 (M+H)⁺, 434 (M-H)⁻. Anal calcd for C₂₄H₂₅N₃O₃S 1.4 HCl: C, 49,24; H, 5.47; n, 8.64. Found: C, 59.36; H, 5.24; N, 8.42.

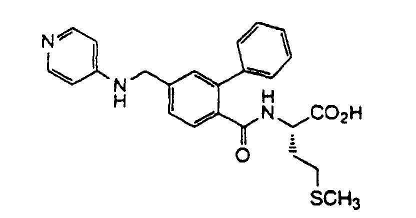

### Example 40

### [4-(4-Pyridylaminomethyl)-2-phenylbenzoyl]methionine

The desired compound was prepared according to the method of Examples 39G and H except substituting 4-aminopyridine for 2-aminopyridine. ¹H NMR (300 MHz, DMSO-d6) d 8.47 (d, 1H), 8.05 (d, 2H), 7.94 (t, 1H), 7.37 (m, 8H), 6.63 (d, 2H), 4.50 (d, 2H), 4.27 (m, 1H), 2.24 (m, 2H), 2.00 (s, 3H), 1.85 (m, 2H); MS (DCl-NH₃) m/e 436 (M+H)⁺, 434 (M-H)⁻. Anal calcd for C₂₄H₂₅N₃O₃S·HCl: C, 61.07; H, 5.55; N, 8.90. Found: C, 61.38; H, 5.66; N, 9.01.

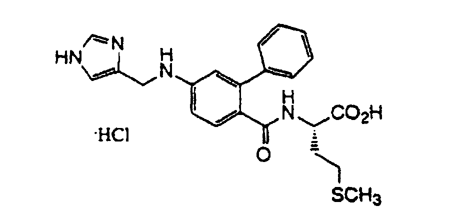

### Example 41

### [4-(1H-imidazol-4-ylmethyl)amino-2-phenylbenzoyl]methionine hydrochloride

### Example 41A

### 4-hydroxymethyl-1H-1-triphenylmethylimidazole

To a mixture in DMF (25 mL) of 4-hydroxymethylimidazole hydrochloride (10.0 g, 74 mmol) and triethylamine (25 mL, 180 mmol) was added a solution of triphenylmethyl chloride (22 g, 79 mmol) in DMF (75 mL) and the thick reaction mixture was spun overnight on the rotary evaporator. The solid was filtered off, washed with DMF and water, and dried overnight over Drierite® to give 4-hydroxymethyl-1H-1-triphenylmethylimidazole (23 g).

### Example 41B

### 1H-1-triphenylmethylimidazole-4-carboxaldehyde

A mechanically-stirred slurry in dioxane (400 mL) of 4-hydroxymethyl-1H-1-triphenylmethylimidazole (9.6 g, 28 mmol), prepared as in Example 41A, was heated to 77 °C to dissolve the solid. The reaction mixture was cooled to ambient temperature and MnO₂ (20.5 g, 236 mmol) was added all at once. The black slurry was warmed to 85 °C and stirred for 5.5 hours. The reaction mixture was filtered through Celite and the filtrate was concentrated in vacuo to give a crystalline solid. Recrystallization from dichloromethane-hexanes to give 1H-1-triphenylmethylimidazole-4-carboxyaldehyde (5.1 g).

### Example 41C

### [4-(1H-trimethylphenylimidazol-4-ylmethyl)amino-2-phenylbenzoyl]methionine methyl ester

1H-1-triphenylmethylimidazole-4-carboxyaldehyde was reductively aminated with 4-amino-2-phenylbenzoyl methionine methyl ester (compound 8) according to the previously described reductive amination procedure.

### Example 41D

### [4-(1H-imidazol-4-ylmethyl)amino-2-phenylbenzoyl]methionine methyl ester

To a solution in dichloromethane (5 mL) of [4-(1H-trimethylphenyl imidazol-4-ylmethyl)amino-2-phenylbenzoyl]methionine (365 mg, 0.54 mmol), prepared as in Example 41C, was added triethylsilane (0.41 mL, 2.57 mmol). The reaction mixture was cooled to 0 °C and trifluoroacetic acid (5 mL) was added dropwise. The reaction mixture was stirred for 1.5 hours at 0 °C and then was concentrated in vacuo and azeotroped with toluene. The residue was partitioned between water and ethyl acetate. The aqueous phase was extracted with ethyl acetate. The initial ethyl acetate extract was diltued with hexane and extracted with water. The second ethyl acetate extract was concentrated in vacuo and the residue was combined with the two aqueous phases. The aqueous solution was frozen and lyophylized to give [4-(1H-imidazol-4-ylmethyl)amino-2-phenylbenzoyl]methionine methyl ester (260 mg).

### Example 41E

### [4-(1H-imidazol-4-ylmethyl)amino-2-phenylbenzoyl]methionine hydrochloride

The desired compound was prepared by saponification of [4-(1H-imidazol-4-ylmethyl)amino-2-phenylbenzoyl]methionine methyl ester prepared as in Example 41D according to the procedure of Example 165. ¹H NMR (300 MHz, DMSO-d₆) δ 9.01 (d, 1H), 8.02 (d, 1H), 7.55 (d, 1H), 7.31 (m, 5H), 7.27 (d, 1H), 6.66 (dd, 1H), 6.60 (d, 1H), 6.59 (br s, 1H), 4.42 (s, 2H), 4.23 (m, 1H), 2.24 (m, 2H), 2.00 (s, 3H), 1.85 (m, 2H); MS (APCl) m/e 425 (M+H)⁺, 423 (M-H)⁻. Anal calcd for C₂₂H₂₄N₄O₃S·2HCl·0.5H₂O: C, 52.18; H, 5.37; N, 11.06. Found: C, 52.36; H, 5.18; N, 10.57.

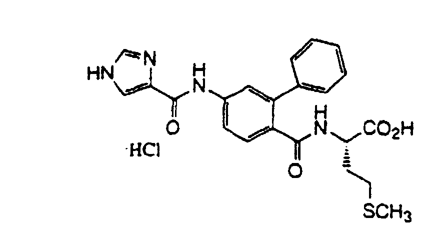

### Example 42

### [4-(1H-imidazol-4-ylcarbonyl)amino-2-phenylbenzoyl]methionine hydrochloride

### Example 42A

### 1H-1-triphenylmethylimidazole-4-carboxylic acid

To a slurry of 1H-1-triphenylmethylimidazole-4-carboxaldehyde (1.0 g, 3.0 mmol), prepared as in Example 41B, *tert*-butyl alcohol (60 mL), 2-methyl-2-butene (15 mL, 140 mmol) was added KH₂PO₄ (2.82 g, 21 mmol) and 80% sodium chlorite (3.1 g in 25 mL H₂O, 27 mmol). The reaction mixture was stirred for 1.5 hours using a mechanical stirrer. The pH was adjusted to 3-3.5 and the white solid was filtered off and rinsed with water. The solid was dried under high vacuum over P₂O₅ for two days to give 1H-1-triphenylmethylimidazole-4-carboxylic acid (956 mg, 91%).

### Example 42B

### [4-(1H-1-triphenylmethylimidazol-4-ylcarbonyl)amino-2-phenylbenzoyl]methionine methyl ester

The desired compound was prepared by coupling of 1H-1-triphenylmethylimidazole-4-carboxylic acid, prepared as in Example 42A, with 4-amino-2-phenylbenzoyl methionine methyl ester (compound 8).

### Example 42C

### [4-(1H-imidazol-4-ylcarbonyl)amino-2-phenylbenzoyl]methionine hyddrochloride

The desired compound was prepared according to the method of Examples 41D and E, except substituting [4-(1H-1-triphenylmethyl imidazol-4-ylcarbonyl)amino-2-phenylbenzoyl]methionine methyl ester, prepared as in Example 42B for [4-(1H-trimethylphenyl imidazol-4-ylmethyl)amino-2-phenylbenzoyl]methionine. ¹H NMR (300 MHz, DMSO-d₆) d 10.43 (s, 1H), 8.50 (s, 1H), 8.47 (d, 1H), 8.17 (s, 1H), 7.86 (m, 2H), 7.40 (m, 6H), 4.30 (m, 1H), 2.24 (m, 2H), 2.00 (s, 3H), 1.85 (m, 2H); MS (DCl-NH₃) m/e 439 (M+H)⁺. Anal calcd for C₂₂H₂₂N₄O₄S·HCl ·H₂O: C, 53.60; H, 5.11; N, 11.36. Found: C, 53.58; H, 5.00; N, 11.01.

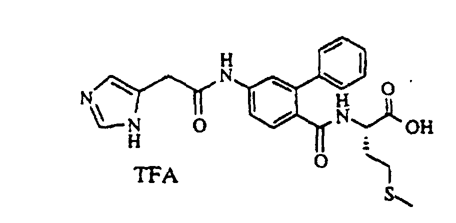

### Example 43

### [4-(1H-imidazole-4-ylacetamido)-2-phenylbenzoyl]methionine trifluoroacetate

### Example 43A

### N-(4-toluenesulfonyl)imidazole-4-yl-acetic acid

To a solution of 4-imidazole acetic acid hydrochloride (1 g, 6.15 mmol) in 6.2 mL of 1 N NaOH and 18 mL of water was added 4-toluenesulfonyl chloride (1.29 g, 6.77 mmol) and the mixture was stirred at ambient temperature. The pH of the mixture was maintained at 8.5 by addition of 1 N NaOH. After 3 hours, a total volume of 12 mL of 1 N NaOH was added and a clear solution was obtained. This solution was extracted with ether and the aqueous solution was acidified to pH 1 with 3 N HCl. The mixture was cooled in an ice bath and *N*-(4-Toluenesulfonyl)imidazole-4-yl-acetic acid was isolated by filtration (white crystals, 1.10 g, 63% yield). m.p. 105-106 °C (decomp); ¹H NMR (CDCl₃) δ 10.0 (br, 1H), 8.04 (s, 1H), 7.82 (d, *J*= 8.2 Hz, 2H), 7.37 (d, *J*= 8.2 Hz, 2H), 7.28 (s, 1H), 3.65 (s, 2H), 2.45 (s, 3H). ¹³C NMR (CD₃OD) δ 173.6, 148.1, 138.9, 138.0, 136.1, 131.6, 128.7, 117.0, 34.0, 21.6.

### Example 43B

### N-(4-toluenesulfonyl)imidazole-4-yl-acetic acid N-methyl-O-methylcarboxamide

To a suspension of *N*-(4-toluenesulfonyl)imidazole-4-yl-acetic acid (911 mg, 3.25 mmol) and N-methyl-O-methylhydroxylamine hydrochloride (317 mg, 3.25 mmol) in 30 mL of methylene chloride was added triethylamine (0.5 mL, 3.62 mmol) and ethyl dimethylaminopropyl carbodiimide hydrochloride (623 mg, 3.25 mmol). The mixture was stirred at ambient temperature for 7 hours and then worked up. The crude product was purified by flash column chromatography (5% methanol-ethyl acetate) to give *N*-(4-Toluenesulfonyl)imidazole-4-yl-acetic acid *N*-methyl-*O*-methylcarboxamide (1.0 g, yield 90%); ¹H NMR (CDCl₃) δ 7.91 (s, 1H), 7.77 (d, 8.4 Hz, 2H), 7.31 (d, 8.4 Hz. 2H), 7.30 (s, 1H), 3.70 (s, 2H), 3,64 (s, 3H), 3.16 (s, 3H), 2.38 (s, 3H); ¹³C NMR (CDCl₃) δ 170.4. 145.8, 137.9, 135.5, 134.4, 130.0, 126.9, 114.9, 60.9, 31.7, 31.3, 21.2.

### Example 43C

### {4-[1-(4-toluenesulfonyl)imidazole-4-yl)acetamido]-2-phenylbenzoyl}methionine methyl ester

To a suspension of *N*-(4-toluenesulfonyl)imidazole-4-yl-acetic acid *N*-methyl-*O*-methylcarboxamide (100 mg, 0.357 mmol), prepared as in Example 43B was added diisopropylethylamine (125 µL) and tetramethylfluoroformamidinium hexafluorophosphate (94 mg, 0.357 mmol, prepared as described in *J. Am. Chem*. *Soc.* **1995,** *117*, 5401-5402). The mixture was stirred for 5 minutes and then 4-amino-2-phenylbenzoylmethionine methyl ester hydrochloride (compound 8, 140 mg, 0.355 mmol) and diisopropylethylamine (65 µL) was added. After 5 hours, the reaction was worked up. The crude product was recrystallized from methylene chloride and hexane to give {4-[1-(4-toluenesulfonyl)imidazole-4-ylacetamido]-2-phenylbenzoyl)methionine methyl ester (92 mg, yield 41%); m.p. 202-203 °C; ¹H NMR (CDCl₃) δ 9.27 (s, 1H, amide), 8.04 (s, 1H, imidazole), 7.85 (d, *J*= 8.2 Hz. 2H, tolyl), 7.70 (d, *J*= 8.5 Hz, 1H), 7.61 (d, *J*= 8.5 Hz, 1H), 7.49 (s, 1H), 7.45 (m, 5H), 7.36 (d, *J*= 8.2 Hz, 2H, tosyl), 7.19 (s, 1H, imidazole), 5.83 (d, *J*= 7.6 Hz, 1H, amide), 4.62 (ddd, *J*= 7.1 Hz, 1H, Met α H), 3.65 (s, 3H, OCH₃), 3.62 (s, 2H, acetyl), 2.44 (s, 3H, tosyl), 2.07 (t, *J*= 7.5 Hz, 2H, CH₂S), 2.00 (s, 3H, SCH₃), 1.84-1.93 (m, 1H, Met CH₂), 164-1.76 (s, 1H, Met CH₂); ¹³C NMR (CDCl₃) δ 172.4, 169.2, 168.2, 146.6, 140.4, 140.2, 138.7, 134.3, 131.1, 130.7, 129.0, 128.4, 128.2, 127.4, 127.3, 120.3, 117.3, 115.5, 52.1, 51.3, 36.1, 30.0, 29.6, 21.2, 14.5.

### Example 43D

### [4-(1H-imidazole-4-ylacetamido)-2-phenylbenzoyl]methionine trifluoroacetate

{4-[1-(4-toluenesulfonyl)imidazole-4-ylacetamido]-2-phenylbenzoyl}methionine methyl ester (33 mg, 0.053 mmol), prepared as in Example 43C was dissolved in a mixture of THF (4 mL) and 0.5 N NaOH (0.6 mL). The mixture was stirred at 0 °C for 2 hours and then evaporated. The residue was acidified with 1 N HCl and the aqueous solution was lyophilized to give a solid mixture. This mixture was purified by preparative HPLC (C18, acetonitrile-water) to give 4-(1H-imidazole-4-yl)acetamido-2-phenylbenzoyl]methionine as a trifluoroacetate salt (17.6 mg, 55% yield); ¹H NMR (CD₃OD) δ 8.85 (s, 1H, imidazole), 7.67 (s, 1H, imidazole), 7.64 (d, *J*= 8.1 Hz, 1H, aminophenyl), 7.52 (d, *J*= 8.1 Hz, 1H, aminophenyl), 7.46 (s, 1H, aminophenyl), 7.33-7.40 (m, 5H, phenyl), 4.48 (dd. *J*= 4.0, 5.5 Hz, 1H, Met α H), 3.96 (s. 2H, acetyl), 2.15-2.22 (m, 1H), 2.01-2.11 (m, 1H), 2.00 (s, 3H), 1.85-1.99 (m, 1H), 1.76-1.81 (m, 1H).

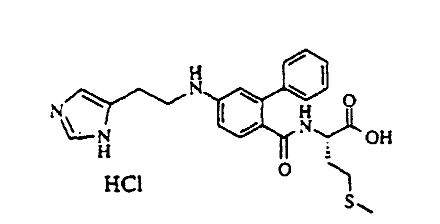

### Example 44

### [4-(1H-imidazole-4-ylethylamino)-2-phenylbenzoyl]methionine hydrochloride

### Example 44A

### {4-[1-(4-toluenesulfonyl)imidazole-4-ylethylamino]-2-phenylbenzoyl}methionine methyl ester

*N*-(4-toluenesulfonyl)imidazole-4-yl-acetic acid *N*-methyl-*O*-methylcarboxamide (1.86 g, 5.77 mmol), prepared as in Example 43B, was dissolved in 15 mL of THF and 15 mL of ether. This solution was cooled to -78 °C and LiAlH₄ (215 mg, 5.81 mmol) was added. The mixture was stirred for 20 minutes and then worked up with 1 N HCl. The mixture was extracted with ether. The ether solution was washed with concentrated sodium bicarbonate and dried. After evaporating solvents, a crude solid was obtained (1.61 g). ¹H NMR showed it contained 28% aldehyde, 20% unreacted carboxamide and 50% of destosylated side product. This mixture was dissolved in 20 mL of methanol and 1 mL of acetic acid. 4-Amino-2-phenylbenzoyl methionine methyl ester hydrochloride (compound 8.640 mg, 1.62 mmol) was added to the above solution and the reaction was stirred at ambient temperature. After 15 minutes, sodium cyanoborohydride (152 mg, 2.42 mmol) was added. The mixture was stirred for 15 hours and then evaporated. The residue was extracted with concentrated sodium bicarbonate and methylene chloride. The methylene chloride solution was dried and evaporated. The residue was purified by flash column chromatography (ethyl acetate-hexane 4:1) to give {4-[1-(4-toluenesulfonyl)imidazole-4-ylethylamino]-2-phenylbenzoyl}methionine methyl ester as a fluffy solid (500 mg, 51%); [α]²⁵_{D} = +0.60 (c = 1.10, CDCl₃); ¹H NMR (CDCl₃) δ 7.95 (s, 1H, imidazole), 7.79 (d, *J*= 8.0 Hz, 2H, tosyl), 7.68 (d, *J*= 8.5 Hz, 1H, aminophenyl), 7.37-7.45 (m, 5H), 7.33 (d, *J*= 8.0 Hz, 2H, tosyl), 7.05 (s, 1H, imidazole), 6.57 (d, *J*= 8.5 Hz, 1H, aminophenyl), 6.41 (s, 1H, aminophenyl), 5.66 (d, *J*= 7.6 Hz, 1H, amide), 4.61 (ddd, *J*= 5.2, 7.2 and 7.6 Hz, 1H, Met α H), 4.46 (t, *J*= 5.6 Hz, 1H, amine), 3.67 (s, 3H, OCH₃), 3.43 (q, *J*= 6.3 Hz, 2H, ethylene), 2.82 (t, *J*= 6.4 Hz, 2H, ethylene), 2.43 (s, 3H), 2.08 (t, *J*= 7.7 Hz, 2H, CH₂S), 2.00 (s, 3H, SCH₃), 1.82-1.90 (m, 1H), 1.58-1.70 (m, 1H); ¹³C NMR (CDCl₃) δ 171.8, 168.3, 149.4, 1461, 142.3, 141.5, 141.0, 136.1, 134.5, 131.0, 130.2, 128.5, 128.3, 127.5, 127.1, 122.3, 113.7, 113.6, 111.0, 52.0, 51.5, 42.2, 31.5, 29.3, 27.2, 21.5, 15.0.

### Example 44B

### [4-(1H-imidazole-4-ylethylamino-)2-phenylbenzoyl]methionine hydrochloride

{4-[1-(4-toluenesulfonyl)imidazole-4-ylethylamino]-2-phenylbenzoyl}methionine methyl ester (303 mg, 0.50 mmol), prepared as in Example 44A, was dissolved in a mixture of THF (4 mL) and 0.5 N NaOH (4.0 mL). The mixture was stirred at 0 °C for 2 hours and then evaporated. After acidification with 1 N HCl, the aqueous solution was lyophilized. The crude solid was purified by reverse phase preparative HPLC to give a trifluoroacetate salt (140 mg, 51%). This trifluoroacetate salt was dissolved in 1 N HCl and the aqueous solution was lyophilized to give [4-(1H-imidazole-4-ylethylamino-)2-phenylbenzoyl]methionine hydrochloride; [α]²⁵_{D} = -25.5 (c = 1.1, H₂O); ¹H NMR (CD₃OD) δ 8.79 (s, 1H, imidazole), 7.48 (d, *J*= 8.4 Hz, 1H), 7.32-7.39 (m, 6H), 6.84 (d, *J*= 8.4 Hz, 1H), 6.77 (s, 1H), 4.45 (dd, *J*= 4.1 and 5.1 Hz, 1H, Met α H), 3.58 (t, *J*= 7.0 Hz, 2H), 3.08 (t, *J*= 7.0 Hz, 2H), 2.16-2.24 (m, 1H), 2.05-2.14 (m, 1H), 2.00 (s, 3H), 1.92-1.98 (m, 1H), 1.72-1.85 (m, 1H); ¹³C NMR (CD₃OD) δ 174.8, 172.4, 145.1, 143.5, 141.3, 134.9, 131.8, 131.3, 130.9, 129.7, 129.6, 129.0, 119.7, 118.1, 116.6, 53.0, 46.5, 31.6. 31.0, 24.2, 15.0.

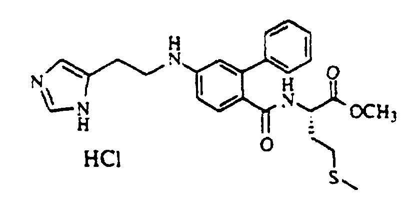

### Example 45

### [4-(1H-imidazole-4-ylethylamino)-2-phenylbenzoyl]methionine methyl ester hydrochloride

{4-[1-(4-toluenesulfonyl)imidazole-4-ylethylamino-]2-phenylbenzoyl}methionine methyl ester (90.2 mg, 0.1488 mmol), prepared as in Example 44A, was dissolved in 5 mL of THF. To this solution was added 3-hydroxy-1,2,3-benzotriazin-4(3H)-one (80.4 mg, 0.5956 mmol) and the mixture was stirred at ambient temperature. After 2 hours, TLC showed the disappearance of starting material. The solution was evaporated and the residue was extracted with ethyl acetate and 1 N HCl. The aqueous solution was neutralized with 1 N NaOH to pH 8.5 and then extracted with ethyl acetate. After evaporating solvents, the residue was dissolved in 1 N HCl and the solution was lyophilized to give [4-(1H-imidazole-4-ylethylamino)-2-phenylbenzoyl]methionine methyl ester hydrochloride (62.6 mg, yield 80%); [α]²⁵_{D} = -34.0 (c = 1.50, H₂O);
¹H NMR (CD₃OD) δ 8.82 (s, 1H, imidazole), 7.53 (d, 1H, *J*= 8.4 Hz, aminophenyl), 7.35-7.46 (m, 6H, imidazole and phenyl), 7.07 (d, 1H, *J*= 8.4 Hz, aminophenyl), 7.01 (s, 1H, aminophenyl), 4.50 (dd, *J*= 4.0 Hz, 1H, Met α H), 3.70 (s, 3H, OCH₃), 3.65 (t, *J*= 7.1 Hz, 2H, ethylene), 3.15 (t, *J*= 7.1 Hz, 2H, ethylene), 2.14-2.23 (m, 1H), 2.04-2.12 (m, 1H), 1.99 (s, 3H, SCH₃), 1.89-1.96 (m, 1H), 1.73-1.82 (m, 1H); ¹³C NMR (CD₃OD) δ 173.6, 172.2, 143.7, 143.3, 140.9, 135.2, 132.8, 131.4, 131.3, 129.8, 129.7, 129.1, 121.2, 118.2, 118.1, 53.0, 52.8, 47.6, 31.3, 30.8, 23.8, 15.0.

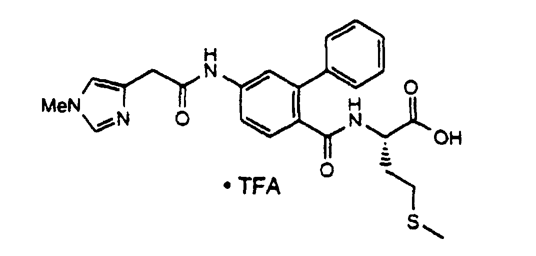

### Example 46

### [4-(1-Methylimidazole-4-ylacetamido-)2-phenylbenzoyl]methionine trifluoroacetate

### Example 46A

### [4-(1-Methylimidazole-4-ylacetamido)-2-phenylbenzoyl]methionine methyl ester

4-Amino-2-phenylbenzoyl methionine methyl ester hydrochloride (compound 8, 111.8 mg, 0.2833 mmol) and *N*-methylimidazole-4-yl-acetic acid hydrochloride (50 mg, 0.2832 mmol) were suspended in 10 mL of methylene chloride. To this solution was added diisopropylethylamine (197 µL, 4.0 eq) and *O*-benzotriazol-1-yl-*N*,*N*,*N*',*N*'-tetramethyluroniumhexafluorophosphate (107.4 mg, 0.2833 mmol). After stirring at ambient temperature for 2 days, the reaction was worked up by washing with dilute HCl (PH = 3.0) and concentrated sodium bicarbonate. After evaporating solvents, the residue was purified by flash column chromatography (CH₂Cl₂-Methanol, 10:1) to give [4-(1-Methylimidazole-4-ylacetamido)-2-phenylbenzoyl]methionine methyl ester (106 mg, 78%); m.p. 69-70 °C; ¹H NMR (CDCl₃) δ 9.89 (s, 1H, amide), 7.67 (d, *J*= 8.4 Hz, 1H), 7.60 (d, *J*= 8.4 Hz, 1H), 7.55 (s, 1H), 7.46 (s, 1H, imidazole), 7.34-7.42 (m, 5H), 6.81 (s, 1H, imidazole), 5.90 (d, *J*= 7.7 Hz, 1H, amide), 4.63 (ddd, *J*= 5.1, 7.3 and 7.7 Hz, 1H, Met α H), 3.72 (s, 3H, OCH₃), 3.64 (s, 5H, N-methyl and imidazole acetyl), 2.10 (t, *J=* 7.6 Hz, 2H), 1.98 (s, 3H), 1.83-1.94 (m, 1H), 1.66-1.75 (m, 1H); ¹³C NMR (CDCl₃) δ 171.7, 169.0, 168.9, 140.3, 140.1, 139.8, 137.3, 135.3, 129.6, 129.3. 128.5, 128.3, 127.5, 120.6, 118.5, 118.0, 52.1, 51.6, 36.3, 33.3, 30.8, 29.4, 15.0; LRMS (EI) for C₂₅H₂₈O₄N₄S 480 (M⁺, 20), 406 (100), 318 (50); HRMS (El) calcd 480.1813, obsd 480.1829.

### Example 46B

### [4-(1-Methylimidazole-4-ylacetamido-)2-phenylbenzoyl]methionine trifluoroacetate

4-[1-Methylimidazole-4-yl]acetamido-2-phenylbenzoyl]methionine methyl ester (70 mg, 0.1458 mmol), prepared as in Example 46A, was dissolved in a mixture of THF (2.0 mL) and 0.5 N LiOH (0.5 mL). The mixture was stirred at 0 °C for 1 hour. After evaporating solvents, the residue was acidified with 1 N HCl. The aqueous solution was lyophilized and the crude solid was purified by reverse phase preparative HPLC to give [4-(1-Methylimidazole-4-ylacetamido-)2-phenylbenzoyl]methionine as a TFA salt (50 mg, 60%). ¹H NMR (CD₃OD) δ 8.83 (s, 1H, imidazole), 7.66 (s, 1H, imidazole), 7.64 (d, *J*= 8.5 Hz, 1H), 7.52 (d, *J*= 8.5 Hz, 1H), 7.48 (s, 1H), 7.32-7.43 (m, 5H), 4.48 (dd, *J*= 4.1 and 9.5 Hz, 1H, Met α H), 3.92 (s, 5H, N-methyl and imidazole acetyl), 2.13-2.22 (m, 1H), 2.00-2.10 (m, 1H), 2.00 (s, 3H), 1.94-2.00 (m, 1H), 1.72-1.84 (m, 1H); ¹³C NMR (CD₃OD) δ 174.9, 172.8, 168.2, 142.4, 141.4, 141.2, 136.7, 132.7, 130.0, 129.7, 129.5, 129.4, 128.8, 122.8, 122.2, 119.2, 53.0, 36.1, 33.0, 31.5, 31.0, 15.0.

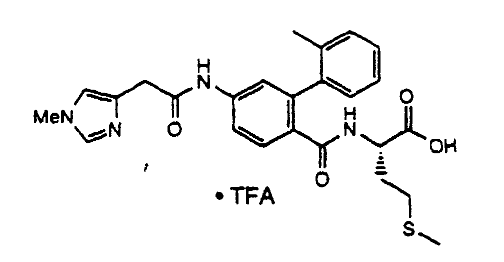

### Example 47

### [4-(1H-1-Methylimidazole-4-ylacetamido)-2-(2-methylphenyl)benzoyl]methiopine trifluoroacetate

### Example 47A

### 4-nitro-2-(2-methylphenyl)benzoic acid methyl ester

The coupling of 4-nitro-2-bromobenzoic acid methyl ester with 2-methylphenylboronic acid in DMF at 100 °C in the presence of Pd(PPh₃)₄ (1.5 % eq) and Na₃PO₄ (2.5 eq) gave 4-nitro-2-(2-methylphenyl)benzoic acid methyl ester as a colorless oil (43% yield after column chromatography purification 6:1 = hexane / ethyl acetate). ¹H NMR (CDCl₃) δ 8.26 (d, *J*=8.6 Hz, 1H), 8.13 (s, 1H), 8.08 (d, *J*=8.6 Hz, 1H), 7.21-7.34 (m, 3H), 7.06 (d, *J*=7.5 hz, 1H), 3.65 (s, 3H), 2.09 (s, 3H); ¹³C NMR (CDCl₃) δ 165.8, 148.5, 143.5, 138.5, 135.9, 134.6, 130.5, 129.3, 127.9, 127.7, 125.1, 121.5, 51.8, 19.3 (expect 12 aromatic C, observed 11); LRMS (El) 271; HRMS (EI) calcd for C₁₅H₁₃NO₄ 271.0844, obsd 271.0852.

### Example 47B

### 4-nitro-2-(2-methylphenyl)benzoic acid

4-Nitro-2-(2-methylphenyl)benzoic acid methyl ester, prepared as in Example 178A, was saponified using aqueous NaOH-CH₃OH to give 4-nitro-2-(2-methylphenyl)benzoic acid; HRMS calcd for C₁₄H₁₁NO₄ 257.0688, obsd 257.0699.

### Example 47C

### [4-Nitro-2-(2-methylphenyl)benzoyl]methionine methyl ester hydrochloride

4-Nitro-2-(2-methylphenyl)benzoic acid (2.31 g, 9 mmol), prepared as in Example 47B, was coupled with L-methionine methyl ester (1.0 eq) in the presence of ethyl dimethylaminopropyl carbodiimide hydrochloride (EDCI, 1.0 eq) and 3-hydroxy-1,2,3-benzotriazin-4(3H)-one (HOBT, 1.0 eq) to give [4-nitro-2-(2-methylphenyl)benzoyl]methionine methyl ester as a pale yellow oil (3.54 9, 98% yield); ¹H NMR showed diastereomers due to restricted carbon-carbon bond rotation; ¹H NMR (CDCl₃) δ 8.26-8.30 (d. *J*= 8.5 Hz, 1H), 8.10 (s, 1H), 8.03-8.09 (m, 1H), 7.27-7.42 (m, 3.5 H), 7.18 (d, *J*= 7.4 Hz, 0.5 H), 6.03 (br, 1H, amide), 4.59-4.67 (m, 1H), 3.67 (s, 3H), 2.23 (s, 1.5H, PhCH₃), 2.06 (s, 1.5 H, PhCH₃), 1.98-2.03 (m, 5H), 1.81-1.93 (m, 1H), 1.59-1.69 (m, 1H).

### Example 47D

### [4-amino-2-(2-methylphenyl)benzoyl]methionine methyl ester hydrochloride

[4-Nitro-2-(2-methylphenyl)benzoyl]methionine methyl ester was reduced to a corresponding amine by stannous chloride in ethyl acetate at 78 °C. The free amine was treated with methylene chloride and 3 N HCl in ether to give [4-amino-2-(2-methylphenyl)benzoyl]methionine methyl ester hydrochloride (85% yield); [α]²⁵_{D} = -28.3 (c = 1.0, methanol); ¹H NMR (CD₃OD) δ 7.74 (d, *J*= 8.2 Hz, 1H), 7.47 (d, *J*= 8.2 Hz, 1H), 7.23-7.30 (m, 5H), 4.47 (m, 1H), 3.69 (s, 3H), 2.06-2.18 (m. 4 H), 1.99 (s, 3H), 1.95-1.97 (m, 2H), 1.74 (m, 1H); ¹³C NMR (CD₃OD) δ 173.3, 170.8, 143.3, 139.7, 138.1, 137.2, 133.5, 131.4, 130.8, 130.6, 129.6, 128.8, 126.2, 123.1, 66.9, 52.9, 31.5, 30.8, 20.4, 15.1.

### Example 47E

### [4-(-H-1-Methylimidazole-4-ylacetamido)-2-(2-methylphenyl)benzoyl]methionine methyl ester

The desired compound was prepared by coupling of 4-amino-2-(2-methylphenyl)benzoylmethionine methyl ester hydrochloride, prepared as in Example 47D, with N-methylimidazole-4-yl-acetic acid
(yield 58%, purified by column chromatography (10:1 CH₂CH₂-CH₃OH), m.p. 69-70 °C; [α]²⁵_{D} = + 17.5 (c = 4.4, CHCl₃); ¹H NMR (CDCl₃) δ 9.91 (s, 1H), 7.90-7.99 (m, 1H), 7.65 (m, 1H), 7.45 (s, 1H), 7.40 (d, *J*= 6.2 Hz, 1H), 7.26-7.31 (m, 3H), 7.15-7.20 (m, 1H), 6.79 (s, 1H), 5.86 (d, *J*= 7.1 Hz, 1H amide), 4.56-4.64 (m, 1H), 3.67 (s, 4H) 3.64 (s, 4H), 2.17 (s, 1.5 H, PhCH₃), 1.93-2.05 (m, 6.5 H), 1.80-1.89 (m, 1H), 1.51-1.61 (m, 1H); ¹³C NMR (CDCl₃) δ 171.7, 171.6, 168.7, 167.1, 166.7, 140.5, 140.4, 140,1, 139.8, 137.5, 136.0, 135.5, 130.5, 130.3, 128.9, 128.7, 128.5, 128.1, 128.0, 126.0. 125.9, 120.5, 118.2, 118.1, 52.1, 51.5, 51.4, 36.4, 33.2, 31.3, 29.1, 19.7, 15.0 (diastereomers shown in NMR data are due to restricted carbon-carbon bond rotation); HRMS calcd for C₂₆H₃₀O₄N₄S 494.1988, obsd 494.1986.

### Example 47F

### [4-(1H-1-Methylimidazole-4-ylacetamido)-2-(2-methylphenyl)benzoyl]methionine trifluoroacetate

[4-(1H-1-Methylimidazole-4-ylacetamido)-2-(2-methylphenyl)benzoyl]methionine methyl ester (72 mg, 0.1472 mmol) was saponified using 0.5 N LiOH (0.58 mL. 0.29 mmol) in 2.0 mL of THF. The acid was purified by reverse phase preparative HPLC to give [4-(1-methylimidazole-4-ylacetamido)-2-(2-methylphenyl)benzoyl]methionine trifluoroacetate (70 mg, 87% yield); ¹H NMR showed a complex due to diastereomers caused by restricted bond rotation; ¹H NMR (CD₃OD) δ 8.81 (s, 1H), 7.68 (m, 2H), 7.46-7.49 (m, 2H), 7.25 (m, 4H), 4.43 (m, 1H), 3.91 (m, 5H), 2.08-2.17 (m, 4H), 1.94-1.99 (m, 5H), 1.69 (m, 1H).

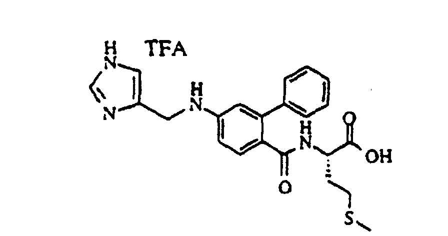

### Example 48

### [4-(1-H-imidazole-4-ylmethylamino)-2-phenylbenzoyl]methionine trifluoroacetate

### Example 48A

### 4-Hydroxymethyl-1-p-toluenesulfonylimidazole

4-Hydroxymethylimidazole hydrochloride (1.0 g, 7.4 mmol) and *p*-toluenesulfonyl chloride were suspended in 1 mL. of distilled water and 3 mL. of THF. Sodium hydroxide (1N) was added and the pH of approximately 9 was maintained over a period of 3 hours. The reaction mixture was extracted with diethyl ether (3X50 mL.). The extracts were combined, dried over magnesium sulfate and concentrated. The residue was purified by flash chromatography (100% ethyl acetate) to give 4-hydroxymethyl-1-*p*-toluenesulfonylimidazole (1.1 g, 58%) as a white solid; m.p. 109-112°C; ¹H NMR (300 MHz, CDCl₃) δ 7.98 (s, 1H, imidazole), 7.82 (d, *J*=8.4 Hz. 2H), 7.34 (d, *J*=8.3 Hz, 2H), 7.22 (s, 1H), 4.51 (s, 2H, CH₂OH), 2.42 (s, 3H, CH₃); ¹³C NMR (75 MHz, CDCl₃) δ 146.68, 144.68, 136.82, 134.72, 130.58, 127.54, 114.28, 57.66, 21.82; MS m/e calc'd for : 252.0569, found 252.0576.

### Example 48B

### (1H-1-p-toluenesulfonylimidazole-4-yl)carboxaldehyde

4-Hydroxymethyl-1-*p*-toluenesulfonylimidazol (0.7 g, 2.8 mmol), prepared as in Example 48A, was dissolved into 7 mL. of methylene chloride and manganese (IV) oxide (2.0 g, 23 mmol) added. The reaction mixture was stirred at room temperature under a nitrogen atmosphere for 20 hours. The reaction mixture was filtered through a celite pad, the pad rinsed and the combined filtrates were evaporated. The residue was purified by flash chromatography (2:3 ethyl acetate/hexanes) to give (1-*p*-toluenesulfonylimidazole-4-yl)carboxaldehyde (0.45 g, 64 %) as a white solid.: ¹H NMR (300 MHz, CDCl₃) δ 9.79 (s, 1H), 8.00 (s, 1H), 7.87 (s, 1H), 7.81 (d, *J*=8.2 Hz, 2H), 7.33 (d, *J*=8.0 Hz, 2H), 2.38 (s, 3H); MS *m*/*e* calc'd for : 250.0412, found 250.0419.

### Example 48C

### [4-(-1H-1-p-toluenesulfonylimidazole-4-ylmethyamino-2-phenylbenzoyl]methionine methyl ester

(1-*p*-toluenesulfonylimidazole-4-yl)carboxaldehyde (0.10 g, 0.4 mmol), prepared as in Example 48B, and 4-amino-2-phenylbenzoyl-methionine methyl ester hydrochloride (0.16 g, 0.4 mmol) were dissolved in 10 mL. of 95% methanol and 5% acetic acid and stirred for 15 minutes. Sodium cyanoborohydride (0.05 g, 0.8 mmol)was added and the reaction was stirred for 0.5 hour. Twice during this time additional (1-*p*-toluenesulfonylimidazole-4-yl)carboxaldehyde (0.165 g, 0.66 mmol) and sodium cyanoborohydride (0.83 g, 1.3 mmol) were added. The reaction was stirred at room temperature under a nitrogen atmosphere for 16 hours. The reaction mixture was concentrated and the residue was taken up in ethyl acetate and washed with a saturated solution of sodium bicarbonate. The organic phase was dried over magnesium sulfate and concentrated. The residue was purified by flash chromatography (3:2 ethyl acetate/hexanes) to give [4-(-1H-1-*p*-Toluenesulfonylimidazole-4-ylmethyamino-2-phenylbenzoyl]methionine methyl ester (0.075 g, 7.5%) as a white solid; ¹H NMR (300 MHz, CDCl₃) δ 7.97 (s, 1H, Imidazole), 7.79 (d, *J*=8.2 Hz, 2H), 7.65 (d, *J*=8.4 Hz, 1H), 7.41-7.34 (m, 8H), 7.18 (s, 1H, Imidazole), 6.60 (d, *J*=8.5 Hz, 1H), 6.46 (s, 1H,), 6.46 (s, 1H), 5.71 (d, 7.65 1H), 4.62 (dd, *J=*6.14, 6.21 Hz, 1H), 4.27 (s, 2H), 3.64 (s, 3H), 2.44 (s, 3H), 2.10 (t, *J*=7.56 Hz, 2H), 2.00 (s, 3H), 1.93-1.82 (m, 1H), 1.69-1.60 (m, 1H).

### Example 48D

### [4-(1-H-imidazole-4-ylmethylamino)-2-phenylbenzoyl]methionine trifluoroacetate

The [4-(-1H-1-*p*-toluenesulfonylimidazole-4-ylmethyamino-2-phenylbenzoyl]methionine methyl ester (0.075 g, 0.13 mmol), prepared in Example 48C, was dissolved into 2 mL. of THF and cooled to 0°C. Lithium hydroxide (2 mL. 0.5M) was slowly added and the reaction mixture was stirred for 4 hours. The pH was adjusted to 4 with 1N HCl and the THF was removed under vacuum. The aqueous layer was lyophilized and the resulting solid was purified by reverse phase preparative HPLC (Waters 25X10 cm, C-18 column, 220 nm UV detector, flow rate 15 mL./min, linear gradient from 5% acetonitrile and 95% water containing 0.1% TFA to 60% acetonitrile in 40 minutes) to give [4-(1-H-imidazole-4-ylmethylamino)-2-phenylbenzoyl]methionine trifluoroacetate as a white solid (0.03 g, 52%); ¹H NMR (300 MHz, DMSO-d₆) δ 14.44 (br s, 2H), 12.60 (s, 1H), 9.01 (s, 1H), 8.07 (d, *J*=7.8 Hz, 1H), 7.56 (s, 1H), 7.30-7.24 (m, 8H), 6.65 (d, *J*=9.2 Hz, 1H), 6.59 (s, 1H), 4.41 (s, 2H), 4.25-4.18 (m, 1H, α CH Met.), 2.27-2.14 (m, 1H), 1.98 (s, 3H), 1.86-1.75 (m, 1H).

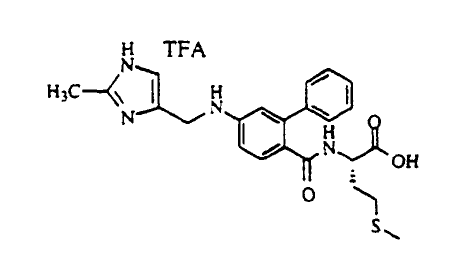

### Example 49

### [4-(1-H-2-Methylimidazole-4-ylmethylamino)-2-phenylbenzoyl]methionine trifluoroacetate

### Example 49A

### 4-Hydroxymethyl-2-methylimidazole

1,3-Dihydroxyacetone (6.2 g, 50 mmol) and ethyl acetimidate hydrochloride (4.5 g, 50 mmol) were added to an autoclave to which 50 mL of liquid ammonia was added. The apparatus was sealed and heated (68-70° C) with stirring for 4 hours. After cooling the reaction mixture was extracted with hot acetonitrile which upon cooling formed a precipitate which was collected to give 4-hydroxymethyl-2-methylimidazole (2.3 g, 41%); ¹H NMR (300 MHz, CDCl₃) δ 6.75 (s, 1H), 4.50 (s, 2H), 2.35 (s, 3H); ¹³C NMR (75 MHz, CDCl₃) δ 143.48, 135.98, 116.25, 55.59, 12.95.

### Example 49B

### 4-Hydroxymethyl-2-methyl-1-p-toluenesulfonylimidazole

4-Hydroxymethyl-2-methylimidazole (0.7 g, 6.25 mmol), prepared as in Example 49A, and *p*-toluenesulfonylchloride (1.2 g, 6.25 mmol) were suspended in 5 mL of distilled water and 3 mL of THF. Sodium hydroxide (1N) was added to maintain a pH of 9 over a period of 3 hours. The reaction mixture was extracted with ethyl acetate (3X50 mL.). The extracts were combined, dried over magnesium sulfate , concentrated and crystallized from ethyl acetate, collected by vacuum filtration and dried to give 4-hydroxymethyl-2-methyl-1-*p*-toluenesulfonylimidazole (0.5 g, 35%) as a white solid; m.p. 140-143°; ¹H NMR (300 MHz, CDCl₃) δ 7.78 (d, 8.25 2H), 7.36 (d, *J*=8.25 Hz, 2H), 7.33 (s, 1H), 4.49 (s, 2H), 2.50 (s, 3H), 2.45 (s, 3H); ¹³C NMR (75 MHz, CDCl₃) δ 146.38, 140.96, 134.85, 130.51, 127.57, 116.05, 57.37, 21.84, 15.02; MS m/e calc'd: 266.0725. found: 266.0714.

### Example 49C

### (2-Methyl-1-p-toluenesulfonylimidazole-4-yl)-carboxaldehyde

4-Hydroxymethyl-2-methyl-1-*p*-toluenesulfonylimidazole (0.75 g, 2.8 mmol), prepared as in Example 49B, was dissolved into 10 mL of methylene chloride and manganese (IV) oxide (2.0 g, 23 mmol) added over an 8 hour period. The reaction mixture was stirred at room temperature for an additional 16 hours. The reaction mixture was filtered through a celite plug and concentrated to leave a slightly yellow oil. The residue was crystallized from ethyl acetate, collected and dried to give (2-methyl-1-*p*-toluenesulfonylimidazol-4-yl)carboxaldehyde (0.44 g, 59%) as a white solid; mp 106-109° C; ¹H NMR (300 MHz, CDCI₃) δ 9.82 (s, 1H), 8.09 (s, 1H), 7.84 (d, *J*= 7.53, 2H), 7.42 (d, *J*= 7.44, 2H), 2.56 (s, 3H), 2.48 (s, 3H); ¹³C NMR (75 MHz, CDCl₃) δ 184.95, 147.32, 139.63, 133.81, 130.83, 127.94, 125.35, 21.93, 15.18.

### Example 49D

### [4-(1H-2-Methyl-1-p-toluenesulfonylimidazol-4-ylmethylamino)-2-phenylbenzoyl]methionine methyl ester

(2-Methyl-1-*p*-toluenesulfonylimidazol-4-yl)carboxaldehyde (0.10 g, 0.38 mmol), prepared as in Example 49C, and 4-amino-2-phenylbenzoyl-methionine methyl ester hydrochloride (0.037 g, 0.09 mmol) were dissolved in 10 mL. of 95% methanol and 5% acetic acid and stirred for 15 minutes. Sodium cyanoborohydride (0.048 g, 0.76 mmol) was then added and the reaction was stirred for 0.5 hour. Additional (2-Methyl-1-*p*-toluenesulfonylimidazol-4-yl)carboxaldehyde (0.10 g, 0.038 mmol) and sodium cyanoborohydride (0.048 g, 0.076 mmol) were then added, followed by-4-amino-2-phenylbenzoylmethionine methyl ester hydrochloride (compound 8, 0.037 g, 1.7 mmol). Additional carboxaldehyde (0,24 g, 0.91 mmol) and 4-amino-2-phenylbenzoylmethionine methyl ester hydrochloride (0.180 g, 0.46 mmol) were then added and the reaction was stirred at room temperature for 0.5 hour. The reaction mixture was concentrated and the residue taken up in ethyl acetate and washed with a saturated solution of sodium bicarbonate. The organic phase was dried over magnesium sulfate and concentrated. The residue was purified by flash chromatography (4:1 ethyl acetate/hexanes) to give [4-(1H-2-methyl-1-*p*-toluenesulfonylimidazol-4-ylmethylamino)-2-phenylbenzoyl]methionine methyl ester (0.185 g, 47%) as a white foam; ¹H NMR (300 MHz, CDCl₃) δ 7.73 (d, *J*=8.28 Hz, 2H), 7.67 (d, *J*=8.52 Hz, 2H), 7.42-7.27 (m, 7H), 6.62 (dd, *J*=7.23, 2.25 Hz, 1H), 6.49 (d, *J*=2.25 Hz, 1H), 5.71 (d, *J*=7.59 Hz, 1H), 4.66-4.59 (m, 1H, α CH Met.), 4.55 (t, *J*=5.46 Hz, 1H), 4.22 (d, *J*=5.34 Hz, 2H), 3.65 (s, 3H), 2.62 (s, 3H), 2.50 (s, 3H), 2.10 (t, *J*=7.65 Hz, 2H), 2.01 (s, 3H), 1.94-1.78 (m, 1H), 1.72-1.60 (m, 1H); ¹³C NMR (75 MHz, CDCl₃) δ 172.26, 168.67, 149.40, 146.34, 141.62, 141.30, 138.38, 134.99, 131.44, 130.58, 128.95, 128.83, 128.02, 127.50, 123.54, 116.28, 114.45, 111.67, 52.51, 52.01, 41.45, 31.68, 29.71, 21.91, 15.49, 15.35.

### Example 49E

### [4-(1-H-2-Methylimidazol-4-ylmethyamino)-2-phenylbenzoyl]methionine trifluoroacetate

[4-(1H-2-methyl-1-*p*-toluenesulfonylimidazol-4-ylmethylamino)-2-phenylbenzoyl]methionine methyl ester (0.1 g, 0.165 mmol), prepared as in Example 49D, was dissolved in 2 mL of THF and cooled to 0°C. Lithium hydroxide (2 mL. 0.5M) was slowly added and the reaction mixture was stirred for 6 hours. Aqueous HCl (3 mL, 0.5M) was added and excess THF-removed under vacuum. The aqueous layer was lyophilized and the resulting solid was purified by reverse phase preparative HPLC (Waters 25X10 cm, C-18 column, 220 nm UV detector, flow rate 15 mL/min, linear gradient from 5% acetonitrile and 95% water containing 0.1% TFA to 60% acetonitrile in 40 minutes) to give [4-(1-H-2-methylimidazol-4-ylmethyamino)-2-phenylbenzoyl]methionine trifluoroacetate as a white solid (0.03 g, 52%); ¹H NMR (300 MHz, CDCl₃) δ 14.12 (br, s, 1H), 13.92 (br, s, 1H), 12.55 (br s 1H), 8.06 (d, *J*=7.8 Hz, 1H), 7.39 (s, 1H), 7.28-7.22 (m, 7H), 6.62 (d, *J*=8.7 Hz, 1H), 6.56 (s, 1H), 4.32 (s, 2H), 4.23-4.16 (m, 1H, α CH Met.), 2.49 (s, 3H), 2.29-2.12 (m, 2H), 1.96 (s, 3H), 1.84-1.73 (m, 2H).

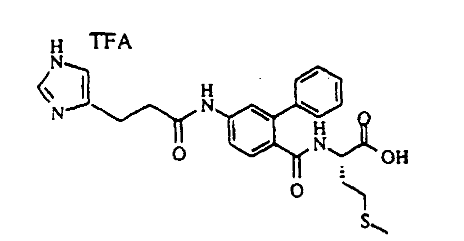

### Example 50

### [4-((1-H-imidazol-4-yl)-3-propylcarbonylamino)-2-phenylbenzoyl]methionine trifluoroacetate

### Example 50A

### trans-Urocanic acid-methyl ester

Urocanic acid (0.6 g, 4.3 mmol) was suspended in methanol and HCl gas bubbled through so refluxing commenced for 1 hour After cooling the precipitate was collected by vacuum filtration, washed with hexanes and dried to give *trans*-urocanic acid-methyl ester (0.74 g, 91%) as a white solid. m.p. 239-242°C; ¹H NMR (300 MHz, CDCl₃) δ 9.26 (s, 1H), 8.07 (s, 1H), 7.59 (d, *J*=16.3 Hz, 1H), 6.89 (d, *J*=16.2 Hz, 1H), 3.74 (s, 3H);

### Example 50B

### 3-(1-H-Imidazol-4-yl)propanoic acid methyl ester

*Trans*-urocanic acid-methyl ester (0.6 g, 3.2 mmol) was dissolved in methanol (20 mL) and hydrogenated at room temperature using 10% Palladium on carbon (0.04 g) under a hydrogen atmosphere (40 psi) for 5.5 hours. The reaction mixture was filtered through a celite plug and concentrated. The residue was crystallized from ethyl ether, collected and dried to give 3-(1-H-imidazol-4-yl)propanoic acid methyl ester (0.56 g, 93%) as a white solid; m.p. 105-108° C; ¹H NMR (300 MHz, CDCl₃) δ 8.99 (s, 1H), 7.40 (s, 1H), 3.58 (s, 3H); MS m/e calc: 154.0742, found: 154.0750.

### Example 50C

### 3-(1-H-1-Triphenylmethylimidazol-4-yl)propanoic acid methyl ester

To a solution of 3-(1-H-imidazol-4-yl)propanoic acid methyl ester (0.5 g, 2.6 mmol), prepared as in Example 50B, and triphenylmethylchloride (0.73 g, 2.6 mmol) in 10 mL of methylene chloride was added triethylamine (0.58 g, 5.2 mmol). The reaction mixture was stirred at room temperature for 3 hours. The organics were washed with distilled water, dried using magnesium sulfate and concentrated under vacuum. The residue was crystallized from ether and hexanes, collected by vacuum filtration and dried to give 3-(1H-1-triphenylmethylimidazol-4-yl)propanoic acid methyl ester (0.81 g, 79%) as a white solid; m.p. 140-141 C°; ¹H NMR (300 MHz, CDCl₃) δ 7.39-7.30 (m, 10H), 7.15-7.77 (m, 6H), 6.55 (s, 1H), 3.62 (s, 3H), 2.87 (t, *J*=7.32 Hz, 2H), 2.66 (t, *J*=7.74 Hz, 2H)

### Example 50D

### 3-(1H-1-Triphenylmethylimidazol-4-yl)propanoic acid

To a 0 °C solution of 3-(1H-1-triphenylmethylimidazol-4-yl)propanoic acid methyl ester (0.6 g, 1.5 mmol), prepared as in Example 50C, was slowly added lithium hydroxide (6 mL. 0.5M) and the reaction mixture was stirred for 2 hours. The THF was removed under vacuum and the aqueous layer was acidified using HCl (6 mL. 0.5M). A white precipitate which formed was collected by vacuum filtration and dried to give 3-(1H-1-triphenylmethylimidazol-4-yl)propanoic acid (0.53 g, 93%) as a white solid; m.p. 182-186° C; ¹H NMR (300 MHz, DMSO-d₆) δ 7.40-7.38 (m, 9H), 7.26 (s, 1H), 7.09-7.07 (m, 6H), 6.64 (s, 1H), 2.67 (t, *J*=6.66 Hz, 2H), 2.49 (t, *J*=6.78 Hz, 2H).

### Example 50E

### [4-((1H-1-Triphenylmethylimidazol-4-yl)-3-propylcarbonylamino)-2-phenylbenzoyl]methionine methyl ester

To a 0 °C solution of 3-(1H-1-triphenylmethylimidazol-4-yl)propanoic acid (0.5 g 1.3 mmol), prepared as in Example 50D, ethyl dimethylaminopropyl carbodiimide hydrochloride (0.27 g, 1.4 mmol), 3-hydroxy-1,2,3-benzotriazin-4(3H)-one (HOBT, 0.18 g, 1.3 mmol), and 4-amino-2-phenylbenzoyl-methionine methyl ester hydrochloride (0.52 g, 1.3 mmol) in 10 mL. of methylene chloride, was added triethylamine (0.13 g, 1.3 mmol) and the reaction mixture was stirred for 16 hours at room temperature under a nitrogen atmosphere. The reaction mixture was washed first with distilled water followed by 0.5N HCl. The organics were dried using magnesium sulfate and concentrated. The residue was purified by flash chromatography (19:1 chloroform/hexanes) to give [((1H-1-triphenylmethylimidazol-4-yl)-3-propylcarbonylamino)-2-phenylbenzoyl]methionine methyl ester (0.38 g, 40%) as a white foam; ¹H NMR (300 MHz, CDCl₃) δ 9.96 (s, 1H), 7.70 (d, 1H), 7.69-7.56 (m, 2H), 7.56-7.23 (m, 17H), 7.08-7.05 (m, 4H), 6.62 (s, 1H), 5.85 (d, *J*=7.68 Hz, 1H), 5.85 (d, *J*=7.68 Hz, 1H), 4.64 (dd, *J*=7.26, 6.23 Hz, 1H), 3.65 (s, 3H), 2.95-2.91 (m, 2H), 2.81-2.77 (m, 2H).

### Example 50F

### [4-(1H-imidazol-4-yl)-3-propylcarbonylamino-2-phenylbenzoyl]methionine trifluoroacetate

To a 0 °C solution of [4-(1H-1-triphenylmethylimidazol-4-yl)-3-propylcarbonylamino-2-phenylbenzoyl]methionine methyl ester (0.16 g, 0.23 mmol), prepared as in Example 50E, in 4.4 mL of THF was slowly added lithium hydroxide (4.4 mL. 0.5M) and the reaction mixture was stirred for 2 hours. The pH was adjusted using 0.5 M HCl and the mixture was extracted with ethyl acetate (3X50 mL.). The extracts were combined, dried over magnesium sulfate and concentrated to an oil. The oil was taken up in methylene chloride (4 mL.) to which trifluoroacetic acid (8 mL) was added which produced a deep yellow color. Immediately after the addition of TFA, triethylsilane was added dropwise until the reaction mixture was nearly colorless. The reaction was stirred for 2 hours at ambient temperature and concentrated to give a solid which was washed with diethyl ether. The solid was collected by vacuum filtration, washed with additional diethyl ether and dried to yield [4-(1H-imidazol-4-yl)-3-propylcarbonylamino-2-phenylbenzoyl]methionine trifluoroacetate (0.073 g, 36%). ¹H NMR (300 MHz, CD₃OD) δ 8,72 (s, 1H), 7.64 (br s, 2H), 7.35-7.50 (m, 8H), 4.50 (br s, 2H), 2.80 (br s, 2H), 2.19 (br s, 2H), 2.00 (s, 3H), 1.82 (br s, 2H). MS m/e 467 (M+H)⁺.

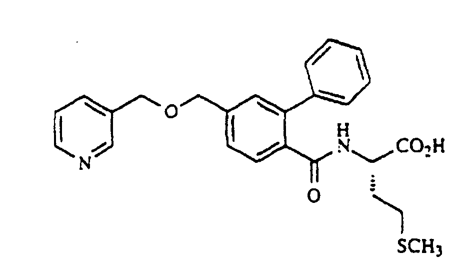

### Example 51

### [4-(3-pyridylmethyloxymethyl)-2-phenylbenzoyl]methionine hydrochloride

### Example 51A

### [4-(3-pyridylmethyloxymethyl)-2-phenylbenzoic acid methyl ester

To a solution in DMF of 3-pyridinemethanol (0.59 mL) was added sodium hydride (60% in mineral oil, 0.19 g), and the mixture was stirred until gas evolution ceased. A solution of 2-phenyl-4-bromomethylbenzoic acid methyl ester (0.98 g) in DMF was then added and the reaction mixture was stirred until the bromide was consumed. The reaction mixture was partitioned between ethyl acetate and water. The organic phase was washed with brine, dried, and concentrated. The residue was purified by chromatography on silica gel (1:1 ethyl acetate-hexanes) to give [4-(3-pyridylmethyloxymethyl)-2-phenylbenzoic acid methyl ester (0.58 g).

### Example 51B

### 4-(3-pyridylmethyloxymethyl)-2-phenylbenzoic acid

To a solution in methanol (5 mL) of [4-(3-pyridylmethyloxymethyl)-2-phenylbenzoic acid methyl ester (0.58 g), prepared as in Example 51A, was added saturated aqueous lithium hydroxide and the reaction mixture was stirred overnight at ambient temperature. The reaction mixture was warmed to 60 °C and stirred for 4 hours. The reaction mixture was concentrated in vacuo and the residue was taken up in water. The aqueous phase was taken to pH 5 with aqueous 3N HCl and extracted with chloroform. The organic phase was concentrated in vacuo to give 4-(3-pyridylmethyloxymethyl)-2-phenylbenzoic acid (0.52 g).

### Example 51C

### [4-(3-pyridylmethyloxymethyl)-2-phenylbenzoyl]methionine methyl ester

The desired compound was prepared by coupling of 4-(3-pyridylmethyloxymethyl)-2-phenylbenzoic acid with methionine methyl ester hydrochloride.

### Example 51D

### [4-(3-pyridylmethyloxymethyl)-2-phenylbenzoyl]methionine

The desired compound was prepared by saponification of [4-(3-pyridylmethyloxymethyl)-2-phenylbenzoyl]methionine methyl ester, prepared as in Example 51C using the procedure of Example 51B; ¹H NMR (CDCl₃, 300 MHz) δ 1.66-2.17 (4H, m), 2.02 (3H, s), 4.62 (1H, m), 4.76 (2H, s), 4.78 (2H, s), 6.31 (1H, d, *J*= 6.3Hz), 7.23-7.44 (7H, m), 7.71 (1H, d, *J*= 7.8Hz). 7.86 (1H, m), 8.34 (1H, m). 8.65 (1H, m), 8.72 (1H, m); MS (DCl/NH₃) m/e 451 (M+H)⁺. Anal calcd for C₂₅H₂₆N₂O₄S·1.45 HCl: C, 59.65; H, 5.50; N, 5.56. Found: C, 59.80; H , 5.11; N. 5.26.

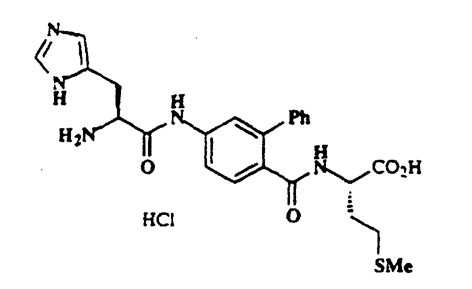

### Example 52

### [4-(L-histidyl)-2-phenylbenzoyl]methionine hydrochloride

### Example 52A

### [4-(bis-tert-butoxycarbonyl-L-histidyl)-2-phenylbenzoyl]methionine methyl ester

Bis-*tert*-butoxycarbonyl-L-His (1.78 g, 5.00 mmol) was added to a solution of [4-amino-2-phenylbenzoyl]methionine methyl ester (compound 8, 1.79 g, 5.00 mmol), 3-hydroxy-1,2,3-benzotriazin-4(3*H*)-one (HOOBT, 2.50 g, 15.0 mmol), 1-(3-dimethylaminopropyl)-3-ethylcarbodiimide (EDC, 2.93 g, 15.0 mmol), and *N*-methylmorpholine (NMM) in DMF (25 mL). The reaction mixture was stirred at ambient temperature for 17 hours and then was concentrated under reduced pressure (50 °C. 0.1 mm Hg) to provide an amber oil. The oil was dissolved in ethyl acetate (25 mL) and the solution was extracted with saturated aqueous NaHCO₃ (3 x 10 mL), followed by brine (10 mL). The combined aqueous layers were back-extracted with ethyl acetate (10 mL), and the combined organic portions were dried (MgSO₄) and then concentrated under reduced pressure to provide a yellow solid. Flash column chromatography (90:8:2 to 70:28:2 hexane-Ethyl acetate-Et₃N) afforded 1.32 g (38%) of [4-(bis-*tert*-butoxycarbonyl-L-histidyl)-2-phenylbenzoyl methionine methyl ester; ¹H NMR (CDCl₃) δ 1.41 (s, 9 H), 1.58 (s, 9 H), 1.61-1.78 (m. 1 H), 1.83-1.95 (m, 1 H), 1.98 (s, 3 H), 2.04-2.13 (comp, 2 H), 2.99 (dd, 1 H), 3.18 (dd. 1 H), 3.63 (s, 3 H), 4.50-4.92 (comp, 2 H), 5.84 (d, 1 H), 6.32 (d, 1 H), 7.21 (s, 1 H), 7.30-7.42 (comp, 5 H), 7.45 (m, 1 H), 7.68 (d, 1 H), 8.02 (s, 1 H), 9.63 (br, 1 H). LRMS (Cl): 696 (M+1)⁺.

### Example 52B

### [4-(L-histidyl)-2-phenylbenzoyl]methionine methyl ester hydrochloride

[4-(bis-*tert*-butoxycarbonyl-L-histidyl)-2-phenylbenzoyl methionine methyl ester (0.992 g, 1.42 mmol), prepared as in Example 52A, was dissolved in 4 M HCl/ dioxane (15 mL), upon which gas evolution was observed. The clear amber solution was stirred for 6 hours, during which time a white precipitate formed. The mixture was treated with ethyl ether and the precipitate was isolated by filtration to provide 0.779 g (100%) of [4-(L-histidyl)-2-phenylbenzoyl methionine methyl ester (believed to be the monohydrochloride salt); ¹H NMR (CD₃OD) δ 1.72-1.87 (m, 1 H), 1.95-2.03 (comp, 4 H), 2.08-2.28 (comp, 2 H), 3.38-3.60 (comp, 2 H), 3.67 (s, 3 H), 4.45-4.57 (comp, 2 H), 7.30-7.46 (comp, 6 H), 7.53 (d, 1 H), 7.69 (d, 1 H), 7.77 (app s, 1 H), 8.90 (s, 1 H). LRMS (Cl): 496 (M+1)⁺.

### Example 52C

### [4-(L-histidyl)-2-phenylbenzoyl]methionine hydrochloride

To a solution of [4-(L-histidyl)-2-phenylbenzoyl methionine methyl ester hydrochloride (98.9 mg, 0.200 mmol), prepared as in Example 52B, in THF/H₂O (4:1, 20 mL) was added LiOH•H₂O (68.5 mg, 1.60 mmol). The solution was stirred for 6 hours and then was treated with 1 M aqueous HCl (20 mL). The mixture was lyopholized to provide a white solid. Recrystallization from methanol afforded [4-(L-histidyl)-2-phenylbenzoyl]methionine hydrochloride (31 mg, 32%) as a white solid. ¹H NMR (D₂O) δ 1.68-1.82 (m, 1 H), 1.88-1.99 (comp, 2 H), 2.02 (s, 3 H), 2.00-2.12 (m, 1 H), 3.28-3.32 (m, 2 H), 3.49 (d, 2 H), 4.28-4.34 (m, 1 H), 4.44 (t, 1 H), 7.36-7.53 (comp, 8 H), 7.57 (m, 1 H), 8.67 (m, 1 H); LRMS (Cl): 482 (M+1)⁺, 701.

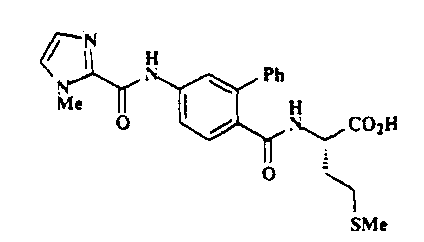

### Example 53

### [4-(1H-1-methylimidazol-2-ylcarboxyamino)-2-phenylbenzoyl]methionine

### Example 53A

### [1-ethoxy-2-(1-methyl-1H-imidazol-2-yl)ethenyl]carbonic acid ethyl ester

Triethylamine (10.2 g, 100.0 mmol) was added to a solution of 1,2-dimethylimidazole (2.45 g, 25.0 mmol) in acetonitrile (25 mL) at 0 °C. Ethyl chloroformate (6.15 g, 55.0 mmol) was added dropwise (1 drop/sec) and the reaction mixture was slowly warmed to ambient temperature. After 4 hours, the reaction mixture was concentrated under reduced pressure, and the residue was treated with 1:1 sat'd aqueous NaHCO₃/H₂O (25 mL). The mixture was extracted with dichloromethane (4 x 25 mL), and the organic extracts were rinsed with brine (25 mL), dried over MgSO₄, filtered, and concentrated under reduced pressure to provide an amber oil. Flash column chromatography (ethyl acetate:CH₂Cl₂:Methanol:HCO₂H ; 40:40:18:2 to 30:30:38:2) afforded 3.13 g (13%) of a 3:1 mixture of formic acid and the desired compound. ¹H NMR (CDCl₃) δ 1.2-1.6 (br, 6 H), 3.7-3.9 (br, 3 H), 4.2-4.6 (br, 4 H), 7.1 (br, 1 H), 7.3 (br, 1 H), 8.2 (br), 10.8 (br). LRMS (Cl): 241 (M+1)⁺, 169 (54318-148C+1)⁺.

### Example 53B

### (1H-1-methylimidazol-2-yl)acetic acid

The forrmic acid contaminated material prepared in Example 53A (3.13 g, ca 3.50 mmol) from above was dissolved in 3 M aqueous HBr (30 mL). The solution was heated to reflux for 30 hours, after which lyopholization afforded 0.783 g (*ca* 100%) of (1H-1-methylimidazol-2-yl)acetic acid. ¹H NMR (CD₃OD) δ 3.40 (s, 3 H), 5.38 (s, 3 H), 7.32-7.36 (comp, 2 H), 7.41-7.46 (comp, 3 H), 7.76-7.78 (m, 1 H), 7.78-7.80 (m, 1 H), 7.87 (d. *J*= 8.8 Hz, 1H). LRMS (Cl): 304 (M+18)⁺, 287 (M+1)⁺.

### Example 53C

### [4-(1H-1-methylimidazol-2-ylcarboxyamino)-2-phenylbenzoyl]methionine methyl ester

Triethylamine (1.23 g, 12.0 mmol) was added dropwise to a solution of [4-amino-2-phenylbenzoyl]methionine, methyl ester hydrochloride (Compound 8, 1.00 g, 2.03 mmol), (1H-1-methylimidazol-2-yl)acetic acid (0.783 g, 3.54 mmol), prepared as in Example 53B, 3-hydroxy-1,2,3-benzotriazin-4(3*H*)-one (1.06 g, 6.37 mmol), and 1-(3-dimethylaminopropyl)-3-ethylcarbodiimide (1.25 g, 6.37 mmol) in DMF (35 mL). The reaction mixture was stirred at ambient temperature for 16 hours and then concentrated under reduced pressure (50 °C, 0.1 mm Hg) to provide an amber oil. Flash column chromatography (ethyl acetate:CH₂Cl₂:Methanol:HCO₂H 30:30.38:2), tollowed by a second chromatography(ethyl acetate:HCO₂H 92:2) afforded 0.891 g (52%) of a ca 1:1 mixture of triethylamine hydrochloride and the desired compound. ¹H NMR (CDCl₃) δ 1.05-1.35 (t, 9 H of TEA•HCl), 1.80-7.92 (m, 1H), 1.94-2.08 (comp, 4 H), 2.12-2.22 (m, 1 H), 2.22-2.37 (m, 1 H), 3.10-3.24 (comp, 6 H of TEA•HCl + ?), 3.72 (s, 2 H), 3.91 (s, 3 H), 4.50-4.61 (m, 1 H), 7.33-7.45 (comp, 7 H), 7.46 (d, 1 H), 7.64-7.70 (comp, 2 H). Note ¹H spectrum poorly resolved such that assignments uncertain. LRMS (Cl): 481 (M+1)⁺.

### Example 53D

### [4-(1H-1-methylimidazol-2-ylcarboxyamino)-2-phenylbenzoyl]methionine

Lithium hydroxide hydrate (1.71 g, 40.0 mmol) was added to a solution of the triethylamine hydrochloride contaminated methyl ester prepared in Example 53C (0.891 g, 1.00 mmol) in THF/H₂O (4:1, 50 mL). The solution was stirred for 5 hours and then extracted with pentane (40 mL then 20 mL). The mixture was *carefully* acidified by the addition of 3 M aqueous HCl and then Iyopholized. Flash column chromatography (ethyl acetate:CH₂Cl₂:Methanol:HCO₂H (30:30:39:1)) followed by filtration of the concentrate through celite with methanol rinses afforded 0.080 g (*ca* 9%) of a 4:1 mixture of HCO₂H and [4-(1H-1-methylimidazol-2-ylcarboxyamino)-2-phenylbenzoyl]methionine. ¹H NMR (CD₃OD): δ 1.2-1.5 (small amount unidentified impurity), 1.8-1.9 (br m, 1H), 1.9-2.1 (br, comp 5 H), 2.1-2.3 (br m, 1 H), 3.6-4.0 (br m, 2 H), 4.0 (s, 3 H), 4.4-4.5 (br, m, 1 H), 7.3-7.5 (br comp, 6 H), 7.5-7.6 (br comp, 2 H), 7.6-7.8 (br m, 2 H). LRMS (Cl): 467 (M+1)⁺.

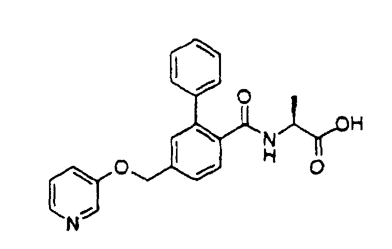

### Example 54

### [4-(3-pyridyloxymethyl)-2-phenylbenzoyl]alanine

### Example 54A

### 4-chloromethyl-2-phenylbenzoic acid methyl ester

To a solution of 10.5 g (43.3 mmol) of methyl 4-hydroxymethyl-2-phenylbenzoate in 50 mL of *N*,*N*-dimethylformamide was added 4.5 mL (62 mmol) of thionyl chloride, and 2.0 g (47 mmol) of lithium chloride. The reaction was complete upon dissolution of the lithium chloride. The solution was poured into 350 mL of water, then extracted with diethyl ether (3 x 100 mL). The combined diethyl ether layers were back extracted with water (2 x 100 mL), saturated aqueous sodium bicarbonate solution (1 x 100 mL). and brine (1 x 100 mL), dried over magnesium sulfate, filtered, and concentrated *in vacuo* to 11.1 g (98%) of 4-chloromethyl-2-phenylbenzoic acid methyl ester as a pale yellow oil.

### Example 54B

### 4-(3-pyridyloxymethyl)2-phenylbenzoic acid methyl ester

To a solution of 11.1 g (42.3 mmol) of 4-chloromethyl-2-phenylbenzoic acid methyl ester, prepared as in Example 54A, in 150 mL of toluene was added 1.7 g (6.4 mmol) of 18-Crown-6, and 8.40 g (63.1 mmol) of 3-hydroxypyridine, potassium salt. The reaction was stirred at ambient temperature for 20 minutes, then heated to reflux under N₂. After 3 hours, the mixture was poured into 100 mL of water. The layers were separated, then the aqueous layer was extracted with ethyl acetate (3 x 100 mL). The combined organic layers were back extracted with 2M aqueous NaOH (2 x 30 mL), brine (1 x 100 mL), dried over magnesium sulfate, filtered, and concentrated to an oil which slowly crystallized. The product was recrystallized from 50 mL of 2-propanol to give 6.78 g of a tan solid. The supematant was concentrated and purified *via* silica gel chromatography (50:50 hexanes: ethyl acetate) to give another 2.18 g of product, for a total yield of 8.96 g (66%). ¹H NMR (300 MHz, *d6*-DMSO) δ 3.60 (s, 3H), 5.33 (s, 2H), 7.28-7.54 (m, 8H), 7.57 (dd, *J*=1.5, 9.0 Hz, 1H), 7.78 (d, *J*=9 Hz, 1H), 8.18 (dd. *J*=1.0, 5.5 Hz, 1H), 8.38 (d, *J*=3.0 Hz, 1H).

### Example 54C

### 4-(3-pyridyloxymethyl)2-phenylbenzoic acid

To 2.60 g (8.14 mmol) of 4-(3-pyridyloxymethyl)2-phenylbenzoic acid methyl ester, prepared as in Example 54B, was added 15 mL of methanol, and a solution of 0.79 g (12 mmol) of 85% KOH in 3 mL of water. The mixture was stirred at reflux for 3 hours, then concentrated *in vacuo.* The residue was taken up in 5 mL of water and treated with 12 mL of 1M aqueous HCl. The precipitated product was filtered and washed with a small amount of water. The combined washings and filtrate were adjusted to pH 4 with 1M HCl, and additional precipitate was collected, then washed with water. The combined precipitates were dried *in vacuo* to give 4-(3-pyridyloxymethyl)2-phenylbenzoic acid (2.48 g, 99%) as an off-white powder. ¹H NMR (300 MHz, *d6*-DMSO) *δ* 5.31 (s, 2H), 7.31-7.56 (m, 9H), 7.76 (d, *J*=7.5 Hz, 1H), 8.19 (dd, *J*=1.0, 6.0 Hz, 1H), 8.39 (d, *J*=3.0 Hz, 1H), 12.8 (br s, 1H).

### Example 54D

### [4-(3-pyridyloxymethyl)-2-phenylbenzoyl]alanine methyl ester

To a solution of 100 mg (0.33 mmol) of 4-(3-pyridyloxymethyl)2-phenylbenzoic acid, L-alanine methyl ester hydrochloride (1.5 mmol), 69 mg (0.36 mmol) of 1-(3-dimethylaminopropyl)-3-ethylcarbodiimide hydrochloride, and 59 mg (0.36 mmol) of 3-hydroxy1,2,3-benzotriazin-4(3*H*)-one in 1 mL of *N*,*N*-dimethylformamide was added 5 drops of triethylamine. The mixture was stirred at ambient temperature for 24 hours, then poured into 10 mL of 0.6M aqueous sodium bicarbonate and extracted with ethyl acetate (3 x 5 mL). The combined ethyl acetate layers were back extracted with water (2 x 5 mL), then brine (1 x 5 mL), dried over magnesium sulfate, filtered, and concentrated *in vacuo.* The product was purified *via* chromatography over silica gel, eluting with an appropriate mixture of hexanes and ethyl acetate.

### Example 54E

### [4-(3-pyridyloxymethyl)-2-phenylbenzoyl]alanine

To approximately 0.3 mmol of [4-(3-pyridyloxymethyl)-2-phenylbenzoyl]alanine methyl ester, prepared as in Example 54D, was added 1 mL of 1.39M NaOH in 5:1 methanol: water. The mixture was heated to reflux for 20 minutes, then 1 mL of water, 1.4 mL of 1M aqueous HCl, and 5 mL of ethyl acetate were added sequentially. The biphasic mixture was stirred, then separated, and the aqueous layer was extracted with additional ethyl acetate (2 x 5 mL). The combined ethyl acetate layers were dried over magnesium sulfate, filtered, and concentrated to give 4-(3-pyridyloxymethyl)-2-phenylbenzoyl-L-alanine as a foam. ¹H NMR (300 MHz, *d6*-DMSO) δ 1.11 (d, *J*= 7.1 Hz, 3H), 4.14 (quintet, *J*= 7.3 Hz, 1H), 5.21 (s, 2H), 7.22-7.45 (m, 10H), 8.10 (d, *J*= 4.1 Hz, 1H), 8.29 (d, *J*= 1.7 Hz, 1H), 8.46 (d, *J*= 7.5 Hz, 1H), 12.42 (br s, 1H); MS (DCl) *m*/*e* 377 (M+H)⁺, 394 (M+NH₄)⁺. Anal calcd for C₂₂H₂₀N₂O₄•0.15HCl: C, 69.19; H, 5.32; N, 7.34. Found: C, 69.22; H, 5.01; N, 7.07.

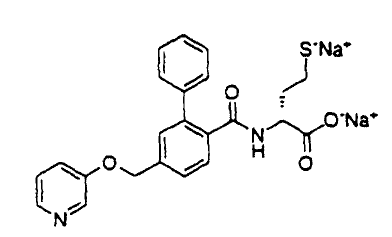

### Example 55

### [4-(3-pyridyloxymethyl)-2-phenylbenzoyl]cysteine disodium salt

### Example 55A

### [4-(3-pyridyloxymethyl)-2-phenylbenzoyl]homocysteine thiolactone

The desired compound was prepared according to the method of Example 54D, except substituting DL homocysteine thiolactone hydrochloride for L-alanine methyl ester hydrochloride.

### Example 55B

### [4-(3-pyridyloxymethyl)-2-phenylbenzoyl]cysteine disodium salt

To 51 mg (0.13 mmol) of [4-(3-pyridyloxymethyl)-2-phenylbenzoyl]homocysteine thiolactone, prepared as in Example 55A, was added 1 mL of 0.25 M NaOH in 9:1 methanol: water. The mixture was heated at reflux for 1 hour, then concentrated *in vacuo* to a white solid. ¹H NMR (300 MHz, *d6*-DMSO) δ 1.68-1.95 (m, 2H), 2.05-2.42 (m, 2H), 3.80-3.95 (m, 1H), 5.27 (s, 2H), 7.28-7.49 (m, 11H), 8.18 (dd, *J*=1.2, 4.6 Hz, 1H), 8.38 (d, J= 2.7 Hz, 1H); MS (DCI) *m*/*e* 405 (-H₂O). Anal calcd for C₂₃H₂₀N₂O₄SNa₂•1.35H₂O: C, 56.29; H, 4.66; N, 5.71. Found: C, 56.33; H, 4.84; N, 5.55.

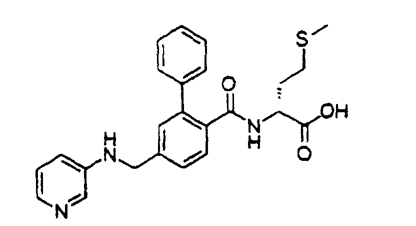

### Example 56

### [4-(3-pyridylaminomethyl)-2-phenylbenzoyl]methionine

### Example 56A

### [4-(3-pyridylamionomethyl)-2-phenylbenzoic acid methyl ester

To a solution of 1.72 g (7.16 mmol) of 4-methoxycarbonyl-3-phenylbenzaldehyde in 21 mL of methanol was added 7 mL of glacial acetic acid, the 875 mg (9.31 mmol) of 3-aminopyridine. The solution was stirred at ambient temperature for 1 hour, then cooled with an ice bath. Next, 750 mg (11.9 mmol) of sodium cyanoborohydride was added in small portions, keeping the ensuing bubbling under control. After 30 minutes, the ice bath was removed, and the reaction was stirred for 18 hours at ambient temperature. The reaction was concentrated *in vacuo,* then the residue was taken up in 75 mL of water and extracted with ethyl acetate (2 x 35 mL). The combined ethyl acetate layers were back extracted with saturated aqueous sodium bicarbonate solution (2 x 35 mL), then brine (1 x 35 mL), dried over magnesium sulfate, filtered, and concentrated to an oil. Purification *via* silica gel chromatography(ethyl acetate) provided 4-(3-pyridylamionomethyl)-2-phenylbenzoic acid methyl ester (2.10 g, 92%) as a colorless oil.

### Example 56B

### [4-(3-pyridylaminomethyl)-2-phenylbenzoyl]methionine

The desired compound was prepared according to the method of Example 54, steps C, D and E, except substituting 4-(3-pyridylamionomethyl)-2-phenylbenzoic acid methyl ester, prepared as in Example 56A, for [4-(3-pyridyloxymethyl)-2-phenylbenzoyl]alanine methyl ester, and substituting D-methionine methyl ester hydrochloride for L-alanine methyl ester hydrochloride. ¹H NMR (300 MHz, *d6*-DMSO) δ 1.75-1.91 (m, 2H), 1.98 (s, 3H), 2.16-2.27 (m, 2H), 4.27 (m, 1H), 4.39 (d, *J*= 6.4 Hz, 2H). 6.62 (t, *J*= 6.4 Hz, 1H), 6.90 (ddd, *J*= 1.4, 2.7, 8.5 Hz. 1H), 7.03 (dd, *J=* 4.6, 8.3 Hz, 1H). 7.30-7.41 (m, 8H), 7.40 (d, *J*= 4.1 Hz, 1H), 7.97 (d, *J*= 2.7 Hz, 1H), 8.50 (d. *J*= 7.8 Hz, 1H), 12.65 (br s, 1H); MS (DCl) *m*/*e* 436 (M+H)⁺. Anal calcd for C₂₄H₂₅N₃O₃S•0.90H₂O: C, 63.81; H, 5.98; N, 9.30. Found: C, 63.82; H, 5.61; N, 9.16.

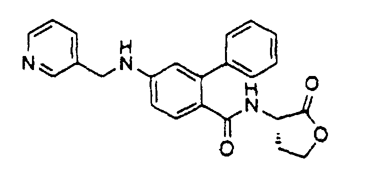

### Example 57

### [4-(3-Pyridylmethylamino)-2-phenylbenzoyl]homoserine lactone

### Example 57A

### 4-Amino-2-phenylbenzoic acid hydrochloride

4-Nitro-2-phenylbenzoic acid (10.5 g, 43.2 mmol) and tin (II) chloride dihydrate (34.1 g, 0.15 mol) were combined and refluxed in 250 mL ethyl acetate for 1 hour. An equal volume of water was added followed by solid NaHCO₃ to pH 8. The mixture was extracted with ethyl acetate. The combined ethyl acetate extracts were washed with brine and concentrated to a thick oil. The oil was diluted with ether and excess anhydrous HCl was added. The resulting precipitate was collected and dried to provide 4-Amino-2-phenylbenzoic acid hydrochloride (3.2 g). MS m/e 214 (M+H)⁺. ¹H NMR (d₆-DMSO, 300 MHz) δ 6.65 (d, *J*= 3 Hz, 1H), 6.79 (m, 1H), 7.20-7.72 (m, 6H).

### Example 57B

### 4-(3-Pyridylmethylamino)-2-phenylbenzoic acid acetic acid salt

3-Pyridinecarboxaldehyde (1.2 mL, 12.8 mmol) and 4-amino-2-phenylbenzoic acrd hydrochloride (3.2 g, 12.8 mmol), prepared in Example 57A, were dissolved in 100 mL. 1 % acetic acid in methanol. After stirring for 10 minutes, NaBH₃CN was added, and stirring was continued for 18 hours. The reaction was evaporated to dryness under reduced pressure and partitioned between ethyl acetate and water. The aqueous phase was extracted with ethyl acetate. The organic extracts were combined, washed with brine, and dried over Na₂SO₄ to give 4-(3-Pyridylmethylamino)-2-phenylbenzoic acid acetic acid salt (4.35 g, 90 %) of the title compound. MS m/e 305 (M+H)⁺. ¹H NMR (d₆-DMSO, 300 MHz) δ 4.41 (s, 2H), 6.45 (d, *J*= 3 Hz, 1H), 6.60 (m, 1H), 7.15-7.90 (m, 10H), 8.48 (m, 1H), 8.60 (m, 1H).

### Example 57C

### [4-(3-Pyridylmethylamino)-2-phenylbenzoyl]homoserine lactone

4-(3-Pyridylmethylamino)-2-phenylbenzoic acid acetic acid salt (0.20 g, 0.55 mmol), prepared as in Example 578, and L-homoserine lactone (0.19 g, 1.37 mmol), 1-(3-dimethylaminopropyl)-3-ethylcarbodiimide hydrochloride (0.27 g, 1.43 mmol), and 3-hydroxy1,2,3-benzotriazin-4(3*H*)-one (0.25 g, 1.65 mmol) were combined in 10 mL DMF. Triethylamine (1.65 mmol) was added, and the reaction was stirred overnight at room temperature. The reaction mixture was diluted with HCl (1 M, 10 mL) and extracted with ethyl acetate. Solid NaHCO₃ was added to pH 8. The aqueous was extraced with ethyl acetate. The combined extracts were washed with brine and dried over Na₂SO₄. Flash chromatography (2 % Methanol in ethyl acetate to 4 % Methanol in ethyl acetate) provided [4-(3-pyridylmethylamino)-2-phenylbenzoyl]homoserine lactone (140 mg). MS m/e 436 (M+H)⁺. ¹H NMR (CDCl₃, 300 MHz) δ 1.73 (m, 2H), 2.65 (m, 2H), 4.1-4.5 (m. 2H), 5.52 (m, 1H), 6.51 (d, *J*= 3 Hz, 1H), 6.65 (m, 1H), 7.25-7.44 (m, 8H), 7.49 (m, 2H), 8.56 (m, 1H), 8.63 (d. *J*= 3 Hz, 1H).

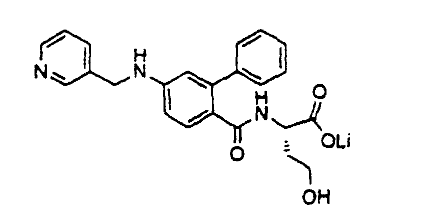

### Example 58

### Lithium 4-(3-pyridylmethylamino)-2-phenylbenzoyl-L-homoserinate

[4-(3-Pyridylmethylamino)-2-phenylbenzoyl]homoserine lactone (55 mg, 0.14 mmol), prepared as in Example 57, was dissolved in 1 mL of methanol and treated with aqueous 1.0 M LiOH (0.15 mmol). After 18 hours at ambient temperature, the mixture was evaporated to provide the title compound in quantitative yield. ¹H NMR (d₆-DMSO, 300 MHz) δ 1.43 (m, 1H), 1.60 (m, 1H), 3.59 (m, 1H), 4.1 (m, 1H), 4.38 (m, 2H), 6.33 (m, 1H), 6.6 (m, 2H), 6.83 (m, 1H), 7.22-7.38 (m, 8H), 7.74 (m, 2H), 8.45 (m, 1H), 8.59 (d, *J*= 3 Hz, 1H).

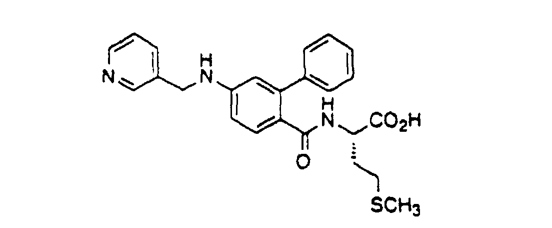

### Example 59

### [4-(3-pyridylmethylamino)-2-phenylbenzoyl]methionine

### Example 59A

### (4-Nitro-2-phenylbenzoyl)methionine methyl ester

4-Nitro-2-phenylbenzoic acid (50.0 g, 205 mmol) and 3-hydroxy1,2,3-benzotriazin-4(3*H*)-one (36.89 g, 226 mmol) were dissolved in 500 mL DMF. 1-(3-dimethylaminopropyl)-3-ethylcarbodiimide hydrochloride (47.3 g, 247 mmol) and methionine methyl ester hydrochloride (53.37 g, 267 mmol) were added followed by triethylamine (31.5 mL, 226 mmol). Additional triethylamine was added to raise the pH to 6-7. After 1 hour at ambient temperature, the reaction mixture was concentrated to 200 mL, diluted with 500 mL ethyl acetate, washed with 1 M HCl, 5 % NaHCO₃, and brine, and dried over Na₂SO₄ to provide (4-nitro-2-phenylbenzoyl)methionine methyl ester which was used directly without further purification. MS m/e 389 (M+H)⁺. ¹H NMR (CDCl₃, 300 MHz) δ 1.78 (m, 2H), 2.01 (s, 3H), 2.60 (m, 2H), 3.69 (s, 3H), 4.69 (m, 1H), 6.02 (d, *J*= 8 Hz, 1H), 7.48 (m, 5H), 7.85 (m, 1H), 8.27 (m, 2H).

### Example 59B

### (4-Amino-2-phenylbenzoyl)methionine methyl ester hydrochloride

Tin(II) dichloride dihydrate (157 g, 696 mmol) was added to a solution of (4-nitro-2-phenylbenzoyl)methionine methyl ester (67.9 g, 175 mmol) in 500 mL ethyl acetate and the reaction mixture was heated at reflux for 1 hour. The reaction mixture was cooled to ambient temperature and stirring was continuted for 18 hours. The reaction mixture was concentrated to 200 mL, and 500 mL H₂O was added. Solid NaHCO₃ was added to pH 8 before extracting with ethyl acetate. The ethyl acetate extract were washed with 5 % NaHCO₃ and brine, dried over Na₂SO₄, and concentrated. The residue was dissolved in ether with a minimum of ethyl acetate added to keep the material in solution and treated with anhydrous HCl. The solid was collected and washed with ether to provide (4-amino-2-phenylbenzoyl)methionine methyl ester hydrochloride in 83 % yield. MS m/e 359 (M+H)⁺. ¹H NMR (d₆-DMSO, 300 MHz) δ 1.83 (m, 2H), 1.99 (s, 3H), 2.23 (m, 2H), 3.63 (s, 3H), 4.33 (m, 1H), 7.03 (m, 2H), 7.35 (m, 6H), 7.48 (d, *J*= 8 Hz, 1H).

### Example 59C

### [4-(3-Pyridylmethylamino)-2-phenylbenzoyl]methionine methyl ester

(4-Amino-2-phenylbenzoyl)methionine methyl ester hydrochloride (5.0 g, 12.7 mmol), prepared as in Example 59B, and 3-pyridinecarboxaldehyde (1.25 mL, 13.3 mmol) were dissolved in 100 mL 1 % acetic acid in methanol. After 10 minutes, sodium cyanoborohydride (0.95 g, 15.9 mmol) was added. After stirring at room temperature 18 hours, the reaction mixture was evaporated and partitioned between 5 % NaHCO₃ and ethyl acetate. The organic layer was washed with 5 % NaHCO₃ and brine, dried over Na₂SO₄, and evaporated to provide [4-(3-pyridylmethylamino)-2-phenylbenzoyl]methionine methyl ester which was used without further purification. MS m/e 450 (M+H)⁺. ¹H NMR (CDCl₃, 300 MHz) δ 1.65 (m, 2H), 1.87 (m, 2H), 2.00 (s, 3H), 3.63 (s, 4H), 4.42 (s, 2H), 4.61 (m, 1H), 5.69 (d, *J*= 7 Hz, 1H), 6.50 (d, *J*= 3 Hz, 1H), 6.63 (m, 1H), 7.45 (m, 6H), 7.68 (m, 2H), 8.55 (m, 1H), 8.62 (d, *J*= 3 Hz, 1H).

### Example 59D

### [4-(3-Pyridylmethylamino-2-phenylbenzoyl]methionine

Excess LiOH (3 M) was added to a solution in methanol of [4-(3-pyridylmethylamino)-2-phenylbenzoyl]methionine methyl ester (5.69 g, 12.7 mmol), prepared as in Example 59C and the reaction mixture was stirred at ambient temperature for 72 hours. The reaction mixture was concentrated and partitioned between ether and water. The aqueous layer was washed with ether, acidified to pH 4∼5 with HCl, and extracted with ethyl acetate. The combined ethyl acetate extracts were washed with brine and dried over Na₂SO₄ to give the title compound in 98 % yield. MS m/e 436 (M+H)⁺. ¹H NMR (d₆-DMSO, 300 MHz) δ 1.91 (m, 2H), 1.99 (s, 3H), 2.22 (m, 2H), 3.36 (bs, 1H), 4.11 (m, 1H), 4.40 (s, 2H), 6.57 (m, 2H), 6.75 (bs, 1H), 7.3 (m, 6H), 7.79 (m, 1H), 7.99 (m, 1H), 8.46 (m, 1H), 8.30 (d, J= 3 Hz, 1H), 12.48 (bs, 1H).

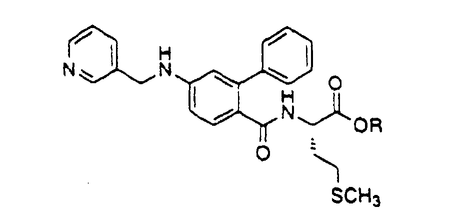

### Examples 60-62

### Example 60

### [4-(3-pyridylmethylamino)-2-phenylbenzoyl]methionine isoamyl ester

[4-(3-pyridylmethylamino)-2-phenylbenzoyl]methionme (200 mg, 0.46 mmol), prepared as in Example 59, carbonyldiimidazole (74 mg, 0.46 mmol), and isoamyl alcohol (40 mg, 0.46 mmol) were combined in 10 mL THF. After 2 hours at room temperature, sodium ethoxide (2.68 M in ethanol, 0.02 mmol) was added. After an additional 18 hours, the mixture was evaporated to dryness, partitioned between ethyl acetate and water, washed with water and brine, dried over Na₂SO₄, and chromatographed (Ethyl acetate) to give [4-(3-pyridylmethylamino)-2-phenylbenzoyl-L-methionine isoamyl ester (90 mg). MS m/e 506 (M+H)⁺. ¹H NMR (CDCl₃, 300 MHz) δ 0.90 (d, *J*= 7 Hz, 6H), 1.48 (m, 2H), 1.64 (m, 4H), 1.88 (m, 1H), 2.01 (s, 3H), 2.11 (t, *J*= 7 Hz, 2H), 4.08 (m, 2H), 4.42 (s, 2H), 4.60 (m, 1H), 5.87 (d, *J*= 8 Hz, 1H), 6.51 (d, *J*= 3 Hz, 1H), 6.63 (m, 1H), 7.28-7.44 (m, 5H), 7.68 (m, 2H), 8.55 (m, 1H), 8.63 (s, 1H).

### Example 61

### [4-(3-pyridylmethylamino)-2-phenylbenzoyl]methionine 1-adamantylethyl ester

The desired compound was prepared according to the method of Example 60, except substituting 2-adamantaneethanol for isoamyl alcohol. MS m/e 598 (M+H)⁺. ¹H NMR (CDCl₃, 300 MHz) δ 1.6 (m, 17H), 1.94 (m, 2H), 2.01 (s, 3H), 2.10 (m, 2H), 4.09 (m, 2H), 4.40 (m, 2H), 4.59 (m, 1H), 5.72 (d, *J*= 7 Hz, 1H), 6.51 (d, *J*= 3 Hz, 1H), 6.63 (m, 1H), 7.3 (m, 8H), 7.68 (m, 1H), 8.60 (m, 2H).

### Example 62

### [4-(3-pyridylmethylamino)-2-phenylbenzoyl]methionine octyl ester

The desired compound was prepared according to the method of Example 60. except substituting octanol for isoamyl alcohol. MS m/e 548 (M+H)⁺. ¹H NMR (CDCl₃, 300 MHz) δ 0.88 (t, *J*= 7 Hz, 3H), 1.28 (m, 10H), 1.6 (m, 2H), 2.01 (s, 3H), 2.10 (m, 2H), 4.02 (m, 2H), 4.4 (m, 2H), 4.61 (m, 1H), 5.71 (d, J=7 Hz, 1H), 6.50 (d. J=3 Hz, 1H), 6.63 (m, 1H), 7.39 (m, 9H), 7.68 (m, 2H), 8.55(m, 1H), 8.63 (m, 1H).
The next example is Example 66.

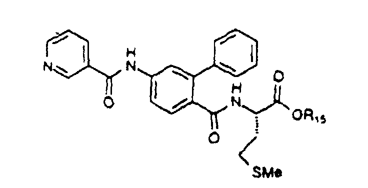

### Examples 66-67

### Example 66

### [4-(3-pyridylcarbonylamino)-2-phenylbenzoyl]methionine methyl ester

Nicotinic acid (345mg, 2.8 mmol) was suspended in 10 mL of dichloromethane and oxalyl chloride (2.8 mL of a 2.0 M soln in methylene chloride) was added by syringe followed by one drop of DMF. The reaction mixture was stirred at 25 °C for 2 hours and then was evaporated and azeotroped with toluene. The resulting acid chloride was then dissoived in dichloromethane and a solution of (4-amino-2-phenylbenzoyl)methionine methyl ester (669mg, 1.87 mmol), prepared as in Example 59B, in 5 mL of dichloromethane was added followed by 4 mL of saturated aqeuous NaHCO₃ and the reaction was stirred at 25 °C for 3 hours. The layers were separated and the organic layer was dried over Na₂SO₄, filtered and evaporated to give 830mg (96%) of [4-(3-pyridylcarbonylamino)-2-phenylbenzoyl]methionine methyl ester as an oil. ¹H NMR (300 mHz, CDCl₃) δ 9.2 (d, 1H), 8.76 (d, 1H), 8.42 (bs, 1H), 8.2 (dt, 1H), 7.72-7.65 (m, 2H), 7.6 (dd, 1H), 7.45 - 7.35 (m, 5H), 6.02 (bd, 1H), 4.65 (dq, 1H), 3.68 (s. 3H), 2.20 (t, 2H). 2.01 (s, 3H), 1.98 - 1.88 (m, 1H), 1.80 - 1.78 (m, 1H). CIMS 464 (M+H)⁺.

### Example 67

### [4-(3-pyridylcarbonylamino)-2-phenylbenzoyl]methionine hydrochloride

[4-(3-pyridylcarbonylamino)-2-phenylbenzoyl]methionine methyl ester (830mg, 1.79 mmol), prepared as in Example 66, was dissolved in 8 mL THF and cooled to 0 °C. LiOH monohydrate (226mg, 5.38 mmol) was added followed by 2 mL of H₂O. The reaction was complete in 2 hours. The solvents were evaporated and the residue was acidified to pH = 3 with 1 N HCl. The resulting precipitate was taken up in ethyl acetate and the solution was washed with water, dried over Na₂SO₄ and evaporated. The residue was crystallized from hot ethanol to give 281 mg (32%) of [4-(3-pyridylcarbonylamino)-2- phenylbenzoylmethionine hydrochloride as a white crystalline solid. ¹H NMR (300 mHz, CD₃OD) δ 9.4 (d, 1H), 9.1 (d, 1H), 9.0 (d, 1H), 8.2 (dd, 1H), 7.85-7.80 (m, 2H), 7.58 (dd, 1H), 7.45 - 7.32 (m, 5H), 4.52 - 4.45 (m, 1H), 2.20 - 2.02 (m, 2H), 2.00 (s, 3H1.90- 1.80 (m, 2H), 1.15 (t, 1H). CIMS 450 (M+H)⁺.

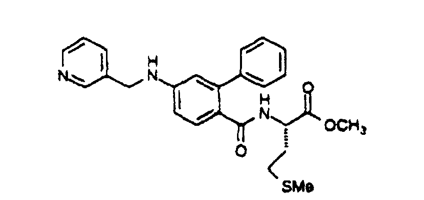

### Example 68

### [4-(3-pyridylmethylamino)-2-phenylbenzoyl]methionine methyl ester

(4-Amino-2-phenylbenzoyl)methionine methyl ester (1.15g, 3.21 mmol), prepared as in Example 59B, and 3-pyridine carboxaldehyde (361mg, 3.37 mmol) were combined in 15 mL methanol and sodium cyanoborohydride (302 mg, 4.81 mmol) was added followed by crushed molecular sieves. The reaction was adjusted to pH = 6 with acetic acid and stirred at 25 °C for 3 hours. The reaction was concentrated and transferred directly to a column of silica gel and purified by flash chromatography (5%methanol-ethyl acetate) to give [4-(3-pyridylmethylamino)-2-phenylbenzoyl]methionine methyl ester (1.38 g, 95%) as an oil that solidified after standing. ¹H NMR (300 mHz, CDCl₃) δ 8.6 (d, 1H). 8.52 (dd, 1H), 7.72 - 7.65 (m, 2H), 7.45 - 7.30 (m, 6H), 6.62 (dd, 1H), 6.48 (d, 1H), 5.72 (bd, 1H), 4.64 (dq, 1H), 4.42 (bs, 2H), 3.64 (s, 3H), 2.25 - 2.05 (m, 3H), 2.00 (s, 3H), 1.95 - 1.80 (m, 1H), 1.72 - 1.60 (m, 1H); CIMS MH⁺ 450.

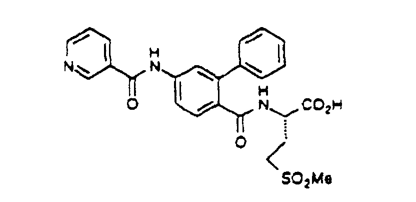

### Example 69

### [4-(3-pyridylcarbonylamino)-2-phenylbenzoyl]-2-amino-4-(methylsulfonyl)butanoic acid

### Example 69A

### [4-(3-pyridylcarbonylamino)-2-phenylbenzoyl]-2-amino-4-(methylsulfonyl)butanoic acid methyl ester

[4-Amino-2-phenylbenzoyl]methionine methyl ester (46 mg, 0.0.092 mmol), prepared as in Example 68, was protected as the HCl pyridinium salt and was dissolved in 5 mL of CH₂Cl₂, cooled to -78 °C and treated with a solution of *m*-chloroperbenzoic acid (44mg, 0.184 mmol) in 2 mL of CH₂Cl₂ and warmed to 0 °C. After 0.5 hours, the reaction was quenched with dimethyl sulfide and evaporated. Purification by flash chromatography (5% methanol-ethyl acetate) gave [4-(3-pyridylcarbonylamino)-2-phenylbenzoyl]-2-amino-4-(methylsulfonyl)butanoic acid methyl ester (43 mg, 88%) as a white solid.

### Example 69B

### [4-(3-pyridylcarbonylamino)-2-phenylbenzoyl]-2-amino-4-(methylsulfonyl)butanoic acid

The [4-(3-pyridylcarbonylamino)-2-phenylbenzoyl]-2-amino-4-(methylsulfonyl)butanoic acid methyl ester prepared in Example 69A (43mg, 0.081 mmol) was dissolved in 3 mL of THF and a solution of LiOH monohydrate was in 1 mL of H₂O was added. The reaction mixture was stirred at 25 °C for 1 hour and then was evaporated. Formic acid (1 mL) was added to acidify to pH = 3. The reaction was then evaporated once again and 1 mL of H₂O was added along with 5 mL of ethyl acetate to dissolve the mixture. The ethyl acetate layer was dried over Na₂SO₄, filtered and evaporated and the residue was lyophilized from acetonitrile-H₂O to give [4-(3-pyridylcarbonylamino)-2-phenylbenzoyl]-2-amino-4-(methylsulfonyl)butanoic acid (36mg, 88%) as a white solid. ¹H NMR (300 mHz, CD₃OD) δ 9.12 (d, 1H), 8.70 (d, 1H), 8.42 (dt, 1H), 7.85 - 7.80 (m, 2H), 7.65 - 7.40 (m, 8H), 4.50 (m, 1H), 2.90 (s, 3H), 2.88 - 2.80 (m, 1H), 2.70 2.58 (m, 1H), 2.35 - 2.20 (m, 1H), 2.10 - 1.95 (m, 1H). HRMS calcd C₂₄H₂₃N₃SO₆ MH⁺ 482.1386, found 482.1373.

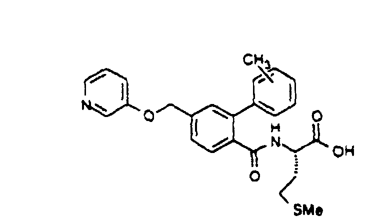

### Examples 70-72

### Example 70

### [4-(3-pyridyloxymethyl)-2-(2-methylphenyl)benzoyl]methionine methyl ester

### Example 70A

### 2-(triluoromethanesulfonyloxy)-4-methylbenzoic acid methyl ester

To a -10 °C solution of 4-methylsalicylic acid methyl ester (22.46g, 0.135 mol) in 100 mL of pyridine was added triflic anhydride (45.8g, 0.162 mol) dropwise by addition funnel while keeping the temperature below 0 °C. The reaction mixture was then warmed to ambient temperature and after 12 hours was poured over a mixture of 100 mL conc. HCl / 300g ice in a large erylenmeyer flask. After the ice melted, the mixture was transferred to a separatory funnel and the aqueous layer was extracted with ethyl acetate (3x100 mL). The ethyl acetate layers were combined and washed with 1 N HCl, then sat'd aqueous NaHCO₃, then brine and then filtered and evaporated to give 35.66g (88%) of 2-(triluoromethanesulfonyloxy)-4-methylbenzoic acid methyl ester as a yellow oil.

### Example 70B

### 2-(trifluoromethanesulfonyloxy)-4-bromomethylbenzoic acid methyl ester

To a stirred solution of 2-(trifluoromethanesulfonyloxy)-4-methylbenzoic acid methyl ester (19.6g, 65.8 mmol), prepared as in Example 70A, in 250 mL CCl₄ was added N-bromosuccinimide (12.29g, 69.1 mmol) followed by 2,2'-azobisisobutyronitrile (108 mg, 0.658 mmol) and the reaction was heated to reflux. After 16 hours, the reaction was evaporated and the residue was purified by flash chromatography over silica gel (10% ethyl acetate-hexanes) to give 19.9 g (80%) of 2-(trifluoromethanesulfonyloxy)-4-bromomethylbenzoic acid methyl ester as a yellow oil.

### Example 70C

### 4-(3-pyridyloxymethyl)-2-(trifluoromethanesulfonyloxy)benzoic acid methyl ester

A solution of 2-(trifluoromethanesulfonyloxy)-4-bromomethylbenzoic acid methyl ester (2.97 g, 7.87 mmol), prepared as in Example 70B, in 20 mL of CH₂Cl₂ was combined with a solution of the potassium alkoxide of 3-OH pyridine (1.57g, 11.8 mmol) in 20 mL of H₂O. Tetrabutylammonium bromide (3.80g, 11.8 mmol) was added and the reaction was stirred vigorously at 25 °C for 1.5 hours The reaction was poured into a separatory funnel and the layers were separated. The aqueous layer was washed with CH₂Cl₂ (2x50 mL) and the CH₂Cl₂ layer was washed twice with water. The organic layers were combined and dried over Na₂SO₄, filtered and evaporated to an oil and purifed by flash chromatography over silica gel to give 4-(3-pyridyloxymethyl)-2-(triluoromethanesulfonyloxy)benzoic acid methyl ester (861 mg, 28%) as a light brown oil.

### Example 70D

### 4-(3-pyridyloxymethyl)-2-(2-methylphenyl)benzoic acid methyl ester

To a solution of 4-(3-pyridyloxymethyl)-2-(trifluoromethanesulfonyloxy)benzoic acid methyl ester (216mg, 0.55 mmol), prepared as in Example 70C, in 4 mL of DMF at 25 °C was added PdCl₂(PPh₃)₂ (38mg, 0.055 mmol, 10 mol%) followed by 2-tolyl boronic acid (113mg, 0.83 mmol) and Cs₂CO₃ (270mg, 0.83 mmol) and the reaction was heated to 80 °C for 12 hours. The reaction was then cooled to ambient temperature, taken up in 50 mL ethyl acetate, and washed with H₂O (5 x 10 mL). The orgarinc phase was dried over Na₂SO₄, filtered and evaporated to an oil. Purification by radial chromatography using a gradient of 25% ethyl acetate-hexanes to 75% ethyl acetate-hexanes gave 4-(3-pyridyloxymethyl)-2-(2-methylphenyl)benzoic acid methyl ester (178 mg, 97%) as an oil.

### Example 70E

### 4-(3-pyridyloxymethyl)-2-(2-methylphenyl)benzoic acid

The 4-(3-pyridyloxymethyl)-2-(2-methylphenyl)benzoic acid methyl ester prepared in Example 70D (160 mg, 0.48 mmol) was dissolved in 5 mL of methanol and 1 mL of saturated aqueous LiOH was added. The reaction was heated to reflux for 1 hour. The reaction was then evaporated and 1 mL of formic acid was added to acidify the crude product to pH =3. The reaction was evaporated again to remove formic acid and 5 mL of ethyl acetate and 1 mL of H₂O were added to completely solubilize the reaction mixture. The aqueous layer was extracted with ethyl acetate (3x5 mL) and all the ethyl acetate layers were combined and dried over Na₂SO₄, filtered and evaporated to give 4-(3-pyridyloxymethyl)-2-(2-methylphenyl)benzoic acid (131mg, 86%) as an oil.

### Example 70F

### [4-(3-pyridyloxymethyl)-2-(2-methylphenyl)benzoyl]methionine methyl ester

The 4-(3-pyridyloxymethyl)-2-(2-methylphenyl)benzoic acid prepared in Example 70E (153mg, 0.48 mmol) was dissolved in 2 mL of DMF and 3-hydroxy1,2,3-benzotriazin-4(3*H*)-one (39mg, 0.24 mmol) was added followed by methionine methyl ester HCl (48mg, 0.24 mmol), (3-dimethylaminopropyl)-3-ethylcarbodiimide hydrochloride (46mg, 0.24 mmol) and triethylamine (0.03mL, 0.32 mmol) and the reaction mixture was stirred for 16 hours at 25 °C. The reaction mixture was taken up in ethyl acetate and washed three times with water and three times with brine. The ethyl acetate layer was dried over Na₂SO₄, filtered and evaporated to an oil. Purification by radial chromatography (2-8 %methanol-chloroform gradient with 0.25% NH₄OH) gave 4-(3-pyridyloxymethyl)-2-(2-methylphenyl)benzoyl]methionine methyl ester (72mg, 32%) as an oil.

### Example 70G

### [4-(3-pyridyloxymethyl)-2-(2-methylphenyl)benzoyl]methionine

The [4-(3-pyridyloxymethyl)-2-(2-methylphenyl)benzoyl]methionine methyl ester prepared in Example 70F (72mg, 0.15 mmol) was dissolved in 3 mL of THF and 1 mL of saturated aqueous LiOH was added. The reaction mixture was stirred at room temperature for 1 hour. The reaction was thoroughly evaporated and formic acid was added until pH = 3 was obtained at which time the reaction was evaporated to dryness and 10 mL of ethyl acetate was added followed by a minimum quantity of H₂O (~1 mL) to completely solubilize the free acid and the water soluble salts, respectively. The layers were separated and the aqueous layer was extracted with ethyl acetate (3x5 mL). The ethyl acetate layers were combined, dried over Na₂SO₄, filtered and evaporated to give 58mg (84%) of [4-(3-pyridyloxymethyl)-2-(2-methylphenyl)benzoyl]methionine as an amorphous solid. ¹H NMR (300 mHz, CD₃OD) δ 8.30 (d, 1H), 8.15 (dd, 1H), 7.68 (bd, 1H), 7.58 - 7.48 (m, 2H), 7.40 - 7.30 (m, 2H), 7.26 - 7.16 (m, 4H), 5.25 (s, 2H), 4.50 - 4.40 (m, 1H), 2.20 - 2.02 (m, 5H), 2.00 (s, 3H), 2.00 - 1.90 (m, 1H), 1.80 - 1.68 (m, 1H) CIMS MH⁺ 451.

### Example 71

### [4-(3-pyridyloxymethyl)-2-(3-methylphenyl)benzoyl]methionine

The desired compound was prepared according to the method of Example 70, except substituting 3-methylphenyl boronic acid for 2-methylphenyl boronic acid. ¹H NMR (300 mHz, CD₃OD) δ 8.30 (d, 1H), 8.15 (d, 1H), 7.68 - 7.48 (m, 6H), 7.40 - 7.16 (m, 4H), 5.25 (s, 2H), 4.50 - 4.40 (m, 1 H), 2.40 (s, 3H), 2.18 - 1.75 (m, 7H); CIMS MH⁺ 451.

### Example 72

### [4-(3-pyridyloxymethyl)-2-(4-methylphenyl)benzoyl]methionine

The desired compound was prepared according to the method of Example 70, except substituting 4-methylphenyl boronic acid for 2-methylphenyl boronic acid. ¹H NMR (300 mHz, CD₃OD) δ 8.30 (d, 1H), 8.15 (d, 1H), 7.58 - 7.44 (m, 4H), 7.40 - 7.28 (m, 3H), 7.24 - 7.10 (m, 3H), 5.25 (s, 2H), 4.42 (dd, 1H), 2.10 - 1.90 (m, 6H), 1.84 - 1.70 (m, 1H). CIMS MH⁺ 451.

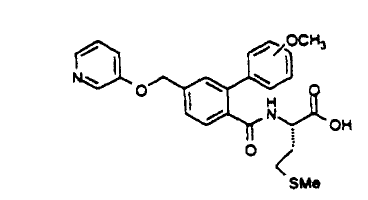

### Examples 73-75

### Example 73

### [4-(3-pyridyloxymethyl)-2-(2-methoxyphenyl)benzoyl]methionine

The desired compound was prepared according to the method of Example 70, except substituting 2-methoxyphenyl boronic acid for 2-methylphenyl boronic acid. ¹H NMR (300 mHz, CD₃OD) δ 8.30 (d, 1H), 8.15 (d, 1H), 7.68 (bd, 1H), 7.54 - 7.50 (m, 2H), 7.38 - 7.32 (m, 3H), 7.22 (dd, 1H), 7.04 - 6.98 (m, 2H), 5.25 (s, 2H), 4.42 (dd, 1H), 3.74 (s, 3H), 2.16 - 2.08 (m, 2H), 2.00 (s, 3H), 1.98 - 1.86 (m, 1H), 1.78 - 1.64 (m, 1H). CIMS MH⁺ 467.

### Example 74

### [4-(3-pyridyloxymethyl)-2-(3-methoxyphenyl)benzoyl]methionine

The desired compound was prepared according to the method of Example 70, except substituting 3-methoxyphenyl boronic acid for 2-methylphenyl boronic acid. ¹H NMR (300 mHz, CD₃OD) δ 8.34 (s, 1H), 8.15 (d, 1H), 7.60 - 7.54 (m, 4H), 7.38 - 7.24 (m, 3H), 7.02 - 6.90 (m, 3H), 5.25 (s, 2H), 4.44 (dd, 1H), 3.82 (s, 3H), 2.18 - 1.90 (m, 6H), 1.92 - 1.82 (m, 1H): CIMS MH⁺ 467.

### Example 75

### [4-(3-pyridyloxymethyl)-2-(4-methoxyphenyl)benzoyl]methionine

The desired compound was prepared according to the method of Example 70, except substituting 4-methoxyphenyl boronic acid for 2-methylphenyl boronic acid. ¹H NMR (300 mHz, CD₃OD) δ 8.34 (s, 1H), 8.15 (bs, 1H), 7.72 - 742 (m, 6H), 7.40 - 7.35 (m, 2H), 6.96 - 6.90 (m, 2H)5.25 (s, 2H), 4.44 (dd, 1H), 3.84 (s, 3H), 2.20 - 1.90 (m, 6H), 1.88 - 1.76 (m, 1H); CIMS MH⁺ 467.

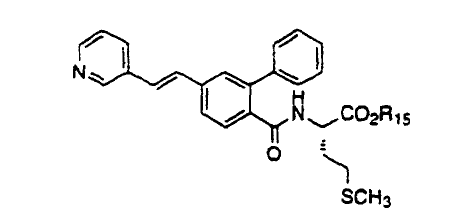

### Examples 76-77

### Example 76

### {4-[2-(pyrid-3-yl)ethenyl]-2-phenylbenzoyl}methionine methyl ester

### Example 76A

### 2-phenyl-4-nitrobenzoic acid methyl ester

A mixture of methyl 2-chloro-4-nitrobenzoate (44.2 g, 205 mmol), phenylboronic acid (27.5 g, 226 mmol), sodium carbonate (2.0 M in water, 123 mL, 246 mmol), and bis(triphenylphosphine) palladium(II) chloride (2.8 g, 4 mmol) in dioxane (300 mL) was degassed by nitrogen, and heated at 90-95 °C for 20 hours. The reaction mixture was diluted with ether (500 mL) and ethyl actate (500 mL), washed with water (2 times, 200 mL each) and brine, dried over anhydrous magnesium sulfate, filtered, and concentrated *in vacuo*. The residue was recrystalized from hexane-ethyl acetate (1/1) to give 2-phenyl-4-nitrobenzoic acid methyl ester as a white solid (43.3 g). The mother liquid from the recrystalization was concentrated *in vacuo*, and the residue was purified by column chromataography (80:15:5 hexane-chloroform-ethyl acetate) to yield an additional 5.2 g of the desired compound (total yield 48.5 g, 92%) ¹H NMR (300 MHz, CDCl₃) δ 8.25 (d. 1H), 8.24 (dd, 1H), 7.94 (dd, 1H), 7.44 (m, 3H) 7.35 (m, 2H), 3.67 (s, 3 H).

### Example 76B

### 2-phenyl-4-aminobenzoic acid methyl ester

A mixture of the 2-phenyl-4-nitrobenzoic acid methyl ester prepared in Example 76A (48.4 g, 188 mmol), palladium (10%) on carbon (2.1 g), and ammonium formate (59.4 g, 941 mmol) in methanol (500 mL) was retluxed for 3 hours. The solvent was removed *in vacuo,* and the residue was desolved in a miminum amount of hot methanol (about 30 mL). To this solution was added chloroform and ether (1/1 ratio, 400 mL), and the mixture was filtered through a plug of silica gel (80 g) and rinsed with chloroform. The filtrate was concentrated *in vacuo* to give pure 2-phenyl-4-aminobenzoic acid methyl ester (42.4 g, 99%). ¹H NMR (300 MHz. CDCl₃) δ 7.80 (d, 1H), 7.40-7.25 (m. 5H), 6.65 (dd, 1H), 6.57 (d, 1H), 3.60 (s 3H).

### Example 76C

### 4-iodo-2-phenylbenzoic acid methyl ester

To a 0 °C suspension of the 2-phenyl-4-aminobenzoic acid methyl ester prepared in Example 76B (4.54 g, 20 mmol) in 6.0 N HCl (20 mL) and acetone (10 mL) was added dropwise a solution of sodium nitrite (1.66 g, 24 mmol) in a minimum amount of water. After 30 minutes, potassium iodide (6.64 g, 40 mmol) in a minimum amount of water was added dropwise to the reaction mixture. The intemal temperature of the reaction mixture was maintained under 5 °C for both additions. The reaction mixture was then stirred at ambient temperature for one hour. The reaction mixture was diluted with ether (200 mL), washed with water, sodium bisulfite (10% aqueous solution), water and brine, dried over anhydrous magnesium sulfate, filtered, and concentrated *in vacuo*. The residue was purified by column chromatography (8% ethyl acetate-hexane) to give 4-iodo-2-phenylbenzoic acid methyl ester (3.98 g, 59%). ¹H NMR (300 MHz, CDCl₃) δ 7.77 (m, 2H), 7.55 (d, 1H), 7.38 (m, 3H), 7.27 (m, 2H), 3.63 (s, 3H). Ms (Cl+): 356 (M+NH₄)⁺.

### Example 76D

### (4-iodo-2-phenylbenzoyl)methionine methyl ester

A mixture of the 4-iodo-2-phenylbenzoic acid methyl ester prepared in Example 76C (2.77 g, 8.20 mmol) in aqueous saturated lithium hydroxide (3 mL) and methanol (10 mL) was heated at 60 °C for 12 hours. The mixture was then acidified with concentrated HCl to pH about 2, and extracted twice with ethyl acetate (50 mL each). The combine extracts was washed with brine, dried over anhydrous magnesium sulfate, filtered, and concentrated *in vacuo*. The residue was suspended in dichloromethane (10 mL), and oxalyl chloride (2.0 M in dichloromethane, 6.2 mL)was added, followed by a small drop of DMF. The mixture was stirred at room temperature for 1 hour, and then was concentrated *in vacuo*, followed by further drying under high vacuum for 10 minutes. To the residue was added dichloromethane (20 mL), L-methionine methyl ester hydrochloride (1.64 g, 8.05 mmol) and triethylamine (3.4 mL, 24.6 mmol) The reaction mixture was stirred at room temperature for 12 hours. The mixture was diluted with ether (50 mL), filtered through silica gel (30 g), and concentrated *in vacuo*. The crude product was purified by column chromatography (40:40:20 hexane-chloroform -ether) to give (4-iodo-2-phenylbenzoyl)methionine methyl ester (3.46 g, 90%). ¹H NMR (300 MHz, CDCl₃) δ 7.76 (m, 2H), 7.42 (m, 6H), 5.88 (br d, 1H), 4.65 (m, 1H), 3.68 (s, 3H), 2.05 (m, 2H), 2.00 (s, 3H), 1.90 (m, 1H), 1.73 (m, 1H).

### Example 76E

### 3-Vinylpyridine

To a slurry of methyltriphenylphosphine chloride (18.0 g, 50.5 mmol) in THF (20 mL) was added slowly sodium bis(trimethylsilyl)amide (1.0 M solution in THF, 50 mL). After the mixture was stirred at room temperature for 30 minutes, the reaction was cooled to 0 °C, and 3-pyridinecarboxaldehyde (4.72 mL, 50.0 mmol) was added slowly to the mixture. After 30 minutes, the reaction mixture was diluted with ether (100 mL) and filtered through silica gel (100 g), rinsed with ether, and concentrated *in vacuo*. The resulting liquid was diluted with 1:1 hexane-ether (50 mL), and was again filtered through silica gel (50 g), rinsed with 70:30 ether-hexane and concentrated *in vacuo* to give3-vinylpyridine (4.31 g, 82%). ¹H NMR (300 MHz, CDCl₃) δ 8.63 (d, 1H), 8.50 (dd, 1H), 7.74 (m, 1H), 7.26 (m, 1H), 6.71 (dd, 1H), 5.83 (d, 1H), 5.39 (d, 1H).

### Example 76F

### {4-[2-(pyrid-3-yl)ethenyl]-2-phenylbenzoyl}methionine methyl ester

A mixture of the (4-iodo-2-phenylbenzoyl)methionine methyl ester prepared in Example 76D (655 mg, 1.40 mmol), the vinylpyridine prepared in Example 76E (221 mg, 1.5 mmol), triethylamine (0.29 mL, 2.10 mmol), [1,1'-bis(diphenylphosphino)ferrocene]palladium(II) chloride, complexed to dichloromethane (1:1) (114 mg, 0.14 mmol) in DMF (2 mL) was degassed with nitrogen, and heated at 100 °C for 14 hours. The reaction mixture was diluted with ether (100 mL), washed with water and brine, dried over anhydrous magnesium sulfate, filtered, and concentrated *in vacuo*. The residue was purified by column chromatography (ethyl acetate) to give {4-[2-(pyrid-3-yl)ethenyl]-2-phenylbenzoyl}methionine methyl ester (366 mg, 56%). ¹H NMR (300 MHz, CDCl₃) δ 8.75 (d, 1H), 8.51 (dd, 1H), 7.85 (dt, 1H), 7.76 (d, 1H), 7.59 (dd, 1H), 7.48 (dd, 1H), 7.44 (m, 5H), 7.31 (dd, 1H), 7.22 (d, 1H), 7.16 (d, 1H), 5.94 (br d, 1H), 4.69 (m, 1H), 3.68 (s, 3H), 2.08 (m, 2H), 2.02 (s, 3H), 1.93 (m, 1H), 1.76 (m, 1H). MS (APCl⁺) m/e 447 (M+H)⁺.

### Example 77

### {4-[2-(pyrid-3-yl)ethenyl]-2-phenylbenzoyl}methionine sodium salt

To a solution of the {4-(2-(pyrid-3-yl)ethenyl]-2-phenylbenzoyl}methionine methyl ester prepared in Example 76 (136 mg, 0.304 mmol) in methanol (2 mL) was added a solution of sodium hydroxide (0.979 N, 0.334 mL). After 14 hours, the solvent was evaporated *in vacuo* to give {4-[2-(pyrid-3-yl)ethenyl]-2-phenylbenzoyl}methionine sodium salt (141 mg, 100%), ¹H NMR (300 MHz, DMSO-d₆) δ 8.90 (d, 1H), 8.46 (dd, 1H), 8.07 (dt, 1H), 7.64 (m, 2H), 7.50-7.35 (m, 10H), 3.78 (m, 1H), 2.10 (m, 2H), 1.98 (s, 3H), 1.76 (m, 2H). MS (APCI⁺) m/e 433 (M+H)⁺.

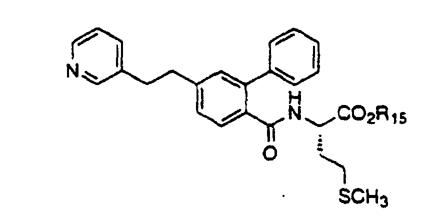

### Examples 78-79

### Example 78

### {4-[2-(pyrid-3-yl)ethyl]-2-phenylbenzoyl}methionine methyl ester

A mixture of the {4-[2-(pyrid-3-yl)ethenyl]-2-phenylbenzoyl}methionine methyl ester prepared in Example 76 (160 mg, 0.36 mmol) and palladium (10%) on carbon (460 mg, 0.43 mmol of palladium) in methanol was flushed with hydrogen, and stirred under a positive hydrogen pressure for 8 hours. The mixture was then filtered through Celite. rinsed with ethyl acetate. and concentrated *in vacuo.* The residue was purified by column chromatography (ethyl acetate) give {4-[2-(pyrid-3-yl)ethyl]-2-phenylbenzoyl}methionine methyl ester (115 mg, 71%). ¹H NMR (300 MHz, COCl₃) δ 8.45 (m, 2H), 7.64 (d, 1H), 7.40 (m, 7H), 7.22 (dd, 2H), 7.10 (d, 1H), 5.87 (br d, 1H), 4.68 (m, 1H), 3.67 (s, 3H), 2.99 (m, 4H), 2.08 (m. 2H), 2.02 (s, 3H), 1.93 (m, 1H), 1.76 (m, 1H). MS (APCl⁺) m/e 449 (M+H)⁺.

### Example 79

### {4-[2-(pyrid-3-yl)ethyl]-2-phenylbenzoyl}methionine sodium salt

The desired compound was prepared by saponification of {4-[2-(pyrid-3-yl)ethyl]-2-phenylbenzoyl}methionine methyl ester, prepared as in Example 78 according to the procedure of Example 77. ¹H NMR (300 MHz, DMSO-d₆) δ 8.45 (d, 1H), 8.40 (dd, 1H), 7.69 (dt, 1H), 7.40-7.20 (m, 9H), 7.07 (br d, 1H), 3.76 (m, 1H), 2.97 (s, 4H), 2.10 (m, 2H), 1.96 (s, 3H), 1.76 (m, 2H). MS (APCl⁺) m/e 435 (M+H)⁺ as the acid form.

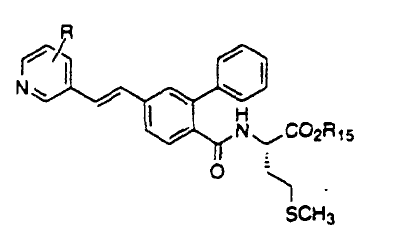

### Examples 80-82

### Example 80

### {4-[2-(4-dimethylaminopyrid-3-yl)ethenyl]-2-phenylbenzoyl}methionine sodium salt

### Example 80A

### 3-bromo-4-dimethylaminopyridine

To a mixture of 4-dimethylaminopyridine (5.00 g, 41 mmol), potassium carbonate (50 g in 50 mL of water), tetrabutylammonium hydrogensulfate (1.4 g, 4.1 mmol) and dichloromethane (100 mL) was added a solution of bromine (4.2 mL, 82 mmol) in dichloromethane (30 mL) via an addition funnel over 30 minutes. After 3 hours, the reaction mixture was diluted with ether (100 mL), washed with water and brine, dried over anhydrous sodium sulfate, filtered, and concentrated *in vacuo.* The residue was then purified by column chromatography (ethyl acetate) to give 3-bromo-4-dimethylaminopyridine (4.89 g, 59%). ¹H NMR (300 MHz, CDCl₃) δ 8.51 (s, 1H), 8.27 (d, 1H), 6.79 (d, 1H), 2.97 (s, 6H).

### Example 80B

### 3-vinyl-4-dimethylaminopyridine

A mixture of the 3-bromo-4-dimethylaminopyridine prepared in Example 80A (1.29 g, 6.39 mmol) vinyltributyltin (2.23 g, 7.02 mmol), bis(triphenylphosphine)palladium(II) chloride (224 mg, 0.32 mmol) in toluene (10 mL) was heated at 100 °C for 12 hours. To the stirring reaction mixture at room temperature was added ether (30 mL), water (0.2 mL) and 1.8-diazabicyclo[5.4.0]undec-7-ene (0.5 mL). The resulting mixture was filtered through silica gel (15 g), rinsed with ethyl acetate, and concentrated *in vacuo*. The residue was purified by column chromatography (50% ethyl acetate-hexanes, then ethyl acetate) to give 3-vinyl-4-dimethylaminopyridine (1.11 g, contaminated with about 10 mol% of tributyltin derivatives). ¹H NMR (300 MHz, CDCl₃) δ 8.39 (s, 1H), 8.26 (d, 1H), 6.77 (dd, 1H), 6.70 (d, 1H), 5.65 (dd, 1H), 5.29 (dd, 1H), 2.87 (s, 6H).

### Example 80C

### {4-[2-(4-dimethylaminopyrid-3-yl)ethenyl]-2-phenylbenzoyl}methionine methyl ester

The desired compound was prepared according to the method of Example 76, except substituting 3-vinyl-4-dimethylaminopyridine, prepared as in Example 80B, for 3-vinylpyridine. ¹H NMR (300 MHz, CDCl₃) δ 8.52 (br s, 1H), 8.30 (br d, 1H), 7.76 (d, 1H), 7.59 (dd, 1H), 7.47 (m, 6H), 7.22 (d, 1H), 7.03 (d, 1H), 6.78 (br d, 1H), 5.95 (br d, 1H), 4.69 (m, 1H), 3.67 (s, 3H), 2.92 (s, 6H), 2.10 (m, 2H), 2.02 (s, 3H), 1.93 (m, 1H), 1.76 (m, 1H). MS (Cl⁺) m/e 490 (M+H)⁺.

### Example 80D

### {4-[2-(4-dimethylaminopyrid-3-yl)ethenyl]-2-phenylbenzoyl}methionine sodium salt

The desired compound was prepared by saponification of {4-[2-(4-dimethylaminopyrid-3-yl)ethenyl]-2-phenylbenzoyl}methionine methyl ester, prepared as in Example 80C, according to the method of Example 77. ¹H NMR (300 MHz, DMSO-d₆) δ 8.42 (s 1H), 8.11 (d, 1H), 7.58 (d, 1H), 7.47-7.07 (m, 10H), 6.79 (d, 1H), 3.76 (m, 1H), 2.97 (s, 6H), 2.02 (m, 2H), 1.88 (s, 3H), 1.68 (m, 2H). MS (APCl⁺) m/e 476 (M+H)⁺ as the acid form.

### Example 81

### {4-[2-(5-bromopyrid-3-yl)ethenyl]-2-phenylbenzoyl}methionine sodium salt

### Example 81A

### 3-vinyl-5-bromopyridine

The desired compound was prepared according to the method of Example 80B, except substituting 3,5-dibromopyridine for 3-bromo-4-dimethylaminopyridine. ¹H NMR (300 MHz, DMSO-d₆) δ 8.56 (d, 1H), 8.53 (d, 1H), 7.87 (t, 1H), 6.64 (dd. 1H), 5.85 (d. 1H), 5.44 (d, 1H).

### Example 81B

### {4-[2-(5-bromopyrid-3-yl)ethenyl]-2-phenylbenzoyl}methionine sodium salt

The desired compound was prepared according to the method of Examples 80C and D, except substituting 3-vinyl-5-bromopyridine, prepared as in Example 215A, for 3-vinyl-4-dimethylaminopyridine. ¹H NMR (300 MHz, CDCl₃) δ δ 8.79 (d, 1 H), 8.59 (d, 1 H), 8.39 (br s, 1 H), 7.66-7.34 (m, 11 H), 3.78 (m, 1 H), 2.13 (m, 2 H), 1.98 (s, 3 H), 1.77 (m, 2 H). MS (APCl +) m/e (⁷⁹Br) 511 (M+H)⁺ as the acid form.

### Example 82

### {4-[2-(5-carboxymethylpyrid-3-yl)ethenyl]-2-phenylbenzoyl}methionine hydrochloride

### Example 82A

### 3-vinyl-5-carboxymethylpyridine

The desired compound was prepared according to the method of Example 80B, except substituting methyl 3-bromonicotinate for 3-brom-4-dimethylaminopyridine. ¹H NMR (300 MHz, DMSO-d₆) δ 9.09 (d, 1H), 8.78 (d, 1H), 8.34 (t, 1H), 6.76 (dd, 1H), 5.93 (d, 1H), 5.49 (d, 1H), 3.97 (s, 3H).

### Example 82B

### {4-[2-(5-carboxymethylpyrid-3-yl)ethenyl]-2-phenylbenzoyl}methionine methyl ester

The desired compound was prepared according to the method of Example 76F, except substituting 3-vinyl-5-carboxymethylpyridine, prepared as in Example 82A, for 3-vinylpyridine. ¹H NMR (300 MHz, CDCl₃) δ 9.11 (d, 1H), 8.87 (d, 1H), 8.45 (t, 1H), 7.78 (d, 1H), 7.59 (dd, 1H), 7.51 (d, 1H), 7.47 (m, 5H), 7.30 (d, 1H), 7.24 (d, 1H), 5.91 (br d, 1H), 4.68 (m, 1H), 3.97 (s, 3H), 3.67 (s, 3H), 2.08 (m, 2H), 2.02 (s, 3H), 1.94 (m, 1H), 1.76 (m, 1H). MS (CI⁺) m/e 505 (M+H)⁺.

### Example 82C

### {4-[2-(5-carboxymethylpyrid-3-yl)ethenyl]-2-phenylbenzoyl}methionine hydrochloride

To a solution of the {4-[2-(5-carboxymethylpyrid-3-yl)ethenyl]-2-phenylbenzoyl}methionine methyl ester prepared in Example 82B (90.4 mg, 0.179 mmol) in methanol (2 mL) was added a solution of sodidum hydroxide (0.979 N, 0.098 mL). After 14 hours, additional sodium hydroxide (0.979 N, 0.036 mL) was added to the reaction mixture. After 5 hours, tlc indicated that no starting material remained. The reaction was then quenched with hodrogen chloride (1.0 *M* in ether, 1 mL), and the solvent was evaporated *in vacuo* to give the title compound (105 mg, 100%) as a mixture of nicotinic acid methyl ester and nicotinic acid (ratio, 1:2). ¹H NMR (300 MHz. DMSO-d₆) δ 9.07,9.04 (2 d's. 1H), 8.98 (d, 1H), 8.67,8.57 (m's, 2H), 7.80-7.30 (m, 11H), 4.11 (m, 1H), 3.95 (s, from the methyl ester), 2.20 (m, 2H), 2.00 (s, 3H), 1.94 (m, 2H), MS (APCl⁺) m/e 491 (M+H)⁺ for the diacid, 505 (M+H)⁺ for nicotinic acid methyl ester.

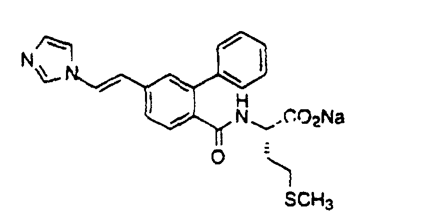

### Example 83

### {4-[2-(1-H-imidazole-1-yl)ethenyl]-2-phenylbenzoyl}methionine sodium salt

The desired compound was prepared according to the method of Examples 76 and 77, except substituting 1-vinylimidazole for 3-vinylpyridine. ¹H NMR (300 MHz, DMSO-d₆) δ 8.02 (m, 2H), 7.71 (s, 1H), 7.53-7.30 (m, 8H), 7.15 (m, 2H), 7.07 (d, 1H), 3.73 (m, 1H), 2.10 (m, 2H), 1.97 (s, 3H), 1.77 (m, 2H). MS (APCl⁺) m/e 444 (M+Na)⁺ as the acid form.

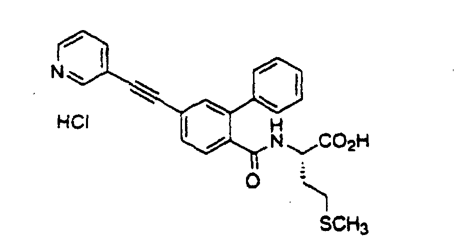

### Example 84

### {4-[(pyrid-3-yl)ethynyl]-2-phenylbenzoyl}methionine hydrochloride

### Example 84A

### {4-[(pyrid-3-yl)ethynyl]-2-phenylbenzoyl}methionine methyl ester

A mixture of (4-iodo-2-phenylbenzoyl)methionine methyl ester, prepared as in Example 76D, (469 mg, 1.0 mmol), [1,1'-bis(diphenylphosphino)ferrocene]palladium(II) chloride, complexed to dichloromethane (1:1) (82 mg, 0.10 mmol), and ethynyltributyltin (315 mg, 1.0 mmol) in DMF (5 mL) was heated at 80 °C for 6 hours at which point thin layer chromatography indicated no starting iodide left. 3-Bromopyridine (0.114 mL. 1.2 mmol) and triethylamine (0.42 mL, 3.0 mmol) were added and the reaction mixture was heated at 100 °C for 14 hours. The reaction mixture was diluted with ether (50 mL) and ethyl acetate (50 mL), washed with water and brine, dried over anhydrous magnesium sulfate, and filtered. To the filtrate was added 3 drops of water, followed by 1,8-diazabicyclo[5.4.0]undec-7-ene (0.2 mL) with swirling. The resulting milky mixture was filtered through silica gel (15 g), rinsed with ethyl acetate, and concentrated *in vacuo*. The residue was then purified by column chromatography (50% ethyl acetate-hexane, then ethyl acetate) to give {4-[(pyrid-3-yl)ethynyl]-2-phenylbenzoyl}methionine methyl ester (109 mg, 24%). ¹H NMR (300 MHz, CDCl₃) δ 8.79 (s, 1H), 8.58 (dd, 1H), 7.82 (dt, 1H), 7.74 (d, 1H), 7,59 (dd, 1H), 7.57 (s, 1H), 7.45 (m, 5H), 7.31 (dd. 1H), 5.93 (br d, 1H), 4.69 (m, 1H), 3.68 (s, 3H), 2.08 (m, 2H), 2.02 (s, 3H), 1.93 (m, 1H), 1.76 (m, 1H). MS (Cl⁺) m/e 445 (M+H)⁺.

### Example 84B

### {4-[(pyrid-3-yl)ethynyl]-2-phenylbenzoyl}methionine hydrochloride

To a solution of the {4-[(pyrid-3-yl)ethynyl]-2-phenylbenzoyl}methionine methyl ester prepared in Example 84A (48 mg, 0.108 mmol) in methanol (2 mL) was added aqueous sodidum hydroxide (0.979 N, 0.120 mL). After 14 hours, the reaction was quenched with hydrochloric acid (3.0 N, 0.1 mL), and the solvent was evaporated *in vacuo* to give {4-[(pyrid-3-yl)ethynyl]-2-phenylbenzoyl}methionine hydrochloride (61 mg, 100%). ¹H NMR (300 MHz, DMSO-d₆) δ 8.95 (d, 1H), 8.89 (d, 1H), 8.84 (dd, 1H), 8.11 (dt, 1H), 7.68 (dd, 1H), 7.63 (d, 1H), 7.57 (dd, 1H), 7.51 (d, 1H), 7.46 (m, 2H), 7.38 (m, 2H), 4.31 (m, 1H), 2.24 (m, 2H), 2.00 (s, 3H), 1.86 (m, 2H). MS (APCI⁺) m/e 431 (M+H)⁺ as the acid form.

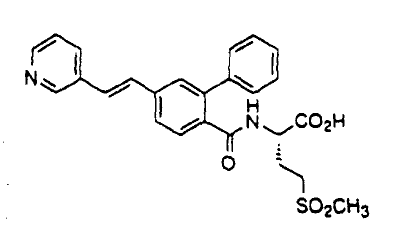

### Example 85

### N-{4-[2-(pyrid-3-yl)ethenyl]-2-phenylbenzoyl]-2-amino-4-(methylsulfonyl)butanoic acid

### Example 85A

### N-{4-[2-(pyrid-3-yl)ethenyl]-2-phenylbenzoyl]-2-amino-4-(methylsulfonyl)butanoic acid methyl ester

To a solution of {4-(2-(pyrid-3-yl)ethenyl]-2-phenylbenzoyl}methionine methyl ester (521 mg, 1.17 mmol), prepared as in Example 76, 4-methylmorpholine *N*-oxide (551 mg, 4.68 mmol), methylsulfonamide (222 mg, 2.34 mmol) and quinuclidine (13 mg, 0.12 mmol) in *tert*-butanol (5 mL) and water (5mL) was added a solution of osmium tetraoxide (2.5 wt% in *tert*-butanol, 0.73 mL, 0.058 mmol) and the mixture was stirred at 45 °C for 18 hours. The reaction mixture was diluted with ethyl acetate (100 mL), washed with water and brine, dried over anhydrous sodium sulfate, filtered, and concentrated *in vacuo.* The residue was purified by column chromatography (ethyl acetate, then 5% methanol-ethyl acetate) to give the desired compound (71 mg, 15%) as the first fraction, and compound diol (285 mg, 56%, a 1:1 mixture of diastereomers) as the second fraction. ¹H NMR (300 MHz, CDCl₃) δ 8.74 (s, 1 H), 8.53 (d, 1 H), 7.87 (dt, 1 H), 7.73 (d, 1 H), 7.60 (dd, 1 H), 7.48 (m, 6 H), 7.32 (dd, 1 H), 7.19 (s, 2 H), 6.02 (br d, 1 H), 4.68 (m, 1 H), 3.70 (s, 1 H), 2.95 9s, 3 H), 2.77 (m, 1 H), 2.65 (m, 1 H), 2.27 (m, 1 H), 1.99 (m, 1 H). MS (Cl +) m/e 479 (M+H)⁺.
For compound 12a:

### Example 85B

### N-{4-[2-(pyrid-3-yl)ethenyl]-2-phenylbenzoyl]-2-amino-4-(methylsulfonyl)butanoic acid

The desired compound was prepared by saponification of the product of Example 85A using the procedure of Example 77. ¹H NMR (300 MHz, DMSO-d₆) δ 8.80 (d, 1H), 8.45 (dd, 1H), 8.07 (dt, 1H), 7.65 (m, 2H), 7.54 (d, 1H), 7.43 (m, 8H), 7.25 (br d, 1H), 3.88 (m, 1H), 2.89 (s, 3H), 2.78 (m, 2H), 1.96 (m, 2H), MS (FAB⁺) m/e 487 (M+Na)⁺ as the acid form.

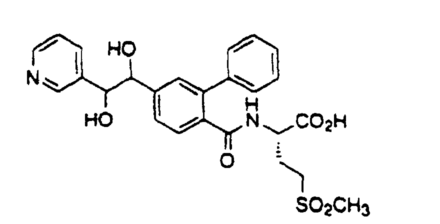

### Example 86

### N-{4-[2-(pyrid-3-yl)-1,2-dihydroxyethyl]-2-phenylbenzoyl]-2-amino-4-(methylsulfonyl)butanoic acid

### Example 86A

### N-{4-[2-(pyrid-3-yl)-1,2-dihydroxyethyl]-2-phenylbenzoyl]-2-amino-4-(methylsulfonyl)butanoic acid methyl ester

The desired product was a side product in Example 85A. ¹H NMR (300 MHz, CDCl₃) δ 8.47 (2 d's, 1H), 8.19,8.15 (2 s's, 1H), 7.61 (2 dt's, 1H), 7.57 (d, 1H), 7.40 (m, 2H), 7.23 (m, 5H), 7.00 (dd, 1H), 6.25 (2 d's, 1H), 4.76 (m, 2H), 4.63 (m, 1H), 3.70,3.68 (2s's. 3H), 2.92,2.93 (2s's, 3H), 2.80-2.52 (m, 2H), 2.24 (m, 1H), 1.97 (m, 1H). MS (Cl⁺) m/e 513 (M+H)⁺.

### Example 86B

### N-{4-[2-(pyrid-3-yl)-1,2-dihydroxyethyl]-2-phenylbenzoyl]-2-amino-4-(methylsulfonyl)butanoic acid

The desired compound was prepared by saponification of the product of Example 86A using the procedure of Example 77. ¹H NMR (300 MHz, DMSO-d₆) δ 8.36 (m, 2H), 7.58,7.55 (2 d's, 1H), 7.39-7.19 (m. 9H), 7.06 (s, 1H), 4.75,4.74 (2 s's, 2H), 3.75 (m, 1H), 2.87 (s, 3H), 2.77 (m, 2H), 1.95 (m, 1H). MS (APCI⁺) m/e 499 (M+H⁺) as the acid form.

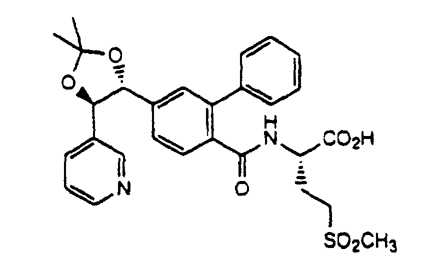

### Example 87

### N-{4-[2-(pyrid-3-yl)-1,2-dihydroxyethyl dimethyl ketal]-2-phenylbenzoyl]-2-amino-4-(methylsulfonyl)butanoic acid

### Example 87A

### N-{4-[2-(pyrid-3-yl)-1,2-dihydroxyethyl dimethyl ketal]-2-phenylbenzoyl]-2-amino-4-(methylsulfonyl)butanoic acid methyl ester

A solution of *N*-{4-(2-(pyrid-3-yl)-1,2-dihydroxyethyl]-2-phenylbenzoyl]-2-amino-4-(methylsulfonyl)butanoic acid methyl ester (79 mg, 0.154 mmol), prepared as in Example 86A, toluenesulfonic acid (20 mg) in 2,2-dimethoxypropane (0.5 mL) and DMF (1 mL) was stirred at 50 °C for 6 hours. The reaction mixture was diluted with ether (100 mL), washed with saturated aqueous sodium bicarbonate, water and brine, dried over anhydrous magnesium sulfate, filtered, and concentrated *in vacuo*. The residue was purified by column chromatography (ethyl acetate) to give the desired compound (71 mg, 84%). ¹H NMR (300 MHz, CDCl₃) δ 8.61 (br s, 1H), 8.50 (d, 1H), 7.66 (m, 2H), 7.45-7.35 (m, 6H), 7.24 (m, 2H), 6.0 (2 d'd, 1H), 4.78 (m, 2H), 4.67 (m, 1H), 3.70 (s, 3H), 3.69 (s, 3H), 2.96 (s, 3H), 2.80-2.60 (m, 2H), 2.28 (m, 1H), 1.99 (m, 1H). MS (Cl⁺) m/e 553 (M+H)⁺.

### Example 87B

### N-{4-[2-(pyrid-3-yl)-1,2-dihydroxyethyl dimethyl ketal]-2-phenylbenzoyl]-2-amino-4-(methylsulfonyl)butanoic acid

The desired compound was prepared by saponification of the product of Example 86A using the procedure of Example 77. ¹H NMR (300 MHz, DMSD-d₆) δ 8.55 (dt, 1H), 8.48 9d, 1H), 7.76,7.73 (2 q's, 1H), 7.47-7.07 (m, 10H), 4.90 (m, 2H), 3.90 (m, 1H), 3.16 (s, 6H), 2.73 (m, 1H), 2.28 (m, 1H), 1.94 (m, 2H). MS (APCl⁺) m/e 561 (M+Na)⁺.

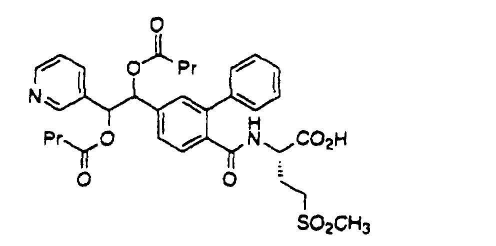

### Example 88

### N-{4-[2-(pyrid-3-yl)-1,2-propionoylethyl]-2-phenylbenzoyl]-2-amino-4-(methylsulfonyl)butanoic acid

### Example 88A

### N-{4-[2-(pyrid-3-yl)-1,2-propionoylethyl]-2-phenylbenzoyl]-2-amino-4-(methylsulfonyl)butanoic acid methyl ester

To a solution of *N*-[4-[2-(pyrid-3-yl)-1,2-dihydroxyethyl]-2-phenylbenzoyl]-2-amino-4-(methylsulfonyl)butanoic acid methyl ester (71 mg, 0.134 mmol), prepared as in Example 86A and 4-dimethylaminopyridine (3 mg) in dichloromethane (3 mL) was added propionic anhydride (0.066 mL, 0.402 mmol) and the reaction was stirred for 4 hours. The reaction mixture was diluted with ether (50 mL), washed with water and brine, dried over anhydrous magnesium sulfate, filtered, and concentrated *in vacuo.* The residue was purified by column chromatography (ethyl acetate) to give *N*-[4-[2-(pyrid-3-yl)-1,2-propionoylethyl]-2-phenylbenzoyl]-2-amino-4-(methylsulfonyl)butanoic acid methyl ester (79 mg. 90%). ¹H NMR (300 MHz, CDCl₃) δ 8.52 (br s, 1H), 8.39 (br s, 1H), 7.60 (d 1H), 7.55 (m, 1H), 7.41 (m, 3H), 7.26 (m, 4H), 7.07 (dd, 1H), 6.12 (m, 3H), 4.62 (m, 1H), 3.681,3.684 (2 s's, 3H), 2.840,2.843 (2 s s, 3H), 2.78-2.57 (m, 2H), 2.34 (m, 4H), 2.25 (m. 1H), 1.97 (m, 1H), 1.63 (m, 4H), 0.92 (m, 6H). MS (Cl⁺) m/e 653 (M+H)⁺.

### Example 88B

### N-{4-[2-(pyrid-3-yl)-1,2-propionoylethyl]-2-phenylbenzoyl]-2-amino-4-(methylsulfonyl)butanoic acid

To a solution of the *N*-[4-[2-(pyrid-3-yl)-1,2-propionoylethyl]-2-phenylbenzoyl]-2-amino-4-(methylsulfonyl)butanoic acid methyl ester prepared in Example 88A (71 mg, 0.109 mmol) in anhydrous ether (2 mL) and THF (2 mL) was added solid potassium trimethylsilanolate (41 mg, 0.327 mmcl). After 1 hour, hydrogen chloride (4.0 N in 1,4-dioxane, 0.1 mL) was added to the reaction mixture and the solvent was evaporated *in vacuo* to give *N*-{4-[2-(pyrid-3-yl)-1,2-propionoylethyl]-2-phenylbenzoyl]-2-amino-4-(methylsulfonyl)butanoic acid (82 mg, 100%). ¹H NMR (300 MHz, DMSO-d₆) δ 8.77-8.60 (m, 3H), 8.28 (br d, 1H), 7.88 (br s, 1H), 7.43-7.22 (m, 8H), 5.05 (d, 1H), 4.91 (d, 1H), 4.30 (m, 1H), 2.94 (s, 3H), 2.92-2.70 (m, 2H), 2.40-2.10 (m, 5H), 1.98 (m, 1H), 1.47 (m, 4H), 1.90-1.72 (m, 6H). MS (FAB⁻) m/e 637 (M-H)- (only major peak above molecular weight 500).

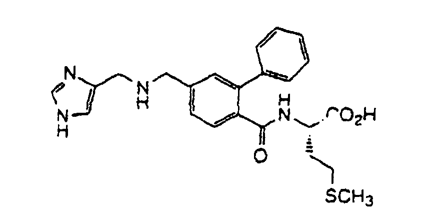

### Example 89

### [4-(1H-imidazol-4-ylmethylaminomethyl)-2-phenylbenzoyl]methionine

### Example 89A

### 4-azidomethyl-1H-1-triphenylmethylimidazole

To a -10 °C solution in toluene (3 mL) of triphenylphosphine (787 mg, 3.0 mmol) was added a solution of diethylazodicarboxylate (0.47 mL, 3.0 mmol) in toluene (3 mL) dropwise over 10 minutes. A slurry of 4-hydroxymethyl-1H-1-triphenylmethylimidazole (684 mg, 2.0 mmol), prepared as in Example 41A, in dichloromethane (10 mL) was added and the reaction mixture was stirred for 30 minutes. A 1 M solution of HN₃ in toluene (10 mL, 10 mmol) was added, the cold bath was removed, and the reaction mixture was stirred for 1 hour. The reaction mixture was diluted with ethyl acetate and extracted with aqueous 1N sodium hydroxide, water and brine, dried over sodium sulfate, filtered, and concentrated in vacuo. Chromatography on silica gel (50% ethyl acetate-chloroform) gave 4-azidomethyl-1H-1-triphenylmethylimidazole (626 mg, 86%).

### Example 89B

### [4-{1H-1-triphenylmethylimidazol-4-ylmethylaminomethyl)-2-phenylbenzoyl]methionine methyl ester

To a solution in THF (3 mL) of 4-azidomethyl-1H-1-triphenylmethylimidazole (220 mg, 0.60 mmol), prepared as in Example 89A, and (4-carboxyaldehyde-2-phenylbenzoyl)methionine methyl ester (186 mg, 0.50 mmol), prepared as in Example 39F, was added triphenylphosphine (157 mg, 0.60 mmol) and the reaction mixture was stirred for 1 hour and at 65 °C for 3 hours. The reaction mixture was cooled to ambient temperature and 2-propanol (2 mL) and sodium cyanoborohydride (94 mg, 1.5 mmol) were added. The reaction mixture was stirred for 0.5 hours and then was poured into aqueous 2N sodium hydroxide. The mixture was extracted twice with ethyl acetate. The combined organic extracts were washed with water, saturated aqueous sodium bicarbonate and brine, dried over sodium sulfate, filtered, and concentrated in vacuo. Chromatography on silica gel (50% methanol-chloroform) followed by another chromatography (ethyl acetate) gave [4-(1H-1-triphenylmethylimidazol-4-ylmethylaminomethyl)-2-phenylbenzoyl]methionine methyl ester (231 mg, 66%).

### Example 89C

### [4-(1H-imidazol-4-ylmethylaminomethyl)-2-phenylbenzoyl]methionine

The desired compound was prepared by saponification of the methyl ester and deprotection of the imidazoleusing the prodecures of Examples 41E and 41D respectively. 1H NMR (300 MHz, D₂O) δ 8.72 (s, 1H), 7.40-7.67 (m, 9H), 4.51 (s, 2H), 4.43 (s, 2H), 4.38 (m, 1H), 2.03 (s, 3H), 1.90-2.09 (m, 3H), 1.79 (m, 1H). MS (Cl NH₃) m/e 439 (M+H)⁺, 359, 227. Anal calcd for C₂₃H₂₆N₄O₃S + 2.4 trifluoroacetic acid: C, 46.89, H, 4.02, N, 7.87. Found: C, 46.79; H, 4.16, N, 7.87.

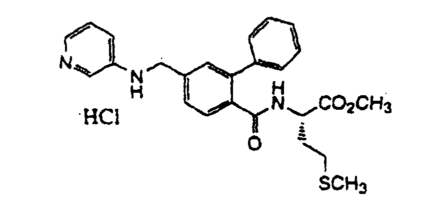

### Example 90

### [4-(pyrid-3-ylaminomethyl)-2-phenylbenzoyl]methionine methyl ester hydrochloride

### Example 90A

### [4-(pyrid-3-ylaminomethyl)-2-phenylbenzoyl]methionine methyl ester

To a solution of (4-carboxyaldehyde-2-phenylbenzoyl)methionine methyl ester (2.5 g, 6.7 mmol), prepared as in Example 39F, in methanol (15 mL) was added 3-aminopyridine (941 mg, 10 mmol) and acetic acid (5 mL). The reaction mixture was stirred for 1 hour and sodium cyanoborohydride (0.85 g, 13.5 mmol) was added. The reaction mixture was stirred for 2 hours and additional sodium cyanoborohydride (0.42 g, 6.7 mmol) was added. Stirring was continued for an additional 2 hours and then the reaction mixture was poured into aqueous 1N sodium hydroxide. The mixture was extracted with ethyl acetate (3x). The combined organic extracts were washed with saturated aqueous sodium bicarbonate (2x), water (3x) and and brine, dried over sodium sulfate, filtered, and concentrated in vacuo. Successive chromatographiss on silica gel (ethyl acetate) gave [4-(pyrid-3-ylaminomethyl)-2-phenylbenzoyl]methionine methyl ester (2.2 g, 73%).

### Example 90B

### [4-(pyrid-3-ylaminomethyl)-2-phenylbenzoyl]methionine methyl ester hydrochloride

To a solution in ethyl acetate (20 mL) of [4-(pyrid-3-ylaminomethyl)-2-phenylbenzoyl]methionine methyl ester (2.96 g, 6.58 mmol), prepared as in Example 90A, was added HCl (1.0 M in ethyl acetate, 20 mL, 20 mmol) dropwise. The reaction mixture was stirred for 10 minutes and then was concentrated and the residue was azeotroped with toluene. Water (50 mL) was added and the milky mixture was concentrated and lyophilized to give [4-(pyrid-3-ylaminomethyl)-2-phenylbenzoyl]methionine methyl ester hydrochloride (3.04 g, 95%). ¹H NMR (300 MHz., DMSO d₆) δ 8.66 (d, 1H), 8.11 (s, 1H), 8.02 (t, 1H), 7.84 (bt, 1H), 7.71 (m, 2H), 7.44 (m, 3H), 7.26 (m, 5H), 4,53 (bd, 2H), 4.36 (ddd, 1 H), 3.64 (s, 3H), 2.23 (m, 2H), 1.98(s, 3H), 3.85 (m, 2H). MS (Cl NH₃) m/e 450 (M+H)⁺, 374, 319, 287. Anal calcd for C₂₅H₂₈ClN₃O₃S + 0.58 H₂O) :C, 60.48; H , 5.92; N, 8.46; Cl, 7.29. Found: C, 60.49; H, 5.58; N, 8.40; Cl 7.84.

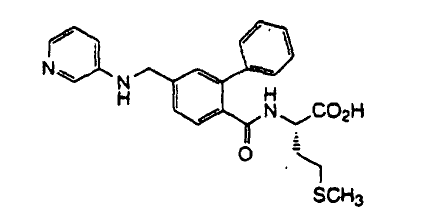

### Example 91

### [4-(pyrid-3-ylaminomethyl)-2-phenylbenzoyl]methionine

To a solution of [4-(pyrid-3-ylaminomethyl)-2-phenylbenzoyl]methionine methyl ester hydrochloride (72 mg, 0.16 mmol) in THF (3 mL) was added a solution of lithium hydroxide hydrate (13 mg, 0.32 mmol) in water (1 mL) and the reaction mixture was stirred for 30 minutes. The THF was evaporated in vacuo and the residue was taken up in aqueous 3N HCl. The solution was concentrated and the residue was purified by preparative HPLC (70% acetonitrile-0.1% aqueous trifluoroacetic acid) to give [4-(pyrid-3-ylaminomethyl)-2-phenylbenzoyl]methionine (39 mg, 50%). ¹H NMR (300 MHz., DMSO-d₆) δ 8.48 (d, 1H), 8.17 (m, 1H), 7.97 (m, 1H), 7.56 (m, 2H), 7.29 - 7.44 (m, 7H), 4.51 (bd. 2H), 4.48 (ddd, 1H), 2.24 (m, 2H), 1.99 (s, 3H), 1.85 (m, 2H). MS (Cl NH₃) m/e 436 (M+H)⁺, 418, 319, 287, 194, 165. Anal calcd for C₂₄H₂₅N₃O₃S (+ 1.26 TFA): C, 55.00; H, 4.57; N, 7.25. Found: C. 54.97; H, 4.58; N, 7.33.
The next example is Example 93.

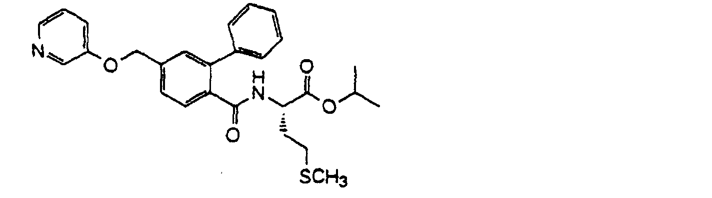

### Example 93

### [4-(3-pyridyloxymethyl)-2-phenylbenzoyl]methionine isopropyl ester

### Example 93A

### N-tert-butoxycarbonymethionine isopropyl ester

To a solution of *N*-*tert*-butoxycarbonylmethionine (2.49 g, 10 mmol) in DMF (50 mL) was added 3-hydroxy-1,2,3-benzotriazin-4(3H)-one (1.74 mg, 11 mmol), 4-dimethylaminopyridine (244 mg, 2.0 mmol),ethyl dimethylaminopropyl carbodiimide hydrochloride (2.11 g, 11 mmol) and isopropanol (2.3 mL. 30 mmol) and the reaction mixture was stirred for 27 hours. The reaction mixture was poured into water and extracted three times with ethyl acetate. The combined organic extracts were washed with aqueous sodium hydroxide (2x), aqueous HCl (2x), water (2x) and brine, dried, filtered, and concentrated in vacuo. Chromatography on silica gel (20% ethyl acetate-hexanes) gave *N*-*tert*-butoxycarbonymethionine isopropyl ester (1.26 g, 43%).

### Example 93B

### methionine isopropyl ester hydrochloride

To a solution of *N*-*tert*-butoxycarbonymethionine isopropyl ester (291 mg, 1.0 mmol), prepared as in Example 93A, in dioxane (1 mL) was added 4N HCl-dioxane (4 mL) and the mixture was stirred for 3 hours. The reaction mixture was concentrated in vacuo to give methionine isopropyl ester hydrochloride (240 mg) which was used without further purification.

### Example 93C

### 4-methyl-2-phenylbenzoic acid methyl ester

The desired compound was prepared according to the method of Example 36A, except substituting 4-methyl-2-hydroxybenzoic acid methyl ester for 2-iodoterephthalate.

### Example 93D

### 4-bromomethyl-2-phenylbenzoic acid methyl ester

A mixture of 4-methyl-2-phenylbenzoic acid methyl ester (2.26 g, 10 mmol), prepared as in Eample 91B, N-bromosuccinimide (1.87 g, 10.5 mmol) and 2,2'-azobisisobutyronitrile (25 mg) in carbon tetrachloride (40 mL) was stirred as reflux for 7 hours. The reaction mixture was poured into ethyl acetate and extracted with water (2x), aqueous sodium hydrogen sulfite and brine, dried, filtered, and concentrated in vacuo. Chromatography on silica gel (10% ethyl acetate-hexane) gave 4-bromomethyl-2-phenylbenzoic acid methyl ester (2.57 g, 84%).

### Example 93E

### 4-(3-pyridyloxymethyl)-2-phenylbenzoic acid methyl ester

To a mechanicallly-stirred 0°C solution of 3-hydroxypyridine (4.4 g, 46 mmol) in DME (20 mL) was added potassium hexamethyldisilazide (0.5 M in toluene, 88.5 mL, 44 mmol) and the mixture was stirred for 15 minutes. 18-Crown-6 (1.46 g, 5.5 mmol) and a solution of 4-bromomethyl-2-phenylbenzoic acid methyl ester (6.8 g, 22 mmol) in toluene (25 mL) was added and the reaction mixture was vigorousely stirred overnight. The reaction mixture was poured into 200 mL of water and the layers were separated. The aqueous phase was extracted with 2 portions of ethyl acetate and the combined organic phases were extracted with water and brine, dried (MgSO₄), filtered and concentrated. The resulting oil was purified by flash chromatography (65% ethyl acetate-hexane) to give 4-(3-pyridyloxymethyl)-2-phenylbenzoic acid methyl ester (3.4 g).

### Example 93F

### 4-(3-pyridyloxymethyl)-2-phenylbenzoic acid

To a solution of 4-(3-pyridyloxymethyl)-2-phenylbenzoic acid methyl ester (3.4 g, 10.6 mmol), prepared as in Example 93E, in methanol, (30 mL) was added aqueous 4N sodium hydroxide and the reaction mixture was heated at reflux for 6 hours. The methanol was distilled off in vacuo and the residue was taken up in water. The aqueous solution was taken to pH4 with HCl and the resulting precipitate was filtered off and dried to give 4-(3-pyridyloxymethyl)-2-phenylbenzoic acid (3.2 g).

### Example 93G

### [4-(3-pyridyloxymethyl)-2-phenylbenzoyl]methionine isopropyl ester

The desired compound was prepared by coupling of 4-(3-pyridyloxymethyl)-2-phenylbenzoic acid, prepared as in Example 93F with methionine isopropyl ester hydrochloride, prepared as in Example 93B using the procedure of Example 53C. ¹H NMR (300 MHz, CD₃OD) δ 8.70 (d, 1H), 8.46 (d, 1H), 8.31 (ddd, 1H), 8.01 (dd, 1H), 7.58 (m, 3H), 7.33 - 7.47 (m, 5H), 5.45 (s, 2H), 5.00 (heptet, 1H), 4.44 (m, 1H), 2.00 - 2.24 (m, 2H), 2.01 (s, 3H), 1.96 (m, 1H), 1.77 (m, 1H), 1.24 (d, 3H), 1.22 (d, 3H). MS (Cl NH₃) m/e 479 (M+H)⁺, 451, 419, 320, 288, 192. Anal calcd for C₂₇H₃₁ClN₂O₄S (+0.60 H₂O): C, 61.67; H, 6.17; N, 5.33. Found: C, 61.67; H, 6.17; N, 5.33.

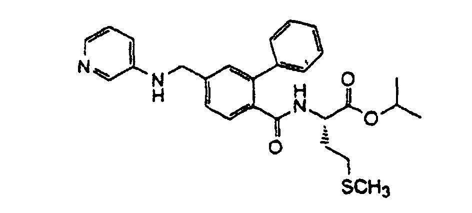

### Example 94

### [4-(3-pyrdylaminomethyl)-2-phenylbenzoyl]methionine isopropyl ester

### Example 94A

### (4-hydroxymethyl-2-phenylbenzoyl)methionine isopropyl ester

The desired compound was prepared according to the method of Example 93C, except substituting 4-hydroxymethylbenzoic acid, prepared as in Example 37D, for 4-(3-pyridyloxymethyl)-2-phenylbenzoic acid.

### Example 94B

### (4-carboxaldehyde-2-phenylbenzoyl)methionine isopropyl ester

The desired compound was prepared by oxidation of the product of Example 94A using the procedure of Example 39B.

### Example 94C

### [4-(3-pyridylaminomethyl)-2-phenylbenzoyl]methionine isopropyl ester

The desired compound was prepared by reductive amination of the product of Example 94B with 3-aminopyridine according to the method of Example 90A. ¹H NMR (304MHz, CD₃OD) δ 7.97 (m, 1H), 7.86 (dd, 1H), 7.43 - 7.52 (m, 5H), 7.31 - 7.42 (m, 5H), 4.98 (heptet, 1H), 4.52 (s, 2H), 4.43 (m, 1H), 2.12 (m, 1H), 2.03 (m, 1H), 1.99 (s, 3H), 1.01 (m, 1H), 1.75 (m, 1H), 1.15 (d, 3H), 1.13 (d, 3H). MS (Cl NH₃) m/e 478 (M+H)⁺, 319, 287. Anal calcd for C₂₇H₃₂ClN₃O₃S(+ 0.58 H₂O): C, 61.83; H, 6.37; N, 8.01. Found: C, 61.82; H, 6.04; N, 7.74.

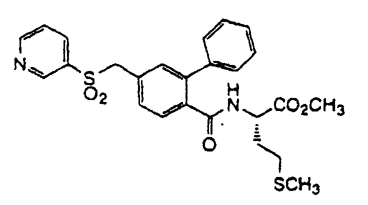

### Example 95

### [4-(3-pyridylsulfonylmethyl)-2-phenylbenzoyl]methionine methyl ester

### Example 95A

### 3-dimethylthiocarbamoylpyridine

To a solution in DMF (50 mL) of 3-hydroxypyridine (4.76 g, 50 mmol) was added 1,1-diazabicyclo[2.2.2]octane (6.80 g, 150 mmol) and dimethylthiocarbamoyl chloride (18.5 g, 150 mmol) and the reaction mixture was stirred for 0.5 hours at ambient temperature and 18 hours at 55 °C. The reaction mixture was cooled to ambient tempertaure and poured into ether. The ethereal solution was washed with aqueous 2N sodium hydroxide (2x), water (2x) and brine, dried, filtered and concentrated in vacuo. Chromatography on silica gel (1:1 ethyl acetate-hexane) gave 3-dimethylthiocarbamoylpyridine (5.46 g).

### Example 95B

### 3-dimethylaminocarbonylthiopyridine

The desired compound was prepared by heating the 3-dimethylthiocarbamoylpyridine prepared in Example 95A at 250 °C for 1.25 hours followed by cooling and chromatography on silica gel (55%, then 75% ethyl acetate-hexane).

### Example 95C

### 3-thiopyridine sodium salt

To a solution of 3-dimethylaminocarbonylthiopyridine (1.23 g, 6.7 mmol), prepared as in Example 95B, in methanol (10 mL) was added aqueous 2N sodium hydroxide and the reaction mixture was stirred at reflux for 2 hours. The reaction mixture was cooled to ambient temperature and evaporated to dryness to give 3-thiopyridine sodium salt as a brown solid which was used without further purification.

### Example 95D

### 4-(3-pyridylthiomethyl)-2-phenylbenzoic acid methyl ester

To a -10 °C suspension in DME (10 mL) of the 3-thiopyridine sodium salt prepared in Example 95C (450 mg, 3.25 mmol) was added catalytic 18-crown-6 and a solution of 3-bromomethyl-2-phenylbenzoic acid (916 mg, 8.3 mmol), prepared as in Example 92B, in DME (5 mL) over 5 minutes. The cold bath was allowed to warm to ambient temperature and the reaction mixture was stirred for 24 hours. The reaction mixture was poured into water and extracted with ethyl acetate (3x). The combined organic extracts were washed with brine, dried, filtered and concentrated. Chromatography on silica gel (40% ethyl acetate-hexane) gave 4-(3-pyridylthiomethyl)-2-phenylbenzoic acid methyl ester (611 mg, 60%).

### Example 95E

### 4-(3-pyridylsulfonylmethyl)-2-phenylbenzoic acid methyl ester

To a 0 °C solution of trifluoroacetic anhydride (2.5 mL, 17.9 mmol) in dichloromethane (10 ml.) was added aqueous 30% hydrogen peroxide (0.56 mL, 5.4 mmol) and a solution of 4-(3-pyridylthiomethyl)-2-phenylbenzoic acid methyl ester (600 mg, 17.4 mmol), prepared as in Example 95D, in dichloromethane (5 mL). The reaction mixture was stirred for 1 hour, then the cold bath was removed and stirring was continued for 0.5 hour. The reaction mixture was partitioned between ether and aqueous 2N sodium hydroxide and the aqueous phase was extracted with ether. The combined ethereal layers were washed with aqueous 2N sodium bisulfite, water and brine, dried, filtered and concentrated in vacuo to give 4-(3-pyridylsulfonylmethyl)-2-phenylbenzoic acid methyl ester (620 mg) which was used without further purification.

### Example 95F

### [4-(3-pyridylsulfonylmethyl)-2-phenylbenzoyl]methionine methyl ester

The desired compound was prepared from 4-(3-pyridylsulfonylmethyl)-2-phenylbenzoic acid methyl ester by saponification of the methyl ester using the procedure of Example 93F, and coupling with methionine methyl ester according to the procedure of Example 53C. mp 152-154 °C. ¹H NMR (300 MHz, CDCl₃) δ 8.95 (d, 1H), 8.87 (dd, 1H), 7.94 (ddd, 1H), 7.66 (d, 1H), 7.43 (m, 4H), 7.30 (m, 2H), 7.21 (dd, 1H), 7.10 (d, 1H), 5.91 (bd, 1H), 4.66 (ddd, 1H), 4.42 (s, 2H), 3.68 (s, 3H), 2.08 (t, 2H). 2.02 (s, 3H), 1.93 (m, 1H), 1.75 (m, 1H), MS (Cl NH₃) m/e 516 (M+NH₄)⁺, 499 (M+H)⁺. Anal calc' for C₂₅H₂₆N₂O₅S₂: C ,60.22; H ,5.25; N ,5.62. Found: C, 60.28; H, 4.94; N, 5.56.

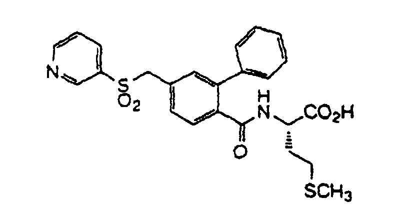

### Example 96

### [4-(3-pyridylsulfonylmethyl)-2-phenylbenzoyl]methionine

The desired compound was prepared by saponification of [4-(3-pyridylsulfonylmethyl)-2-phenylbenzoyl]methionine methyl ester, prepared as in Example 95, according to the method of Example 38. ¹H NMR (300 MHz., DMSO d₆) δ 12.68 (bs, 1H), 8.92 (m, 2H), 8.59 (bd, 1H), 8.18 (ddd, 1H), 7.68 (m, 1H), 7.32 (m, 7H), 7.18 (d, 1H), 4.94 (s, 1H), 4.29 (ddd, 1H), 2.22 (m, 2H), 1.99 (s, 3H), 1.85 (m, 2H). MS (CI NH₃)m/e 502 (M+NH₄)⁺, (485 M+H)⁺. Anal calcd for C₂₄H₂₄N₂O₅S₂ (+ 0.45 H2O): C, 58.51; H, 5.09; N, 5.69. Found: C, 58.51; H. 4.82; N, 5.69.

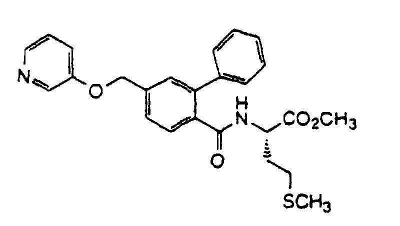

### Example 97

### [4-(3-pyridyloxymethyl)-2-phenylbenzoyl]methionine methyl ester

### Example 97A

### 4-methyl-2-phenylbenzoic acid

To a solution of 4-methyl-2-phenylbenzoic acid methyl ester (1.83 g, 8.09 mmol), prepared as in Example 91A, in methanol (16 mL) was added aqueous 4N sodium hydroxide (5 mL) and the reaction mixture was stirred for 60 hours, after which additional aqueous 4N sodium hydroxide (5 mL) was added and the mixture was heated at reflux for 5 hours. The reaction mixture was cooled to ambient temperature and the methanol was evaporated in vacuo. The aqueous residue was acidified with 4N sulfuric acid and extracted with ethyl acetate (3x). The combined organic extracts were dried, filtered, and concentrated in vacuo to give 4-methyl-2-phenylbenzoic acid (1.67 g) as a white solid.

### Example 97B

### 4-bromomethyl-2-phenylbenzoic acid

A mixture of 4-methyl-2-phenylbenzoic acid (1.66 g, 7.82 mmol), N-bromosuccinimide (1.40 g, 8.21 mmol) and 2,2'-azobisisobutyronitrile (25 mg) in carbon tetrachloride (30 mL) was stirred at reflux for 1 hour. The reaction mixture was poured into ethyl acetate and extracted with water (3x) and brine, dried, filtered, and concentrated in vacuo to give 4-bromomethyl-2-phenylbenzoic acid (2.26 g).

### Example 97C

### (4-bromomethyl-2-phenylbenzoyl)methionine methyl ester

To a solution in dichloromethane (25 mL) of 4-bromomethyl-2-phenylbenzoic acid (2.16 g, 7.42 mmol), prepared as in Example 97B, was added oxalyl chloride (0.84 mL, 965 mmol) and 2 drops on DMF and the reaction mixture was stirred for 2 hours. The reaction mixture was concentrated in vacuo and azeotroped with toluene. The residue was dissolved in dichloromethane (15 mL) and then was added to a solution in dilchloromethane (15 mL) of methionine methyl ester hydrochloride (1.78 g, 8.90 mmol) and diisopropylethylamine (3.10 g, 17.81 mmol) (prepared at -10 °C) dropwise. The reaction mixture was stirred for 30 minutes and then was poured into ether and extracted with water, aqueous 3N HCl (2x) and brine, dried, filtered and concentrated in vacuo. Chromatography on silica gel (5% ethyl acetate-chloroform) to give (4-bromomethyl-2-phenylbenzoyl)methionine methyl ester (2.42 g, 75%).

### Example 97D

### [4-(3-pyridyloxymethyl)-2-phenylbenzoyl]methionine methyl ester

To a 0 °C suspension in DMF (2 mL) of sodium hydride (90% in mineral oil, 38 mg, 0.95 mmol) was added a solution of 3-hydroxypyridine (95 mg, 1.0 mmol) in DMF (2 mL) dropwise and the mixture was stirred for 0.5 hours. A solution of (4-bromomethyl-2-phenylbenzoyl)methionine methyl ester (218 mg, 0.5 mmol) in DMF (1 mL) was added and the reaction mixture was stirred for 18 hours. The reaction mixture was poured into aqueous 2N sodium hydroxide and the mixture was extracted with ethyl acetate (3x). The combined organic extracts were dried, filtered and concentrated in vacuo. Chromatography on silica gel (60% ethyl acetate-hexanes, then ethyl acetate) gave [4-(3-pyridyloxymethyl)-2-phenylbenzoyl]methionine methyl ester (58 mg). ¹H NMR (300MHz, DMSO d₆) δ 12.66 (bs, 1H), 8.58 (d, 1H), 8.38 (d, 1H), 8.17 (dd, 1H), 7.30 - 7.56 (m, 10H), 5.29 (s, 2H), 4.29 (ddd, 1H), 2.23 (m, 2H), 1.98 (s, 3H), 1.84 (m, 2H). MS (Cl NH₃) m/e 454 (M + NH₄)⁺, 437 (M+H)⁺. Anal calcd for C₂₄H₂₄N₂O₄S (+ 0.41 H2O): C , 64.94; H, 5.64; N, 6.31. Found: C, 64.94; H, 5.35; N,S 6.14.

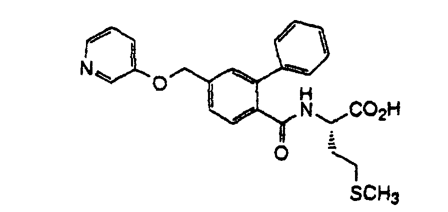

### Example 98

### [4-(3-pyridyloxymethyl)-2-phenylbenzoyl]methionine

The desired compound was prepared by saponification of [4-(3-pyridyloxymethyl)-2-phenylbenzoyl]methionine methyl ester, prepared as in Example 97, according to the procedure of Example 38. ¹H NMR (300 MHz., CDCl₃) δ 8.41 (dd, 1H), 8.26 (dd, 1H), 7.74 (d, 1H), 7.47 (dd, 1H), 7.43 (m, 6H), 7.14 (m, 2H), 5.92 (bd, 1H), 5.18 (s, 2H), 4.67 (ddd, 1H), 3.67 (s, 3H), 2.08 (t, 2H). 2.01 (s, 3H), 1.92 (m, 1H), 1.73 (m, 1H). MS (Cl NH₃) m/e 451 (M+H)⁺, 320, 288. Anal calcd for C₂₅H₂₆N₂O₄S: C, 66.65; H, 5.82; N. 6.22. Found: C, 66.53; H, 5.71; N, 6.16.

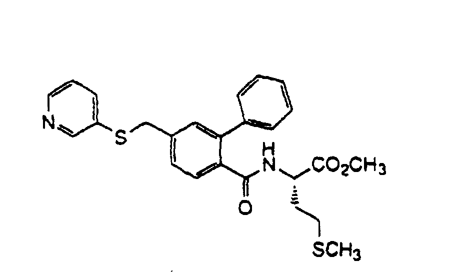

### Example 99

### [4-(3-pyridylthiomethyl)-2-phenylbenzoyl]methionine methyl ester

The desired compound was prepared by reaction of (4-bromomethyl-2-phenylbenzoyl)methionine methyl ester, prepared as in Example 97C, with 3-thiopyridine sodium salt, prepared as in Example 95C, according to the method of Example 97D. ¹H NMR (300MHz, CDCl₃) δ 8.56 (m, 1H), 8.45, (dd, 1H;), 7.66 (d, 1H), 7.38 (ddd, 1H), 7.30 - 7.47 (m, 6H), 7.21 (m, 2H), 5.87 (bd, 1H), 4.65 (ddd, 1H), 4.14 (s, 2H), 3.67 (s, 3H), 2.06 (m, 2H), 2.01 (s, 3H), 1.92 (m, 1H), 1.74 (m, 1H). MS (Cl NH₃) m/e 467 (M+H)⁺, 304. Anal calcd for C₂₅H₂₆N₂O₃S₂: C, 64.35; H, 5.62; N, 6.00. Found: C, 64.21; H, 5.61; N, 6.00.

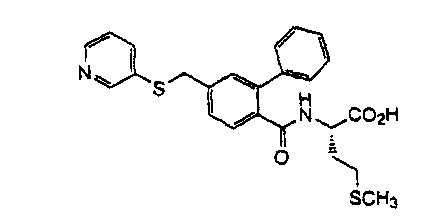

### Example 100

### [4-(3-pyridylthiomethyl)-2-phenylbenzoyl]methionine

The desired compound was prepared by saponification of [4-(3-pyridylthiomethyl)-2-phenylbenzoyl]methionine methyl ester, prepared as in Example 99 using the procedure of Example 38. ¹H nmr (300MHz., DMSO-d₆): δ 8.54, m, 1H; 8.39, dd, 1H; 7.83, m, 2H; 7.29 - 7.47, m, 8H; 4.39, s, 2H; 4.24, m, 1H; 2.25, m, 2H; 1.98, s, 3H; 1.85, m, 2H. MS (CI NH₃): 453 (MH⁺); 304, 194. EA: calc'd for C₂₄H₂₄N₂O₃S₂: C 63.69; H 5.34; N 6.19; found C 63.35; H 5.20; N 6.02.

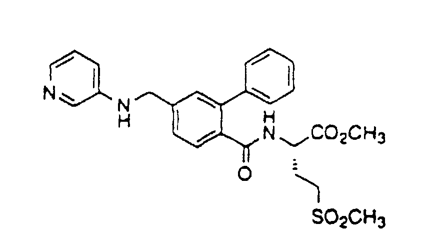

### Example 101

### N-[4-(3-pyridylaminomethyl)-2-phenylbenzoyl]-2-amino-4-(methylsulfonyl)butanoic acid

### Example 101A

### 2-amino-4-(methylsulfonyl)butanoic acid methyl ester

To a 0 °C suspension of 2-amino-4-(methylsulfonyl)lbutanoic acid (5.05 g, 27.9 mmol) in methanol (50 mL) was added thionyl chloride (3.0 mL, 41.8 mmol). The cold bath was allowed to warm to ambient temperature and the reaction mixture was stirred for 48 hours. The reaction mixture was dilted with water and taken to pH 6 with solid potassium carbonate. The aqueous mixture was extracted with dichloromethane (3x). The combined organic extracts were dried, filtered and concentrated in vacuo to give the methyl ester (1.14 g).

### Example 101B

### (4-carboxaldehyde-2-phenylbenzoyl)-2-amino-4-(methylsulfonyl)butanoic acid methyl ester

To a mechanically-stirred solution of 4-hydroxymethyl-2-phenylbenzoic acid (2.28 g, 10 mmol), prepared as in Example 37D, in dichloromethane (50 mL) was added MnO₂ and the reaction mixture was stirred overnight at ambient temperature. The reaction mixture was filtered and partitioned between ethyl acetate and aqueous 3N HCl, and the mixture was stirred vigorously for 0.5 hours. The mixture was filtered through Celite with ethyl acetate rinsings. The combined organic layers were dried, filtered and concentrated. Chromatography on silica gel gave 4-carboxaldehyde-2-phenylbenzoic acid (1.65 g).

The desired compound was prepared by coupling of 2-amino-4-(methylsulfonyl)butanoic acid, prepared as in Example 101A, and the 4-carboxaldehyde-2-phenylbenzoic acid, according to the method of Example 53C, escept tht no triethylamine was required.

### Example 101C

### N-[4-(3-pyridylaminomethyl)-2-phenylbenzoyl]-2-amino-4-(methylsulfonyl)butanoic acid methyl ester

The desired compound was prepared by reductive amination of the product of Example 101B with 3-aminopyridine. To a solution in methanol (3 mL) of (4-carboxyaldehyde-2-phenylbenzoyl)-1-amino-4-(methylsulfonyl) butanoic acid methyl ester (0.80 mmol), was added 3-aminopyridine (118 mg, 1.25 mmol) and acetic acid (0.90 mL). The reaction mixture was stirred for 30-40 minutes at ambient temperature and sodium cyanoborohydride (152 mg, 2.45 mmol) was added and stirring was continued for 2 hours. The reaction mixture was partitioned between ethyl acetate and aqueous 2N NaOH. The aqueous phase was extracted with ethyl acetate. The combined organic extracts were washed with 2N NaOH, twice with water, twice with brine, dried over Na₂SO₄, filtered, and concentrated in vacuo, followed by chromatography on silica gel (ethyl acetate). ¹H NMR (300MHz., CDCl₃) δ 8.09, (m, 1H; 8.00 (bd, 1H; 7.68 (d, 1H), 7.34 - 7.44 (m, 7H), 7.07 (dd, 1H), 6.88 (ddt, 1H), 5.99 (bd, 1H), 4.68 (ddd, 1H), 4.45 (bd, 2H), 4.24 (bs, 1H), 3.68 (s, 3H), 2.83 (s, 3H), 2.57 - 2.85 (m, 2H), 2.27 (m, 1H), 1.98 (m, 1H). MS (Cl NH₃⁻) m/e 482 (M+H)⁺. Anal calcd for C₂₅H₂₇N₃O5_{S}: C, 62.36; H, 5.65; N, 8.73. Found: C. 61.88; H. 5.69; N .8.60.

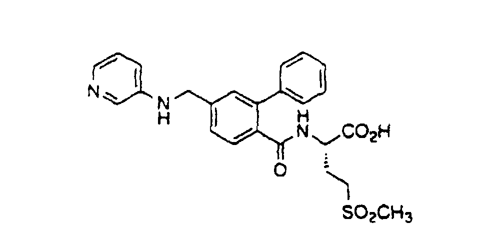

### Example 102

### N-[4-(3-pyridylaminomethyl)-2-phenylbenzoyl]-2-amino-4-(methylsulfonyl)butanoic acid

The desired compound was prepared by saponification of the product of Example 101C according to the procedure of Example 39H ¹H NMR (300 MHz., D₂O) δ 7.95 (m, 1H), 7.92 (m, 1H) 7.40 - 7.64 (m. 10H), 4.58 (s, 2H), 4.22, (ddd, 1H), 3.01 (s, 3H). 2.71 (m, 1H), 2.48 (m, 1H), 2.17 (m, 1H), 1.93 (m, 1H). MS FAB(⁺) m/e 468 (M+H)⁺. FAB(-); 466 (M-H)⁺.

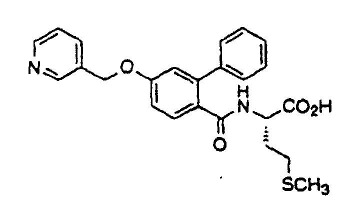

### Example 103

### [4-(3-pyridylmethyloxy)-2-phenylbenzoyl]methionine

### Example 103A

### 2,4-dihydroxybenzoic acid methyl ester

To a solution in methanol (50 mL) of 2,4-dihydroxybenzoic acid (1.54 g, 10 mmol) was added sulfuric acid (0.5 mL) and trimethyl orthoformate (1.6 mL, 15 mmol) and the reaction mixture was stirred at reflux for 36 hours. The reaction mixture was cooled to ambient temperature and diluted with water. The methanol was evaporated in vacuo. The residue was diluted with water and extracted with ether (3x). The combined ether extracts were washed with saturated aqueous sodium bicarbonate (2x) and brine, dried. filtered and concentrated in vacua to give 2,4-dihydroxybenzoic acid methyl ester (1.34 g) as a white solid.

### Example 103B

### 4-(3-pyridylmethyoxy)-2-hydroxybenzoic acid methyl ester

A mixture in acetone (40 mL) and water (10 mL) of 2,4-dihydroxybenzoic acid methyl ester (1.19 g, 7.08 mmol), prepared as in Example 103A, 3-chloromethylpyridine hydrochloride (2.32 g, 14.2 mmol) and potassium carbonate (2.44 g, 21.2 mmol) was Stirred at reflux for 24 hours, then acetone (10 mL) and piperidine (1 g) were added and reflux was continued for 12 hours. The reaction mixture was cooled to ambient temperature, poured into water, and extracted with ethyl acetate (3x). The combined organic extracts were washed with aqueous sodium hydroxide and water, dried, filtered and concentrated in vacuo. The residue was recrystallized from aqueous ethanol to give 4-(3-pyridylmethyloxy)-2-hydroxybenzoic acid methyl ester (0.57 g, 31%).

### Example 103C

### 4-(3-pyridylmethyloxy)-2-trifluoromethanesulfonyloxybenzoic acid methyl ester

To a -10 °C solution in pyridine (3 mL) of 4-(3-pyridylmethyloxy)-2-hydroxybenzoic acid methyl ester (0.56 g, 2.16 mmol), prepared as in Example 103B, was added triflic anhydride (0.73 mL, 4.32 mmol). The cold bath was allowed to warm to ambient temperature and the reaction mixture was stirred for 96 hours. The reaction mixture was poured into water, made basic with aqueous 2N sodium hydroxide and extracted with ethyl acetate. The combined organic extracts were washed with water (2x) and brine, dried, filtered and concentrated. Purification by chromatography on silica gel (60% ethyl acetate-hexanes) gave 4-(3-pyridylmethyloxy)-2-trifluoromethanesulfonyloxybenzoic acid methyl ester (519 mg, 61%).

### Example 103D

### 4-(3-pyridylmethyloxy)-2-phenylbenzoic acid methyl ester

The desired compound was prepared according to the method of Example 39A except substituting 4-(3-pyridylmethyloxy)-2-trifluoromethanesulfonyloxybenzoic acid methyl ester, prepared as in Example 103C for 2-iodoterephthalate.

### Example 103E

### 4-(3-pyridylmethyloxy)-2-phenylbenzoic acid

The desired compound was prepared by saponification of the product of Example 103D using the procedure of Example 97A.

### Example 103F

### [4-(3-pyridylmethyloxy)-2-phenylbenzoyl]methionine methyl ester

The desired compound was prepared by according to the procedure used in step C of the preparation of compound 8, except substituting 4-(3-pyridylmethyloxy)-2-phenylbenzoic acid, prepared as in Example 103E, for 4-nitro-2-phenylbenzoic acid.

### Example 103G

### [4-(3-pyridylmethyloxy)-2-phenylbenzoyl]methionine methyl ester

The desired compound was prepared by saponification of the compound of Example 103F using the procedure of Example 39H. ¹H NMR (300 MHz, DMSO-d₆) δ 8.69 (bs, 1H), 8.55 (bd, 1H), 8.39 (d, 1H), 7.88 (dt, 1H), 7.40 (m, 6H), 7.07 (dd, 1H), 7.03 (d, 1H), 5.17 (s, 2H), 4.28 (ddd, 1H), 2.25 (m, 2H), 2.00 (s, 3H), 1.84 (m, 2H). MS (Cl, NH₃) m/e 454 (M+NH₄)⁺, 437 (M+H)⁺,419, 320, 288. Anal calcd for C₂₄H₂₄N₂O₄S (+ 0.23 H₂O): C, 65.42; H, 5.59; N, 5.99. Found: C, 65.41; H; 5.42; N, 5.99.

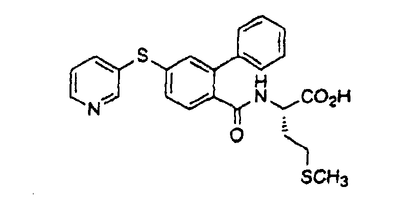

### Example 104

### [4-(3-pyridyl)thio-2-phenylbenzoyl]methionine

### Example 104A

### 3-pyridylthio-2-phenylbenzoic acid tert-butyl ester

To a mixture in DMF (2 mL) of 4-nitro-2-phenylbenzoic acid tert-butyl ester (403 mg, 1.35 mmol), prepared by esterification of 4-nitro-2-phenylbenzoic acid (compound 8, step B), and 3-thiopyridine sodium salt (224 mg, 1.68 mmol), prepared as in 232C, was stirred at 100 °C for 60 hours. The reaction mixture was cooled to ambient temperature and diluted with saturated aqueous sodium bicarbonate. The mixture was extracted with ether (3x). The combined ether extracts were dried over magnesium sulfate, filtered, and concentrated in vacuo to give a brown oil. Chromatography on silica gel (10% ethyl acetate-hexanes) gave 3-pyridylthio-2-phenylbenzoic acid *tert*-butyl ester as a colorless oil (248 mg, 51%).

### Example 104B

### 3-pyridylthio-2-phenylbenzoic acid

To a 0 °C solution in dichloromethane (1 mL) of 3-pyridylthio-2-phenylbenzoic acid *tert*-butyl ester (245 mg, 0.67 mmol), and triethylsilane (390 mg, 3.4 mmol) was added trifluoroacetic acid (1.53 g, 13.4 mmol) and the reaction mixture was wamred to ambient temperature and stirred for 18 hours. The reaction mixture was concentrated and azeotroped with toluene (3x) to give 3-pyridylthio-2-phenylbenzoic acid (209 mg) as a translucent film which was used without further purification.

### Example 104C

### [4-(3-pyridyl)thio-2-phenylbenzoyl]methionine

The desired compound was prepared according to the method of Examples 103F and G, except substituting 3-pyridylthio-2-phenylbenzoic acid for 4-(3-pyridyloxymethyl)-2-phenylbenzoic acid. 1H NMR (300 MHz, DMSO-d6) d 1.60 (m,. 1H), 1.85 (m, 1H), 2.00 (s, 3H), 2.10 (m, 2H), 4.50 (m, 1H), 5.85 (m, 1H), 7.25-7.40 (m, 8H), 7.60-7.80 (m, 2H). 8.45 (dd, 1H), 8.65 (dd, 1H). MS (Cl, NH₃) m/e 407 (M+H)⁺.

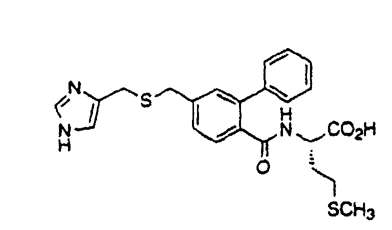

### Example 105

### [4-(1H-imidazol-4-ylmethylthiomethyl)-2-phenylbenzoyl]methionine

### Example 105A

### 1H-1-triphenylmethylimidazol-4-ylmethylthiolacetic acid

The desired compound was prepared according to the method of Example 89A, except substituting thiolacetic acid for HN₃.

### Example 105B

### 1H-1-triphenylmethylimidazol-4-ylmethylthiol sodium salt

A mixture of 1H-1-triphenylmethylimidazol-4-ylmethylthiolacetic acid (1.80 g, 4.5 mmol), prepared as in Example 105A, and sodium hydroxide (204 mg, 5.0 mmol) in 3:1 methanol-water was stirred for 18 hours at ambient temperature. The resulting tan solid was filtered and dried to give 1H-1-triphenylmethylimidazol-3-ylmethylthiol sodium salt which was used without further purification.

### Example 105C

### 4-(1H-1-triphenylmethylimadazol-4-ylmethyl)-2-phenyl benzoic acid methyl ester

A solution in DME of 1H-1-triphenylmethylimidazol-4-ylmethylthiol sodium salt (946 mg, 2.5 mmol), prepared as in Example 105B, and 4-bromomethyl-2-phenylbenzoic acid methyl ester (305 mg, 1.0 mmol) was stirred at 50 °C for 18 hours. The reaction mixture was concentrated and the residue purified by chromatography on silica gel (1:1 ethyl acetate-hexanes) to give 4-(1H-1-triphenylmethylimadazol-4-ylmethyl)-2-phenyl benzoic acid methyl ester.

### Example 105D

### 4-(1H-1-triphenylmethylimadazol-4-ylmethyl)-2-phenyl benzoic acid

A mixture of 4-(1H-1-triphenylmethylimadazol-3-ylmethyl)-2-phenyl benzoic acid methyl ester (200 mg, 0.34 mmol) and sodium hydroxide (69 mg, 1.7 mmol) in 3:1 methanol-water (0.18 mL) was stirred at raflux for 8 hours. The reaction mixture was concentrated and the residue taken up in water. The aqueous solution was taken to pH 5 and extracted. The organic extracts were dried over magnesium sulfate, filtered and concentrated in vacuo to give 4-(1H-1-triphenylmethylimadazol-4-ylmethyl)-2-phenyl benzoic acid (160 mg) as a solid.

### Example 105E

### [4-(1H-1-triphenylmethylimidazol-4-ylmethylthiomethyl)-2-phenylbenzoyl]methionine methyl ester

The desired compound was prepared by according to the procedure used in step C of the preparation of compound 8, except substituting 4-(1H-1-triphenylmethylimadazol-3-ylmethyl)-2-phenyl benzoic acid, prepared as in Example 105C, for 4-nitro-2-phenylbenzoic acid.

### Example 105F

### [4-(1H-1-triphenylmethylimidazol-4-ylmethylthiomethyl)-2-phenylbenzoyl]methionine

The desired compound was prepared by saponification of [4-(1H-imidazol-4-ylmethylthiomethyl)-2-phenylbenzoyl]methionine methyl ester, prepared as in Example 105E.

### Example 105G

### [4-(1H-imidazol-4-ylmethylthiomethyl)-2-phenylbenzoyl]methionine

The desired compound was prepared by deprotection of the compound of Example 105F using the procedure of Example 41D. ¹H NMR (300 MHz, DMSO-d6) δ 1.85 (m, 1H), 2.00 (s, 3H), 2.20-2.40 (m, 2H), 3.80 (s, 2H), 3.85 (s, 2H), 4.30 (m, 1H), 7.40 (m, 8H), 7.50 (s, 2H), 8.50 (d, 1H), 8.90 (s, 1H), 13.0 (br s, 1H). MS (Cl, NH₃) m/e 456 (M+H)⁺.

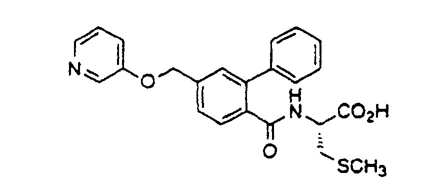

### Example 106

### [4-(3-pyridyloxymethyl)-2-phenylbenzoyl]cysteine

### Example 106A

### Cysteine methyl ester hydrochloride

To a 0 °C slurry in methanol of L-cysteine (1.23 g, 9.1 mmol) was added thionyl chloride (0.75 mL, 10.3 mmol). The cold bath was removed and the reaction mixture was stirred for 15 minutes and then ovemight at 45 °C. The reaction mixture was cooled to ambient temperature and concentrated to a white solid. The white solid was azeotroped with methanol to give cysteine methyl ester hydrochloride.

### Example 106B

### [4-(3-pyridyloxymethyl)-2-phenylbenzoyl]cysteine methyl ester

The desired compound was prepared by coupling of cysteine methyl ester hydrochloride and 4-(3-pyridylmethyloxy)-2-phenylbenzoic acid, using the procedure of Example 52A.

### Example 106C

### [4-(3-pyridyloxymethyl)-2-phenylbenzoyl]cysteine

The desired compound was prepared by saponification of the product of Example 106B. ¹H NMR (300 MHz, DMSO-d₆) d 8.77 (d, 1H), .58 (d, 1H), 8.35 (d, 1H), 7.85 (dd. 1H), 7.63 (dd. 1H), 7.52 (m, 5H), 7.36 (m, 3H), 5.38 (s, 2H), 4.44 (m. 1H), 3.90 (dd. 1H), 3.72 (dd. 1H), 2.05 (s, 3H). MS (DCI-NH₃) m/e 423 (M+H)⁺, 440 (M+NH₄)⁺.

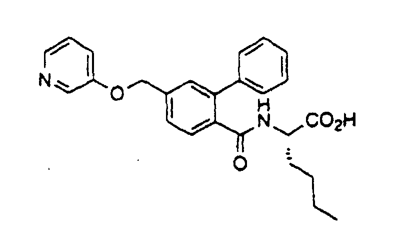

### Example 107

### N-[4-(3-pyridyloxymethyl)-2-phenylbenzoyl]norleucine

The desired compound was prepared according to the method of Example 106, except substituting norleucine methyl ester hydrochloride for cysteine methyl ester hydrochloride. ¹H NMR (300 MHz, DMSO-d₆) δ 8.60 (d, 1H), 8.53 (d, 1H), 8.37 (d, 1H), 7.90 (dd, 1H), 7.70 (dd, 1H), 7.52 (d, 1H), 7.51 (s, 1H), 7.42 (m, 3H), 7.38 (m, 3H), 5.38 (s, 2H), 4.16 (m, 1H), 1.60 (m, 2H), 1.20 (m, 2H), 1.10 (m, 2H), 0.82 (t, 3H). MS (DCl-NH₃) m/e 419 (M+H)⁺, 436 (M+NH₄)⁺.

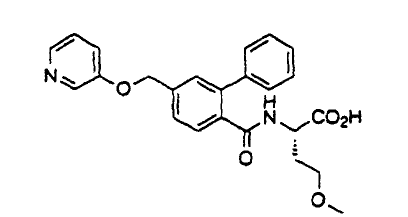

### Example 108

### N-[4-(3-pyridyloxymethyl)-2-phenylbenzoyl]-2-amino-3-methoxybutyric acid

The desired compound was prepared according to the method of Example 106, except substituting L-2-amino-3-methoxybutyric acid methyl ester hydrochloride for cysteine methyl ester hydrochloride. ¹H NMR (300 MHz, DMSO-d₆) δ 8.57 (d, 1H), 8.42 (d, 1H), 8.22 (d, 1H), 7.60 (dd, 1H), 7.50 (m, 2H), 7.40 (m, 7H), 5.33 (s, 2H), 4.24 (m, 1H), 3.17 (s, 3H), 3.15 (m, 2H), 1.93 (m, 1H), 1.77 (m, 1H). MS (APCI) m/e 421 (M+H)⁺. 419 (M-H)⁻. Anal calcd for C₂₄H₂₄N₂O₅. 0.5 H₂O: C, 67.12; H, 5.87; N, 6.52. Found: C, 67.38; H, 5.57; N, 6.72.
The next example is Example 136.

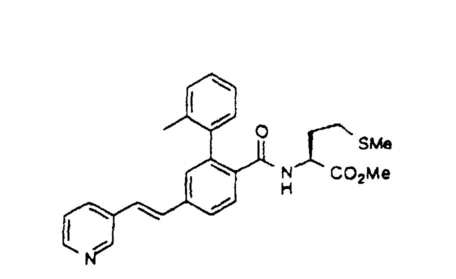

### Example 136

### {4-[2-(pyrid-3-yl)ethenyl]-2-(2-methylphenyl)benzoyl}methionine methyl ester

The desired compound was prepared according to the method of Example 76, except substituting 2-methylphenylboronic acid for phenylboronic acid. ¹H NMR (300 MHz, CDCl₃) δ 8.74 (d, 1H), 8.52 (d, 1H), 8.01 (dd, 1H), 7.88 (dd, 1H), 7.62 (dd, 1H), 7.40-7.28 (m. 6H), 7.19,7.18 (2 d's, 2H), 5.95 (d, 1H), 4.65 (m, 1H), 3.67 (s, 3H), 2.23.2.11 (2 s's, 3H), 2.10-2.00 (m, 2H), 2.03 (s, 3 H), 1.89 (m, 1H), 1.61 (m, 1H). MS (Cl⁺) m/e 484 (M+H)⁺.

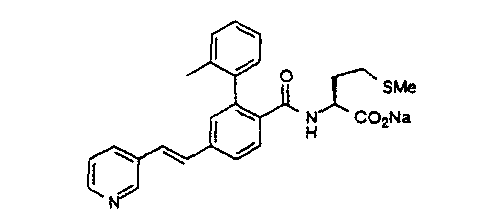

### Example 137

### {4-[2-(pyrid-3-yl)ethenyl]-2-(2-methylphenyl)benzoyl}methionine sodium salt

To a solution of {4-[2-(pyrid-3-yl)ethenyl]-2-(2-methylphenyl)benzoyl}methionine methyl ester, prepared as in Example 136, (3.285 g, 7.13 mmol) in methanol (10 mL) was added a solution of sodium hydroxide (0.979 N, 7.35 mL). After 15 hours, the solvent was evaporated *in vacuo* to give the title compound (3.35 g, 100%). ¹H NMR (300 MHz, DMSO-d₆) δ 8.79 (d, 1H), 8.46 (dd, 1H), 8.05 (dt, 1H), 7.70-7.53 (m, 3H), 7.48 -7.37 (m, 4H), 7.27-7.18 (m, 3H),6.97 (m, 1H), 3.50 (m, 1H), 2.21,2.03 (2 s's, 3H), 2.00-1.92 (m, 2H), 1.93 (s, 3H), 1.70 (m, 1H), 1.58 (m, 1H). MS (APCl) m/e 445 (M-H)⁻ as the acid form.
The next example is Example 139.

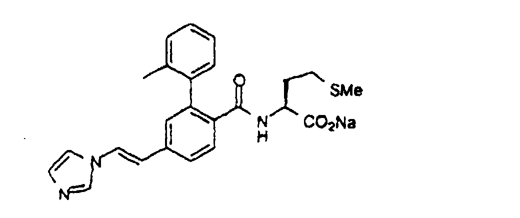

### Example 139

### {4-[2-(1H-1-imidazolyl)ethenyl]-2-(2-methylphenyl)benzoyl}methionine sodium salt

### Example 139A

### {4-[2-(1H-1-imidazolyl)ethenyl]-2-(2-methylphenyl)benzoyl}methionine methyl ester

The desired compound was prepared according to the method of Example 136, except substituting 1-vinylimidizole for 3-vinylpyridine.

### Example 139B

### {4-[2-(1H-1-imidazolyl)ethenyl]-2-(2-methylphenyl)benzoyl}methionine sodium salt

The desired compound was prepared by saponification of the compound of Example 139A according to the method of Example 137. ¹H NMR (300 MHz, DMSO-d₆): δ 8.00 (d, 1H), 7.95 (d, 1H), 7.69 (s, 1H), 7.61-7.51 (m, 2H), 7.37-6.92 (m, 8H), 2.20.2.00 (2 s's, 3H), 2.44-1.92 (m, 2H), 1.93 (s, 3H), 1.70 (m, 1H), 1.58 (m, 1H). MS (APCl) m/e 434 (M-H)- as the acid form.

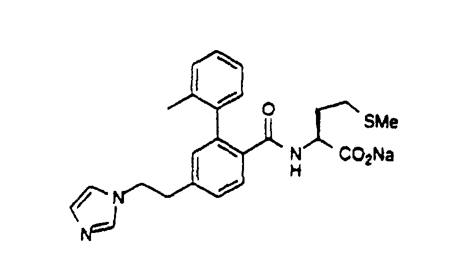

### Example 140

### {4-[2-(1H-1-imidazolyl)ethyl]-2-(2-methylphenyl)benzoyl}methionine sodium salt

### Example 140A

### {4-[2-(1H-1-imidazolyl)ethyl]-2-(2-methylphenyl)benzoyl}methionine methyl ester

A mixture of the product of Example 139A (171 mg, 0.38 mmol) and palladium (10%) on carbon (489 mg, 0.46 mmol of palladium) in methanol was flushed with hydrogen, and stirred under a hydrogen balloon for 5 hours. The mixture was then filtered through Celite, rinsed with ethyl acetate, and concentrated *in vacuo*. The residue was purified by column chromatography (5% methanol-ethyl acetate) to give the title compound (97 mg, 56%).

### Example 140B

### {4-[2-(1H-1-imidazolyl)ethyl]-2-(2-methylphenyl)benzoyl}methionine sodium salt

The desired compound was prepared by saponification of the product of Example 140A using the procedure of Example 137. ¹H NMR (300 MHz, DMSO-d₆) δ 7.62 (s, 1H), 7.51 (s, 1H), 7.44 (d, 1H), 7.36-7.14 (m, 5H), 6.98-6.82 (m, 3H). MS (APCl -) m/e 436 (M-H)⁻ as the acid form.

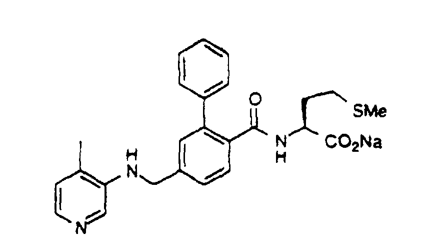

### Example 141

### [4-(4-methylpyrid-3-ylaminomethyl)-2-phenylbenzoyl]methionine sodium salt

### Example 141A

### 4-methyl-3-aminopyridine

A mixture of 4-methyl-3-nitropyridine (414.4 mg, 3 mmol) and palladium (10%) on carbon (100 mg) in methanol (5 mL) was flushed with hydrogen, and stirred under a hydrogen balloon for 5 hours. The mixture was then filtered through Celite, rinsed with methanol, and concentrated *in vacuo.* The residue was used without further purification.

### Example 141B

### 4-(4-methylpyrid-3-ylaminomethyl)-2-phenylbenzoic acid methyl ester

A mixture of 4-methyl-3-aminopyridine (3.0 mmol), prepared as in Example 141A, 4-carboxaldehyde-2-phenylbenzoic acid methyl ester (480 mg, 2 mmol), prepared as in Example 39B, molecular sieves (size 4Å, 1 g) and p-toluenesulfonic acid (10 mg) in toluene (3 mL) were stirred at 80 °C for 6 hours. After the reaction was cooled to room temperature, THF (2 mL), sodium borohydride (200 mg, 6 mmol), and ethanol (2 mL) was added to the reaction mixture sequentially. After 15 hours at room temperature, the reaction mixture was filtered through Celite and rinsed with ethyl acetate (80 mL). The organic phase was washed with saturated aqueous ammonium chloride, water and brine, dried over anhydrous sodium sulfate, filtered and concentrated *in vacuo*. The residue was then purified by column chromatography (ethyl ether) to give the title compound (454 mg, 66%).

### Example 141C

### [4-(4-methylpyrid-3-ylaminomethyl)-2-phenylbenzoyl]methionine methyl ester

A solution of the product of Example 141B (446 mg, 1.3 mmol) and aqueous saturated lithium hydroxide (3 mL) in methanol (5 mL) was heated at 60 °C for 15 hours. The reaction mixture was then neutralized with hydrogen chloride (4 N in dioxane, 5 mL). The reaction mixture was concentrated *in vacuo* to dryness. To the residue was added sequentially *L*-methionine methyl ester hydrochloride (311 mg, 1.56 mmol), 3-hydroxy 1,2,3-benzotriazin-4(3*H*)-one (318 mg, 1.95 mmol), 1-(3-dimethylaminopropyl)-3-ethylcarbodiimide (374 mg, 1.95 mmol), THF (10 mL) and pyridine (1 mL). After 15 hours, the reaction mixture was diluted with ethyl acetate, washed with water and brine, dried over anhydrous magnesium sulfate, filtered and concentrated *in vacuo*. The residue was then purified by column chromatography (ethyl acetate) to give the title compound.

### Example 141D

### [4-(4-methylpyrid-3-ylaminomethyl)-2-phenylbenzoyl]methionine sodium salt

The desired compound was prepared by saponification of the product of Example 141C using the procedure of Example 137. ¹H NMR (300 MHz, DMSO-d₆) δ 7.72 (s, 1H), 7.70 (d, 1H), 7.41-7.36 (m, 7H), 7.15 (d, 1H), 6.96 (d, 1H), 5.93 (br t, 1H), 4.49 (d, 2H), 3.78 (m, 1H), 2.17 (s, 3H), 2.16-2.02 (m, 2H), 1.95 (s, 3H), 1.85-1.08 (m, 2 H). MS (APCl +) m/e 450 (M+H)⁺.

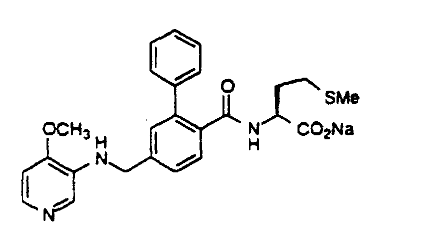

### Example 142

### {4-(4-methoxypyrid-3-ylaminomethyl)-2-phenylbenzoyl]methionine sodium salt

The desired compound was prepared according to the method of Example 141, except substituting 4-methoxy-3-nitropyridine for 4-methyl-3-nitropyridine. ¹H NMR (300 MHz, DMSO-d₆) δ 7.75 (d, 1H), 7.66 (s, 1H), 7.40-7.30 (m, 7H), 7.13 (d, 1H), 6.83 (d, 1H), 5.77 (t, 1H), 4.42 (d, 1H), 3.86 (s, 3H), 3.73 (m, 1H), 2.10 (m, 2H), 1.95 (s, 3H), 1.75 (m, 2H). MS (APCl⁺) m/e 466 (M+H)⁺.

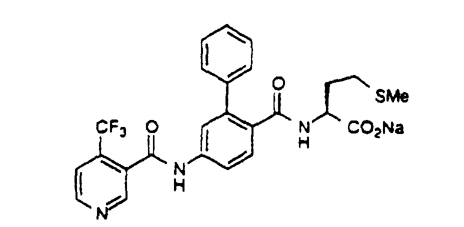

### Example 143

### [4-(4-trifluoromethylpyrid-3-ylcarbonylamino)-2-phenylbenzoyl]methionine sodium salt

### Example 143A

### [4-(4-trifluoromethylpyrid-3-ylcarboxyamino)-2-phenylbenzoyl]methionine methyl ester

A mixture of 4-trifluoromethylnicotinic acid (100 mg, 0.523 mmol), (4-amino-2-phenylbenzoyl)methionine methyl ester hydrochloride (206 mg, 0.52 mmol), prepared as in Example 59B, 3-hydroxy1,2,3-benzotriazin-4(3*H*)-one (120 mg, 0.628 mmol), 1-(3-dimethylaminopropyl)-3-ethylcarbodiimide (125 mg, 0.628 mmol) in THF (5 mL) was stirred 15 hours. The reaction mixture was diluted with ethyl acetate , washed with water and brine, dried over anhydrous magnesium sulfate, filtered and concentrated *in vacuo*. The residue was purified by column chromatography (50% ethyl acetate-hexane) to give the title compound (157 mg, 57%).

### Example 143B

### [4-(4-trifluoromethylpyrid-3-ylcarbonylamino)-2-phenylbenzoyl]methionine sodium salt

The desired compound was prepared by saponification of the product of Example 143A using the procedure of Example 137. ¹H NMR (300 MHz, DMSO-d₆) δ 10.99 (br s, 1H), 9.03 (s, 1H), 8.97 (d, 1H), 7.89 (d, 1H), 7.72 (d, 1H), 7.68 (dd, 1H), 7.48 (d, 1H), 7.41-32 (m, 5H), 7.12 (d, 1H), 3.77 (m, 1H), 2.10 (m, 2H), 2.01 (s, 3H), 1.75 (m, 2H). MS (APCl⁺) m/e 518 (M+H)⁺.

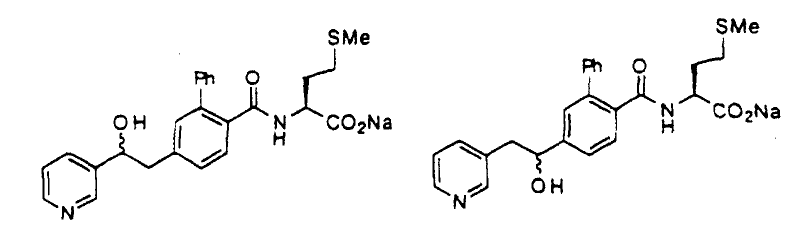

### Example 144

### {4-[2-(3-pyridy)-2-hydroxyethyl]-2-phenylbenzoyl}methionine sodium salt and {4-[2-(3-pyridy)-1-hydroxyethyl]-2-phenylbenzoyl}methionine sodium salt

### Example 144A

### 4-[2-(3-pryidyl)ethenyl]-2-phenylbenzoic acid methyl ester

A mixture of the 4-iodo-2-phenylbenzoic acid methyl ester (6.11 g, 18.1 mmol), prepared as in Example 76C, 3-vinylpyridine (2.85 g, 27.1 mmol), prepared as in Example 76E, [1,1'-bis(diphenylphosphino)ferrocene]palladium(II) chloride, complexed to dichloromethane (1:1) (444 mg, 0.543 mmol) and triethylamine (5.05 g, 36.2mmol), in 1-methyl-2-pyrrolidinone (30 mL) was degassed with nitrogen and heated at 80 °C for 18 hours. The reaction mixture was diluted with ether, filtered through silica gel, and rinsed with ethyl acetate. The filtrate was washed with water and brine, dried over anhydrous magnesium sulfate, filtered and concentrated *in vacuo*. The residue was purified by column chromatography (30% ethyl acetate-hexane) to give the title compound (4.82 g, 84%).

### Example 144B

### 4-[2-(3-pryidyl)-1,2-dihydroxyethyl]-2-phenylbenzoic acid methyl ester

To a solution of the product of Example 144A (575 mg, 1.83 mmol), 4-methylmorpholine *N*-oxide (642 mg, 5.48 mmol), methylsulfonamide (174 mg, 1.83 mmol) amd quinuclidine (203 mg, 1.83 mmol) in *tert*-butanol (5 mL) and water (5mL) was added a solution of osmium tetraoxide (2.5 wt% in *tert*-butanol, 1.2 mL, 0.091 mmol). The mixture was then stirred at 70 °C for 5 hours. The reaction mixture was diluted with ethyl acetate, washed with water and brine, dried over anhydrous sodium sulfate, filtered, and concentrated *in vacuo*. The residue was purified by column chromatography (ethyl acetate and 2% methanol-ethyl acetate) to give the title compound (323 mg, 51%).

### Example 144C

### 4-[2-(3-pyridyl)-1,2-dihydroxyethyl thio ketal]-2-phenylbenzoic acid methyl ester

A. solution of the product of Example 144B (250 mg, 0.716 mmol) and 1,1'-thiocarbonyldiimidazole (171 mg, 0.86 mmol) in THF (5 mL) was stirred at 50 °C for 5 hours. The reaction mixture was diluted with ether, washed with saturated aqueous ammonium chloride, water and brine, dried over anhydrous magnesium sulfate, filtered and concentrated *in vacuo.* The residue was purified by column chromatography (50% ethyl acetate-hexane) to give the title compound (227 mg, 81%).

### Example 144D

### 4-[2-(3-pryidyl)-1-hydroxyethyl]-2-phenylbenzoic acid methyl ester and 4-[2-(3-pryidyl)-2-hydroxyethyl]-2-phenylbenzoic acid methyl ester

A solution of the product of Example 144C (220 mg, 0.562 mmol), tributyltin hydride (0.30 mL, 1.1 mmol) and azobisisobutyronitrile (AIBN, 10 mg) in toluene was heated at 110 °C for 2 hours. The reaction mixture was diluted with ether, washed with 10% aqueous sodium hydroxide, water and brine, dried over anhydrous magnesium sulfate, filtered and concentrated *in vacuo*. The residue was purified by column chromatography (20%, then 50% ethyl acetate-hexane, then ethyl acetate) to give the bis-deoxy compound as the first fraction (53 mg, 30%), and the desired product as the second fraction (117 mg, 63%, a mixture of two regioisomers).

### Example 144E

### {4-[2-(3-pyridy)-2-hydroxyethyl]-2-phenylbenzoyl}methionine sodium salt and {4-[2-(3-pyridy)-1-hydroxyethyl]-2-phenylbenzoyl}methionine sodium salt

The desired compounds were prepared from the product of Example 144D according to the method of Examples 141C and D. ¹H NMR (300 MHz, DMSO-d₆) δ 8.61-8.37 (m, 2H), 7.79-7.60 (m, 1H), 6.02-7.00 (m, 10H), 3.88 (m, 1H),3.77 (m, 1H), 2.95 (m, 2H), 2.15-2.02 (m, 2H), 2.00,1.99,1.96,1.95 (4 s's, 3H), 1.90-1.70 (m, 2H). MS (APCl⁺) m/e 451 (M+H)⁺.

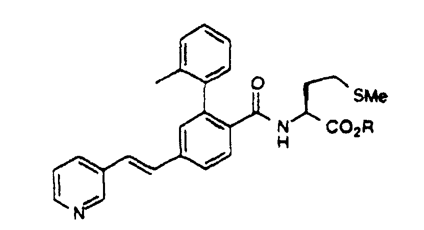

### Examples 145-149

### Example 145

### {4-[2-(pyrid-3-yl)ethenyl]-2-(2-methylphenyl)benzoyl}methionine butyl ester

{4-[2-(pyrid-3-yl)ethenyl]-2-(2-methylphenyl)benzoyl}methionine (138 mg, 0.30 mmol) was heated at 100°C for 2 hours in n-butanol (5 mL) with 1 drop of H₂SO₄. The reaction was evaporated to dryness, partitioned between ethyl acetate and 5% NaHCO₃, washed with water and brine, and dried over Na₂SO₄ to provide the title compound in 86% yield. ¹H NMR (CDCl₃, 300 MHz) δ 0.92 (t, 3H), 1.35 (m, 2H), 1.60 (m, 2H), 1.86 (m, 1H), 2.1 (m, 9H), 4.08 (m, 2H), 4.62 (m, 1H), 5.97 (d, 1H), 7.18-8.04 (m, 11H), 8.53 (s, 1H), 8.77 (s, 1H). MS m/e 503 (M+H)⁺.

### Example 146

### N-(4-(3-Pyridylethylenyl)-2-(2-tolyl)benzoyl)-L-methionine octadecyl ester

{4-[2-(pyrid-3-yl)ethenyl]-2-(2-methylphenyl)benzoyl}methionine (50 mg, 0.11 mmol), 1-octadecanol (30 mg, 0.11 mmol), and carbonyldiimidazole (18 mg, 0.11 mmol) were combined and dissolved in THF (2 mL) and heated to reflux for 18 hours. The mixture was diluted with ethyl acetate and washed with 5% NaHCO₃ and brine and dried over Na₂SO₄. Flash chromatography (50% ethyl acetate-hexane, provided the title compound (35.4 mg). MS m/e 699 (M+H)⁺.

### Example 147

### {4-[2-(pyrid-3-yl)ethenyl]-2-(2-methylphenyl)benzoyl}methionine dimethylaminoethyl ester

The desired compound was prepared according to the method of Example 145, except substituting N,N-dimethylethanolamine for n-butanol. ¹H NMR (CDCl₃, 300 MHz) δ 1.62 (m, 1H), 1.87 (m, 1H), 2.1 (m, 12H), 2.40 (m, 4H), 2.72 (m, 1H), 4.28 (m, 1H), 4.59 (m, 1H), 6.05 (m, 1H), 7.18-8.03 (m, 11H), 8.52 (m, 1H), 8.75 (m, 1H). MS m/e 518 (M+H)⁺.

### Example 148

### {4-[2-(pyrid-3-yl)ethenyl]-2-(2-methylphenyl)benzoyl}methionine acetlyloxymethyl ester

{4-[2-(pyrid-3-yl)ethenyl]-2-(2-methylphenyl)benzoyl}methionine (75 mg, 0.17 mmol), bromomethyl acetate (26 mg, 0.17 mmol), and potassium iodide (9 mg, 0.06 mmol) were dissolved in DMF (2 mL), treated with sodium hydride (60% suspension in mineral oil, 6.7 mg, 0.17 mmol), and heated at 100°C for 8 hours. The mixture was diluted with ethyl acetate, washed with 5% NaHCO₃ and brine and dried over sodium sulfate. Chromatography on silica gel (50% ethyl acetate-hexane) afforded the title compound. ¹H NMR (CDCl₃, 300 MHz) δ 1.60 (m, 1H), 1.89 (m, 1H), 2.0-2.2 (m, 11H), 4.64 (m, 1H), 5.72 (m, 2H), 5.91 (m, 1H), 7.15-7.64 (m, 9H), 8.02 (m, 2H), 8.57 (m, 1H), 8.79 (m, 1H). MS m/e 519 (M+H)⁺.

### Example 149

### {4-[2-(pyrid-3-yl)ethenyl]-2-(2-methylphenyl)benzoyl}methionine pivaloyloxymethyl ester

The desired compound was prepared according to the method of Example 148, except substituting chioromethyl pivalate for bromomethyl acetate. ¹H NMR (CDCl₃, 300 MHz) δ 1.19 (s, 9H), 1.59 (m, 1H), 1.84 (m, 1H), 2.1 (m, 8H), 4.63 (m, 1H), 5.72 (m, 2H), 5.88 (m, 1H), 7.15-7.41 (m, 8H), 7.62 (m, 1H), 8.0 ( m, 2H), 8.54 (m, 1H), 8.77 (m, 1H). MS m/e 561 (M+H)⁺.

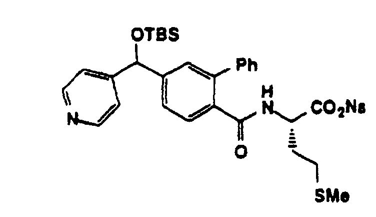

### Example 150

### [4-(4-pyridyl-t-butyldimethylsilyloxymethyl)-2-phenylbenzoyl]methionine sodium salt

### Example 150A

### 4-(4-Pyridylhydroxymethyl)-2-phenylbenzoic acid methyl ester

A solution of 4-bromopyridine (0.32 g, 2.0 mmol) in ether (10 mL) was cooled to -78°C and treated with butyllithium. After 10 minutes, 4-carboxaldehyde-2-phenylbenzoic acid methyl ester (0.53 g, 2.2 mmol), in ether (5 mL) was added. Stirring was continued for 15 minutes before allowing the reaction to warm to ambient temperature over 2 hours. The reaction mixture was evaporated to dryness. The residue was dissolved in ethyl acetate and washed with water and brine, dried and concentrated. Chromatography on silica gel (ethyl acetate) gave the title compound (769 mg).

### Example 150B

### 4-(4-Pyridyl-t-butyldimethylsilyloxymethyl)-2-phenylbenzoic acid methyl ester

4-(4-Pyridylhydroxymethyl)-2-phenylbenzoic acid methyl ester (769 mg, 2:41 mmol), prepared as in Example 150A, diisopropylethylamine (0.84 mL, 4.8 mmol), and *t*-butyldimethylsilyl triflate (1.1 mL, 4.8 mmol) were dissolved in methylene chloride (50 mL) and stirred for 18 hours. TLC indicated the presence of the alcohol so additional base (1 mL) and triflate (0.5 mL) were added. After 15 minutes, all starting alcohol was consumed. The reaction mixture was washed with water, 5% NaHCO₃, and brine, dried over Na₂SO₄, and concentrated. Chromatography on silica gel (20% ethyl acetate-hexane) provided the desired compound in a 93% yield.

### Example 150C

### 4-(4-Pyridyl-t-butyldimethylsilyloxymethyl)-2-phenylbenzoic acid

4-(4-Pyridyl-*t*-butyldimethylsilyloxymethyl)-2-phenylbenzoic acid methyl ester (0.97 g, 2.24 mmol), prepared as in Example 150B, was dissolved in methanol. Saturated aqueous LiOH (1 mL) was added, and the solution was reluxed overnight. The reaction was evaporated to dryness and partititioned between ethyl acetate and water. The organic layer was dried over Na₂SO₄ to provide the title compound in 27% yield.

### Example 150D

### [4-(4-Pyridyl-t-butyldimethylsilyloxymethyl)-2-phenylbenzoyl)methionine methyl ester

The desired compound was prepared by coupling of the product of Example 150C and methionine methyl ester hydrochloride.

### Example 150E

### [4-(4-pyridyl-t-butyldimethylsilyloxymethyl)-2-phenylbenzoyl]methionine sodium salt

[4-(4-pyridyl-*t*-butyldimethylsilyloxymethyl)-2-phenylbenzoyl)methionine methyl ester (25.0 mg, 44 µmol), prepared as in Example 150D, was dissolved in methanol (5 mL) and stirred with NaOH (1.0 M, 44 µmol) at 55°C for 72 hours. The reaction was evaporated to dryness and lyophilized from water to provide the title compound (19.4). ¹H NMR (d₆-DMSO, 300 MHz) δ 0.03 (s, 6H), 0.91 (s, 9H), 1.75 (m. 1H), 1.96 (m, 3H), 2.09 (m, 1H), 2.5 (m. 2H), 3.75 (m, 1H), 6.02 (s, 1H), 7.2-7.5 (m, 10H), 8.50 (m. 2H). MS m/e 551 (M+H)⁺.

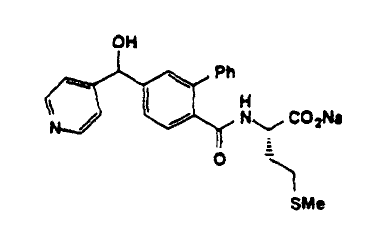

### Example 151

### [4-(4-pyridylhydroxymethyl)-2-phenylbenzoyl]methionine sodium salt

The desired compound was prepared from 4-(4-Pyridylhydroxymethyl)-2-phenylbenzoic acid methyl ester, prepared as in Example 297A according to the method of Examples 150C, D, and E. ¹H NMR (CDCl₃, 300 MHz) δ 1.78 (m, 1H), 1.96 (m, 3H), 2.09 (m, 3H), 3.73 (m, 1H), 5.80 (s, 1H), 7.13 (m, 1H), 7.2-7.5 (m, 10H), 8.48 (m, 2H). MS m/e 437 (M+H)⁺.

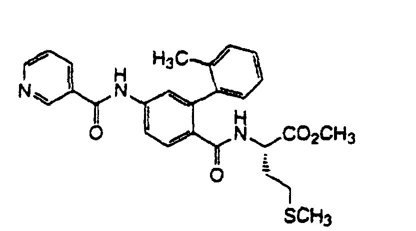

### Example 152

### [4-(3-pyridylcarbonylamino)-2-(2-methylphenyl)benzoyl]methionine methyl ester

To a stirred solution of the [4-amino-2-(2-methylphenyl)benzoyl]methionine methyl ester (85 mg, 0.23 mmol) in CH₂Cl₂ (5 mL) was added nicotinic acid chloride hydrochloride (81 mg, 0.46 mmol) and saturated NaHCO₃ (2 mL). The reaction was stirred at ambient temperature for 2 hours. The reaction was diluted with CH₂Cl₂ (10 mL), the layers were separated and the organic layer washed with saturated aqueous NaHCO₃ (5 mL), dried (MgSO₄) and concentrated in vacuo. Flash chromatography (CH₂Cl₂-methanol 50:1) and crystallization from ethyl acetate gave the desired compound (87 mg, 80%) as a white powder. ¹H NMR (300 MHz, CDCl₃) δ 9.10 (dd, 1H, *J*= 2.4, 1.0 Hz), 8.80 (dd, 1H, *J*= 4.7, 1.7 Hz), 8.21 (ddd, 1 H, *J*= 7.8, 2.4, 1.7 Hz), 8.09-8.00 (m, 2H), 7.71-7.66 (m, 1H), 7.64-7.61 (m, 1H), 7.46 (ddd: 1H, *J*=7.8, 4.7, 1.0 Hz), 7.35-7.20 (m, 4H), 5.92 (bd, *J*= 7.5 Hz), 4.67-4.57 (m. 1H), 3.66 (s. 3H), 2.23-2.01 (4s and m, 8H), 2.13-2.00 (m, 1H), 1.65-1.52 (m, 1H). MS *m*/*z* 478 (M+1)⁺.

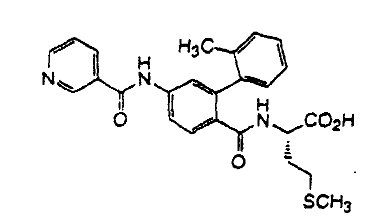

### Example 153

### [4-(3-pyridylcarbonylamino)-2-(2-methylphenyl)benzoyl]methionine

To a stirred solution of the product of Example 152 (140 mg, 0.29 mmol) in THF (6 mL) was added a solution of LiOH·H₂O (37 mg, 88 mmol) in H₂O (1 mL) and the resulting solution stirred for 2 hours at ambient temperature. The reaction was concentrated in vacuo and 1 N HCl was added to the residue. The resulting precipitate was filtered and washed with H₂O. Lyopholization gave the title compound (87 mg, 59%) as a white powder. ¹H NMR (300 MHz, DMSO-d6, 90 °C) δ 9.12 (d, 1H, *J*= 2.4 Hz), 8.74 (dd. 1H, *J*= 4.9, 1.9 Hz), 8.31 (dt, 1H, *J*= 7.9, 1.8 Hz), 7.84 (dd, 1H, *J*= 7.9, 1.8 Hz), 7.63 (s, 1H), 7.61 (d, 1H, *J*= 2.4 Hz), 7.54 (dd, 1H, *J*= 7.9, 4.9 Hz), 7.45 (d, 1H, *J*= 7.9 Hz), 7.23-7.21 (m, 2H), 7.19-7.15 (m, 2H), 4.30-4.26 (m, 1H), 2.28-2.22 (m, 1H), 2.20-2.14 (m, 1H), 2.11 (s, 3H), 1.98 (s, 3H), 1.88-1.81 (m, 1H), 1.75-1.68 (m, 1H). MS *m*/*z* 464 (M+1)⁺, 446. Anal calcd for C₂₅H₂₅N₃O₄S ·HCl·0.5 H₂O (509.01): C, 58.99; H, 5.35; N, 8.26. Found: C, 59.38; H, 5.49; N, 7.89.

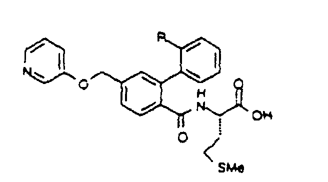

### Examples 154-156

### Example 154

### [4-(3-pyridyloxymethyl)-2-(2-trifluoromethylphenyl)benzoyl]methionine

### Example 154A

### 4-hydroxymethyl-2-aminobenzoic acid methyl ester

To a solution of dimethylaminoterphthalate (3.07 g, 14.7 mmol) in 30 mL of a 2:1 mixture of THF : Et₂O at -78°C was added neat DIBAL (6.27 g, 44.1 mmol, 3.0 eq.) and the reaction was warmed to 0 °C over 4 hours. The reaction was quenched with 5 mL of methanol followed by 5 mL of saturated aqueous sodium tartrate. The mixture was stirred overnight and then was taken up in ethyl acetate. The layers were separated and the ethyl acetate layer was washed with saturated aqueous NaHCO₃and brine and then dried over Na₂SO₄, filtered and evaporated to an oil. Purification by chromatography on silica gel (50% ethyl acetate-hexane) gave the desired compound (1.03 g, 39%) of **1b** as a colorless oil.

### Example 154B

### 4-hydroxymethyl-2-aminobenzoic acid methyl ester

To a stirred solution of the product of Example 154A (152 mg, 0.84 mmol) in acetone (20 mL) and 3N H₂SO₄ (20 mL) at -15 °C was added a solution of NaNO₂ (1.34 g, 79.4 mmol) in H₂O (10 mL) dropwise by addition funnel. After the addition was complete, urea (210 mg, 3.52 mmol) was added followed by a solution of KI (5.11 g, 30.8 mmol) in H₂O (5 mL), the ice bath was removed, and the reaction warmed to ambient temperature. After 2 hours, the reaction was quenched with saturated aqueous NaHSO₃ and the acetone was evaporated. The aqueous layer was extracted with ethyl acetate (3x). The combined ethyl acetate layers were dried over Na₂SO₄, filtered and evaporated to an oil. Purification by chromatography on silica gel (25% ethyl acetate-hexane) gave the iodide (4.31 g, 84%) as a light yellow oil.

### Example 154C

### 4-(3-pyridyloxymethyl)-2-iodobenzoic acid methyl ester

To a solution of the iodide prepared in Example 154B (6.01g, 20.6 mmol) in DMF (30 mL) was added SOCl₂ and LiCl and the reaction was stirred at 25 °C for 5 minutes. The reaction mixture was taken up in ethyl acetate, washed with H₂O (3x) and brine (4x), dried over Na₂SO₄, filtered and evaporated to an oil. The benzyl chloride (6.39 g, 20.6 mmol) was dissolved in toluene and 18-crown-6 (8.17 g, 30.9 mmol) was added followed by the potassium salt of 3-pyridinol and the reaction was heated to reflux. The reaction was complete in 2 hours. The reaction mixture was cooled to ambient temperature and washed with H₂O (3x), dried over Na₂SO₄, filtered and evaporated to an oil. Purification by chromatography on silica gel (gradient of 50% ethyl acetate-hexanes to 75% ethyl acetate-hexanes) gave the desired pyridyl ether (3.01 g, 40%).

### Example 154D

### 4-(3-pyridyloxymethyl)-2-(2-trifluoromethylphenyl)benzoic acid methyl ester

To a solution of the pyridyl ether prepared in Example 154C (365 mg, 0.96 mmol) in DMF (4 mL) at 25 °C was added PdCl₂(PPh₃)₂ (67 mg, 0.096 mmol, 10 mol%) followed by 2-trifluoromethyl boronic acid (366 mg, 1.93 mmol) and Cs₂CO₃ (629 mg, 1.93 mmol) and the reaction was heated at 80 °C for 12 hours. The reaction was then cooled and taken up in ethyl acetate. The organic phase was washed with H₂O (5x), dried over Na₂SO₄, filtered and evaporated to an oil. Purification by radial chromatography (gradient of 25% ethyl acetate-hexanes to 75% ethyl acetate-hexanes) gave the desired compound (261mg, 70%) as an oil.

### Example 154E

### 4-(3-pyridyloxymethyl)-2-(2-trifluoromethylphenyl)benzoic acid

The product of Example 154D (241 mg, 0.62 mmol) was dissolved in methanol (5 mL) and saturated aqueous LiOH (1 mL) was added. The reaction was heated at reflex for 1 hour. The reaction mixture was then evaporated and formic acid (1 mL) was added to acidify the crude product to pH3. The reaction was evaporated again to remove formic acid and ethyl acetate (5 mL) and H₂O (1 mL) were added to completely solubilize the reaction mixture. The aqueous layer was extracted with ethyl acetate (3x) and the ethyl acetate layers were combined and dried over Na₂SO₄, filtered and evaporated to give the acid (231 mg, 100%).

### Example 154F

### [4-(3-pyridyloxymethyl)-2-(2-trifluoromethylphenyl)benzoyl]methionine methyl ester

The product of Example 154E (231mg, 0.62 mmol) was dissolved in DMF (4 mL) and HOOBT (152mg, 0.93 mmol) was added followed by methionine methyl ester HCl (185mg, 0.93 mmol), EDCl (179mg, 0.93 mmol) and Et₃N (0.18mL, 1.24 mmol). The reaction was stirred for 12 hours at 25 °C and then was taken up in ethyl acetate and washed with H₂O (3x) and brine (3x). The ethyl acetate layer was dried over Na₂SO₄, filtered and evaporated to an oil. Purification by radial chromatography (25% ethyl acetate-hexanes to 50% ethyl acetate-hexanes to 5% methanol-ethyl acetate) gave the desired compound (291mg, 91%) as an oil.

### Example 154G

### [4-(3-pyridyloxymethyl)-2-(2-trifluoromethylphenyl)benzoyl]methionine methyl ester

The product of Example 154F (291mg, 0.56 mmol) was dissolved in THF(4 mL) and saturated aqueous LiOH (1 mL). Water (1 mL) was added and the reaction mixture was stirred at room temperature for 1 hour. The reaction mixture was thoroughly evaporated and formic acid was added to pH3 The reaction was evaporated to dryness and ethyl acetate (10 mL) was added followed by a minimum quantity of H₂O (∼1 mL) to completely solubilize the free acid and the water soluble salts, respectively. The layers were separated and the aqueous layer was extracted with ethyl acetate (3x). The ethyl acetate layers were combined and dried over Na₂SO₄, filtered, and evaporated and then lyophilized to give the title compound (242mg, 86%) as an amorphous white solid. ¹H NMR (300 MHz, CD₃OD) δ 8.30 (bs, 1H), 8.14 (m, 1H), 7.76 - 7.33 (m, 9H), 5.28 (s, 2H), 4.87 - 4.40 (m, 1H), 2.40 - 2.06 (m, 2H), 2.04 - 1.94 (m, 4H contains methionine SMe), 1.92 - 1.80 (m, 1H). MS (Cl) 505 (M+H)⁺ 505. Anal calcd for C₂₅H₂₃O₄N₂SF₃: 0.65 H₂O solvate: C, 58.17; H, 4.74; N , 5.43. Found: C, 58.17; H, 4.80; N, 5.31. HRMS FAB Calcd m/z MH⁺ for C₂₅H₂₃O₄N₂SF₃ 505.1409, found 505.1408.

### Example 155

### [4-(3-pyridyloxymethyl)-2-(2-ethylphenyl)benzoyl]methionine methyl ester

The desired compound was prepared according to the method of Example 154, except substituting 2-ethylphenylboronic acid for 2-trifluoromethylphenylboronic acid. ¹H NMR (300 MHz, CD₃OD) δ 8.30 (bs, 1H), 8.14 (d, *J*= 4.4 Hz, 1H), 7.71 - 7.17 (m, 9H), 5.29 (s, 2H), 4.87 - 4.43 (m, 1H), 2.54 - 2.37 (m 2 H), 2.24 - 1.84 (m, 7H, contains SMe), 1.90 - 1.82 (m, 1H), 1.04 and 0.97 (rotameric triplets, *J*= 7.3 Hz, 3H), MS (Cl) 465 (M+H)⁺. Anal calcd for C₂₆H₂₈O₄N₂S: 0.22 H₂O solvate: C, 66.65; H, 6.12; N, 5.98. Found: C, 66.64; H, 6.22; N, 5.85. HRMS FAB Calcd m/z MH⁺ for C₂₆H₂₈O₄N₂S 465.1848, found 465.1865.

### Example 156

### [4-(3-pyridyloxymethyl)-2-(2-ethylphenyl)benzoyl]methionine methyl ester

The desired compound was prepared according to the method of Example 154, except substituting 2-chlorophenylboronic acid for 2-trifluoromethylphenylboronic acid. ¹H NMR (500 MHz, CD₃OD) δ 8.31 (bs, 1H), 8.14 (d, *J*=4.4 Hz, 1H), 7.70 - 7.34 (m, 9H), 5.29 (s, 2H), 4.48 - 4.45 (m, 1H), 2.30 - 2.22 (m 1 H), 2.20 - 2.15 (m, 1H), 2.05 - 1.95 (m, 4H,contains SMe), 1.86 - 1.76 (m, 1H). MS (CI) 471 (M+H)⁺. Anal calcd for C₂₄H₂₃O₄N₂SCI: C, 61.21; H, 4.92; N ,5.95. Found : C, 61.31; H, 5.20; N, 5.61. HRMS FAB Calcd m/z MH⁺ for C₂₄H₂₃O₄N₂SCI 471.1145, found 471.1165.

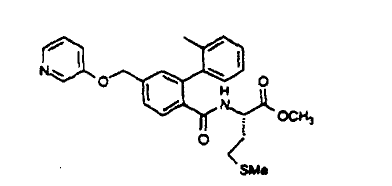

### Example 157

### [4-(3-pyridyloxymethyl)-2-(2-methylphenyl)benzoyl]methionine methyl ester

The desired compound was prepared by saponification of 4-(3-pyridyloxymethyl)-2-(2-methylphenyl)benzoic acid methyl ester, prepared as in Example 70D, followed by coupling with methionine methyl ester hydrochloride and saponification as described in Examples 154E-G. ¹H NMR (300 MHz, CDCl₃) δ 8.40 (bs, 1H), 8.25 (dd, *J*= 4.1, 1.9 Hz, 1H), 7.99 (dd, *J*= 22.8, 8.1 Hz, 1H), 7.53 - 7.50 (m, 1H), 7.36 - 7.21 (m, 7H), 5.91 (bd, *J*= 7.7 Hz, 1H (NH)), 5.18 (s, 2H), 4.70 - 4.58 (m, 1H), 3.66 (s, 3H, OMe), 2.18 - 2.00 (m, 5H), 1.95 -1.82 (m, 1H), 1.65 - 1.55 (m, 4H, contains SMe). MS (Cl) 465 (M+H)⁺. Anal calcd for C₂₆H₂₈O₄N₂S: 0.30 H₂O solvate: C 66.45; H ,6.13; N, 5.96. Found: C, 66.45; H, 6.15; N, 5.97. HRMS FAB Calcd m/z MH⁺ for C₂₆H₂₈O₄N₂S 465.1848, found 465.1869.

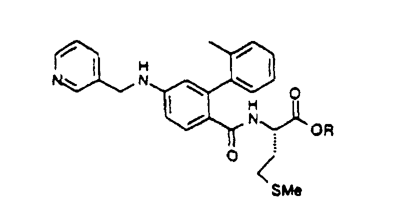

### Example 158

### [4-(3-pyridylmethylamino)-2-(2-methylphenyl)benzoyl]methionine methyl ester

### Example 158A

### 4-nitro-2-(2-methylphenyl)benzoic acid

To a solution in 4:1 THF-water (20 mL) wasadded saturated aqueous LiOH (4 mL) and the reaction was stirred at reflux for 2 hours. The THF was evaporated and the residue was acidified with 2 mL of formic acid, stripped and partitioned between ethyl acetate and H₂O. The ethyl acetate layer was dried over Na₂SO₄, filtered and evaporated to give the acid as an oil which solidified upon standing.

### Example 158B

### [4-nitro-2-(2-methylphenyl)benzoyl]methionine methyl ester

The product of Example 158A (604 mg, 2.35 mmol) and HOOBT (574 mg, 3.52 mmol) were dissolved in DMF (10 mL) and methionine methyl ester HCl (679 mg, 3.52 mmol) and EDCl (676 mg, 3.52 mmol) were added followed by Et₃N (476 mg, 0.65 mL, 4.7 mmol). The reaction was stirred for 12 hours and then was taken up in ethyl acetate and washed successively with brine (3x) and water (3x). The ethyl acetate layer was dried over Na₂SO₄, filtered and evaporated. Purification by chromatography on silica gel (5% methanol-ethyl acetate) gave the desired compound (940mg, 98%).

### Example 158C

### [4-amino-2-(methylphenyl)benzoyl]methionine methyl ester

To a solution of the product of Example 158B (940 mg, 2.33 mmol) in ethyl acetate (50 mL) was added SnCl₂.2H₂O (1.85 g, 8.18 mmol) and the reaction was heated at reflux for 1 hour. The reaction mixture was cooled and basified to pH8 gradually with solid NaHCO₃, stirred overnight, and extracted with ethyl acetate. The ethyl acetate extract was concentrated and the residue was purified by chromatography on silica gel (5% methanol-ethyl acetate) to give the aniline (450mg, 52%) as an oil.

### Example 158D

### [4-(3-pyridylmethylamino)-2-(2-methylphenyl)benzoyl]methionine methyl ester

The aniline prepared in Example 158C (180 mg, 0.48 mmol) and 3-pyridine carboxaldehyde (55 mg, 0.51 mmol) were combined in methanol (4 mL) and sodium cyanoborohydride (48 mg, 0.77 mmol) was added followed by 100 mg of crushed molecular sieves. The reaction was adjusted to pH6 with acetic acid and stirred at 25 °C for 3 hours. The reaction was concentrated and transferred directly to a column of silica gel and purified by flash chromatography (5% methanol-ethyl acetate) to give the title compound (182 mg, 82%) as an oil that solidified after standing. ¹H NMR (300 MHz, CD₃OD) δ 8.54 (d, *J*= 2.4 Hz, 1H), 8.40 (dd, *J*= 5.1, 1.3 Hz, 1H), 7.84 (bd, *J*= 8.4 Hz, 1H), 7.65 - 7.55 (m, 1H), 7.40 (dd, *J*= 7.8, 4.7 Hz, 1H), 7.30 - 7.10 (m, 4H), 6.66 (dd, *J*= 8.8, 2.3 Hz, 1H), 6.37 (d, *J*= 2.3 Hz, 1H), 4.45 (s, 2H), 3.64 (s, 3H), 2.10 - 1.98 (m, 8H), 1.90 - 1.78 (m, 1H), 1.65 - 1.55 (m, 1H). MS (Cl) 464 (M+H)⁺. HRMS FAB Calcd m/z MH⁺ for C₂₆H₂₉O₃N₃S 464.2008, found 464.2023.

### Example 159

### [4-(3-pyridylmethylamino)-2-(2-methylphenyl)benzoyl]methionine

The desired compound was prepared by saponification of the product of Example 158 according to the method of Example 154G. ¹H NMR (300 MHz, CD₃OD) δ 8.81 (bs, 1H), 8.76 (bd, *J*= 11.8 Hz, 1H), 8.64 - 8.61 (m, 1H), 8.07 (dd, *J*= 8.5, 6.1 Hz, 1H), 7.65 - 7.58 (m, 1H), 7.28 - 7.18 (m, 4H), 6.70 (dd, *J*= 8.5, 2.4 Hz, 1H), 6.40 (d, *J*= 2.3 Hz, 1H), 4.68 (s, 2H), 4.44 - 4.38 (m, 1H), 2.14 - 1.99 (m, 8H), 1.90 - 1.80 (m, 1H), 1.65 - 1.55 (m. 1H). MS (Cl) 450 (M+H)⁺. Anal calcd for C₂₅H₂₈O₃N₃SCl: 1.10 H₂O and 0.80 HCl solvate: C, 56.12; H, 5.84; N, 7.85. Found: C, 56.11; H, 5.85; N, 8.03. HRMS FAB Calcd m/z MH⁺ for C₂₅H₂₇O₃N₃S 450.1851, found 450.1864.

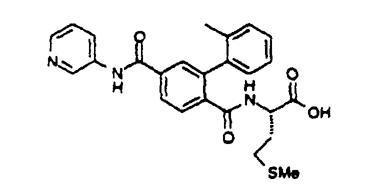

### Example 160

### [4-(3-pyridylaminocarbonyl)-2-(2-methylphenyl)benzoyl]methionine

The desired compound was prepared by saponification of [4-(3-pyridylaminocarbonyl)-2-(2-methylphenyl)benzoyl]methionine according to the method of Example 154G. ¹H NMR (300 MHz, CD₃OD) 8.90 (bs, 1H), 8.32 - 8.25 (m, 2H), 8.11-8.05 (m, 1H), 7.85 (bs, 1H), 7.78 (d, *J*=8.1 Hz, 1H), 7.45(dd, *J*= 8.5, 4.8 Hz, 1H), 7.28 - 7.18 (m, 5H), 4.50 - 4.40 (m, 1H), 2.20 - 1.65 (m, 10 H). MS (Cl) 464 (M+H⁺).

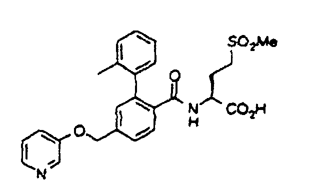

### Example 161

### N-[4-(3-pyridyloxymethyl)-2-(2-methylphenyl)benzoyl]-2-amino-4-(methylsulfonyl)butanoic acid

The desired compound was prepared according to the method of Example 85, except substituting [4-(3-pyridyloxymethyl)-2-(2-methylphenyl)benzoyl]methionine methyl ester, prepared as in Example 157, for {4-[2-(pyrid-3-yl)ethenyl]-2-phenylbenzoyl}methionine methyl ester. ¹H NMR (300MHz, DMSO-d6) δ 8.40 (1H, d, *J*=7 Hz), 8.37 (1H, d, *J*=7 Hz), 7.57 (2H, bs), 7.47 (1H, dd, *J*=8, 3Hz), 7.33 (1H, dd. *J*=7, 5 Hz), 7.30 (1H, s), 7.21 (2H, bs), 7.16 (2H, m), 5.25 (2H, s), 4.21 (1H, bs), 2.92 (3H, s), 2.83 (1H, m), 2.70 (4H, m), 2.05 (3H, bs), 1.90 (2H, m). MS (DCl, NH₃) m/e 483 (M+H)⁺. Anal calcd for C₂₅H₂₈N₂O₇S•1H₂O: C, 59.99; H, 5.64; N 5.60. Found C, 59.93; H, 5.60; N, 5.45.
The next example is Example 167.

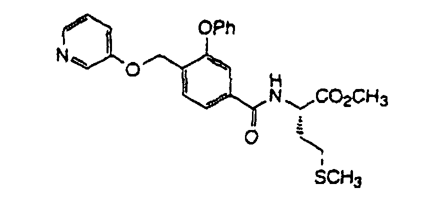

### Example 167

### [4-(3-pyridyloxymethyl)-3-phenoxybenzoyl]methionine

The desired compound was prepared according to the method of Examples 36C-H, except substituting 4-carbonylmethoxy-2-phenoxybenzoic acid for 4-carbonylmethoxy-3-phenoxybenzoic acid. ¹H NMR (300 MHz, DMSO-d6) δ 2.03 (s, 3H), 2.0-2.3 (m, 2H), 2.5-2.6 (m, 2H), 4.4-4.5 (m, 1H), 5.25 (s, 2H), 7.03 (d, *J*= 8 Hz, 2H), 7.17 (t, *J*= 8 Hz, 1H), 7.33-7.55 (m, 5H), 7.7-7.8 (m. 2H), 8.20 (d. *J*= 4 Hz, 1H), 8.35 (d, *J*= 3 Hz, 1H), 8.71 and 8.83 (d, *J*= 8 Hz, 1H).

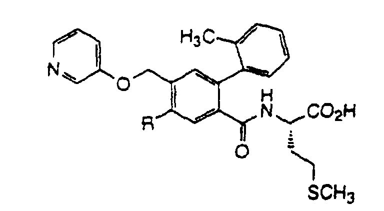

### Examples 169 and 170

### Example 169

### [4-(3-pyridyloxymethyl)-2-(2-methylphenyl)-5-butylbenzoyl]methionine

### Example 169A

### 2-amino-5-bromoterephthalate dimethyl ester

To a -12 °C suspension in dichloromethane of 2-aminoterephthalate (10.46 g, 50 mmol) and pyridine (8.1 mL, 100 mmol) was added as solution of bromine (2.6 mL, 52.5 mmol) in dichloromethane (25 mL) over 0.5 hours and the reaction mixture was warmed slowly to ambient temperature and stirred overnight. Aqueous workup followed by recrystallization from 95% ethanol gave the desired compound (11 g, 77%).

### Example 169B

### 2-(2-methylphenyl)-5-aminoterephthalate dimethyl ester

A solution of palladium acetate (260 mg, 1.16 mmol) and triphenylphosphine (1.21 g, 4.63 mmol) was stirred for 10 minutes at ambient temperature and then the product of Example 169A (11.1 g, 38.6 mmol), 2-methylphenylboronic acid (5.77 g, 42.4 mmol), ethanol (18 mL) and aqueous 2M sodium carbonate (157 mL) were added. The reaction mixture was warmed to reflux and stirred for 18 hours. The reaction mixture was cooled to ambient temperature and diluted with ether. The aqueous phase was extracted with ether. The combined organic layers were washed with water, dried. filtered and concentrated in vacuo to give an orange oil. Chromatography on silica gel (25% ethyl acetate-hexanes) gave the desired compound (9.6 g, 83%) as a yellow solid.

### Example 169C

### 2-(2-methylphenyl)-5-iodoterephthalate dimethyl ester

A mixture of the product of Example 169B (7.00 g. 23.4 mmol) and aqueous 3M HCl (50 mL) in acetone (500 mL) was cooled to 0 °C and a solution of NaNO₂ (1.78 9. 25.7 mmol) in water (20 mL) was added dropwise. The reaction mixture was stirred for 1 hour and then urea (0.53 g, 8.88 mmol) and a solution of Kl (6.79 g, 40.9 mmol) in water (20 mL) was added at a rate such that the reaction temperature remained below 0 °C. The reaction mixture was stirred for 0.5 hours, then the cold bath was removed and stirring was continued for 2 hours. The reaction mixture was diluted with water (400 mL) and NaHSO3 was added until the brown color disappeared. The reaction mixture was filtered and the solid was recrystallized from 20:1 aqueous ethanol to give the desired compound (6.46 g, 67%). mp 105-109 °C.

### Example 169D

### 2-(2-methylphenyl)-5-iodoterephthalate 1-methyl ester

The desired compound was prepared by reaction of a solution of the product of Example 169C in THF with aqueous LiOH at 0 °C according to the method of Example 38.

### Example 169E

### 2-(2-methylphenyl)-4-hydroxymethyl-5-iodobenzoic acid methyl ester

The desired compound was prepared by reduction of the product of Example 169D using the procedure of Example 36 C.

### Example 169F

### 2-(2-methylphenyl)-4-bromomethyl-5-iodobenzoic acid methyl ester

To a -10 °C solution in dichloromethane of the product of Example 169E (830 mg, 2.17 mmol) and carbon tetrabromide (864 mg, 2.60 mmol) was added triphenylphosphine (626 mg, 2.39 mmol) and the reaction mixture was warmed to 0 °C over 1 hour. The cold bath was then removed and stirring was continued for 2 hours. The reaction mixture was concentrated in vacuo and purified by chromatography on silica gel (5% ethyl acetate-hexanes) to give the desired compound (1.1 g) which also contained some triphenylphosphine.

### Example 169G

### 4-(3-pyridyloxymethyl)-2-(2-methylphenyl)-5-iodobenzoic acid methyl ester

To a solution in dichloromethane (10 mL) of benzyltriethylammonium bromide (1.18 g, 4.34 mmol) was added 3-hydroxypyridine potassium salt (586 mg, 4.34 mmol) and the mixture was stirred for 15 minutes. A solution of the product of Example 169F (960 mg, 2.17 mmol) in dichloromethane (4 mL) was added and the reaction mixture was stirred overnight. The reaction mixture was washed with water, dried, filtered and concentrated in vacuo. Chromatography on silica gel (35% ethy acetate-hexanes) gave the desired compound (480 mg, 49%).

### Example 169H

### 4-(3-pyridyloxymethyl)-2-(2-methylphenyl)-5-butylbenzoic acid methyl ester

To a solution of tributylborane (0.10 mL, 0.41 mmol) in degassed DMF was added a solution of the procuct of Example 169G (150 mg, 0.33 mmol) in DMF (1 mL) followed by *bis*(diphenylphosphinoferrocenyl)palladium(II) chloride (8 mg, 0.01 mmol) and potassium phosphate (212 mg, 1.0 mmol) and the reaction mixture was stirred at 65 °C for 3 hours. The reaction mixture was cooled to ambient temperture and poured into water. The aqueous phase was extracted with ethy acetate (2x). The combined organic layers were washed with water and brine, dried, and filtered through a plug of silica gel (ethyl acetate) to give the desired compound (162 mg) which was used without further purification.

### Example 169I

### 4-(3-pyridyloxymethyl)-2-(2-methylphenyl)-5-butylbenzoic acid

The desired product was prepared by saponification of the methyl ester in the product of Example 169H using the method of Example 97A.

### Example 169J

### [4-(3-pyridyloxymethyl)-2-(2-methylphenyl)-5-butylbenzoyl]methione methyl ester

The desired compound was prepared by coupling to the product of Example 1691 with methionine methyl ester hydrochloride using the procedure used in step C of the preparation of compound 8.

### Example 169K

### [4-(3-pyridyloxymethyl)-2-(2-methylphenyl)-5-butylbenzoyl]methione

The desired compound was prepared by saponification of the product of Example 169J using the method of Example 39H. ¹H NMR (300MHz, DMSO-d₆) δ 8.36 (d, 1H), 8.18 (dd, 1H), 8.09 (bd, 1H), 7.48 (m, 1H), 7.37 (s, 1H), 7.32 (dd, 1H), 7.27 (s, 1H), 7.19 (m, 2H), 7.11 (m, 2H), 5.28 (s, 2H), 4.21 (m, 1H), 2.74 (dd, 2H), 1.98 - 2.20 (m, 5H), 1.96 (s, 3H), 1.37 - 1.90 (m, 6H), 0.92 (t, 3H). MS (Cl, NH₃) m/e 507, 489. Anal calcd for C₂₉H₃₄N₂O₄S·0.50 H₂O: C, 67.55; H,6.84; N, 5.43. Found: C, 67.55; H, 6.69; N, 5.33.

### Example 170

### [4-(3-pyridyloxymethyl)-2-(2-methylphenyl)-5-isobutylbenzoyl]methionine

To a -78 °C solution in ether (1 mL) of *tert*-butyllithium (1.7 M in ether, 0.75 mL, 1.28 mmol) was added a solution of iodoisobutane (0.74 mL, 0.64 mmol) in ether (1 mL) and the mixture was stirred for 30 minutes. 9-methoxy-9-borabicyclo[3.3.1]nonane (1.0 M in ether, 0.66 mL, 0.66 mmol) was added and the reaction mixture was warmed to 30 °C and stirred for 30 minutes. A solution of the product of Example 169G (218 mg, 0.53 mmol) in DMF (4 mL) was then added, followed by *bis*(diphenylphosphinoferrocenyl)palladium (II) chloride (13 mg, 0.016 mmol) and potassium phosphate (338 mg, 1.59 mmol). The reaction mixture was stirred at 65 °C under a stream of nitrogen for 2 hours. Workup as described in Example 169H, followed by saponification, coupling and saponification as described in Example 169I-J gave the title compound. ¹H NMR (300MHz, DMSO-d₆) δ 8.35 (d, 1H), 8.18 (dd, 1H), 8.06 (bd, 1H), 7.49 (dq, 1H), 7.35 (s, 1H), 7.33 (dd, 1H), 7.27 (s, 1H), 7.18 (m, 2H), 7.03 (m, 2H), 5.25 (s, 1H), 4.22 (m, 1H), 2.63 (bd, 2H), 2.12 (heptet, 1H), 2.03 (m, 4H), 1.96 (s, 3H), 1.64 - 1.90 (m, 3H), 0.96 (d, 6H). MS (Cl, NH₃) m/e 507, 489, 221, 204. Anal calcd for C₂₉H₃₄N₂O₄S·0.50 H₂O: C, 67.90; H, 6.82: N, 5.46. Found: C, 67.91; H, 6.68; N ,5.40.

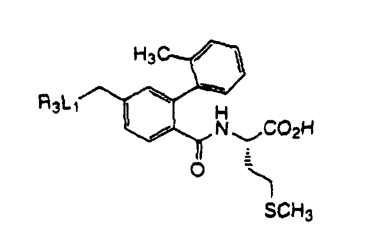

### Examples 171 and 172

### Example 171

### [4-(3-pyridylaminomethyl)-2-(2-methylphenyl)benzoyl]methionine

The desired compound was prepared according to the method of Example 56, except substituting [4-(3-pyridylaminomethyl)-2-(2-methylphenyl)phenylbenzoyl] methionine for [4-(3-pyridylaminomethyl)-2-phenylbenzoyl]methionine. ¹H (300 MHz., DMSO d₆): δ 8.08, (d, 1H), 7.96, (d, 1H), 7.73, (d, 1H) 7.44, (m, 2H) 7.19, (m, 3H) 7.11, (m, 2H) 7.03, (dd, 1H) 6.89, (m, 1H) 6.56, (t, 1H) 4.39, (d, 2H) 4.20, (ddd, 1H) 1.96 - 2.22, (m, 5H) 1.95, (s, 3H) 1.63 - 1.90, (m, 2H) MS (DCl, NH₃): 450 (MH)⁺, 100%. anal. calc for C₂₅H₂₇N₃O₃S•0.62 H₂O: C 65.17 H 6.18 N 9.12. Found: C 65.18 H 5.85 N 9.04.

### Example 172

### [4-(3-pyridylthiomethyl)-2-(2-methylphenyl)benzoyl methionine

To a solution in DMF (2 mL) of 4-chloromethyl-2-(2-methylphenyl)benzoic acid methyl ester (275 mg, 1.0 mmol) was added 3-pyridinethiol potassium salt (224 mg, 1.5 mmol). The reaction mixture was stirred for 30 minutes and then was poured into water. The mixture was extracted with ethyl acetate (3x). The combined organic extracts were washed with water and brine, dried, filtered and concentrated in vacuo.
Chromatography on silica gel (40% ethyl acetate-hexanes) gave 4-(3-pyridylthiomethyl)-2-(2-methylphenyl)benzoic acid methyl ester which was converted to the title compound by saponification of the methyl ester, coupling with methionine methyl ester hydrochloride, and saponification as described in Examples 169I-J. ¹H NMR (300MHz, DMSO-d₆) δ 12.53 (bs, 1H), 8.49 (d, 1H), 8.38 (dd, 1H), 8.11 (d, 1H), 7.79 (dt, 1H), 7.43 (s, 2H), 7.31 (dd, 1H), 7.19 (m, 2H), 7.11 (m, 2H), 7.05 (m, 1H), 4.36 (s, 2H), 4.20 (m, 1H), 1.92 - 2.23 (m, 8H), 1.66 - 1.90 (m, 2H). MS (Cl, NH₃) m/e 467, 449. Anal calcd forC₂₅H₂₆N₂O₃S₂: C, 64.35; H, 5.62; N, 6.00. Found: C, 64.00; H ,5.62; N, 5.89.

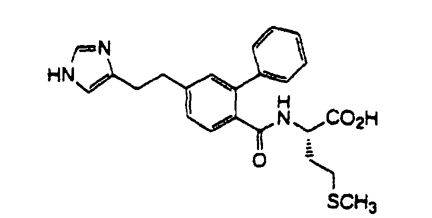

### Example 175

### {4-[2-(1H-imidazol-4-yl)ethyl]-2-phenylbenzoyl}methionine

### Example 175A

### Diethyl (2-phenyl-4-carboxymethylbenzyl)phosphonate

A slurry in THF of diethylphosphite (1.75 g, 12.7 mmol) and sodium hydride (60% in mineral oil, 305 mg, 12.7 mmol) was stirred for 1.5 hours and then 4-chloromethyl-2-phenylbenzoic acid methyl ester was added and the reaction mixture was stirred for 18 hours at ambient temperature and 18 hours at reflux. The reaction mixture was concentrated in vacuo and the residue was partitioned between ethyl acetate and brine. The organic phase was dried over magnesium sulfate, filtered and concentrated in vacuo to give a yellow oil. Chromatography on silica gel (1:1 ethyl acetate-hexanes) gave the desired compound (3.19 g, 77%) as a colorless oil.

### Example 175B

### 4-[2-(1H-1-triphenylmethylimidazol-3-yl)ethenyl]-2-phenylbenzoic acid methyl ester

To a 0 °C slurry in THF (2 mL) of sodium hydride (60% in mineral oil, 61.3 mg, 1.5 mmol) was added a solution of the product of Example 175A (402 mg, 1.5 mmol) in THF (3 mL) and the mixture was stirred for 2 hours. A solution of 1H-1-triphenylmethylimidazole-4-carboxaldehydre (761 mg, 2.35 mmol) in THF (2 mL) was added and the reaction mixture was heated at reflux for 8 hours. The reaction mixture was cooled to ambient temperature and concentrated in vacuo. The residue was partitioned between ethyl acetate and brine. The organic phase was dried over magnesium sulfate, filtered and concentrated in vacuo to give a yellow oil. Chromatography on silica gel (1:1 ethyl acetate-hexanes) gave the desired compound (740 mg, 90%) as a white solid.

### Example 175C

### 4-[2-(1H-1-triphenylmethylimidazol-3-yl)ethyl]-2-phenylbenzoic acid methyl ester

The desired compound was prepared by catalytic (10% Pd/C) hydrogenation of the product of Example 175B.

### Example 175D

### {4-[2-(1H-1-triphenylmethylimidazol-4-yl)ethyl]-2-phenylbenzoyl}methionine

The desired compound was prepared by saponification of the product of Example 175C using aqueous lithium hydroxide and methanol at reflux, followed by coupling to the acid with methionine ethyl ester hydrochloride using the procedure of step c in the preparation of compound 8, and saponification of the ethyl ester using aqueous lithium hydroxide in aqueous THF.

### Example 175E

### {4-[2-(1H-imidazol-4-yl)ethyl]-2-phenylbenzoyl}methionine

A mixture of the product of Example 175D (15 mg, 0.02 mmol), trifluoroacetic acid (26 mg, 0.23 mmol) and triethylsilane (27 mg, 0.23 mmol) in dichloromethane (1 mL) was stirred for 18 hours. The reaction mixture was concentrated in vacuo and the residue was partitioned between water and 1:1 ethyl acetate-hexanes. The aqueous phase was freeze-dried to give the title compound as the trifluoroacetate salt. ¹ H NMR (300 MHz, DMSO-d₆) δ 1.9 (m, 3H), 2.0 (s, 3H), 2.3 (m, 2H), 4.1 (m, 1H), 4.2-4.4 (m, 4H), 7.3-7.5 (m, 8H), 7.7 (br d, 1H), 8.5 (d, 1H), 9.0 (s, 1H), 12 (br s, 1H). MS (DCl, NH₃) m/e 424 (M+H)⁺.

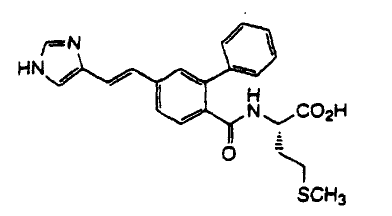

### Example 176

### {4-[2-(1H-imidazol-4-yl)ethyl]-2-phenylbenzoyl}methionine

The desired compound was prepared according to the method of Examples 175A, B, D and E. ¹H NMR (300 MHz, DMSO-d6) δ 1.9 (m, 3H), 2.0 (s, 3H), 2.3 (m, 2H), 4.6 (m, 1H), 5.8 (d, 1H), 6.1 (d, 1H), 7.3-7.5 (m, 8H), 7.7 (br d, 1H), 8.5 (d, 1H), 9.0 (s, 1H), 12 (br s, 1H). MS (DCl, NH₃) m/e 390 (M+H)⁺.

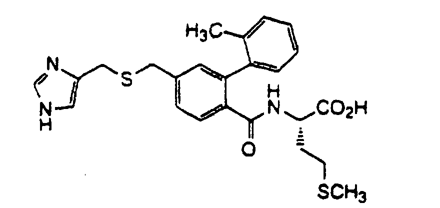

### Example 177

### [4-(1H-imidazol-4-ylmethylthiomethyl)-2-(2-methylphenyl)benzoyl]methionine

The desired compound was prepared according to the method of Example 105, except substituting 4-bromomethyl-2-(2-methylphenyl)benzoic acid methyl ester for 4-bromomethyl-2-phenylbenzoic acid methyl ester. ¹H NMR (300 MHz, DMSO-d₆) δ 1.8 (m, 3H), 2.0 (s, 3H), 2.5 (m, 2H), 3.8 (s, 2H), 3.9 (s, 2H), 4.3 (m, 1H), 7.3 (m, 8H), 7.5 (br d, 1H), 8.5 (d, 1H), 8.9 (br s, 1H), 2 (br s, 1H). MS (DCl, NH₃) m/e 456 (M+H)⁺.

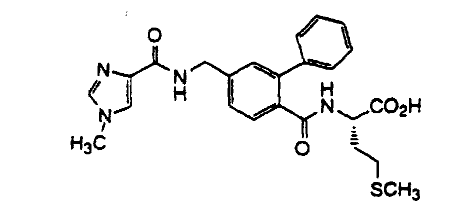

### Example 178

### [4-(1H-1-methylimidazol4-ylcarbonylaminomethyl)-2-phenylbenzoyl]methionine

### Example 178A

### 4-azidomethyl-2-phenylbenzoic acid methyl ester

A mixture of 4-bromomethyl-2-phenylbenzoic acid methyl ester (1.5 g, 4.9 mmol), sodium azide (1.28 g, 19.7 mmol) and tetrabutylammonium iodide (1.3 g, 4.9 mmol) in DMF (16 mL) was heated at 75 °C for 18 hours. The reaction mixture was diluted with water and extracted twice with ethyl acetate. The combined organic extracts were dried over magnesium sulfate, filtered and concentrated in vacuo. Chromatography on silica gel (15% ethyl acetate-hexanes) to give the desired azide as a colorless oil (1.01 g).

### Example 178B

### [4-azidomethyl-2-phenylbenzoyl]methionine methyl ester

The desired compound was prepared by saponification of the product of Example 178A using sodium hydroxide in refluxing aqueous methanol, followed by coupling of the resulting acid with methionine methyl ester hydrochloride using the procedure of step C in the preparation of compound 8.

### Example 178C

### [4-aminomethyl-2-phenylbenzoyl]methionine methyl ester

A solution of the product of Example 178B (1.00 g, 2.5 mmol) and triphenylphosphine (0.98 g, 3.75 mmol) in THF (10 mL) was heated at reflux for 4 hours. The reaction mixture was cooled to ambient temperature, water (0.45 mL) was added, and the reaction mixture was stirred for 18 hours. The reaction mixture was concentrated in vacuo and the residue was partitioned between water and ethyl acetate. The organic phase was washed with brine, dried over sodium sulfate, filtered and concentrated in vacuo. Chromatography on silica gel (25% ethyl acetate-hexane, then 1% ammonia-ethyl acetate) gave the desired compound as a colorless oil (900 mg, 97%).

### Example 178D

### [4-(1H-1-methylimidazol4-ylcarbonylaminomethyl)-2-phenylbenzoyl]methionine

The desired compound was prepared by coupling of the product of Example 178C with 1H-1-methylimidazole-4-carboxylic acid using the procedure used in step C of the preparation of compound 8, followed by saponification of the methyl ester using sodium hydroxide in aqueous THF. ¹H NMR (300 MHz, DMSO-d6) δ 1.8 (m, 3H), 2.1 (s, 3H), 2.5 (m, 2H), 3.6 (m, 1H), 3.8 (m, 3H), 4.8 (m, 1H), 4.9 (d, 2H), 7.3 (m, 8H), 7.4 (d; 1H), 8.3 (d, 1H), 12.0 (br s, 1H). MS (DCl, NH₃) m/e 467 (M+H)⁺.

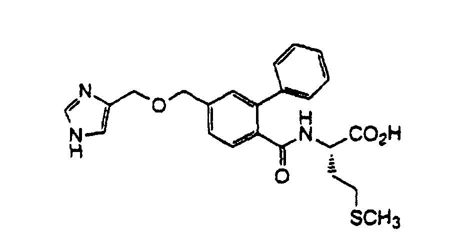

### Example 179

### [4-(1H-imidazol-4-ylmethyloxymethyl)-2-phenylbenzoyl]methionine

The desired compound was prepared according to the method of Examples 105C-F, except substituting 1H-1-triphenylmethylimidazol-4-ylmethanol sodium salt for 1H-1-triphenylmethylimidazol-4-ylmethylthiol sodium salt, and 4-chloromethyl-2-phenylbenzoic acid methyl ester for 4-bromomethyl-2-phenylbenzoic acid methyl ester. ¹H NMR (300 MHz. DM50-d6) δ 1.6 (m, 3H), 2.1 (s, 3H), 2.4 (m, 2H), 3.6 (s, 2H), 3.7 (s, 2H), 4.3 (m, 1H), 7.2 (m, 8H), 7.5 (br d, 1H), 8.5 (d, 1H), 8.9 (br s. 1H), 12 (br s, 1H). MS (DCl, NH₃) m/e 440 (M+H)⁺.

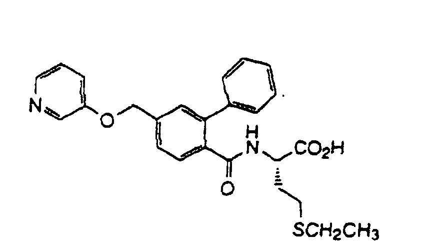

### Example 180

### 2-N-[4-(3-pyridyloxy)-2-phenylbenzoyl]ethionine

The desired compound was prepared according to the method of Example 98. except subsituting ethionine methyl ester hydrochloride for methionine methyl ester hydrochloride.

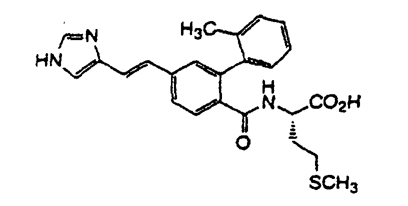

### Example 181

### {4-[2-(1H-imidazol-4-yl)ethenyl]-2-(2-methylphenyl)methionine

The desired compound was prepared according to the method of Example 176, except substituting 4-chloromethyl-2-(2-methylphenyl)benzoic acid methyl ester for 4-chloromethyl-2-phenylbenzoic acid methyl ester. 1H NMR (300 MHz, DMSO-d6) δ 1.90 (s, 3H), 2.10-2.20 (m, 8H), 4.20 (m, 2H), 7.10-7.40 (m, 7H), 7.50 (m, 2H), 7.70 (d, 1H), 8.20 (brd, 1H), 8.80 (d, 1H). MS (DCI-NH₃) 436 (M+H)⁺.

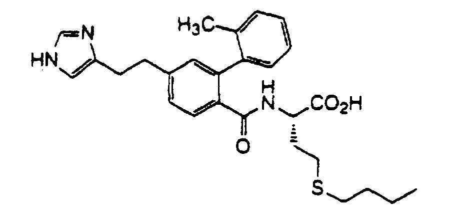

### Example 182

### 2-N-{4-[2-(1H-imidazol-4-yl)ethyl]-2-(2-methylphenyl)benzoyl}amino-4-(thiobutyl)butanoic acid

### Example 182A

### 2-N-tert-butoxycarbonylamino-4-hydroxybutanoic acid benzyl ester

To a 0 °C solution in THF (103 mL) of N-tert-butoxycarbonylaspartic acid 1-benzyl ester (10.0 g, 30.9 mmol) was added borane-THF (1 M in THF, 61.8 mL, 61.8 mmol) over 10 minutes. The cold bath was then removed and the reaction mixture was stirred for 3 hours. The reaction mixture was cooled to 0 °C and quenched with brine. The layers were separated and the aqueous phase was extracted with ethyl acetate. The combined organic layers were washed with brine, dried over magnesium sulfate, filtered, and concentrated in vacuo. Chromatography on silica gel (15% ethyl acetate-hexanes) gave the desired compound as a colorless oil (4.11 g, 43%).

### Example 182B

### 2-N-tert-butoxycarbonylamino-4-methanesulfonyloxybutanoic acid benzyl ester

To a 0 °C solution in dichloromethane (151 mL) of the product of Example 182A (14.68 g, 45.4 mmol) was added triethylamine (9.19 g, 90.8 mmol) and methanesulfonyl chloride (5.72 g, 50 mmol) and the reaction mixture was stirred for 2 hours. The reaction mixture was concentrated in vacuo and the residue was partitioned between ethyl acetate and brine. The organic phase was dried over magnesium sulfate, filtered and concentrated in vacuo. Chromatography on silica gel (25% ethyl acetate-hexanes) gave the desired compound (15.99 g, 91%).

### Example 182C

### 2-N-tert-butoxycarbonylamino-4-(thiobutyl)butanoic acid benzyl ester

To a suspension in THF (6 mL) of sodium hydride (60% in mineral oil, 568 mg, 14.2 mmol) was added butanethiol (1.23 g, 14.2 mmol) and the mixture was stirred for 0.5 hours. A solution of the product of Example 182B (1.83 g, 4.73 mmol) in THF (15.7 mL) was added and the reaction mixture was stirred for 3 hours. The reaction mixture was cooled to 0 °C, quenched with water and extracted with ethyl acetate. The organic phase was dried over magnesium sulfate, filtered and concentrated in vacuo. Chromatography on silica gel (5% ethyl acetate-hexanes) gave the desired compound 1.74 g, 97%).

### Example 182D

### 2-amino-4-(thiobutyl)butanoic acid methyl ester hydrochloride

To a solution in methanol (5 mL) of the product of Example 182C (1.00 g, 2.62 mmol) was added aqueous 1M lithium hydroxide (13.1 mL) and the reaction mixture was stirred for 4 hours. The methanol was evaporated and the aqueous residue was extracted with ethyl acetate. The organic extract was discarded and the aqueous phase was adjusted to pH 2 with aqueous 1N HCl and extracted with ethyl acetate. The ethyl acetate extract was dried over magnesium sulfate, filtered and concentrated in vacuo to give a colorless oil. The oil was dissolved in methanol (25 mL) and thionyl chloride (1.56 g, 13.1 mmol) was added. The reaction mixture was stirred at reflux for 4 hours, cooled to ambient temperature and concentrated to a colorless oil which was used without further purification.

### Example 183

### 2-N-{4-[2-(1H-imidazol-4-yl)ethyl]-2-(2-methylphenyl)benzoyl}amino-4-(thiobutyl)butanoic acid

The desired compound was prepared from the product of Example 182D and 4-[2-(1H-imidazol-4-yl)ethyl]-2-(2-methylphenyl)benzoic acid methyl ester accoring to the method of Example 175D and E. 1H NMR (300 MHz, DMSO-d6) δ 1.2 (m, 4H), 1.4-1.6 (m, 5H), 1.8-2.1 (m, 5H), 3.2 (s, 3H), 3.5 (br s, 1H), 4.3 (m, 4H), 7.0-7.4 (m, 7H), 8.2 (brd, 1H), 8.9 (d, 1H), 12 (br s, 2H). MS (DCI-NH₃) 480 (M+H)⁺.

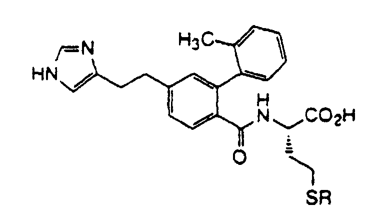

### Examples 184-189

The compounds of Examples 184-189 were prepared according to the method of Examples 182-183, except substituting the desired thiol for butanethiol.

| Example | R | Physical Data ¹H NMR (300 MHz. DMSO-d6) MS (DCl-NH₃) m/e |
|---|---|---|
| 184 | propyl | ¹H NMR δ 1.1 (m, 3H), 1.3-1.6 (m, 4H), 1.8-2.1 (m, 5H), 3.3 (s, 3H), 3.4 (br s, 1H), 4.3 (m, 4H), 7.0-7.4 (m, 7H), 8.2 (brd, 1H), 8.9 (d, 1H), 12 (br s, 1H). MS 466 (M+H)⁺. |
| 185 | methyl | ¹H NMR δ 1.6 (m, 4H), 1.9 (s, 4H), 2.2-2.4 (m, 1H), 3.0 (s, 3H), 4.2 (br m, 4H), 7.0-7.5 (m, 7H), 8.1 (brd, 1H), 8.9 (d, 1H), 12.0 (br s, 2H). MS 438 (M+H)+. |
| 186 | pentyl | ¹H NMR δ 1.3 (m, 5H), 1.4-1.6 (m, 6H), 1.8-2.2 (m. 5H), 3.1 (s, 3H), 3.5 (br s, 1H), 4.2 (m, 4H), 7.0-7.4 (m, 7H), 8.2 (brd, 1H), 8.9 (d, 1H), 12.0 (br s, 2H). MS 494 (M+H)⁺. |
| 187 | isopropyl | ¹H NMR δ 1.1 (d, 6H), 1.8 (m, 3H), 2.2-2.4 (m, 2H), 2.8 (septet, 1H), 3.0 (s, 3H), 3.4 (br s, 1H), 4.2 (m, 4H), 7.0-7.2 (m, 7H), 8.2 (brd, 1H), 8.9 (d, 1H), 12.0 (br s, 2H). MS 466 (M+H)⁺. |
| 188 | cyclopentyl | ¹H NMR δ 1.1 (m, 9H), 1.8 (m, 3H), 2.0-2.3 (m, 2H), 3.0 (s, 3H), 3.4 (br s, 1H), 4.1 (m, 4H), 7.0-7.3 (m, 7H), 8.2 (brd, 1H), 8.9 (d, 1H), 12 (br s. 2H). MS 492 (M+H)⁺. |
| 189 | cyclohexyl | ¹H NMR δ 1.1 (m, 11H), 1.7 (m, 3H), 1.9-2.4 (m, 2H), 3.1 (s, 3H), 3.2 (br s, 1H), 4.1 (m, 4H), 7.0-7.4 (m, 7H), 8.2 (brd, 1H), 8.8 (d, 1H), 12 (br s, 2H). MS 506 (M+H)⁺. |

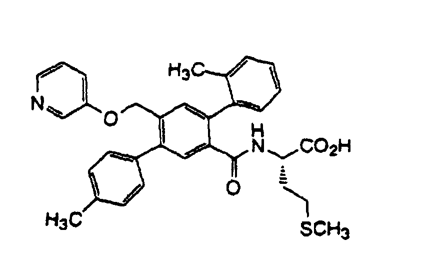

### Example 190

### [4-(3-pyridyloxymethyl)-5-(4-methylphenyl)-2-(2-methylphenyl)benzoyl methionine

### Example 190A

### [4-(3-pyridyloxymethyl)-5-(4-methylphenyl)-2-(2-methylphenyl)benzoyl methionine methylester

To a solution of tetrakis(triphenylphosphine) palladium (0) (2 mg) in toluene (1 mL) was added a solution of 4-(3-pyridyloxymethyl)-5-iodo-2-(2-methylphenyl)benzoic acid methyl ester (100 mg, 0.22 mmol), prepared as in Example 169G, in toluene (3 mL). The mixture was stirred for 10 minutes, then a solution of 4-methylphenylboronic acid (33 mg, 0.24 mmol) in ethanol (2 mL) and aqueous 2M sodium carbonate were added. The reaction mixture was stirred overnight at reflux and additional catalyst (20 mg), boronic acid (20 mg) and base (0.5 mL) were added and reflux was continued for 4 hours. The reaction mixture was cooled to ambient temperature, diluted with ether, washed with water and brine, dried over sodium carbonate, filtered and concentrated in vacuo. Chromatography on silica gel (30% ethyl acetate-hexanes) gave the desired compound (98 mg).

### Example 190B

### [4-(3-pyridyloxymethyl)-5-(4-methylphenyl)-2-(2-methylphenyl)benzoyl methionine

The desired compound was prepared by saponification of the compound of Example 190A, followed by coupling with methionine methyl ester hydrochloride and saponification of the methyl ester as described above. ¹H NMR (300MHz. CDCl₃,) δ 8.31 (1H, d, *J*= 9 Hz), 8.19 (1H, d, *J*= 3 Hz), 7.90 (1H, d, *J*= 4 Hz), 7.42 (1H, s), 7.40-7.20 (10H, m), 6.07 (1H, d, *J*= 9 Hz), 5.08 (2H, m), 4.62 (1H, m), 2.40 (3H, s), 2.25-2.10 (5H, m), 2.02 (3H, s), 2.00-1.55 (2H, m). MS (DCl, NH₃) m/e 541 (M+H)⁺. Anal calcd for C₃₂H₃₂N₂O₄S·0.50 H₂O: C, 69.92; H, 6.05; N, 5.10. Found: C, 69.94; H, 6.20; N, 4.90.

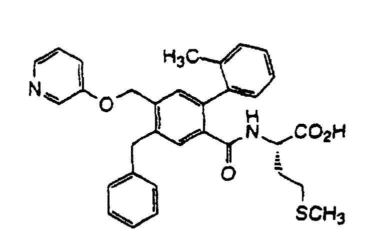

### Example 191

### [4-(3-pyridyloxymethyl)-5-phenylmethyl-2-(2-methylphenyl)benzoyl methionine

### Example 191A

### [4-(3-pyridyloxymethyl)-5-phenylmethyl-2-(2-methylphenyl)benzoyl methionine methyl ester

To a solution in DMF (2 mL) of bis(diphenylphosphinoferrocenyl)palladium(II) chloride (30 mg) and cesium chloride (213 mg, 0.654 mmol) was added a solution of 4-(3-pyridyloxymethyl)-5-iodo-2-(2-methylphenyl)benzoic acid methyl ester (100 mg, 0.22 mmol), prepared as in Example 169G, and 9-benzyl-9-borabicyclo[3.3.1]nonane (0.5 M in THF, 1.31 mL, 10.6 mmol) and the reaction mixture was stirred at 70 °C for 3 hours. The reaction mixture was cooled to ambient temperature and partitioned between water and ethyl acetate. The organic phase was washed with water and brine, dried over sodium carbonate, filtered and concentrated in vacuo. Chromatography on silica gel (30% ethyl acetate-hexanes) gave the desired compound.

### Example 191B

### [4-(3-pyridyloxymethyl)-5-methylphenyl-2-(2-methylphenyl)benzoyl methionine

The desired compound was prepared by saponification of the compound of Example 191A, followed by coupling with methionine methyl ester hydrochloride and saponification of the methyl ester as described above. ¹H NMR (300MHz, CDCl₃) δ 8.27 (1H, d, *J*= 3 Hz), 8.19 (1H, dd, *J*= 6, 2 Hz), 7.88 (1H, s), 7.40-7.00 (12H, m), 6.00 (1H, d, *J*= 9 Hz), 5.08 (1H, d, J= 12 Hz), 5.01 (1H, d, *J*= 12 Hz), 4.62 (1H, m), 4.15 (2H, s), 2.20-2.05 (5H, m), 2.02 (3H, s), 1.92 (1H, m) 1.60 (1H, m). MS (DCl, NH₃) m/e 541 (M+H)⁺. Anal calcd for C₃₂H₃₂N₂O₄S·0.25 H₂O: C, 70.50; H ,6.01; N, 5.14. Found: C, 70.23; H, 5.84; N 4.94.

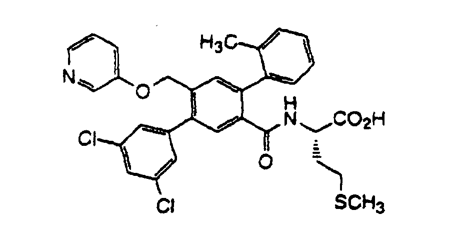

### Example 192

### [4-(3-pyridyloxymethyl)-5-(3,5-dichlorolphenyl)-2-(2-methylphenyl)benzoyl methionine

The desired compound was prepared according to the method of Example 190, except substituting 3,5-dichlorophenylbornic acid for 4-methylphenylboronic acid. ¹H NMR (300MHz, CDCl₃) δ 8.37 (1H, d, *J*= 9 Hz), 8.21 (1H, d, *J*= 3 Hz), 7.85 (1H, d. *J*= 4 Hz), 7.44 (1H, s), 7.40-7.20 (9H, m), 6.08 (1H, d, *J*= 9 Hz), 5.03 (2H, s), 4.62 (1H, m), 2.25-2.05 (5H, m), 2.02 (3H, s), 1.95 (1H, m) 1.64 (1H, m). MS (DCI, NH₃) m/e 595 (M+H)⁺. Anal calcd for C₃₁H₂₈Cl₂N₂O₄S·0.20 H₂O: C, 62.15; H. 4.78; N 4.68. Found: C, 61.86; H, 4.38; N 4.38.

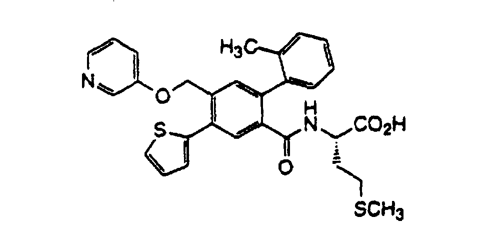

### Example 193

### [4-(3-pyridyloxymethyl)-5-(2-thienyl)-2-(2-methylphenyl)benzoyl methionine

The desired compound was prepared according to the method of Example 190, except substituting 2-thienylboronic acid for 4-methylphenylboronic acid. ¹H NMR (300MHz, CDCl₃) δ 8.38 (1H, d, *J*= 3 Hz), 8.20 (1H, dd, *J*= 3, 1 Hz), 8.03 (1H, s), 7.43 (1H, s), 7.39 (1H, dd, *J*= 6,2 Hz), 7.38-7.20 (6H, m), 7.15(1H, dd, *J*= 3.1 Hz), 7.08 (1H, m), 6.07 (1H, d. *J*= 9 Hz), 5.10 (2H, m), 4.61 (1H, m), 2.20-2.05 (5H, m), 2.02 (3H, s), 1.93 (1H, m) 1.62 (1H, m). MS (DCl, NH₃) m/e 533 (M+H)⁺. Anal calcd for C₂₉H₂₈N₂O₄S₂ 0.25 H₂O: C. 64.84; H, 5.35; N,.4.21. Found: C, 64.55; H, 4.83; N, 4.85.

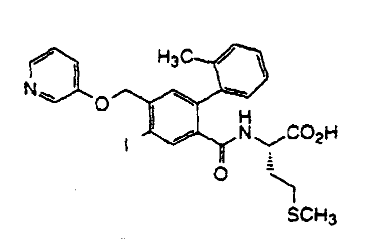

### Example 194

### [4-(3-pyridyloxymethyl)-5-iodo-2-(2-methylphenyl)benzoyl methionine

The desired compound was prepared by saponification of 4-(3-pyridyloxymethyl)-5-iodo-2-(2-methylphenyl)benzoic acid methyl ester, prepared as in Example 169G, followed by coupling with methionine methyl ester hydrochloride and saponification of the methyl ester as described above. ¹H NMR (300MHz, DMSO-d6) δ 12.68 (1H, bs), 8.45 (1H, d, *J*=9 Hz), 8.37 (1H, d, *J*=3 Hz), 8.20 (1H, d, *J*= 4 Hz), 7.93 (1H, s), 7.48 (1H, m), 7.37 (2H, m), 7.30-7.00 (4H, m), 5.20 (2H, s), 4.22 (1H, m), 2.30-2.00 (5H, m), 1.96 (3H, s), 1.80 (2H, m). MS m/e (DCl, NH₃) m/e 577 (M+H)⁺. Anal calcd for C₂₅H₂₅IN₂O₄S: C, 51.85; H, 4.40; N, 4.84. Found: C, 51.91; H, 4.47; N, 4.69.

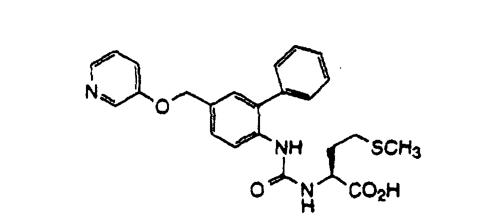

### Example 195

### [4-(3-pyridyloxymethyl)-2-phenylphenylaminocarbonyl]methionine

To a solution in toluene of 4-(3-pyridyloxymethyl)-2-phenylbenzoic acid (126 mg) was added diphenylphosphoryl azide (0.11 mL) and triethylamine (0.075 mL) and the reaction mixture was heated at 100 °C for 20 minutes. A solution in dichloromethane of methionine methyl ester (prepared by addition of triethylamine to the dichloromethane solution) was added and the reaction mixture was stirred at 100 °C for 1 hour and then overnight at ambient temperature. The precipitated product was isolated by filtration and rinsed with ethyl acetate. The filtrate was concentrated and purified by chromatography on silica gel (80% ethyl acetate-hexanes) to give the methyl ester (100 mg).
Saponification of the methyl ester using saturated aqueous lithium hydroxide in methanol gave the title compound. ¹H NMR (DMSO, 300 MHz) δ 1.77 (1H, td, *J*= 7.5, 14.7 Hz), 1.95 (1H, tdd, *J*= 5.7, 7.5, 14.7 Hz), 2.04 (3H, s), 2.45 (2H, t, *J*= 7.5 Hz), 4.22 (1H, td, *J*= 5.7, 7.5 Hz), 5.14 (2H, s), 6.98 (1H, d, *J*= 8.1 Hz), 7.25 (1H, d, *J*= 2.1 Hz), 7.29-7.53 (8H, m), 7.60 (1H, s), 7.92 (1H, d, *J*= 8.7 Hz), 8.16 (1H, dd, *J*= 0.9, 5.1 Hz), 8.35 (1H, d, *J*= 2.7 Hz). MS (FAB/APCI) m/e 452 (M+H)⁺, 450 (M-H)⁻, 486 (M-CI)⁻. Anal calcd for C₂₄H₂₅N₃O₄S·0.30 H₂O: C, 63.08; H, 5.65; N, 9.20. Found: C, 63.11; H, 5.42; N, 8.67.

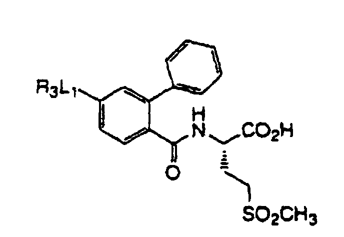

### Examples 196-200

All reactions were performed either in a Manual solid phase synthesis flask using a 120o rotary shaker or on an Advanced ChemTech Model 396 Multiple Peptide Synthesizer (Advanced ChemTech Inc.; Louisville, Kentucky) at ambient temperature. After the reactions were performed the finished compounds were cleaved from the resin. Usually, 80-90 mg of the dried resin containing the desired amide; urea; or secondary amine was treated with a 1.50 mL solution of 95/5 (v:v) trifluoroacetic acid/water for 1.5 h at ambient temperature. The spent resin was removed by filtration and the resulting cleavage solution evaporated in-vacuo. In most cases, 5- 20 mg of crude compound was obtained, Compounds obtained had the desired MW as determined by electrospray mass spectroscopy and had an HPLC purity of 40.90%, or were further purified by partition chromatography to afford compounds of 40-60% HPLC purity. Two types of gradients were used for the reverse phase HPLC. For the amides and ureas a gradient starting with 100% water-0.1% Trifluoroacetic acid and finishing with 100% acetonitrile-0.1% Trifluoracetic acid during a 30 minute period was used. For the secondary amines a gradient beginning with 100% water-5mmol ammonium acetate and finishing with 80% acetonitrile-water-5mmol ammonium acetate during 25 minutes was used.

80 mg of resin (substitution 0.40 mmol/g) containing [4-amino-2-phenylbenoyl]methionine-Wang-polystyrene resin was shaken for 3 min. with 1.0 mL. of N-methylpyrrolidone (NMP). The solvent was drained and the resin was treated 2x (3 min) with 1 mL. NMP. To the now swollen resin were then added 0.20 mL NMP; 0.20 mL of a 1.92 M diisopropylethylamine (DIEA)/NMP solution (15 eq.); 1.00 mL of a 0.180 mM/NMP solution of the desired carboxylic acid (5 eq.); and finally 0.20 mL of a 0.90 M Bromo-tris-pyrrolidino-phosphonium hexafluorophosphate (PyBrop; 5 equiv.)1/NMP solution. The reaction slurry was then mixed for 6 h and drained. The resin was then washed with NMP (3x; 1.0 mL; 3 min. ea); isopropanol (IPA; 5x; 1.0 mL; 3 min. ea.); NMP (3x; 1.0 mL; 3 min. ea.); methanol (MEOH; 2x; 1.0 ml; 3 min. ea.); and finally diethyl ether (2x; 1.0 mL; 3 min. ea.). The resin was then dried and subjected to cleavage conditions described above.

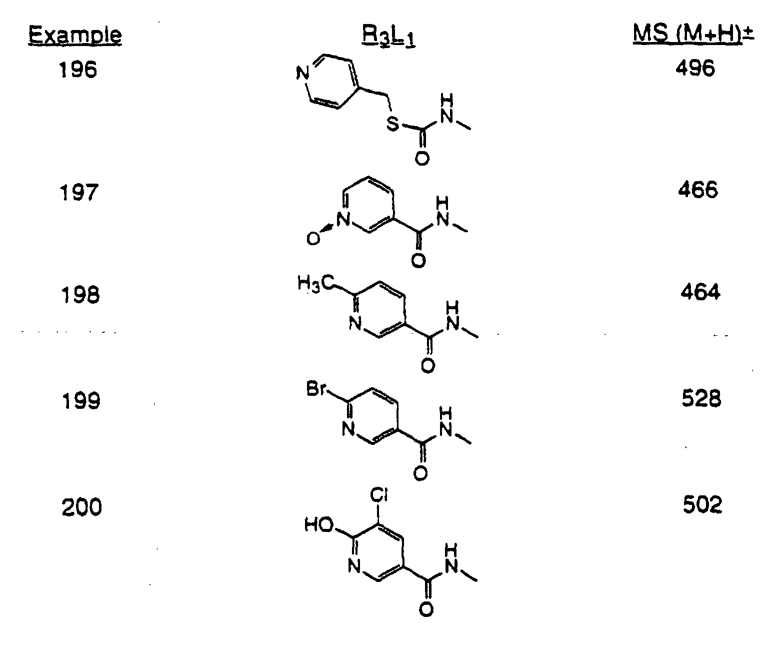

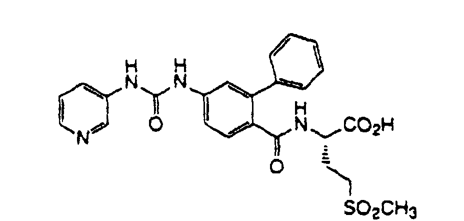

### Example 201

90 mg of resin (substitution 0.39 mmol/g.) containing [4-amino-2-phenylbenzoyl]methionine-Wang-polystyrene resin was shaken with 1.0 mL. dimethylformamide (DMF) for 3 min. The solvent was drained and the resin was then washed with DMF (3x; 1.0 mL; 3 min. ea.); tetrahydrofuran (THF; 4x; 1.0 mL; 3 min. ea.); THF/dichloromethane (DCM) 1:1 (v:v) (4x; 1.0 mL; 3 min. ea.). The resin was then treated with 0.20 mL of DCM/THF (1:1) and a 1.0 mL solution of 0.50 M p-Nitrophenylchloroformate/0.50 M DIEA in a 1:1 solvent mixture of DCM/THF. The resin suspension was then shaken for 15 min. and to the suspension was then added .020 mL of neat DIEA. After shaking for an additional 15 min,; the solvents were drained away and the resin was then washed with DCM/THF (1:1) (4x; 1.0 mL; 3 min. ea.) The resin was then treated with 0.20 mL of DMF and 1.0 mL of a DMF solution containing 0.50 M of the desired primary or secondary amine and 0.50 M of DIEA. The suspension was shaken for 30 min. The solvent was drained off and the resin was then washed with DMF (4x; 1.0 mL; 3 min. ea); THF (4x; 1.0 mL; 3 min. ea.); DCM/THF (4x; 1.0 mL; 3 min. ea); diethyl ether (4x; 1.0 mL; 3 min. ea.). The resin was then dried and subjected to cleavage from the resin as described above to give the desired compound. MS m/e 465 (M+H)⁺.

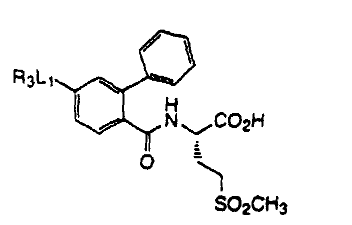

### Examples 203-206

Typically 80 mg of resin (substitution of 0.40 mmol/g) containing 4-formyl-2-phenylbenzamide-L-Methionine-Wang-polystyrene resin was swollen with 1.0 mL of dimethyl acetamide (DMA) for 3 mm. The soivent was drained and the resin was then washed with additional DMA (2x; 1.0 mL; 3 min. ea.). The resin was then suspended in 0.20 mL of DMA and to the suspension was then added a 1.0 mL solution containing 0.48 mM of the desired primary amine (10 eq.) in a 3:1 (v:v) solution of DMA/acetic acid. The resin was shaken for 2 h and was then treated with 0.25 mL of a 2.4 mM solution of sodium cyanoborohydride (10 eq.) in DMA. The resin-slurry was shaken for an additional 2 h. The solvents were drained and the resin was then washed with DMA ( 6x; 1.0 mL; 3 min. ea.); DMF ( 6x; 1.0 mL; 3 min. ea.); IPA (6x; 1.0 mL; 3 min. ea.); DMF ( 6x; 1.0 mL; 3 min. ea.); MEOH ( 6x; 1.0 mL; 3 min. ea.); diethyl ether (6x; 1.0 mL; 3 min. ea.). The resin was dried and then subjected to cleavage as described above.

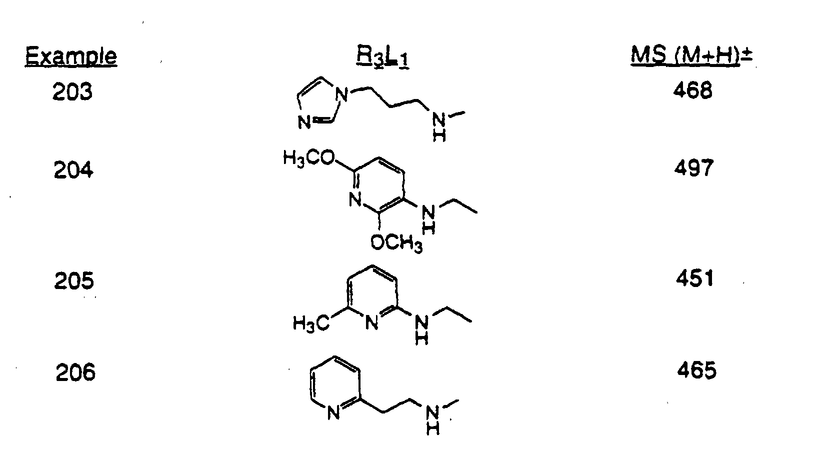

### Examples 207-222

The following compounds were prepared using the materials and methods described above.

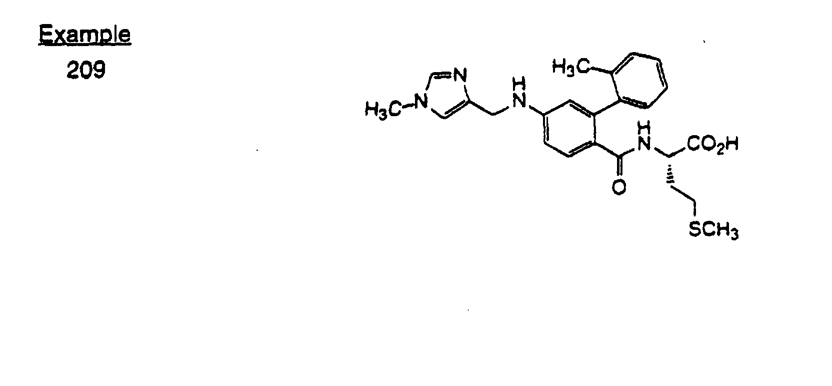

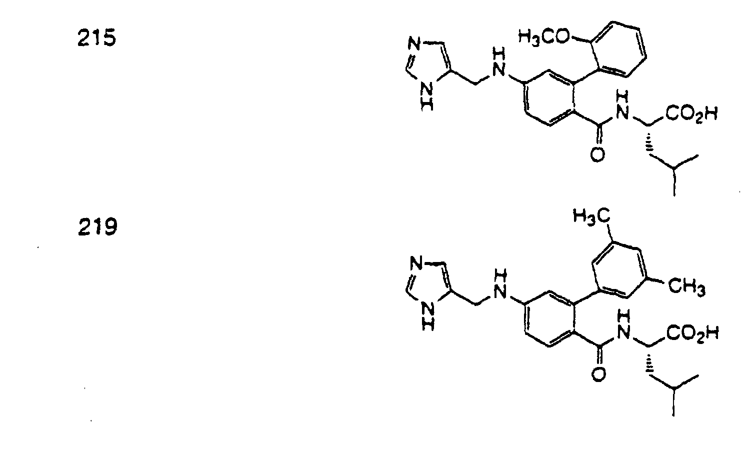

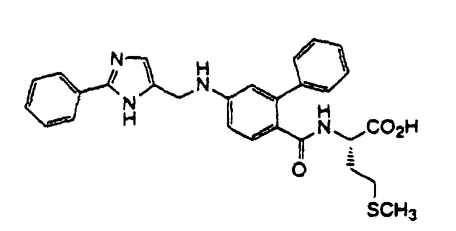

### Example 207A

### 4-[N-(1-H-2-Phenylimidazole-4-yl)methylamino-2-phenylbenzoyl]methionine methyl ester.

1-p-toluenesulfonylimidazole-4-carboxaldehyde (0.05 g, 0.3 mmol) and N-(4-amino-2-phenylbenzoyl)-methionine methyl ester hydrochloride (0.057 g, 0.08 mmol) were dissolved in 10 mL of 95% methanol and 5% acetic acid and stirred for 10 mins. before adding 2 equivalents of sodium cyanoborohydride (0.034 g, 0.54 mmol) The reaction was stirred for 1/2 hour and an additional amount of carboxaldehyde (0.10 g, 0.58 mmol), 2 equivalents sodium cyanoborohydride (0.073 g, 1.2 mmol) and amine hydrochloride salt (0.172 g, 0.44 mmol) were added. The reaction was stirred at room temperature for 1 h. The reaction mixture was concentrated and the residue taken up in ethyl acetate and washed with a saturated solution of sodium bicarbonate. The organic phase was dried over magnesium sulfate and concentrated The residue was purified twice by flash chromatography (19:1 chloroform/methanol) to give the ester as a white foam (0.22 g, 74%).: ¹H NMR (300 MHz, CDCl₃) δ 7.64 (d, J=6.78 Hz, 2H), 7.43 (d, J=8.43 Hz, 1H), 7.24-7.32 (m, 8H), 6.83 (s, 1H), 6.46 (d, J=8.43 Hz, 1H), 6.39 (d, J=1.7 Hz, 1H), 5.91 (d, J=7.59 Hz, 1H), 4.74 (s, J=broad Hz, 1H), 4.59 (dd, J=7.5, 5.76 Hz. 1H), 4.22 (s, 2H), 3.59 (s, 3H), 2.08 (t, J=6.93 Hz, 2H), 1.97 (s, 3H), 1.80-1.89 (m, 1H), 1.58-1.73 (m, 1H).

### Example 207B

### 4-[N-(1-H-2-Phenylimidazole-4-yl)methylamino-2-phenylbenzoyl]methionine.

The above protected sulfonamide (0.1 g, 0.19 mmol) was dissolved in 2 mLs. of THF and cooled to 0°C. Lithium hydroxide (2 mLs. 0.5M) was slowly added to the reaction mixture and stirred for 3 h. The pH was adjusted using 0.5 M HCl and a white precipitate was collected by vacuum filtration and purified twice by reverse phase preparative HPLC (Waters 25X10 cm, C-18 column, 220 nm UV detector, flow rate 15 mLs/min, linear gradient from 5% acetonitrile and 95% water containing 0.1% TFA to 60% acetonitrile in 40 minutes). Fractions containing pure compound were combined and lyophilized to yield the title compound (0.021 g, 18%) as a TFA salt. : ¹H NMR (300 MHz, methanol-d₄) δ 8.04 (d, J=7.8 Hz, 1H), 7.79 (dd, J=7.3 Hz, 2H), 7.60-7.62 (m, 4H), 7.27-7.30 (m, 6H), 6.69 (dd, J=8.4 Hz, 1H), 6.63 (d, J=2.1, 1H) 4.42 (s, 2H), 4.19-4.26 (m, 1H), 2.14-2.32 (m, 2H), 1.98 (s, 3H), 1.75-1.87 (m, 2H).

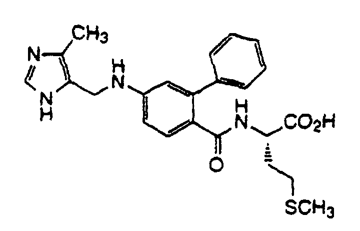

### Example 208A

### 4-[N-(5-Methyl-1-p-toluenesulfonylimidazole-4-yl)-methylamino-2-phenylbenzoyl] methionine methylester.

1-p-toluenesulfonylimidazole-4-carboxaldehyde (0.2 g, 0.76 mmol) and N-(4-amino-2-phenylbenzoyl)-methionine methyl ester hydrochloride (0.224 g, 0.57 mmol) were dissolve in 5 mLs of 95% methanol and 5% acetic acid and stirred for 15 mins. before adding 2 equivalents of sodium cyanoborohydride (0.95 g, 1.5 mmol) The reaction was stirred for 1/2 hour and the addition of reagents added twice more without the addition of sodium cyanoborohydride the second time. The reaction was concentrated and the residue purified twice by flash chromatography first using (19:1 chloroform/methanol) followed by 4:1 chloroform/acetonitrile to give 2 (0.74 g, 74%) as a white solid. : ¹H NMR (300 MHz, CDCl₃) δ 8.09 (s, 1H), 7.76 (d, J=8.37 Hz. 2H), 7.64 (d, J=8.52 Hz, 2H), 7.43-7.24 (m, 8H), 6.60 (dd, J=8.43, 2.16 Hz, 1H); 6.46 (d, J=2.16 Hz, 1H), 5.79 (d, J=7.68 Hz, 1H), 4.61 (dd, J=6.2, 6.21 Hz, 1H), 4.10 (s, 2H), 3.63 (s, 3H), 2.44 (s, 3H), 2.25 (s, 3H), 2.09 (t, J=7.38 Hz, 2H), 193-1.83 (m, 1H), 1.71-1.59 (m, 1H); 13C NMR (75 MHz, CDCl₃) δ 172.2, 168.7, 149.5, 146.5, 141.7, 141.3, 137.4, 136.9, 134.8, 131.3, 130.6, 128.9, 128.7, 127.9, 127.7, 124.0, 114.3, 111.8, 52.5, 51.9, 40.2, 31.8, 29.6, 21.9, 15.4, 9.5.

### Example 208B

### 4-[N-(5-Methyl-1-H-imidazole-4-yl)methylamino-2-phenylbenzoyl]methionine

The above protected sulfonamide **2** (0.5 g, 0.8 mmol) was dissolved into 8 mLs. of THF and cooled to 0oC. Lithium hydroxide (8 mL, 0.5M) was slowly added to the reaction mixture and stirred for 4 h. Excess THF was removed under vacuum and the pH was adjusted using HCl and the aqueous phase extracted with ethyl acetate. The organic layer was washed with a saturated solution of sodium bicarbonate, dried over magnesium sulfate and concentrated to an oil. The residue was purified by reverse phase preparative HPLC (Waters 25X10 cm, C-18 column, 220 nm UV detector, flow rate 15 mLs/min, linear gradient from 5% acetonitrile and 95% water containing 0.1% TFA to 60% acetonitrile in 40 minutes). Fractions containing pure compound were combined and lyophilized to yield the title compound as a TFA salt. : ¹H NMR (300 MHz, methanol-d4) δ 8.68 (s, 1H), 7.42 (d, J=8.34 Hz, 1H), 7.27-7.36 (m, 6H), 6.65 (dd, J=8.46, 1.7 Hz, 1H), 6.58 (d, J=2.3 Hz, 1H), 4.41-4.46 (m, 3H), 2.35 (s, 3H), 2.03-2.23 (m, 2H), 2.00 (s, 3H), 1.74-1.99 (m, 2H).
The next example is Example 210.

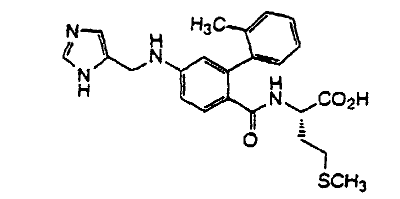

### Example 210A

### 4-[N-(1-Triphenylmethylimidazole-4-yl)methylamino-2-(2-methylphenyl)methionine methyl ester

1-Triphenylmethylimidazole-4-carboxaldehyde (0.3 g, 0.7 mmol) and N-(4-amino-2-(2-methylphenylbenzoyl)-methionine methyl ester hydrochloride (0.25 g. 0.7 mmol) were dissolved in 10 mLs of 95% methanol and 5% acetic acid and stirred for 30 mins. before adding 1 equivalent of sodium cyanoborohydride (.044 g, 0.7 mmol) The reaction was stirred over a period of 3 hours while additional aldehyde was added until all of the amine hydrochloride had disappeared. The reaction mixture was concentrated and the residue taken up in ethyl acetate and washed with a saturated solution of sodium bicarbonate. The organic phase was dried over magnesium sulfate and concentrated The residue was purified by flash chromatography (1:1 ethyl acetate/hexanes) to give the title compound as a white foam (0.36 g, 74%). : ¹H NMR (300 MHz, CDCl₃) δ 7.45 (s, 1H), 7.25-7.35 (m, 13H), 7.09-7.13 (m, 6H), 6.74 (s, 1H), 6.65 (dd, J=9.3, 1.5 Hz, 1H), 6.34 (d, J=2.5 Hz, 1H), 5.69 (t, J=7.35 Hz, 1H), 4.56-4.61 (m, 1H), 4.27 (d, J=4.8 Hz, 2H), 3.64 (s, 3H), 2.00-2.15 (m, 8H), 1.79-1.86 (m, 1H), 1.48-1.56 (m, 1H); ¹³C NMR (75 MHz, CDCl₃) δ 172.4, 167.6 167.2, 150.3, 142.4, 141.4, 141.2, 139.1, 138.2, 136.6, 132.3, 132.0, 130.8, 130.6, 129.9, 129.2, 128.9, 128.3, 126.5, 126.4, 121.9, 121.4, 119.3, 114.2, 112.0, 75.6, 52.4, 51.7, 41.8, 32.0, 29.6, 20.1, 15.4.

### 4-[N-(1-H-imidazole-4-yl)methylamino-2-(2-methylphenyl)benzoyl)]-methionine

### Example 210B

The above protected peptidomimetic (0.15 g, 0.22 mmol) was dissolved into 3.5 mLs. of THF and cooled to 0°C. Lithium hydroxide (18.1 mg dissolved in 3.5 mLs. of water) was slowly added to the reaction mixture and stirred for 1 h. The pH was adjusted using 1 N HCI and placed under vacuum to remove excess THF. The residue was taken up in ethyl acetate and dried over magnesium chloride and excess solvent removed under vacuum. The residue was taken up in 4 mL. of methylene chloride and 8 mL. of trifluoroacetic acid and the reaction mixture was immediately quenched with triethylsilane until colorless. The reaction was stirred for an addition 2 hrs and concentrated to an oil. The residue was taken up in methylene chloride and 3 N HCl added The solids were vacuum filtered collected and dried under vacuum to give desired product. (0.050 g, 50%) as a HCl salt.: ¹H NMR (300 MHz, CD3OD) δ 8.65 (s, 1H), 7.58-7.66 (m, 1H), 7.49 (s, 1H), 7.11-7.25 (m, 4H), 6.80 (dd, J=8.6, 2.4 Hz, 1H), 6.50 (d, J=1.9 Hz, 1H), 4.53 (s, 2H), 4.39-4.40 (m, 1H), 2.04-2.12 (m, 5H), 1.98 (s, 3H), 1.81-1.90 (m, 1H), 1.46-1.64 (m, 1H).

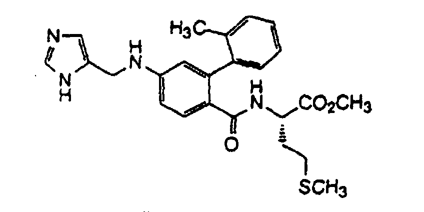

### Example 211

### 4-[N-(1-H-imidazole-4-yl)methylamino-2-(2-methylphenylbenzoyl)]methionine methylester.

The above protected peptidomimetic from Example 210B (0.135g, 0.22 mmol) was dissolved into 4 mls. of methylene chloride and 8 mls. of trifluoroacetic acid and the reaction mixture was immediately quenched with triethylsilane until colorless. The reaction was stirred for an addition 2 hr. and concentrated to an oil. The residue was taken up in methylene chloride and 3 N HCl added The solids were vacuum filtered collected and dried under vacuum to give desired product. (0.050 g, 50%) as a HCl salt.: ¹H NMR (300 MHz, CD3OD) δ 8.84 (s, 1H), 7.57-7.65 (m, 1H), 7.47 (s, 1H), 7.11-7.26 (m, 4H), 6.73 (d, J=8.64 Hz, 1H), 6.43 (s, 1H), 4.50 (s, 2H), 4.39-4.46 (m, 1H), 3.64 (s, 3H), 2.04-2.12 (m, 5H), 1.98 (s, 3H), 1.84-1.94 (m, 1H), 1.57-1.62 (m, 1H).

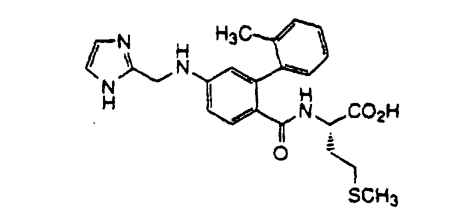

### Example 212A

### 4-[N-(1-Triphenylmethylimidazole-2-yl)methylamino-2-phenylbenzoyl]methoinine.

1-Triphenylmethylimidazole-2-carboxaldehyde (0.5 g, 1.5 mmol) and N-(4-amino-2-(2-methylphenylbenzoyl)methionine methyl ester hydrochloride salt (0.25 g, 0.7 mmol) were dissolve in 30 mLs of 95% methanol and 5% acetic acid and stirred for 30 mins. before adding 1 equivalent of sodium cyanoborohydride (.09 g, 1.5 mmol) The reaction was stirred over a period of 3 hours while additional aldehyde was added until all of the amine hydrochloride had disappeared. The reaction mixture was concentrated and the residue taken up in ethyl acetate and washed with a saturated solution of sodium bicarbonate. The organic phase was dried over magnesium sulfate and concentrated The residue was purified by flash chromatography (19:1 chloroform/methanol) to give the title compound as a white foam (0.55 g, 55%). : ¹H NMR (300 MHz, CDCl₃) δ 7.53 (d, J=8.5 Hz, 1H), 7.23-7.43 (m, 15H), 7.13-7.16 (m, 6H), 7.03 (d, J=1.4 Hz, 1H), 6.87 (d, J=1.1 Hz, 1H), 6.2 (dd, J=8.5, 2.2 Hz, 1H), 6.10 (d, J=2.2 Hz, 1H), 5.61 (d, J=7.7 Hz, 1H), 5.05 (br.s, 1H,), 4.59 (dd, J=9.9, 4.7 Hz, 1H), 3.63 (s, 3H), 3.33 (br.s, 2H), 2.05 (t, J=7.7 Hz, 2H), 2.00 (s, 3H), 1.80-1.91 (m, 2H), 1.24-1.29 (m, 2H).

### Example 212B

### 4-[N-(1-H-imidazole-2-yl)methylamino-2-phenylbenzoyl)-methionine

The above protected peptidomimetic (0.45 g, 0.66 mmol) was dissolved into 3.5 mLs. of THF and cooled to 0°C. Lithium hydroxide (18.1 mg dissolved in 3.5 mLs. of water) was slowly added to the reaction mixture and stirred for 2 h. The pH was adjusted using 1 N HCl and placed under vacuum to remove excess THF. The residue was taken up in ethyl acetate and dried over magnesium chloride and excess solvent removed under vacuum. The residue was taken up in 4 mls. of methylene chloride and 8 mls. of trifluoroacetic acid and the reaction mixture was immediately quenched with triethylsilane until colorless. The reaction was stirred for an addition 2 hrs and concentrated to an oil. The residue was purified by reverse phase preparative HPLC (Waters 25X10 cm, C-18 column, 220 nm UV detector, flow rate 15 mLs/min, linear gradient from 5% acetonitrile and 95% water containing 0.1% TFA to 60% acetonitrile in 40 minutes). Fractions containing pure compound were combined and lyophilized to yield the acid as a TFA salt.: ¹H NMR (300 MHz, DMSO-d6) δ 8.11 (d, 1H), 7.60 (s, 1H), 7.3-7.4 (m, 6H), 6.82 (br.s, 1H), 6.55-6.60 (m, 2H), 4.69 (s, 2H), 4.20-4.30 (m, 1H), 2.10-2.30 (m, 2H), 2.07 (s, 3H), 1.75-1.86 (m, 2H).

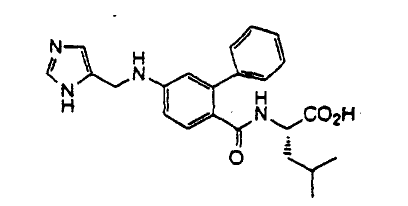

### Example 213

### 4-[N-(iimidazol-4-yl)methylamino-2-phenylbenzoyl]leucine.

To a solution of 2-phenyl-4-amino-benzoyl leucinemethyl ester hydrochloride (0.25 g, 0.74 mmoles) in methanol was added tritylimidazole-4-carboxaldehyde (0.25 g, 0.74 mmoles) followed by 1 mL of glacial acetic acid and the reaction stirred for 30 min. This was followed by the addition of NaCNBH₃ (0.046 g, 0.74 mmoles) and the reaction was stirred at room temperature for 2 h. The solvent was evaporated and the residue dissolved in ethyl acetate (~3 mL), and chromatographed on a silica gel column (1.05" x 23") to afford a white solid. (0.30 g, 61 %). ¹H NMR (300 MHz, CDCl₃) δ 0.79 (dd, 6 H), 1.10-1.35 (m, 3 H), 3.64 (s, 3 H), 4.28 (d, 2 H), 4.51 (m, 1 H), 4,66 (br s, 1 H), 5.45 (d, 1 H), 6.49 (s, 1 H), 6.64 (dd, 1 H), 6.74 (s, 1 H), 7.12 (m, 6 H), 7.09-7.27 (m, 15 H), 7.48 (s, 1 H), 7.69 (d. 1 H).
The above compound (0.30 g. 0.45 mmoles) was dissolved in 15 mL of THF/H₂O (3:2) and LiOH (0.035 g, 0.91 mmoles) was added. The reaction was stirred at room temperature for 2 h. The THF was evaporated and the residue dissolved in 15 mL water, and 1N HCl added to lower the pH to 2. The compound is then extracted with ethyl acetate (3 x 25 mL), dried and the solvents evaporated to afford the free carboxylic acid as a white solid. (0.25 g, 84 %).
The above compound (0.25 g, 0.38 mmoles) was dissolved in 10 mL dichloromethane and 3 mL trifluoroacetic acid followed by the addition of 1.5 mL of triethylsilane and the reaction stirred at room temperature for 2 h. The solvents were evaporated and ether added followed by the addition of 6 N HCl in ether to precipitate the desired compound which was collected by rapid filtration. (0.12 g, 77 %) ¹H NMR (300 MHz, DMSO-d₆) δ 0.76 (dd. 6 H), 1.38 (m, 2 H), 1.55 (m, 1 H), 4.19 (m, 1 H), 4.42 (d, 2 H), 6.63 (s, 1 H), 6.73 (d, 1 H), 7.31-7.36 (m, 6 H), 7.63 (s, 1 H), 8.23 (d, 1 H), 9.09 (s, 1 H).

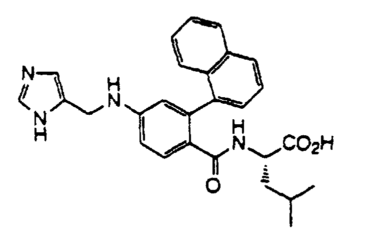

### Example 214

### 4-[N-(imidazol-4-yl)methylamino-2-(1-naphthyl)benzoyl]leucine.

To a solution of 2-(1-naphthyl)-4-aminobenzoylleucinemethyl ester hydrochloride (0.59 g, 1.5 mmoles) in methanol was added tritylimidazole-4-carboxaldehyde ( 0.61 g, 1.80 mmoles) followed by 1 mL of glacial acetic acid and the reaction stirred for 30 min. This was followed by the addition of NaCNBH₃ (0.34 g, 5.4 mmoles) and the reaction was stirred at room temperature for 2 h. The solvent was evaporated and the residue dissolved in ethyl acetate (~3 mL), and chromatographed on a silica gel column (1.05" x 23") to afford a white solid. (0.72 g, 67 %). ¹H NMR (300 MHz, CDCl₃) δ 0.13 (m, 0.8 H), 0.44 (m, 5.2 H), 0.57 (m, 2 H), 0.93 (m, 1 H), 3.40 (s, 1 H), 3.57 (s, 2 H), 4.24 (m, 1 H), 5.37-5.47 (dd, 1 H), 6.49 (s, 1 H), 7.08 (m, 2 H), 7.09-7.11 (m, 6 H), 7.30-7.32 (m, 11 H), 7.49-7.57 (m, 5 H), 7.80 (m, 2 H), 8.08 (d, 1 H).
The above compound (0.24 g, 0.34 mmoles) was dissolved in 10 mL of THF/H₂O (2:1) and LiOH (0.029 g, 0.68 mmoles) was added. The reaction was stirred at room temperature for 2 h. The THF was evaporated and the residue dissolved in 15 mL water, and 1N HCl added to lower the pH to 2. The compound is then extracted with ethyl acetate (3 x 25 mL), dried and the solvents evaporated to afford the free carboxylic acid as a white solid. (0.20 g, 84 %).
The above compound (0.20 g, 0.29 mmoes) was dissolved in 10 mL dichloromethane and 3 mL trifluoroacetic acid followed by the addition of 1.5 mL of triethylsilane and the reaction stirred at room temperature for 2 h. The solvents were evaporated and ether added followed by the addition of 6 N HCl in ether to precipitate the desired compound, which was collected by rapid filtration. (0.096 g, 74 %) ¹H NMR (300 MHz, DMSO-d₆) δ 0.37 (m, 3 H), 0.65 (m, 3 H), 1.08 (m, 2 H), 1.25 (m, 1 H), 3.89 (m, 1 H), 4.37 (d, 2 H), 6.53 (s, 1 H), 6.79 )m, 1 H), 7.01 (m, 1 H), 7.42-7.62 (m, 8 H), 7.86-7.94 (m, 2 H), 8.86 (s, 1 H).

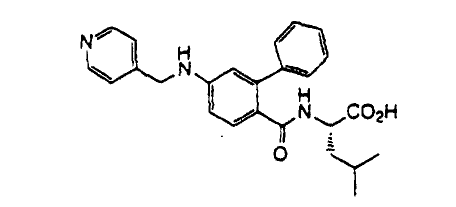

### Example 216

### 5-[N-(4-pyridinyl)methylamino-2-phenylbenzoyl]leucine.

To a solution of 2-phenyl-5-aminobenzoylleucine methyl ester hydrochloride (0.50 g, 1.47 mmoles) in methanol was added pyridine-4-carboxaldehyde (0.164 g, 1.53 mmoles). This was followed by the addition of 1-2 mL of glacial acetic acid, and NaCNBH₃ (0.16 g, 2.56 mmoles) and the reaction was stirred at room temperature for 30 min. The solvents were evaporated and the residue dissolved in 25 mL ethyl acetate and washed with saturated NaHCO₃ (30 mL), concentrated to 3 mL and chromatographed on a silica gel column (1.05" x 23") using ethyl acetate:hexanes (4:1) to afford the protected compound as a yellowish solid. (0.40 g, 63 %). ¹H NMR (300 MHz, CDCl₃) δ 0.76 (m, 6 H), 7.10-7.17 (m, 2 H), 1.27-1.32 (m, 1 H), 3.62 (s, 3 H), 4.43-4.53 (overlapping m & s, 3 H); 5.60 (d, 1 H), 6.61-6.65 (dd, 1 H), 6.97 (d, 1 H), 7.13 (d, 1 H), 7.25-7.35 (m, 8 H), 8.55 (m, 2 H).
The above compound (0.22 g, 0.51 mmoles) was dissolved in 10 mL THF/H₂O (2:1), cooled to 0 °C and LiOH (0.04 g, 1.02 mmoles) added. The reaction was stirred at 0 ∞C for 1 h, followed by stirring at room temperature for 2 h. The solvents were evaporated and the residue passed through a bed of silica gel and eluted with CH₂Cl₂:CH₃OH (9:1) to afford the desired compound as a white solid. (0.19 g, 90 %) ¹H NMR (300 MHz, DMSO-d₆) δ 0.79 (t, 6 H), 1.19 (m, 2 H), 1.42 (m, 1 H), 4.06 (m, 1 H), 4.36 (d, 2 H), 6.53 (dd, 1 H), 6.81 (m, 2 H), 7.04 (d, 1H), 7.17-7.31 (m, 6 H), 7.59 (d, 1 H), 8.46 (d, 2 H).

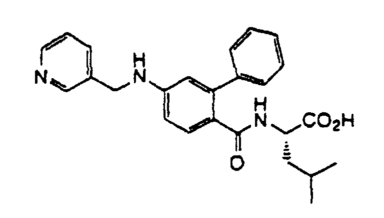

### Example 217

### 5-[N-(3-pyridinyl)methylene(amino-2-phenylbenzoyl)]leucine.

To a solution of 2-phenyl-5-aminobenzoylleucine methyl ester hydrochloride (0.25 g, 0.74 mmoles) in methanol was added pyridine-3-carboxaldehyde (0.08 g, 0.78 mmoles). This was followed by the addition of 1-2 mL of glacial acetic acid, and NaCNBH₃ (0.09 g, 1.28 mmoles) and the reaction was stirred at room temperature for 30 mm. The solvents were evaporated and the residue dissoived in 25 mL ethyl acetate and washed with saturated NaHCO₃ (30 mL), concentrated to 3 mL and chromatographed on a silica gel column (1.05" x 23") using ethyl acetate:hexanes (4:1) to afford as a yellowish solid. (0.14 g, 46 %). ¹H NMR (300 MHz, DMSO-d₆) δ 0.74 (d, 3 H), 0.81 (d, 3 H), 1.37 (m, 2 H), 1.54 (m, 1 H), 3.61 (s, 3 H), 4.21 (m, 1 H), 4.34 (d, 2 H), 6.68 (m, 3 H), 7.19 (m, 2 H), 7.22-7.29 (m, 4 H), 7.38 (m, 1 H), 7.76 (d, 1 H), 8.46 (m, 2 H), 8.60 (s, 1 H).
The above compound (0.14 g, 0.33 mmoles) was dissolved in 10 mL THF/H₂O (2:1), cooled to 0 ∞C and LiOH (0.03 g, 0.67 mmoles) added. The reaction was stirred at 0 °C for 1h, followed by stirring at room temperature for 2h. The solvents were evaporated and the residue passed through a bed of silica gel and eluted with CHCl₃:CH₃OH (9:1) to afford the desired compound as a white solid. (0.12 g, 86 %) ¹H NMR (300 MHz, DMSO-d₆) δ 0.79 (d, 6 H), 1.23 (m, 1 H), 1.34 (m, 1 H), 1.54 (m, 1 H), 3.84 (m, 1 H), 4.33 (d, 2 H), 6.63 (d, 1 H), 6.75 (s, 2 H), 7.05 (m, 2 H), 7.18-7.35 (m, 6 H), 7.76 (d, 1 H), 8.43 (, 1 H), 8.59 (s, 1 H).

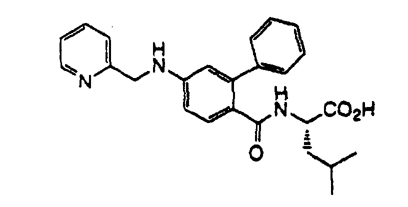

### Example 218

### 5-[N-(2-pyridinyl)methylamino-2-phenylbenzoyl]leucine

To a solution of 2-phenyl-5-aminobenzoylleucine methyl ester hydrochloride (0.25 g, 0.74 mmoles) in methanol was added pyridine-2-carboxaldehyde (0.08 g, 0.77 mmoles). This was followed by the addition of 1-2 mL of glacial acetic acid, and NaCNBH₃ (0.09 g, 1.28 mmoles) and the reaction was stirred at room temperature for 30 min. The solvents were evaporated and the residue dissolved in 25 mL ethyl acetate and washed with saturated NaHCO₃ (30 mL), concentrated to 3 mL and chromatographed on a silica gel column (1.05" x 23") using ethyl acetate:hexanes (4:1) to afford the ester as a yellowish solid. (0.20 g, 63 %). ¹H NMR (300 MHz, DMSO-d₆) δ 0.73 (d, 3H), 0.79(d, 3 H), 1.36 (m, 2 H), 1.52 (m, 1 H), 3.60 (s, 3 H), 4.21 (m, 1 H), 4.41 (d, 2 H), 6.65 (d, 2 H), 6.76 (t, 1 H), 7.19 (d, 1 H), 7.22-7.28 (m, 6 H), 7.72 (d, 1 H), 7.81 (dd, 1 H), 8.53 (m, 2 H).
The above compound (0.20 g, 0.46 mmoles) was dissolved in 10 mL THF/H₂O (3:2), cooled to 0°C and LiOH (0.04 g, 0.93 mmoles) added. The reaction was stirred at 0°C for 1 h, followed by stirring at room temperature for 2 h. The solvents were evaporated and the residue passed through a bed of silica gel and eluted with CHCl₃:CH₃OH (9:1) to afford the desired compound as a white solid. (0.19 g, 98 %) ¹H NMR (300 MHz, DMSO-d₆) δ 0.79 (dd, 6 H), 1.27 (m, 2 H), 1.50 (m, 1 H), 3.89 (m, 1 H), 4.38 (d, 2 H), 6.60 (m, 1 H), 6.69 (s, 1 H), 7.03 (d, 1 H), 7.17-7.36 (m, 7 H), 7.38 (d, 1 H), 7.73 (t, 1 H), 8.51 (d, 1 H).

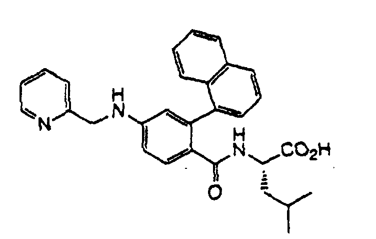

### Example 220

### 4-[N-(2-pyridinyl)methylene(amino-2-(1-naphthyl)benzoyl)]leucine.

To a solution of 2-(1-naphthyl)-4-amino-benzoyl leucinemethylester hydrochloride (0.20 g, 0.51 mmoles) in 15 mL methanol was added pyridine-2-carboxaldehyde (0.06 g, 0.51 mmoles). This was followed by the addition of 1-2 mL of glacial acetic acid, and NaCNBH₃ (0.05 g, 0.77 mmoles) and the reaction was stirred at room temperature for 30 min. The solvents were evaporated and the residue dissolved in 25 mL ethyl acetate and washed with saturated NaHCO₃ (30 mL), concentrated to 3 mL and chromatographed on a silica gel column (1.05" x 23") using ethyl acetate:hexanes (4:1) to afford the ester as a yellowish solid. (0.15 g, 61 %). ¹H NMR (300 MHz, CDCl₃) δ 0.13 (m, 0.8 H), 0.48 (m, 5.2 H), 0.57-0.64 (m, 2 H), 0.96-1.1 (m, 1H), 3.4 (s, 1H), 3.61 (s, 2 H), 4.21 (m, 1 H), 4.50 (m, 2 H), 5.40 (m, 1 H), 5.48 (br t, 1 H), 6.57 (d, 1 H), 6.82 (dd, 1 H), 7.23-7.52 (m, 7 H), 7.85 (m, 1 H), 7.92-8.04 (m, 3 H), 8.57 (d, 1 H).
The above compound (0.15 g, 0.31 mmoles) was dissolved in 10 mL THF/H₂O (2:1), cooled to 0°C and LiOH (0.03 g, 0.62 mmoles) added. The reaction was stirred at 0 °C for 1 h, followed by stirring at room temperature for 2 h. The solvents were evaporated and the residue passed through a bed of silica gel and eluted with CHCl₃:CH₃OH (9:1) to afford the desired compound as a white solid. (0.13 g, 86 %) ¹H NMR (300 MHz, DMSO-d₆) δ 0.45 (m, 7 H), 0.81 (m, 1 H), 1.08 (m, 1 H), 3.77 (m, 1 H), 4.39 (s, 2 H), 6.22 (d, 1 H), 6.50 (m, 1 H), 6.72 (d, 1 H), 7.15 (br s, 1 H), 7.18-7.51 (m, 8 H), 7.81 (m, 1 H), 7.88 (m, 2 H), 8.48 (m, 1 H).

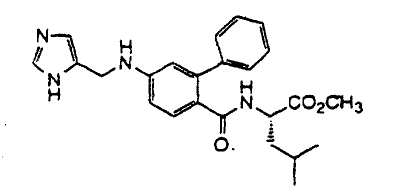

### Example 221

### 4-[N-(1-H-imidazole-2-yl)methylamino-2-phenylbenzoyl)leucine methyl ester.

This compound was synthesized starting from the ester described in the first part of Example 213. The ester (0.45 g, 0.68 mmoles) was dissolved in 10 mL dichloromethane and 3 mL TFA added, followed by the addition of 1.5 mL triethylsilane. The colorless solution was stirred at room temperature for 2 h, following which the solvent was evaporated, the residue dissolved in ethyl acetate and washed with saturated NaHCO3 (25 mL). The organic layer was dried and evaporated to afford the title compound as a white solid. (0.24 g, 84 %). ¹H NMR (300 MHz, DMSO-d₆) δ 0.76 (d, 3 H), 0.81 (d. 3 H0, 1.38 (m, 2 H), 1.52 (m, 1 H), 3.60 (s, 3 H), 4.19 (m, 1H), 4.42 (d, 2 H), 6.67 (s, 1 H), 6.67 (dd, 1 H), 7.22-7.35 (m, 6 H), 7.55 (s, 1 H), 8.18 (d, 1H), 9.04 (s, 1H).

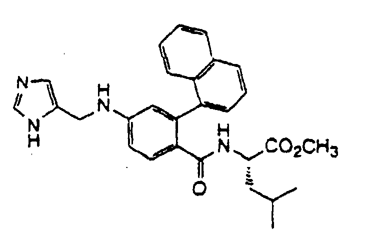

### Example 222

### 4-[N-(1-H-imidazole-2-yl)methylamino-2-(1-naphthyl)benzoyl)leucine methyl ester.

This compound was synthesized starting from the ester described in the first part of Example 214. The ester (0.40 g, 0.56 mmoles) was dissolved in dichloromethane (10 mL) and 3 mL. TFA was added, followed by the addition of 1.5 mL triethylsilane. The colorless solution was stirred at room temperature for 2h, following which the solvent was evaporated, the residue dissolved in ethyl acetate and washed with saturated NaHCO₃ (25 mL). The organic layer was dried and evaporated to afford the title compound as a white solid. (0.21 g, 81 %). ¹H NMR (300 MHz, DMSO-d₆) δ 0.38-0.74 (m, 6 H), 0.91 (m, 2 H), 1.11 (m, 1 H), 3.46 (s, 3 H), 3.94 (m, 1 H), 4.41 (d, 2 H), 6.55(s, 1 H), 6.79 (m, 1 H), 7.34-7.57 (m, 8 H), 7.83-7.94 (m, 3 H), 9.05 (s, 1 H).

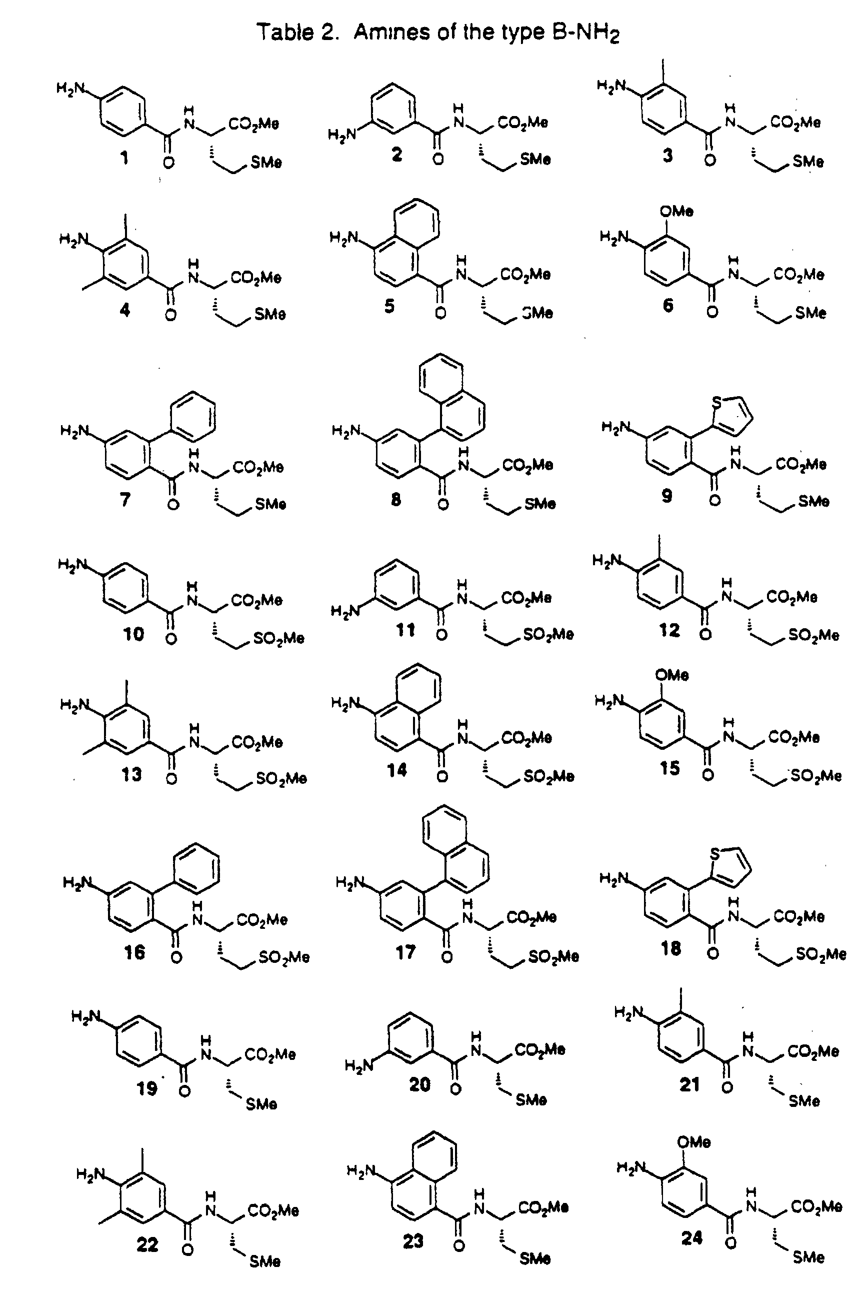

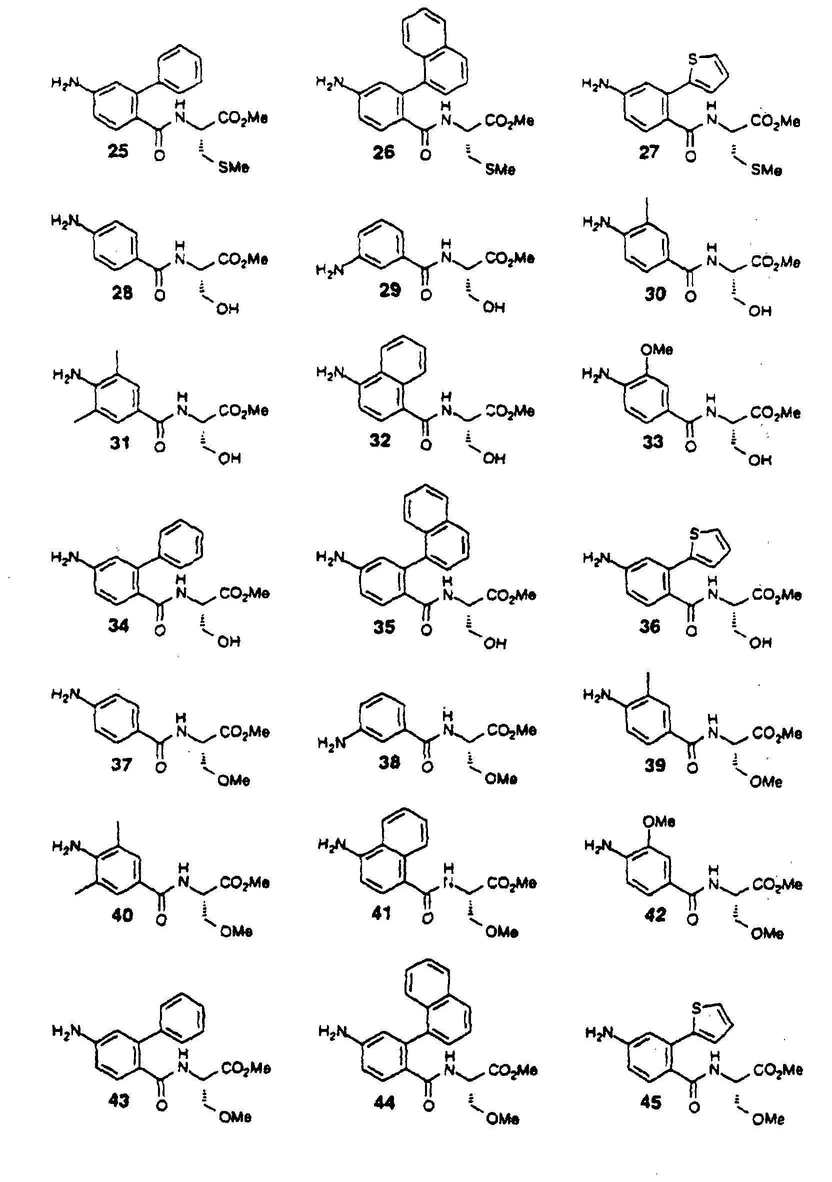

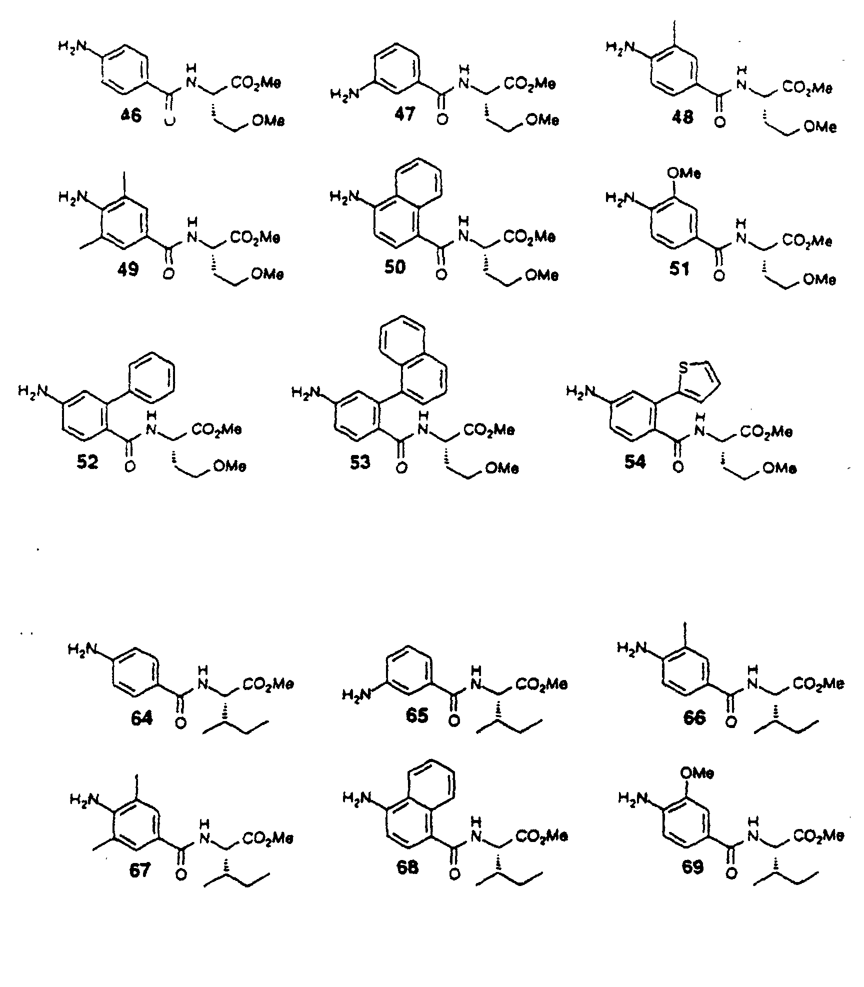

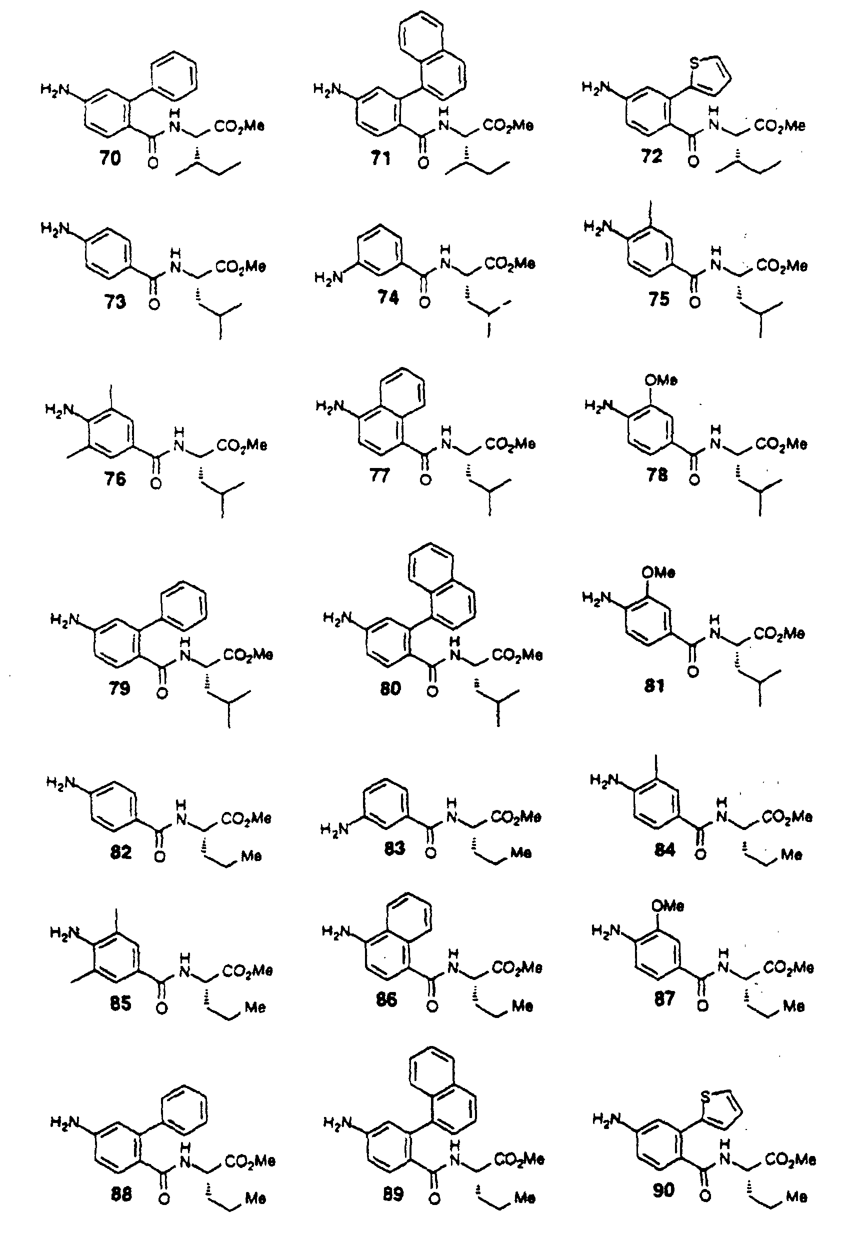

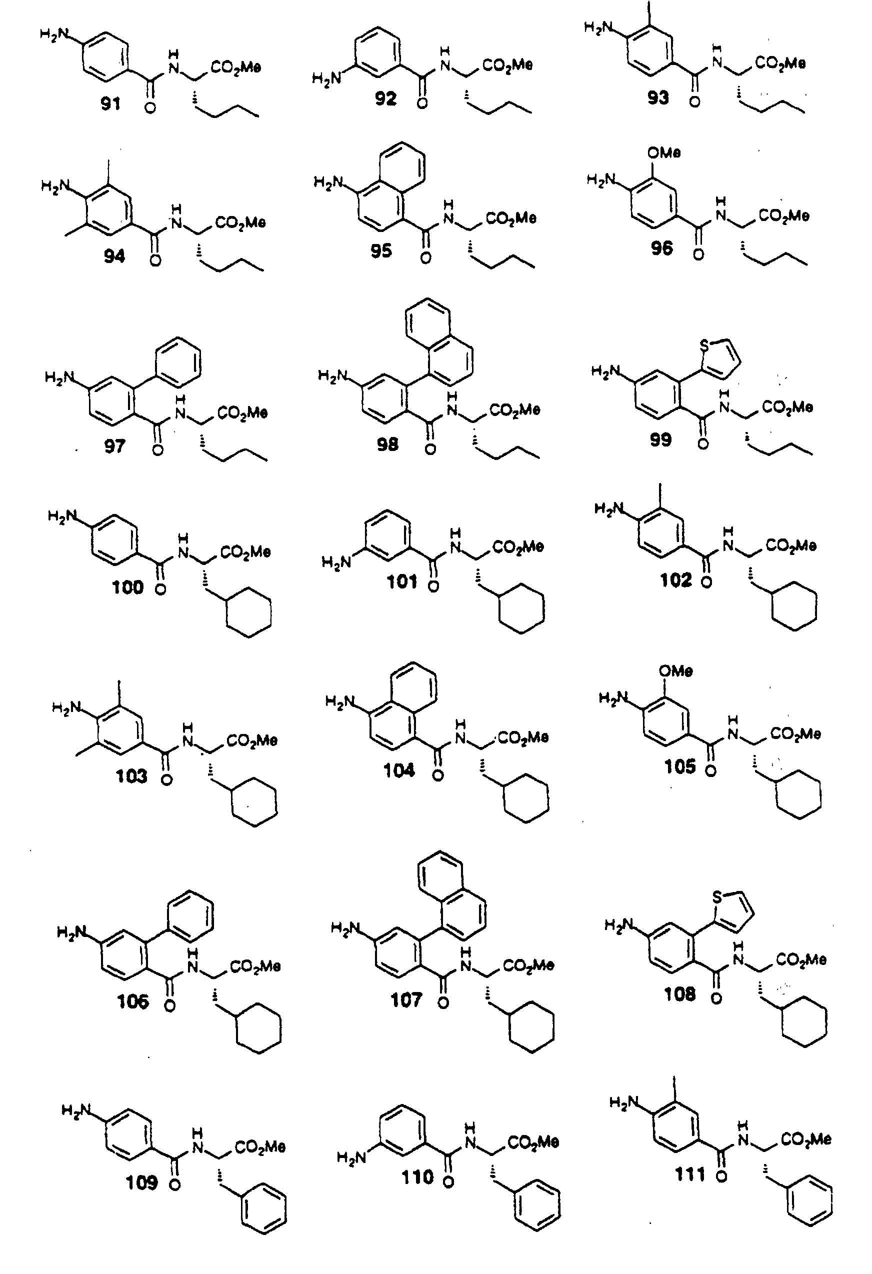

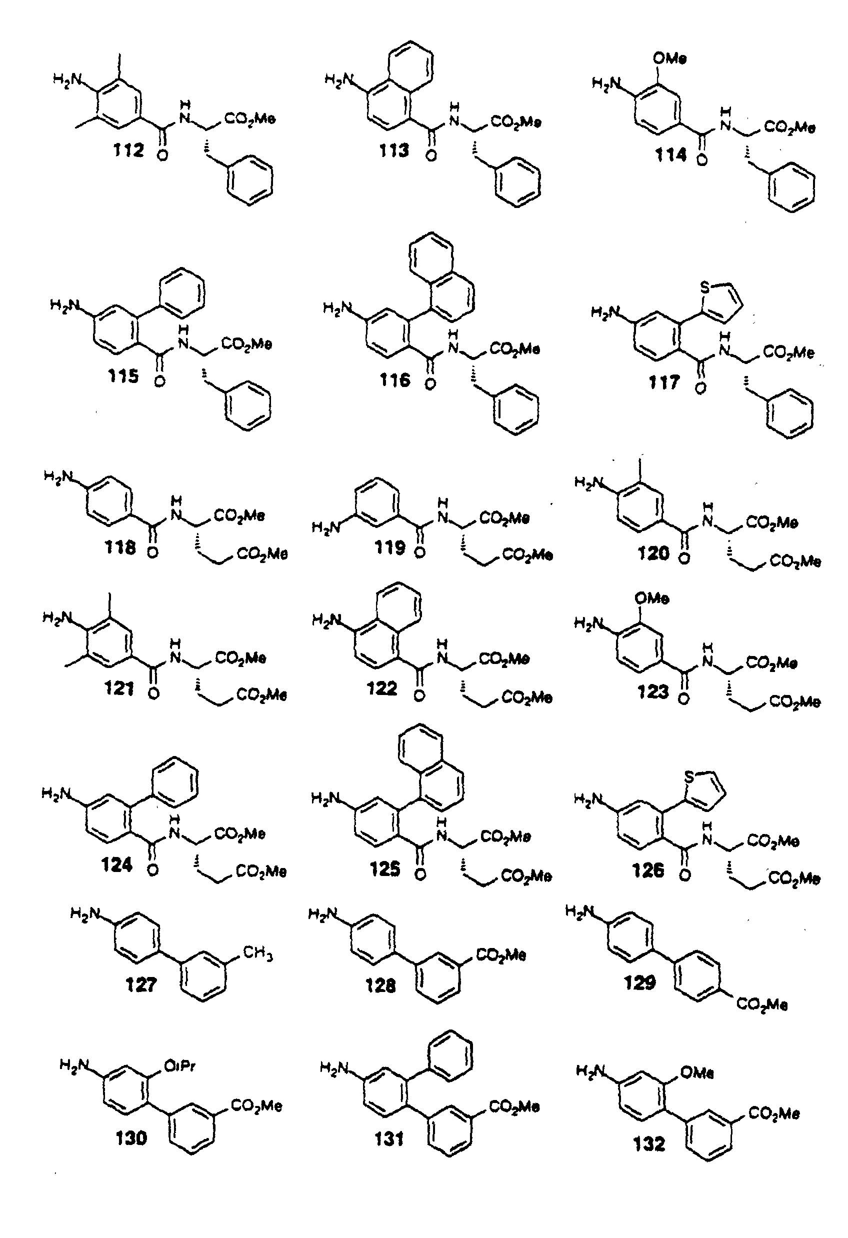

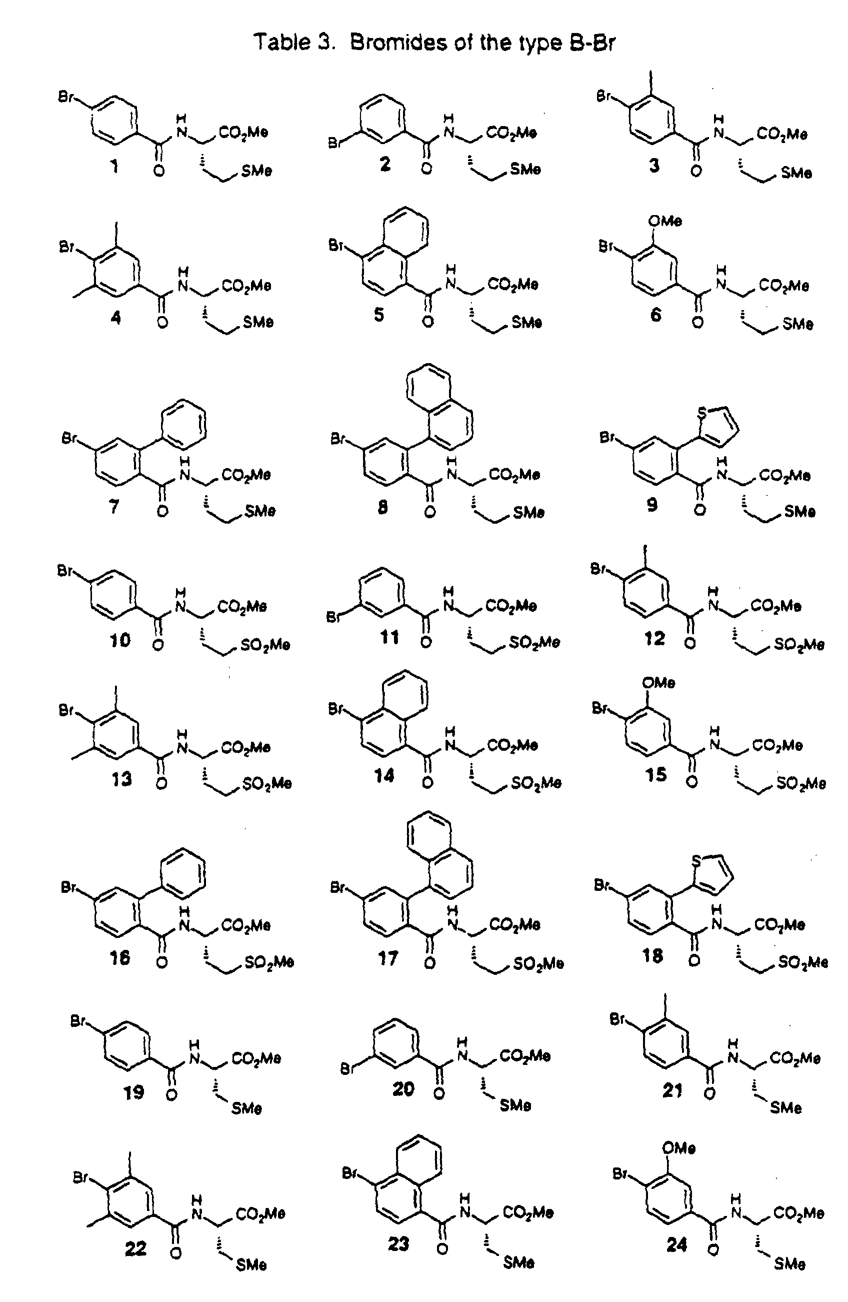

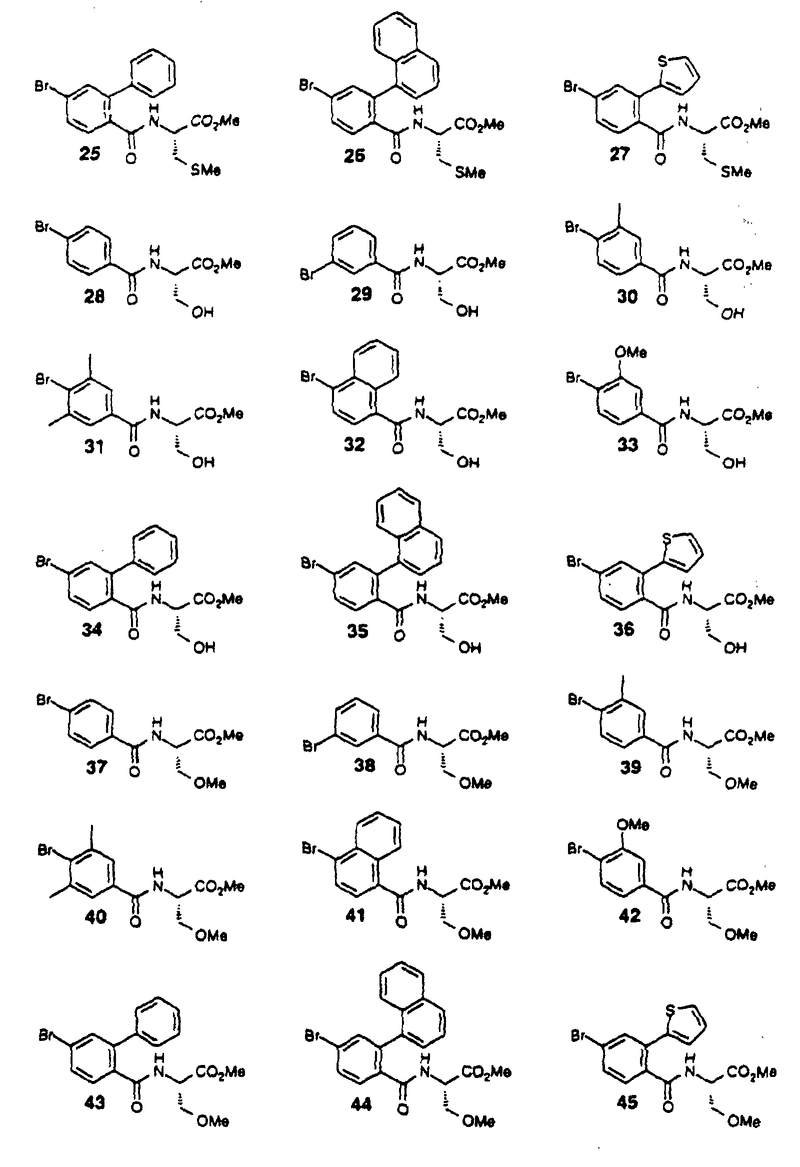

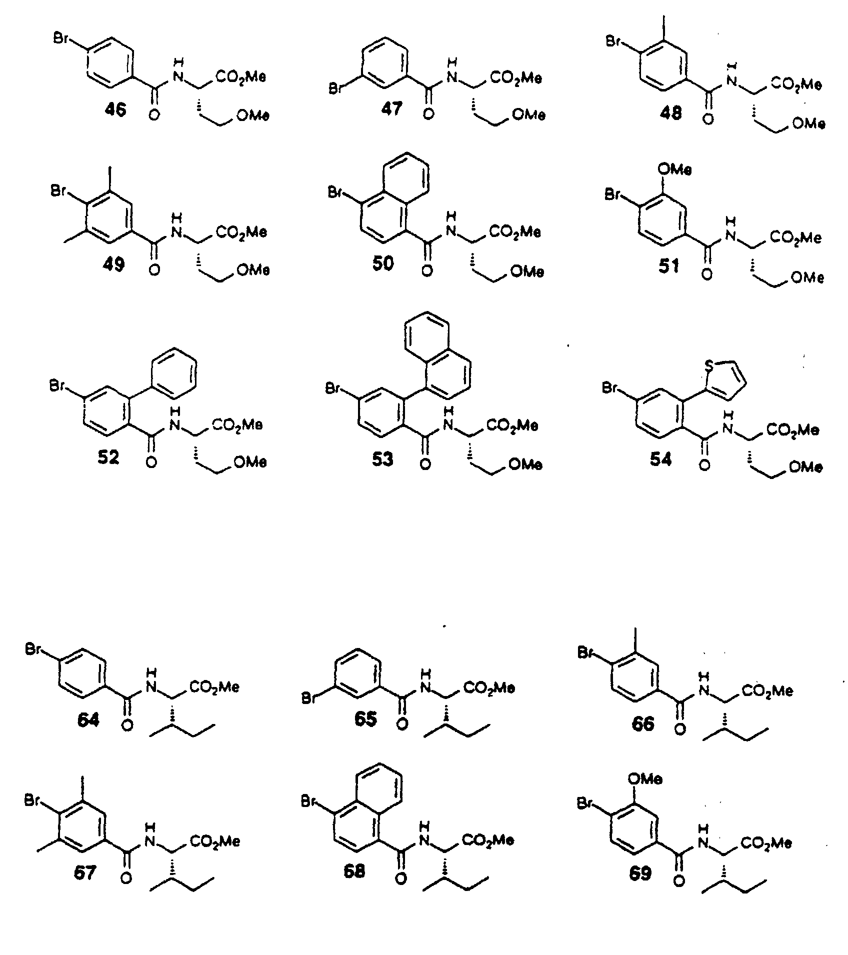

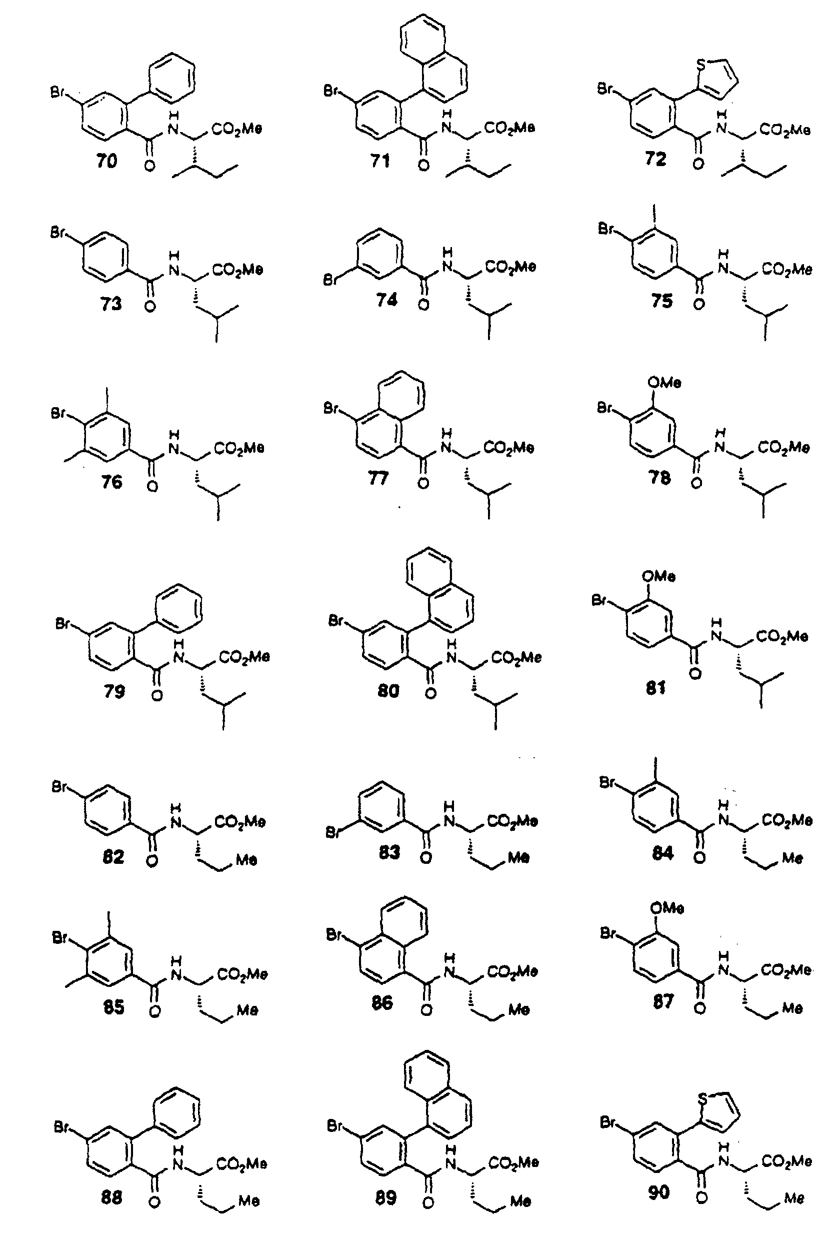

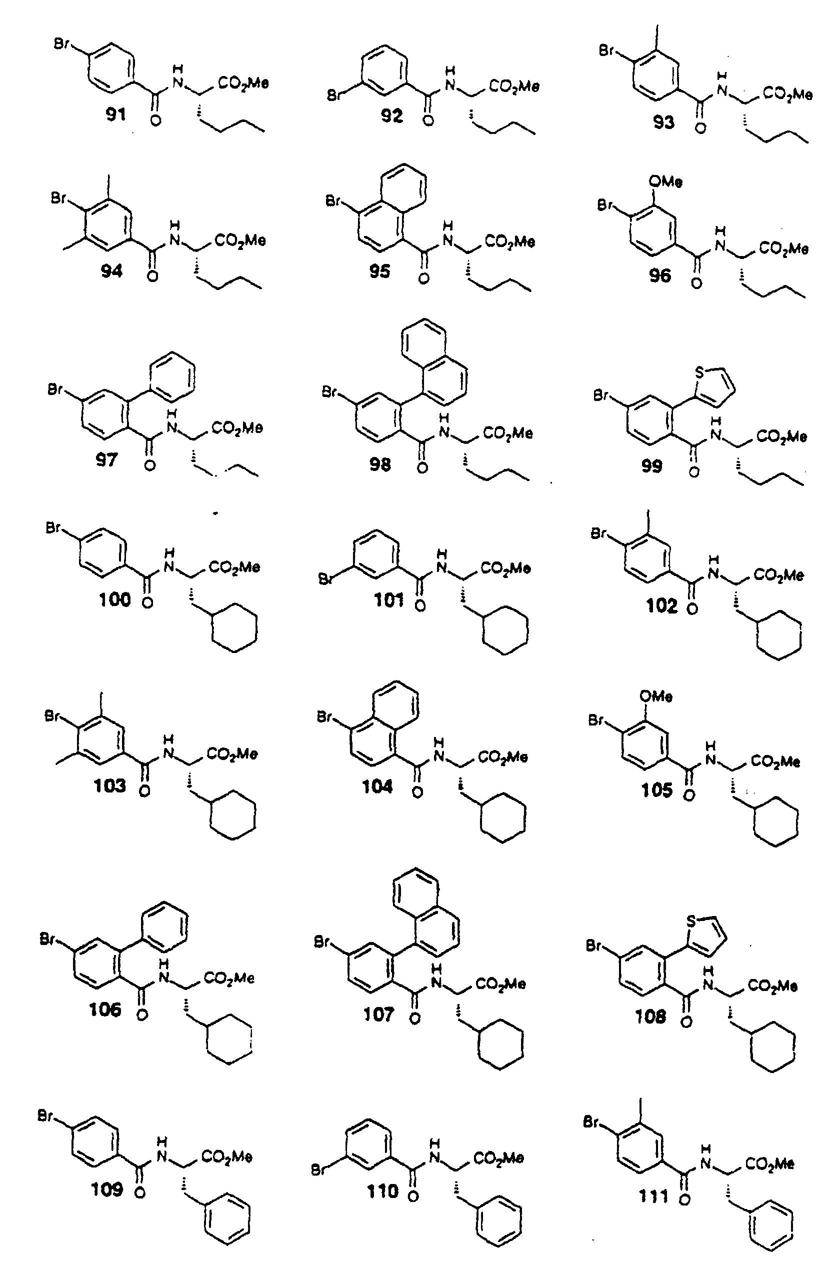

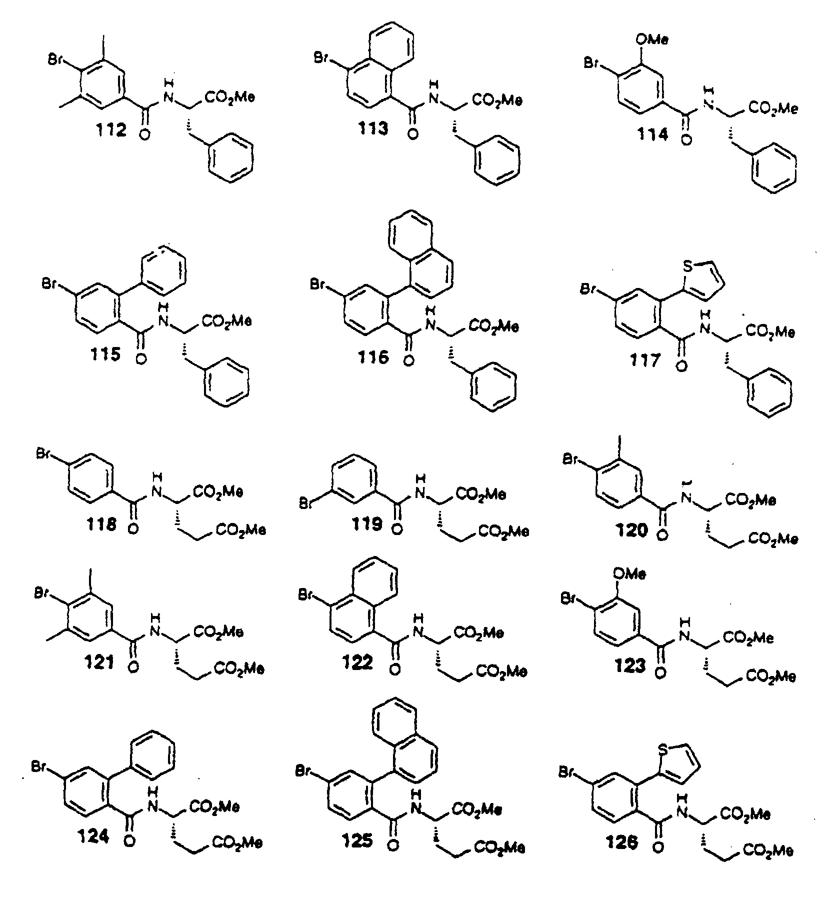

## Claims

1. A compound having the formula 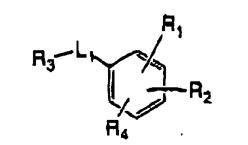 or a pharmaceutically acceptable salt thereof wherein
**R**_{**1**} is selected from the group consisting of
(a) hydrogen,
(b) loweralkyl,
(c) alkenyl,
(d) alkoxy,
(e) thioalkoxy,
(f) halo,
(g) haloalkyl,
(h) aryl - L₂- wherein L₂ is absent or is selected from the group consisting of
-CH₂-,
-CH₂CH₂-,
-CH(CH₃)-,
-O-,
-S(O)_{q} wherein q is 0,1 or 2,
- N(R)- wherein R is hydrogen or loweralkyl, and
-C(O)-
and aryl is selected from the group consisting of
phenyl,
naphthyl,
tetrahydronaphthyl,
indanyl and
indenyl and the aryl group is unsubstituted or substituted, and
(i) heterocyclic - L₃ - wherein L₃ is absent or is selected from the group consisting of
- CH₂-,
- CH₂CH₂-,
-CH(CH₃)-,
-O-,
-S(O)_{q} wherein q is 0, 1 or 2,
-N(R)- wherein R is hydrogen or loweralkyl, and
-C(O)-.
and heterocyclic is a monocyclic heterocyclic wherein the heterocyclic is unsubstituted or substituted with one, two or three substituents independently selected from the group consisting of
loweralkyl,
hydroxy,
hydroxyalkyl,
halo,
nitro,
oxo (=O),
amino,
N-protected amino,
alkoxy,
thioalkoxy and
haloalkyl;
**R**_{**2**} is selected from the group consisting of -C(O)NH-CH(R₁₄)-C(O)OR₁₅ wherein R₁₄ is selected from
loweralkyl,
cycloalkyl,
cycloalkylalkyl,
alkoxyalkyl,
thioalkoxyalkyl,
hydroxyalkyl,
aminoalkyl,
carboxyalkyl,
alkoxycarbonylalkyl,
arylalkyl or
alkylsulfonylalkyl and
R₁₅ is hydrogen or a carboxy-protecting group,
**R**_{**3**} is pyridyl, substituted pyridyl, imidazolyl, or substituted imidazolyl;
**R**_{**4**} is selected from the group consisting of
hydrogen,
lower alkyl,
haloalkyl
halogen,
aryl,
arylakyl,
heterocyclic, and
(heterocyclic)alkyl;
**L**_{**1**} is absent or is selected from the group consisting of
(a) -L₄-N(R₄)-L₅- wherein
L₄ is absent or selected from
C₁-to-C₁₀-alkylene and
C₂-to-C₁₀-alkenylene
wherein the alkylene group or the alkenylene group is unsubstituted or substituted with one, two, three or four substitutents independently selected from loweralkyl,
alkenyl,
hydroxy,
amino,
N-protected amino,
carboxy,
alkoxycarbonyl,
oxo,
thioxo,
imino,
=N-OH,
=N-O-loweralkyl,
=N-O-aryl, and
=N-O-heterocyclic wherein the heterocyclic is unsubstituted or substituted with one, two or three substituents independently selected from the group consisting of
loweralkyl,
halo,
nitro,
haloalkyl,
oxo,
hydroxy,
hydroxyalkyl,
amino,
N-protected amino,
alkoxy, and
thioalkoxy,
R₄ is hydrogen or loweralkyl and
L₅ is absent or is selected from,
-CH₂-,
-CH₂CH₂- and
-CH(CH₃)-,
(b) -L₄-O-L₅- wherein L₄ and L₅ are defined above,
(c) -L₄-S(O)ₙ-L₅- wherein n is 0, 1 or 2 and L₄ and L₅ are defined above,
(d) -L₄-L₆-C(W)-N(R₅)-L₅- wherein L₄, L₅ and R₅ are defined above ,
W is O or S, and
L₆ is absent or is selected from
-O-,
-S- and
-N(R₆)- wherein R₆ is hydrogen or loweralkyl, and
(e) -L₄-L₆-S(O)ₘ-N(R₇)-L₅ -wherein L₄, L₅ and L₆ are defined above.
m is 1 or 2, and
R₇ is hydrogen or loweralkyl,
(f) -L₄-N(R₇)-C(W)-L₇-L₅- wherein W, R₇, L₄ and L₅ are defined above, and L₇ is absent or is selected from -O- and -S- and
(g) -L₄-N(R₇)-S(O)ₚ-L₇-L₅- wherein p is 1 or 2 and L₄, R₇, L₅ and L₇ are defined above,
(h) C₂-C₄-alkylene optionally substituted with 1 or 2 hydroxy groups,
(i) C₂-to-C₄-alkenylene, and
(j) C₂-to-C₄-alkynylene,
wherein 'lower alkyl' indicates C₁ to C₁₀ alkyl.

2. A compound or pharmaceutically acceptable salt thereof as defined in claim 1
wherein **R**_{**1**} is aryl -L₂-. wherein
L₂ is absent or is selected from -CH₂- and -O-, and
aryl is selected from phenyl and naphthyl
wherein the aryl group is unsubstituted or substituted.

3. A compound or pharmaceutically acceptable salt thereof as defined in claim 2
wherein **R**_{**1**} is substituted or unsubstituted phenyl.

4. A compound or pharmaceutically acceptable salt thereof as defined in claim 3
wherein
**R**_{**2**} is selected from the group consisting of
(a) -C(O)NH-CH(R₁₄)-C(O)OR₁₅ wherein R₁₄ is selected from
loweralkyl,
cycloalkyl,
cycloalkylalkyl,
alkoxyalkyl,
thioalkoxyalkyl,
hydroxyalkyl,
aminoalkyl,
carboxyalkyl,
alkoxycarbonylalkyl,
arylalkyl or
alkylsulfonylalkyl and
R₁₅ is hydrogen or a carboxy-protecting group, and
(b) -C(O)NH-CH(R₁₄)-C(O)NHSO₂R₁₆ wherein R₁₄ is defined above and R₁₆ is selected from lower alkyl,
haloalkyl,
substituted or unsubstituted aryl,
substituted or unsubstituted heterocyclic.

5. A compound or pharmaceutically acceptable salt thereof as defined in claim 3
wherein **R**_{**2**} is selected from the group consisting of 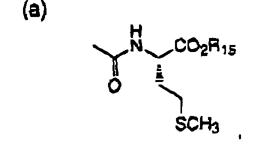 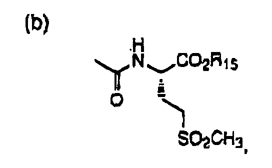 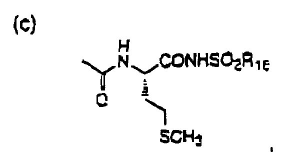 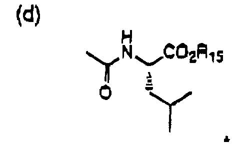 and

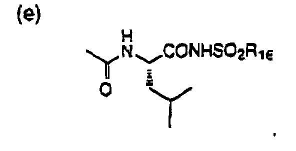

6. A compound or pharmaceutically acceptable salt thereof as defined by claim 5
wherein R₃ is pyridyl or pyridyl substituted with 1 or 2 substituents independently selected from
lower alkyl,
halogen,
haloalkyl,
hydroxy,
alkoxy,
alkoxycarbonyl,
aryl, and
arylalkyl.

7. A compound or pharmaceutically acceptable salt thereof as defined by claim 1
wherein R₃ is imidazolyl or imidazolyl substituted with lower alkyl.

8. A compound of any one of Claims 1 to 7 for use in a method of inhibiting protein isoprenyl transferases in a mammal in need of such treatment.

9. A composition for inhibiting protein isoprenyl transferases comprising a pharmaceutical carrier and a therapeutically effective amount of a compound of Claim 1.

10. A compound of anyone of Claims 1 to 7, alone or in combination with another chemotherapeutic agent for use in a method for inhibiting or treating cancer in a mammal.

11. A composition for the treatment of cancer comprising a compound of Claim 1 in combination with another chemotherapeutic agent and a pharmaceutically acceptable carrier.

12. A compound of any one of Claims 1 to 7, for use in a method for inhibiting post-translational modification of the oncogenic Ras protein by protein farnesyltransferase, protein geranylgeranyltransferase or both in a mammal.

13. A composition for inhibiting post-translational modification of the oncogenic Ras protein by protein farnesyltransferase, protein geranylgeranyltransferase or both comprising a compound of Claim 1 in combination with a pharmaceutically acceptable carrier.

14. A compound of any one of Claims 1 to 7, for use in a method for treating or preventing restenosis in a mannal.

15. A composition for treating or preventing restenosis in a mammal comprising a compound of Claim 1 in combination with a pharmaceutically acceptable carrier.

## Patentansprüche

1. Verbindung mit der Formel 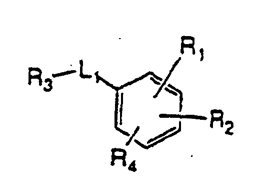 oder ein pharmazeutisch akzeptables Salz derselben, bei der
R₁ ausgewählt ist aus der Gruppe bestehend aus
(a) Wasserstoff,
(b) niederem Alkyl,
(c) Alkenyl,
(d) Alkoxy,
(e) Thioalkoxy,
(f) Halogen,
(g) Halogenalkyl,
(h) Aryl-L₂-,
wobei L₂ nicht vorhanden ist oder ausgewählt ist aus der Gruppe bestehend aus
-CH₂-,
-CH₂CH₂-,
-CH(CH₃)-,
-O-,
-S(O)_{q}, wobei q 0, 1 oder 2 ist,
-N(R)-, wobei R Wasserstoff oder niederes Alkyl ist, und
-C(O)-
und Aryl ausgewählt ist aus der Gruppe bestehend aus Phenyl,
Naphthyl,
Tetrahydronaphthyl,
Indanyl und
Indenyl,
wobei die Arylgruppe unsubstituiert oder substituiert ist, und
(i) Heterocyclus-L₃-,
wobei L₃ nicht vorhanden ist oder ausgewählt ist aus der Gruppe bestehend aus
-CH₂-,
-CH₂CH₂-,
-CH(CH₃)-,
-O-,
-S(O)_{q}, wobei q 0, 1 oder 2 ist,
-N(R)-, wobei R Wasserstoff oder niederes Alkyl ist, und
-C(O)-
und Heterocyclus ein monocyclischer Heterozyklus ist, wobei der Heterozyklus unsubstituiert oder mit einem, zwei oder drei Substituenten substituiert ist, die jeweils unabhängig ausgewählt sind aus der Gruppe bestehend aus
niederem Alkyl,
Hydroxy,
Hydroxyalkyl,
Halogen,
Nitro,
Oxo (=O),
Amino,
N-geschütztem Amino,
Alkoxy,
Thioalkoxy und
Halogenalkyl,
R₂ ausgewählt ist aus der Gruppe bestehend aus
-C(O)NH-CH(R₁₄)-C(O)OR₁₅,
wobei R₁₄ ausgewählt ist aus
niederem Alkyl,
Cycloalkyl,
Cycloalkylalkyl,
Alkoxyalkyl,
Thioalkoxyalkyl,
Hydroxyalkyl,
Aminoalkyl,
Carboxyalkyl,
Alkoxycarbonylalkyl,
Arylalkyl oder
Alkylsulfonylalkyl und
R₁₅ Wasserstoff oder eine Carboxy-Schutzgruppe ist,
R₃ Pyridyl, substituiertes Pyridyl, Imidazolyl oder substituiertes Imidazolyl ist,
R₄ ausgewählt ist aus der Gruppe bestehend aus
Wasserstoff,
niederem Alkyl,
Halogenalkyl,
Halogen,
Aryl,
Arylalkyl,
Heterocyclus und
(Heterocyclus)-Alkyl,
L₁ nicht vorhanden ist oder ausgewählt ist aus der Gruppe bestehend aus
(a) -L₄-N(R₄)-L₅-, wobei
L₄ nicht vorhanden ist oder ausgewählt ist aus C₁- bis C₁₀-Alkylen und C₂- bis C₁₀-Alkenylen,
wobei die Alkylengruppe oder die Alkenylengruppe unsubstituiert oder mit einem, zwei, drei oder vier Substituenten substituiert ist, die jeweils unabhängig ausgewählt sind aus
niederem Alkyl,
Alkenyl,
Hydroxy,
Amino,
N-geschütztem Amino,
Carboxy,
Alkoxycarbonyl,
Oxo,
Thioxo,
Imino,
=N-OH,
=N-O-niederes Alkyl,
=N-O-Aryl und
=N-O-Heterocyclus, wobei der Heterozyklus unsubstituiert oder mit einem, zwei oder drei Substituenten substituiert ist, die jeweils unabhängig ausgewählt sind aus der Gruppe bestehend aus
niederem Alkyl,
Halogen,
Nitro,
Halogenalkyl,
Oxo,
Hydroxy,
Hydroxyalkyl,
Amino,
N-geschütztem Amino,
Alkoxy und
Thioalkoxy,
R₄ Wasserstoff oder niederes Alkyl ist und
L₅ nicht vorhanden ist oder ausgewählt ist aus
-CH₂-,
-CH₂CH₂- und
-CH(CH₃)-,
(b) -L₄-O-L₅-, wobei L₄ und L₅ wie oben definiert sind,
(c) -L₄-S(O)ₙ-L₅-, wobei n gleich 0, 1 oder 2 ist und L₄ und L₅ wie oben definiert sind,
(d) -L₄-L₆-C(W)-N(R₅)-L₅-,
wobei L₄, L₅ und R₅ wie oben definiert sind,
W O oder S ist und
L₆ nicht vorhanden ist oder ausgewählt ist aus
-O-,
-S- und
-N(R₆)-, wobei R₆ Wasserstoff oder niederes
Alkyl ist, und
(e) -L₄-L₆-S(O)ₘ-N(R₇) -L₅-,
wobei L₄, L₅ und L₆ wie oben definiert sind,
m 1 oder 2 ist und
R₇ Wasserstoff oder niederes Alkyl ist,
(f) -L₄-N(R₇) -C(W) -L₇-L₅-,
wobei W, R₇, L₄ und L₆ wie oben definiert sind und L₇ nicht vorhanden ist oder ausgewählt ist aus -Ound -S- und
(g) -L₄-N(R₇)-S(O)ₚ-L₇-L₅-,
wobei p 1 oder 2 ist, und L₄, R₇, L₅ und L₇ wie oben definiert sind,
(h) C₂- bis C₄-Alkylen, das gegebenenfalls mit 1 oder 2 Hydroxygruppen substituiert ist,
(i) C₂- bis C₄-Alkenylen und
(j) C₂- bis C₄-Alkinylen,
wobei "niederes Alkyl" C₁- bis C₁₀-Alkyl bezeichnet.

2. Verbindung oder pharmazeutisches akzeptables Salz derselben gemäß Anspruch 1, bei der R₁ Aryl-L₂- ist, wobei
L₂ nicht vorhanden ist oder ausgewählt ist aus -CH₂- und -O-, und
Aryl ausgewählt ist aus Phenyl und Naphthyl,
wobei die Arylgruppe unsubstituiert oder substituiert ist.

3. Verbindung oder pharmazeutisch akzeptables Salz derselben gemäß Anspruch 2, bei der R₁ substituiertes oder unsubstituiertes Phenyl ist.

4. Verbindung oder pharmazeutisch akzeptables Salz derselben gemäß Anspruch 3, bei der R₂ ausgewählt ist aus der Gruppe bestehend aus
(a) -C(O)NH-CH(R₁₄)-C(O)OR₁₅, wobei R₁₄ ausgewählt ist aus
niederem Alkyl,
Cycloalkyl,
Cycloalkylalkyl,
Alkoxyalkyl,
Thioalkoxyalkyl,
Hydroxyalkyl,
Aminoalkyl,
Carboxyalkyl,
Alkoxycarbonylalkyl,
Arylalkyl oder
Alkylsulfonylalkyl und
R₁₅ Wasserstoff oder eine Carboxy-Schutzgruppe ist, und
(b) -C(O)NH-CH(R₁₄)-C(O)NHSO₂R₁₆, wobei R₁₄ wie oben definiert ist und R₁₆ ausgewählt ist aus
niederem Alkyl,
Halogenalkyl,
substituiertem oder unsubstituiertem Aryl,
substituiertem oder unsubstituiertem Heterozyklus.

5. Verbindung oder pharmazeutisch akzeptables Salz derselben gemäß Anspruch 3, bei der R₂ ausgewählt ist aus der Gruppe bestehend aus 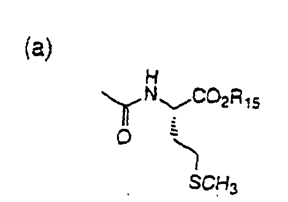 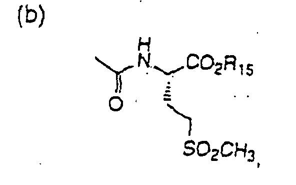 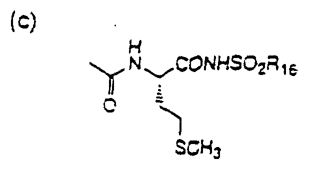 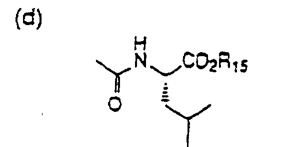 und

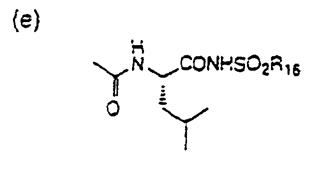

6. Verbindung oder pharmazeutisch akzeptables Salz derselben gemäß Anspruch 5, bei der R₃ Pyridyl oder mit 1 oder 2 Substituenten substituiertes Pyridyl ist, die jeweils unabhängig ausgewählt sind aus
niederem Alkyl,
Halogen,
Halogenalkyl,
Hydroxy,
Alkoxy,
Alkoxycarbonyl,
Aryl und
Arylalkyl.

7. Verbindung oder pharmazeutisch akzeptables Salz derselben gemäß Anspruch 1, bei der R₃ Imidazolyl oder mit niederem Alkyl substituiertes Imidazolyl ist.

8. Verbindung nach einem der Ansprüche 1 bis 7 zur Verwendung in einem Verfahren zum Inhibieren von Protein-Isoprenyl-Transferasen in einem Säugetier bei Bedarf an einer solchen Behandlung.

9. Verbindung zur Inhibierung von Protein-Isoprenyl-Transferasen, die einen pharmazeutischen Träger und eine therapeutisch wirksame Menge einer Verbindung gemäß Anspruch 1 umfasst.

10. Verbindung nach einem der Ansprüche 1 bis 7 allein oder in Kombination mit einem anderen chemotherapeutischen Mittel zur Verwendung in einem Verfahren zur Inhibierung oder Behandlung von Krebs in einem Säugetier.

11. Zusammensetzung zur Behandlung von Krebs, die eine Verbindung gemäß Anspruch 1 in Kombination mit einem anderen chemotherapeutischen Mittel und einem pharmazeutisch akzeptablen Träger umfasst.

12. Verbindung nach einem der Ansprüche 1 bis 7 zur Verwendung in einem Verfahren zur Inhibierung von post-translationaler Modifikation des onkogenen RAS-Proteins durch Protein-Farnesyl-Transferase, Protein-Geranylgeranyl-Transferase oder beidem in einem Säugetier.

13. Zusammensetzung zur Inhibierung von post-translationaler Modifikation des onkogenen RAS-Proteins durch Protein-Farnesyl-Transferase, Protein-Geranylgeranyl-Transferase oder beidem, die eine Verbindung gemäß Anspruch 1 in Verbindung mit einem pharmazeutisch akzeptablen Träger umfasst.

14. Verbindung nach einem der Ansprüche 1 bis 7 zur Verwendung in einem Verfahren zur Behandlung oder Verhinderung von Restenosis in einem Säugetier.

15. Zusammensetzung zur Behandlung oder Verhinderung von Restenosis in einem Säugetier, die eine Verbindung gemäß Anspruch 1 in Kombination mit einem pharmazeutisch akzeptablen Träger umfasst.

## Revendications

1. Composé de formule 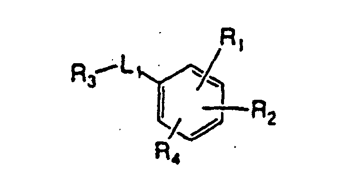 ou un de ses sels pharmaceutiquement acceptables, formule dans laquelle
R₁ est choisi dans le groupe consistant en
(a) un atome d'hydrogène,
(b) un groupe alkyle inférieur,
(c) un groupe alcényle,
(d) un groupe alkoxy,
(e) un groupe thio-alkoxy,
(f) un groupe halogéno,
(g) un groupe halogénalkyle,
(h) un groupe aryl-L₂- dans lequel L₂ est absent ou est choisi dans le groupe consistant en des groupes
-CH₂-,
-CH₂CH₂,
-CH(CH₃)-,
-O-,
-S(O)_{q} dans lequel q est égal à 0, 1 ou 2,
-N(R)- dans lequel R représente un atome d'hydrogène ou un groupe alkyle inférieur, et
-C(O)-
et le groupe aryle est choisi dans le groupe consistant en les groupes
phényle,
naphtyle,
tétrahydronaphtyle,
indanyle et
indényle, le groupe aryle étant non substitué ou substitué, et
(i) un groupe hétérocyclique -L₃- dans lequel L₃ est absent ou est choisi dans le groupe consistant en des groupes
-CH₂-,
-CH₂CH₂-,
-CH(CH₃)-,
-O-
-S(O)_{q} dans lequel q est égal à 0, 1 ou 2,
-N(R)- dans lequel R représente un atome d'hydrogène ou un groupe alkyle inférieur, et
-C(O)-,
et le groupe hétérocyclique est un groupe hétérocyclique monocyclique, le groupe hétérocyclique étant non substitué ou substitué avec un, deux ou trois substituants choisis, indépendamment, dans le groupe consistant en des substituants
alkyle inférieur,
hydroxy,
hydroxyalkyle,
halogéno,
nitro,
oxo (=O),
amino,
amino N-protégé,
alkoxy,
thio-alkoxy et
halogénalkyle ;
R₂ est choisi dans le groupe consistant en des groupes
-C(O)NH-CH(R₁₄)-C(O)OR₁₅ dans lequel R₁₄ est choisi entre des groupes
alkyle inférieur,
cycloalkyle,
cycloalkylalkyle,
alkoxyalkyle,
thio-alkoxyalkyle,
hydroxyalkyle,
amino-alkyle,
carboxyalkyle,
alkoxycarbonylalkyle,
arylalkyle ou
alkylsulfonylalkyle et
R₁₅ représente un atome d'hydrogène ou un groupe protecteur de la fonction carboxy,
R₃ représente un groupe pyridyle, pyridyle substitué, imidazolyle ou imidazolyle substitué ;
R₄ est choisi dans le groupe consistant en
un atome d'hydrogène,
un groupe alkyle inférieur,
un groupe halogénalkyle,
un groupe halogéno,
un groupe aryle,
un groupe arylalkyle,
un groupe hétérocyclique, et
un groupe (hétérocyclique)alkyle ;
L₁ est absent ou est choisi dans le groupe consistant en des groupes
(a) -L₄-N(R₄)-L₅- dans lequel
L₄ est absent ou est choisi entre des groupes
alkylène en C₁ à C₁₀ et
alcénylène en C₂ à C₁₀
dans lesquels le groupe alkylène et le groupe alcénylène sont non substitués ou substitués avec un, deux, trois ou quatre substituants choisis, indépendamment, entre des substituants alkyle inférieur,
alcényle,
hydroxy,
amino,
amino N-protégé,
carboxy,
alkoxycarbonyle,
oxo,
thioxo,
imino,
=N-OH,
=N-O-alkyle inférieur,
=N-O-aryle, et
=N-O-hétérocyclique dans lequel le groupe hétérocyclique est non substitué ou substitué avec un, deux ou trois substituants choisis, indépendamment, dans le groupe consistant en des substituants
alkyle inférieur,
halogéno,
nitro,
halogénalkyle,
oxo,
hydroxy,
hydroxyalkyle,
amino,
amino N-protégé
alkoxy, et
thio-alkoxy,
R₄ représente un atome d'hydrogène ou un groupe alkyle inférieur et
L₅ est absent ou est choisi entre des groupes
-CH₂-,
-CH₂CH₂- et
-CH(CH₃)-,
(b) -L₄-O-L₅- dans lequel L₄ et L₅ répondent aux définitions précitées,
(c) -L₄-S(O)ₙ-L₅- dans lequel n est égal à 0, 1 ou 2 et L₄ et L₅ répondent aux définitions précitées,
(d) -L₄-L₆-C(W)-N(R₅)-L₅- dans lequel L₄, L₅ et R₅ répondent aux définitions précitées,
W représente un atome de O ou S, et
L₆ est absent ou est choisi entre des groupes
-O-,
-S- et
-N(R₆)- dans lequel R₆ représente un atome d'hydrogène ou un groupe alkyle inférieur, et
(e) -L₄-L₆-S(O)ₘ-N(R₇)-L₅- dans lequel L₄, L₅ et L₆ répondent aux définitions précitées,
m est égal à 1 ou 2, et
R₇ représente un atome d'hydrogène ou un groupe alkyle inférieur,
(f) -L₄-(R₇)-C(W)-L₇-L₅- dans lequel W, R₇, L₄ et L₆ répondent aux définitions précitées, et L₇ est absent ou est choisi entre des groupes -O- et -S- et
(g) -L₄-N(R₇)-S(O)ₚ-L₇-L₅- dans lequel p est égal à 1 ou 2 et L₄, R₇, L₅ et L₇ répondent aux définitions précitées,
(h) alkylène en C₂ à C₄, facultativement substitué avec 1 ou 2 groupes hydroxy,
(i) alcénylène en C₂ à C₄, et
(j) alcynylène en C₂ à C₄,
l'expression "alkyle inférieur" désignant un groupe alkyle en C₁ à C₁₀.

2. Composé ou un de ses sels pharmaceutiquement acceptables répondant à la définition suivant la revendication 1, dans lequel R₁ représente un groupe aryl-L₂- dans lequel
L₂ est absent ou est choisi entre des groupes - CH₂- et -O-, et
le groupe aryle est choisi entre les groupes phényle et naphtyle,
le groupe aryle étant non substitué ou substitué.

3. Composé ou un de ses sels pharmaceutiquement acceptables répondant à la définition suivant la revendication 2, dans lequel R₁ représente un groupe phényle substitué ou non substitué.

4. Composé ou un de ses sels pharmaceutiquement acceptables répondant à la définition suivant la revendication 3, dans lequel
R₂ est choisi dans le groupe consistant en des groupes
(a) -C(O)NH-CH(R₁₄)-C(O)OR₁₅ dans lequel R₁₄ est choisi entre des groupes
alkyle inférieur,
cycloalkyle,
cycloalkylalkyle,
alkoxyalkyle,
thio-alkoxyalkyle,
hydroxyalkyle,
amino-alkyle,
carboxyalkyle,
alkoxycarbonylalkyle,
arylalkyle ou
alkylsulfonylalkyle, et
R₁₅ représente un atome d'hydrogène ou un groupe protecteur de la fonction carboxy, et
(b) -C(O)NH-CH (R₁₄)-C(O)NHSO₂R₁₆
dans lequel R₁₄ répond à la définition précitée et R₁₆ est choisi entre des groupes alkyle inférieur,
halogénalkyle,
aryle substitué ou non substitué,
hétérocyclique substitué ou non substitué.

5. Composé ou un de ses sels pharmaceutiquement acceptables répondant à la définition suivant la revendication 3, dans lequel R₂ est choisi dans le groupe consistant en

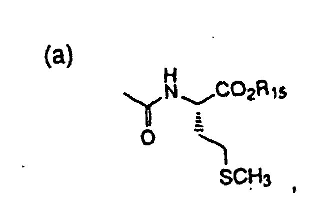

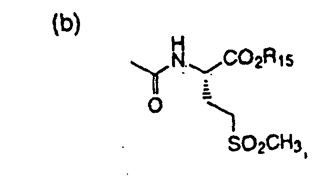

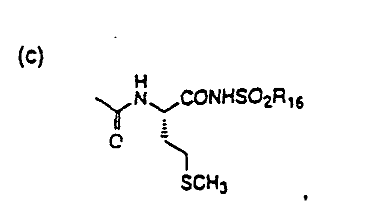

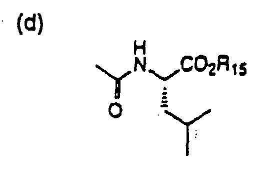

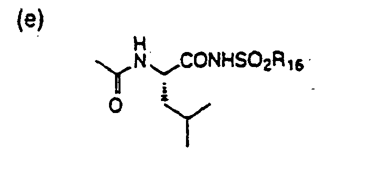

6. Composé ou un de ses sels pharmaceutiquement acceptables répondant à la définition suivant la revendication 5, dans lequel R₃ représente un groupe pyridyle ou pyridyle substitué avec 1 ou 2 substituants choisis, indépendamment, entre des substituants
alkyle inférieur,
halogéno,
halogénalkyle,
hdyroxy,
alkoxy,
alkoxycarbonyle,
aryle, et
arylalkyle.

7. Composé ou un de ses sels pharmaceutiquement acceptables répondant à la définition suivant la revendication 1, dans lequel R₃ représente un groupe imidazolyle ou imidazolyle substitué avec un substituant alkyle inférieur.

8. Composé suivant l'une quelconque des revendications 1 à 7, destiné à être utilisé dans une méthode pour inhiber des protéines-isoprényl-transférases chez un mammifère nécessitant un tel traitement.

9. Composition destinée à inhiber des protéines-isoprényl-transférases, comprenant un support pharmaceutique et une quantité thérapeutiquement efficace d'un composé suivant la revendication 1.

10. Composé suivant l'une quelconque des revendications 1 à 7, seul ou en association avec un autre agent chimiothérapeutique destiné à être utilisé dans une méthode pour inhiber ou traiter un cancer chez un mammifère.

11. Composition pour le traitement d'un cancer, comprenant un composé suivant la revendication 1 en association avec un autre agent chimiothérapeutique et un support pharmaceutiquement acceptable.

12. Composé suivant l'une quelconque des revendications 1 à 7, destiné à être utilisé dans une méthode pour inhiber la modification en post-traduction de la protéine Ras oncogène par là protéine farnésyl-transférase, la protéine géranylgéranyl-transférase ou ces deux agents chez un mammifère.

13. Composition destinée à inhiber la modification en post-traduction de la protéine Ras oncogène par la protéine farnésyl-transférase, la protéine géranylgéranyl-transférase ou ces deux agents, comprenant un composé suivant la revendication 1 en association avec un support pharmaceutiquement acceptable.

14. Composé suivant l'une quelconque des revendications 1 à 7, destiné à être utilisé dans une méthode pour le traitement ou la prévention d'une resténose chez un mammifère.

15. Composition pour le traitement ou la prévention d'une resténose chez un mammifère, comprenant un composé suivant la revendication 1 en association avec un support pharmaceutiquement acceptable.
